(19) Europäisches Patentamt
European Patent Office
Office européen des brevets

(11) **EP 4 252 780 A1**

(12) **EUROPEAN PATENT APPLICATION**
published in accordance with Art. 153(4) EPC

(43) Date of publication:
**04.10.2023 Bulletin 2023/40**

(21) Application number: **21897261.0**

(22) Date of filing: **29.11.2021**

(51) International Patent Classification (IPC):
***A61K 47/54*** *(2017.01)* ***A61K 38/00*** *(2006.01)*
***A61K 38/16*** *(2006.01)* ***A61K 9/00*** *(2006.01)*

(52) Cooperative Patent Classification (CPC):
**A61K 9/00; A61K 38/00; A61K 38/16; A61K 47/54**

(86) International application number:
**PCT/IB2021/022237**

(87) International publication number:
**WO 2022/112849 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200163363**

(71) Applicant: **D&D Pharmatech Inc.**
**Seongnam-si, Gyeonggi-do 13486 (KR)**

(72) Inventors:
- **SHIN, Jae hee**
  **Seoul 05610 (KR)**
- **LIM, Sung Mook**
  **Seoul 06378 (KR)**
- **PARK, Eun Ji**
  **Seoul 06279 (KR)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) BIOLOGICALLY ACTIVE MATERIAL CONJUGATE HAVING BIOTIN MOIETY, FATTY ACID MOIETY, OR COMBINATION THEREOF COUPLED THERETO

(57) The present invention relates to a biologically active material conjugate in which a biological active material is conjugated with a biotin moiety, a fatty acid moiety, or a combination thereof and, more specifically, to a biologically active material conjugate having a biotin moiety and a fatty acid moiety coupled thereto. With a biotin moiety and a fatty acid moiety coupled thereto, the biologically active material according to the present invention exhibits an excellent in-vivo oral absorption rate and has an excellent pharmacokinetic effect.

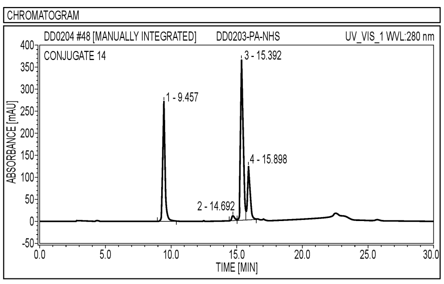


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 9.457 | 55.698 | 271.548 | 32.95 | 35.39 | N.A. |
| 2 | | 14.692 | 2.766 | 10.837 | 1.64 | 1.41 | N.A. |
| 3 | | 15.392 | 80.389 | 363.431 | 47.55 | 47.37 | N.A. |
| 4 | | 15.898 | 30.204 | 121.463 | 17.87 | 15.83 | N.A. |
| TOTAL: | | | 169.057 | 767.278 | 100.00 | 100.00 | |

*FIG. 1A*

EP 4 252 780 A1

## Description

### [Technical Field]

The present invention relates to a biologically active material conjugate in which a biologically active material is conjugated with a biotin moiety, a fatty acid moiety, or a combination thereof, specifically, a biologically active material conjugate in which a biologically active material is conjugated with a biotin moiety, a fatty acid moiety, or a combination thereof that exhibits an excellent in-vivo oral absorption rate and has an excellent pharmacokinetic effect, and more specifically, a biologically active material conjugate having a biotin moiety and a fatty acid moiety coupled thereto.

### [Background Art]

In order for a drug to act effectively, high bioavailability must be ensured. Bioavailability refers to the degree of a drug used at a target site after drug administration, and the degree is different depending on the administration method, target environment, and the like. A drug may be lost or degraded in the course of delivery from the site of administration to the target, depending on the mode of administration.

Typically, drug delivery of therapeutic agents including proteins and polypeptides, etc. is divided into parenteral administration and oral administration. Parenteral administration methods include intravenous injection, intramuscular injection, subcutaneous injection, sublingual administration, etc., where oral administration method means ingestion of the drug orally. Most therapeutic agents, such as proteins and polypeptides, are administered by a parenteral method due to considerations of bioavailability, target environment and delivery process, etc., and it is known that parenteral administration method exhibits a direct and rapid effect. However, parenteral administration may cause pain or discomfort to the patient, and side effects such as infection by injection and air embolism may appear depending on the route. On the other hand, oral administration is convenient in that [the drug] is administered directly by mouth, and there exists an advantage in that a sustained effect can be exhibited. Accordingly, many pharmaceutical companies have attempted to administer therapeutic agents by oral administration, but there is a problem in that [a drug administered by] oral administration passes through the digestive tract, so resistance to an acidic environment and enzymatic degradation, etc. is required. In particular, it is known that proteins and peptides have a low bioavailability of about 0.1% when administered orally.

In order to solve the problems of oral administration, attempts have been made to prepare separate [oral] formulations using surfactants and absorption enhancers, etc. together, or to increase the delivery of the drug by micronizing drug particles and adjusting the number of administrations. Such oral insulin and oral GLP-1 analogs are being developed by large pharmaceutical companies, and in addition, various research and development activities for oral administration of interferon alpha and the like are in progress. However, peptides and protein drugs are materials that are difficult to administer orally; various attempts have been made to solve this problem, but it has not been clearly resolved so far. In particular, peptides and protein drugs have a problem in that the oral absorption rate is not high when administered orally, and whereas various attempts are being made to resolve this problem, there has not been a clear solution as of yet.

### [Detailed Description of the Invention]

### [Problem to be Solved]

The object of the present invention is to provide a biologically active material conjugate in which a biologically active material is conjugated with a biotin moiety, a fatty acid moiety, or a combination thereof, and more specifically, a biologically active material conjugate having a biotin moiety and a fatty acid moiety coupled thereto, that exhibits an excellent in-vivo oral absorption rate and has an excellent pharmacokinetic effect.

### [Means of Solving the Problem]

One aspect of the present invention provides a biologically active material conjugate conjugated with a biotin moiety, a fatty acid moiety, or a combination thereof, and a method for preparing the same. Another aspect of the present invention provides a pharmaceutical formulation comprising a biologically active material conjugate conjugated with a biotin moiety, a fatty acid moiety, or a combination thereof.

Yet another aspect of the present invention provides a formulation for oral administration, the formulation comprising a biologically active material conjugate conjugated with a biotin moiety, a fatty acid moiety, or a combination thereof.

A specific aspect of the present invention provides a biologically active material conjugate having a biotin moiety

and a fatty acid moiety coupled thereto, and a method for preparing the same.

Yet another aspect of the present invention provides a pharmaceutical formulation for preventing or treating diabetes, obesity, fatty liver disease, irritable bowel syndrome, neurodegenerative disease, bone disease, osteoporosis, human growth hormone deficiency, cancer or non-alcoholic fatty liver disease, the formulation comprising a biologically active material conjugate having a biotin moiety and a fatty acid moiety coupled thereto.

**[Effects of the Invention]**

The biologically active material conjugate conjugated with a biotin moiety, a fatty acid moiety, or a combination thereof according to one embodiment of the present invention has a water-soluble biotin coupled thereto, giving excellent effect. Specifically, the effects include improving oral absorption, improving pharmacokinetic effects, protecting against degradation of physiologically active materials from enzymes, promoting intestinal membrane permeation of physiologically active materials, or active transport and absorption through sodium-dependent multivitamin transporters

Further, the biologically active material conjugate conjugated with a biotin moiety and a fatty acid moiety according to one embodiment of the present invention exhibits further improved effects in terms of each of the effects above, compared to a conjugate conjugated solely with a biotin moiety or with a fatty acid moiety.

**[Brief Description of the Drawings]**

FIG. 1 is a purification chromatogram of Conjugates 14 through 17 according to one embodiment of the present invention.

FIG. 2 is a purification chromatogram of Conjugates 18 through 19 according to one embodiment of the present invention.

FIG. 3 is a chromatogram for the final products of Conjugates 20 and 24 according to one embodiment of the present invention.

FIG. 4 is a diagram illustrating changes in blood glucose levels after administration of glucose for Conjugates 20 and 24 according to one embodiment of the present invention.

FIG. 5 is a chromatogram for the final products of Conjugates 51 through 52 according to one embodiment of the present invention.

FIG. 6 is a purification chromatogram of Conjugates 53 through 54 according to one embodiment of the present invention.

FIG. 7 is a diagram illustrating changes in blood glucose levels after administration of glucose for Conjugates 53 and 54 according to one embodiment of the present invention.

FIG. 8 is a purification chromatogram of Conjugates 55 through 59 according to one embodiment of the present invention.

FIG. 9 is a chromatogram for the final product of Conjugate 56 according to one embodiment of the present invention.

FIG. 10 is a diagram illustrating body weight change after 2 weeks of subcutaneous injection of Conjugates 58 through 59 according to one embodiment of the present invention.

FIG. 11 is a purification chromatogram of Conjugates 60 through 64 according to one embodiment of the present invention.

FIG. 12 is a diagram illustrating feed intake levels after oral administration of Conjugates 33, 36, 39, 42, 61, 62, 63 and 64 according to one embodiment of the present invention.

FIG. 13 is a purification chromatogram of Conjugate 66 according to one embodiment of the present invention.

FIG. 14 is a diagram illustrating the blood glucose regulating ability after oral administration of Conjugates 65 and 66 according to one embodiment of the present invention.

FIG. 15 is a purification chromatogram of Conjugate 69 according to one embodiment of the present invention.

FIG. 16 is a diagram illustrating the intracellular accumulation of Conjugates 68 and 69 according to one embodiment of the present invention.

**[Best Mode for Carrying Out the Invention]**

One aspect of the present invention provides a biologically active material conjugate conjugated with a biotin moiety and a fatty acid moiety, and a method for preparing the same.

**[Modes for Carrying Out the Invention]**

Hereinafter, embodiments and working examples of the present invention will be described in detail so that those skilled in the art to which the present invention belongs can readily carry out the present invention.

However, the present invention may be embodied in many different forms and is not limited to the embodiments and working examples described herein. Throughout the specification of the present invention, when a part "comprises" a certain component, it means that other components may be further comprised, rather than excluding other components, unless otherwise stated.

The terms "about", "substantially", etc. to the extent used throughout the specification of the present invention are used to refer to values equal to or close to the numerical values inherent to the manufacturing and material tolerances stated, and are used to aid in understanding the present invention or prevent an unconscionable infringer from unfair use of the disclosure. The term "step of -(doing) " or "step of" as used throughout the specification of the present invention does not mean "step for ~".

Throughout the specification of the present invention, the term "combination thereof' comprised in Markush type expressions refers to a mixture or combination of at least one selected from a group comprising the component elements stated in the Markush type expression, and means that at least one selected from a group comprising the components elements is comprised. Throughout the specification of the present invention, the statement "and/or B" means "and B, or A or B."

One aspect of the present invention provides a biologically active material conjugate conjugated with a biotin moiety, a fatty acid moiety, or a combination thereof, and a method for preparing the same. One specific aspect of the present invention provides a biologically active material conjugate conjugated with a biotin moiety and a fatty acid moiety, and a method for preparing the same.

Typically, peptide and protein drugs correspond to Class 3 of the Biopharmaceutical Classification System (BCS), being highly water soluble and having restrictions on absorption sites in the gastrointestinal tract. Peptide and protein drugs have high hydrophilicity and large molecular weight, can be degraded by gastric acid of low pH, and have low intestinal absorption rate due to attack by enzymes such as trypsin. Typically, the oral bioavailability (BA) of peptide and protein drugs is about 0.1%, making it difficult to use them as pharmaceutical formulations. In order to address this problem, a technique of passing through the stomach using an enteric capsule is used, but this method is limited in that the absorption rate of peptides and proteins cannot be fundamentally improved.

In contrast, the biologically active material conjugate bonded to a biotin moiety, fatty acid moiety or combination thereof according to one embodiment of the present invention is able to promote absorption in the intestines by increasing intestinal membrane permeation.

Further, the biologically active material conjugate bonded to a biotin moiety, fatty acid moiety or combination thereof according to one embodiment of the present invention is able to exhibit outstanding pharmacokinetic effects.

Further, the biologically active material conjugate bonded to a biotin moiety, fatty acid moiety or combination thereof according to one embodiment of the present invention is able to protect against degradation of a biologically active material such as a peptide by enzymes, and is able to ultimately promote the permeation of the intestinal membrane by a biologically active material and its absorption in the intestine.

Further, the biologically active material conjugate bonded to a biotin moiety, fatty acid moiety or combination thereof according to one embodiment of the present invention, by being bonded to biotin, which is a type of water soluble vitamin, can be absorbed by active transport through a sodium-dependent multivitamin transporter.

Further, the biotin moiety, fatty acid moiety or combination thereof according to one embodiment of the present invention may be bonded to an active site or an inactive site of the biologically active material, and thus does not inhibit the activity of the biologically active material.

In the present invention, "unsubstituted or substituted" means unsubstituted or substituted. "Substituted" means having one or more substituents, and a substituent refers to a chemical moiety that is covalently bonded or fused to any atom of a main group such as alkylene or heteroalkylene. In the present invention, "halo" means fluorine, chlorine, bromine, iodine, and the like.

In the present invention, "alkyl" refers to a monovalent moiety obtained by removing a hydrogen atom from a carbon atom of an aliphatic or alicyclic, saturated or unsaturated hydrocarbon compound, for example, methyl, ethyl, propyl, butyl, pentyl, hexyl, n-propyl, n-butyl, n-pentyl, isopropyl, isobutyl, sec-butyl, tert-butyl, isopentyl, neopentyl and the like.

In the present invention, "heteroalkyl" is an alkyl containing one or more heteroatoms, and the heteroatom is a heteroatom positioned at any one carbon atom of the alkyl to replace C, CH, $CH_2$ or $CH_3$.

In the present invention, "alkylene" means a divalent moiety obtained by removing a hydrogen atom from a carbon atom of an aliphatic or alicyclic, saturated or unsaturated hydrocarbon compound.

In the present invention, "alkenylene" means a monovalent moiety obtained by removing a hydrogen atom from a carbon atom of an aliphatic, or alicyclic, saturated or unsaturated hydrocarbon compound. In the present invention,

"heteroalkylene" means an alkylene containing one or more hetero atoms. In the present invention, "aryl" means a monovalent moiety obtained by removing a hydrogen atom from an aromatic ring atom of an aromatic compound having a ring atom. For example, "$C_{5-10}$ aryl" means a monovalent moiety obtained by removing a hydrogen atom from an aromatic ring atom of an aromatic compound having 5 to 10 ring atoms of carbon. Examples of aryl include groups derived from benzene, acenaphthene, fluorene, phenalene, acephenanthrene and aceanthrene.

In the present invention, "heteroaryl" is an aryl comprising one or more heteroatoms, for example, pyridine, pyrimidine, benzothiophene, furyl, dioxalanyl, pyrrolyl, oxazolyl, pyridyl, pyridazinyl, pyrimidinyl, isobenzofuran, indole, isoindole, indolizine, indoline, isoindoline, purine, benzodioxane, quinoline, isoquinoline, quinolizine, benzoxazine, benzodiazine, pyridopyridine, quinoxaline, quinazoline, cinnoline, phthalazine, naphthyridine, pteridine, perimidine, pyridoindole, oxantrene, phenoxatiin, phenazine, phenoxazine, and the like.

In the present invention, "arylene" means a divalent moiety obtained by removing a hydrogen atom from an aromatic ring atom of an aromatic compound having a ring atom.

In the present invention, "heteroarylene" means an arylene containing one or more heteroatoms.

In the present invention, "alkenyl" is an alkyl having one or more carbon-carbon double bonds, for example, vinyl ($-CH=CH_2$), 1-propenyl ($-CH=CHCH_3$), isopropenyl, butenyl, pentenyl, hexenyl, and the like.

In the present invention, "alkynyl" is an alkyl group having one or more carbon-carbon triple bonds, and examples thereof include ethynyl and 2-propynyl.

In the present invention, when a part of the general formula is defined as a specific compound, it comprises forms in which the compound is combined with other components.

According to one embodiment of the present invention, the biotin moiety may be represented by General Formula A below.

[General Formula A]

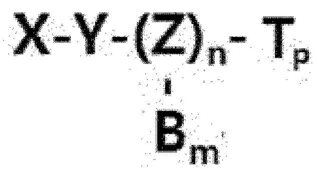

where, in General Formula A,

X is a functional group capable of binding to a biologically active material;
Y is a spacer;
Z is a binding unit;
B may be represented by the following Chemical Formula A-1;

[Chemical Formula A-1]

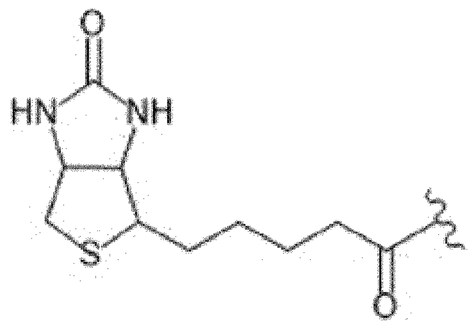

;

Z is connected with the 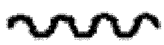 of Chemical Formula A-1;
T is a terminal group;
m is an integer of 1 to 10;
n is 0 or an integer of 1 to 10, where, when n=0, Y bonds directly with B or T; and,
p is an integer of 0 or 1.

According to one embodiment of the present invention, in General Formula A, X is a functional group capable

of binding to a biologically active material. Although not limited thereto, the functional group is a functional group capable of reacting with a thiol group, a carboxyl group and/or an amine group, for example, maleimide, succinimide, N-hydroxysuccinimide, aldehyde, carboxyl group, carboxyl ester, succinimidyl ester, tetrafluorophenyl, -O-tetrafluorophenyl (TFP,2,3,5,6-tetrafluorophenyl), tetrafluorophenyl ester, pentafluorophenyl (PFP), pentafluorophenyl ester, -O-benzotriazole, benzotriazole, sulfotetrafluorophenyl (STP), sulfodichlorophenyl (SDP), nitrophenol, and nitrophenyl carbonate (NPC).

In one embodiment of the present invention, the functional group X may retain its structure or may be eliminated or modified when bound to a biologically active material.

The Y is a spacer and may have a structure having cleavability in the body. Without being limited thereto, for example, Y may be a direct bond, or may include a substituted or unsubstituted alkylene, -O-, -C(O)NR-, -C(O)O- or -C(O) -, -NR-, -NOR-, or the like. More specifically, Y may be a direct bond, or the structure of Y may comprise at least one of the group comprising substituted or unsubstituted $C_{1-50}$ linear alkylene, substituted or unsubstituted $C_{1-50}$ non-linear alkylene, substituted or unsubstituted $C_{1-50}$ Linear heteroalkylene, substituted or unsubstituted $C_{1-50}$ nonlinear heteroalkylene, substituted or unsubstituted $C_{1-50}$ arylene, substituted or unsubstituted $C_{1-50}$ heteroarylene, -O-, -C (O), -C(O)NR-, -C(O)O-, -S-, -NR- or -NOR-, wherein R is hydrogen, or unsubstituted $C_{1-50}$ alkyl, substituted or unsubstituted $C_{1-50}$ aryl, or an ethylene glycol repeating unit ($-(CH_2CH_2O)_n-$, where n is an integer of at least 1 but not more than 20).

Z is a binding unit capable of bonding with B, and may comprise, for example, but is not limited to, an amino acid, polypeptide, alkylene, amine, or polyamidoamine structure.

Non-limiting examples of the amino acid may comprise lysine, 5-hydroxylysine, 4-oxalicine, 4-thialysine, 4-selenalysine, 4-thiahomolysine, 5,5-dimethyllysine, 5,5-difluorolysine, trans-4-dihydrolysine (trans-4-dehydrolysine), 2,6-diamino-4-hexinoic acid, cis-4-dihydrolysine (cis-4-dehydrolysine), 6-N-methyllysine, diaminopimelic acid, ornithine, 3-methylornithine, $\alpha$-methylornithine, citrulline, homocitrulline, arginine, aspartate, asparagine, glutamate, glutamine, histidine, ornithine, proline, serine, threonine, and the like.

In the present invention, when n is 0, B or T may be bonded directly with X or Y (spacer).

In the present invention, the T is a terminal group, and may be hydrogen or $NH_2$, but is not limited hereto.

In the present invention, when the p is 0, B may be a terminal.

In the present invention, the X-Y may together form a biologically active material binding site. According to an embodiment of the present invention, in General Formula A, m may be an integer of 1 to 10, and specifically may be an integer or 1 to 8, 1 to 5, or 1 to 4.

In one aspect of the present invention, the X may be selected from the group consisting of maleimide, succinimide, N-hydroxysuccinimide, succinimidyl succinate, succinimidyl glutarate, succinimidyl methyl ester, succinimidyl pentyl ester, Succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyl, $C_{6-20}$ aryldisulfide, $C_{5-20}$ heteroaryldisulfide, isocyanate, thioester, iminoester, and derivatives thereof.

In a specific aspect of the present invention, the X is maleimide, N-hydroxysuccinimide, succinimidyl carbonate, p-nitrophenyl carbonate, thiol, aminooxyl, aldehyde or amine.

In a specific aspect of the present invention, the X is maleimide, N-hydroxysuccinimide, aldehyde or amine.

In one aspect of the present invention, the Y is absent, or is a substituted or unsubstituted linear or branched $C_{1-50}$ alkylene, substituted or unsubstituted linear or branched $C_{1-50}$ heteroalkylene, substituted or unsubstituted $C_{6-50}$ arylene, or substituted or unsubstituted $C_{6-50}$ heteroarylene, and if substituted, comprises at least one selected from the group comprising =O, $-C(O)NH_2$, -OH, -COOH, -SH, =NH and $-NH_2$.

In one aspect of the present invention, the Y comprises -C(O)-. In one aspect of the present invention, the Y comprises -C(O)NH-.

In one aspect of the present invention, the Y is a substituted linear or branched $C_{1-50}$ heteroalkylene, and comprises at least one -C(O)-.

In one aspect of the present invention, the Y is $-(C(O))_q-(CH_2)_r-(C(O)NH)_s-(CH_2)_r-(OCH_2CH_2)_t-(C(O))_q$, wherein q, r, s and t are independently selected, q and s are 0 or 1, r is an integer of 1 to 20, and t is an integer of 0 to 20.

In one aspect of the present invention, the Y is $-(CH_2)_rC(O)NHNH-$, where r is an integer of 1 to 20. In one aspect of the present invention, the Y comprises $-C(O)-(OCH_2CH_2)_u-NH-$ as a repeating unit, where u is an integer of 1 to 20.

In one aspect of the present invention, the Y comprises $-C(O)-(OCH_2CH_2)_u-NH-$ as a repeating unit, where u is an integer of 2 to 4.

In one aspect of the present invention, the Y comprises an amino acid as a component.

In a specific aspect of the present invention, the Y comprises glutamic acid, glutamine, glycine, isoleucine, or lysine as a component, where each amino acid may exist in bonded form.

In a specific aspect of the present invention, the Y comprises glutamic acid or lysine as a component. In an aspect of the present invention, the Y comprises a fatty acid as a component.

In a specific aspect of the present invention, the Y comprises a $C_{12-24}$ fatty acid, and the fatty acid exists in a

bonded form.

In one aspect of the present invention, the Y is a direct bond.

In one aspect of the present invention, the Z is any one of the following, each of which may be independently selected.

A) forms an amino acid or a derivative thereof together with X or separately from X;

B) is a substituted or unsubstituted linear or branched $C_{1-50}$ heteroalkyene,

where, if substituted, comprises at least one selected from the group comprising =O, $-C(O)NH_2$, -OH, -COOH, -SH, =NH and $-NH_2$.

In one aspect of the present invention, Z is linked to B through -NH-.

In one aspect of the present invention, the Z is a hydrophilic amino acid or a derivative thereof.

In a specific aspect of the present invention, the Z may be selected from the group composed of lysine, arginine, histidine, glutamine, asparagine, threonine, cysteine, serine and derivatives thereof.

In one aspect of the present invention, the Z comprises at least one glycerol, at least one polyethylene glycol, or a combination thereof. in one aspect of the present invention, the Z comprises

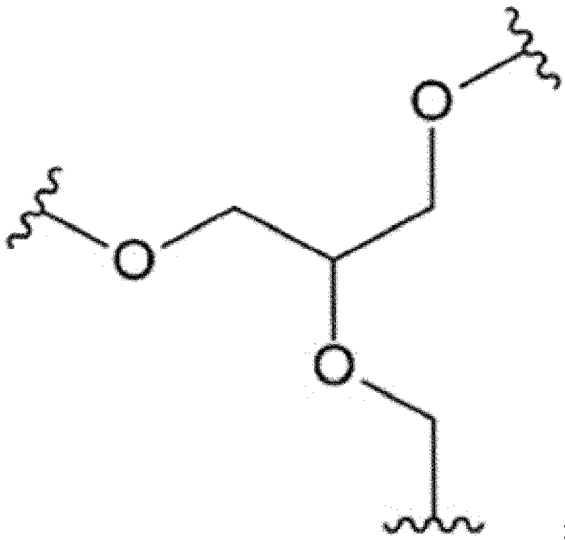

;

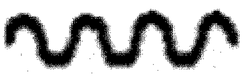 represents a binding site; and at least one

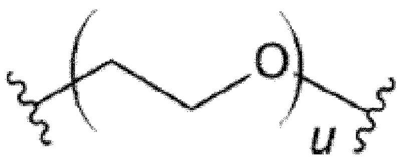

binds to at least one of the binding sites, where u is an integer of 1 to 20.

In one aspect of the present invention, the Z comprises

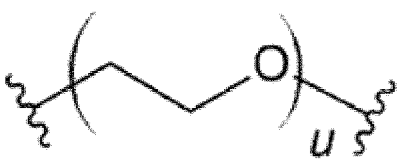

,

and - $(CH_2)_3NH$- is further bonded to

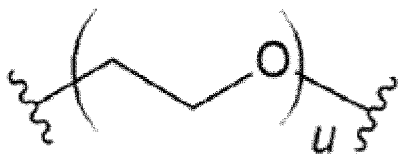

.

In an embodiment of the present invention, the biotin moiety is selected from the group composed of:

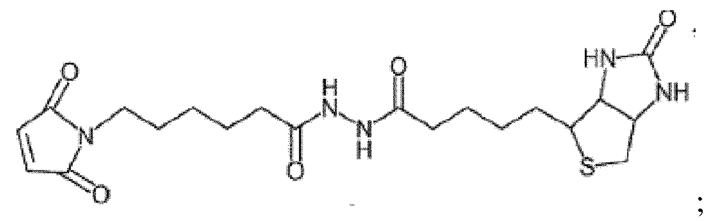

;

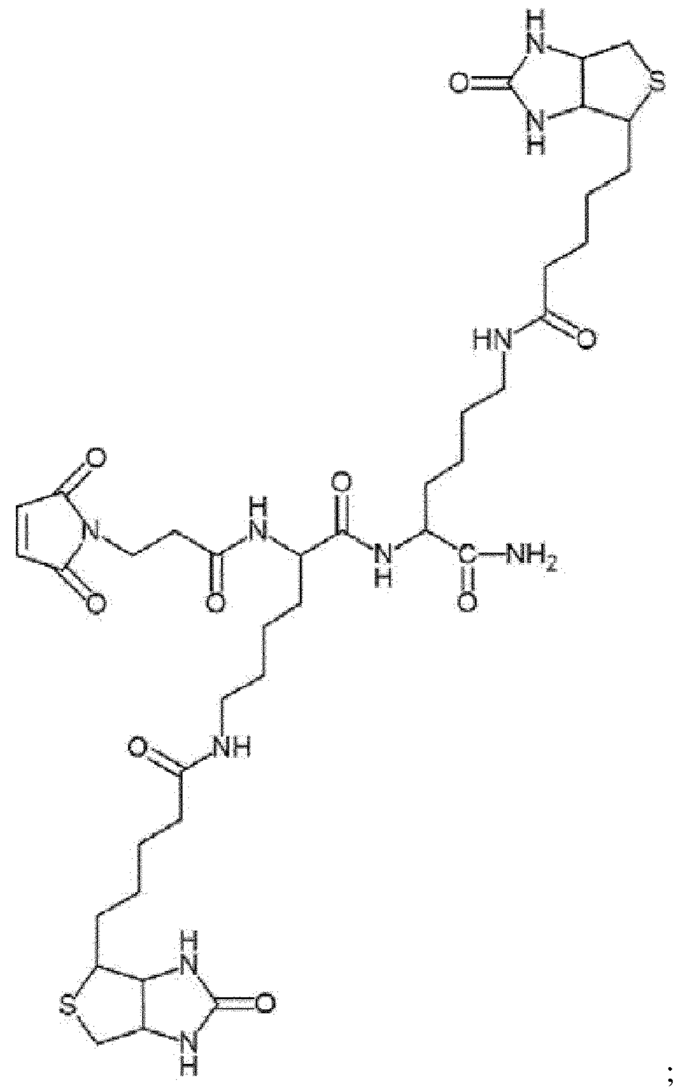

;

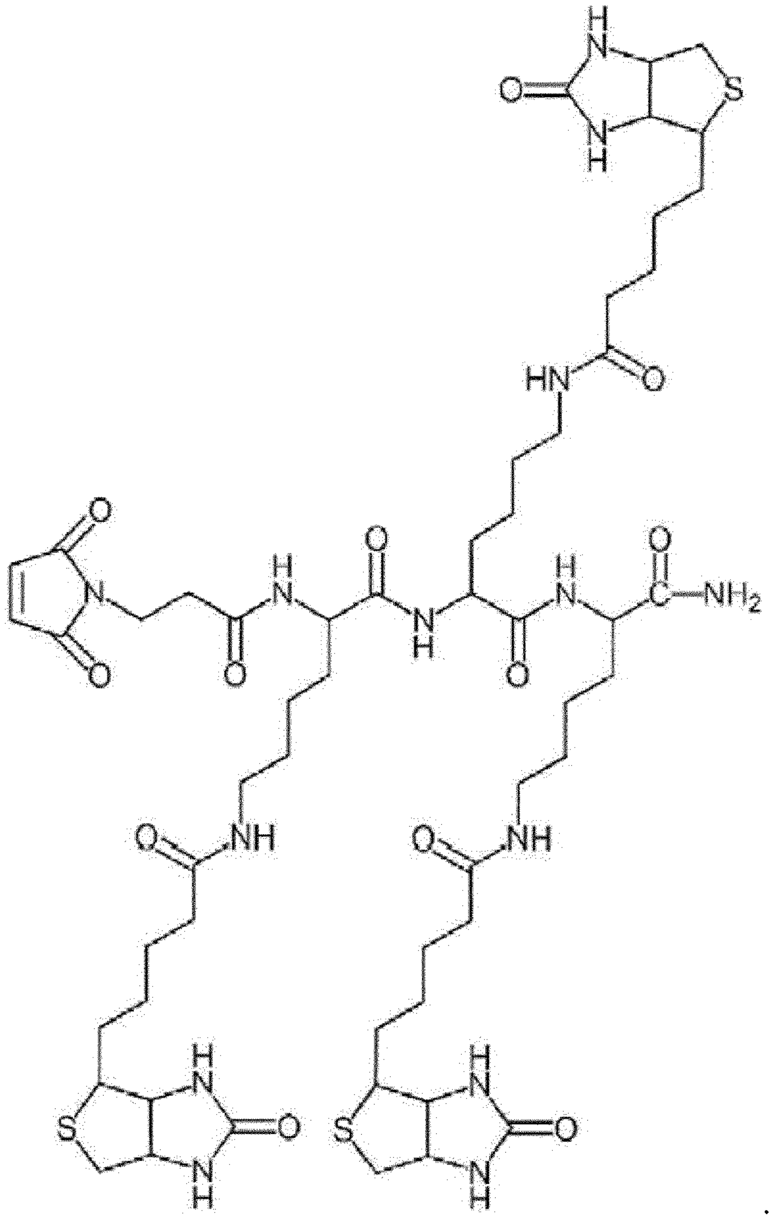

;

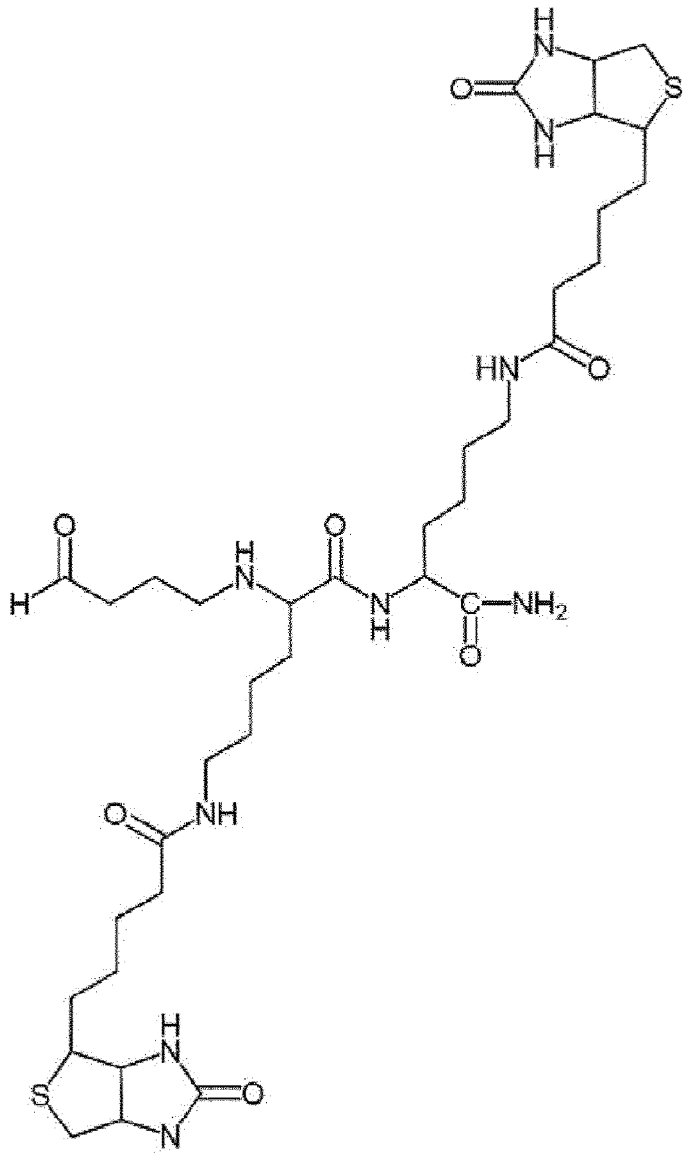

;

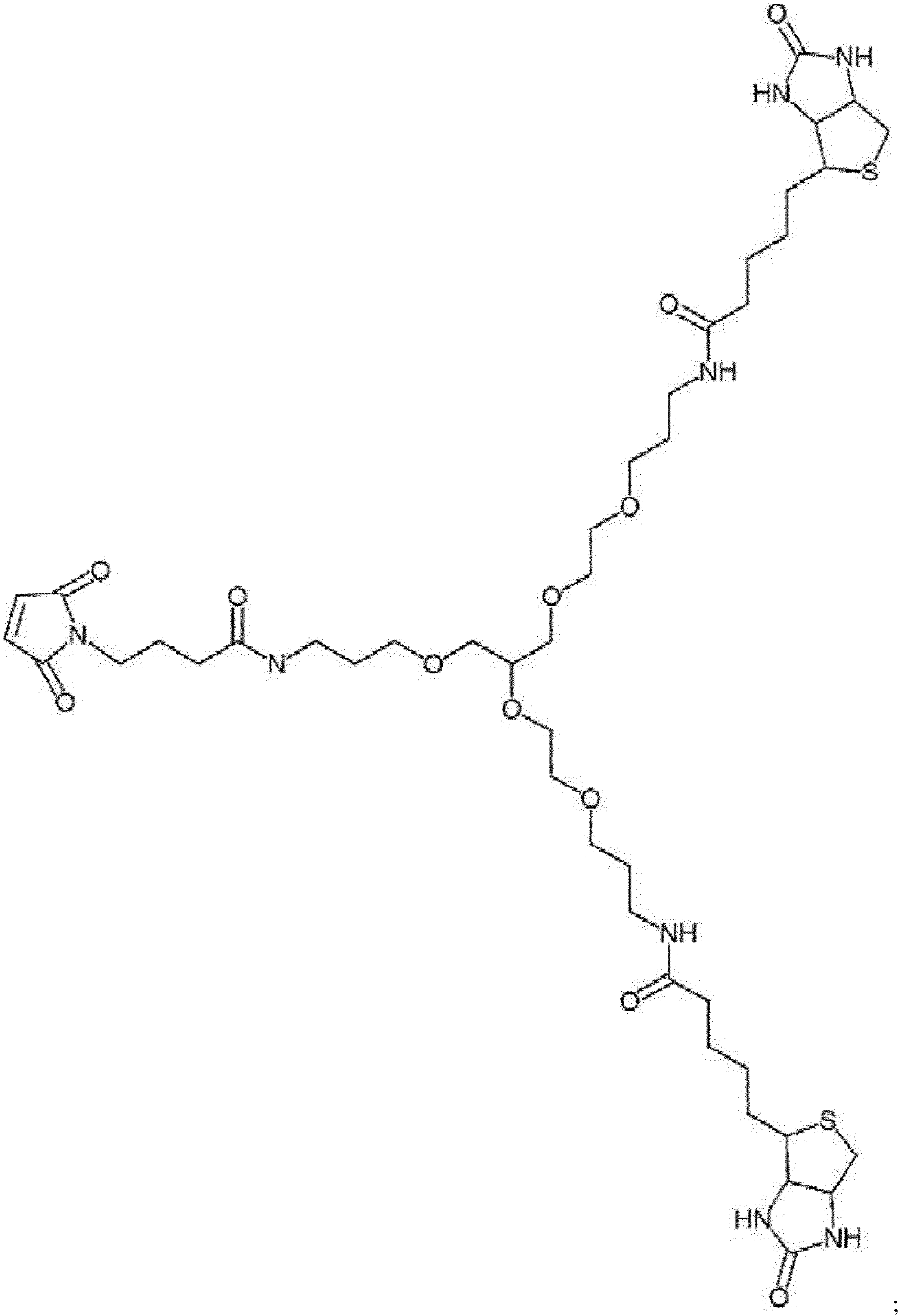

;

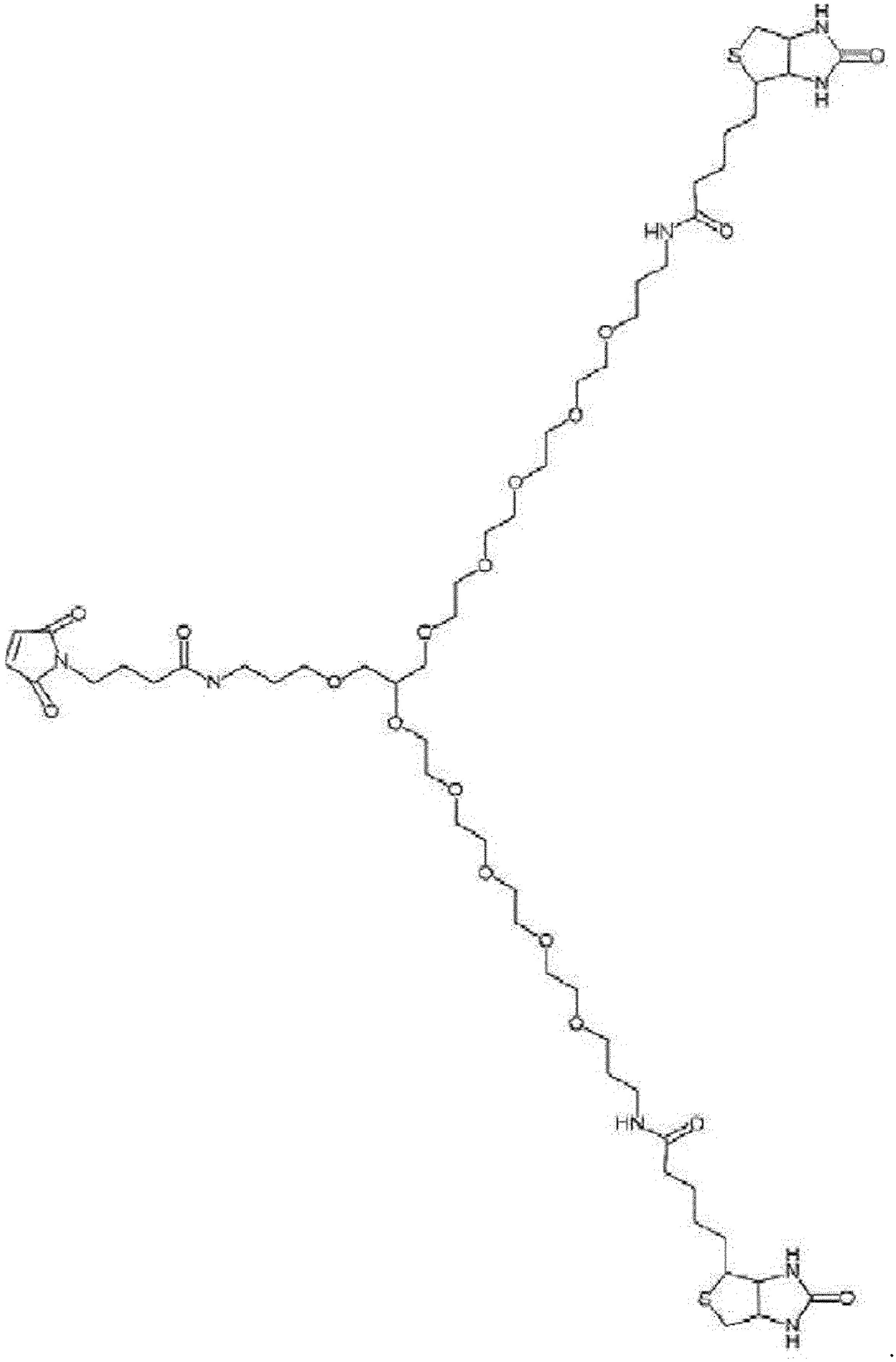

;

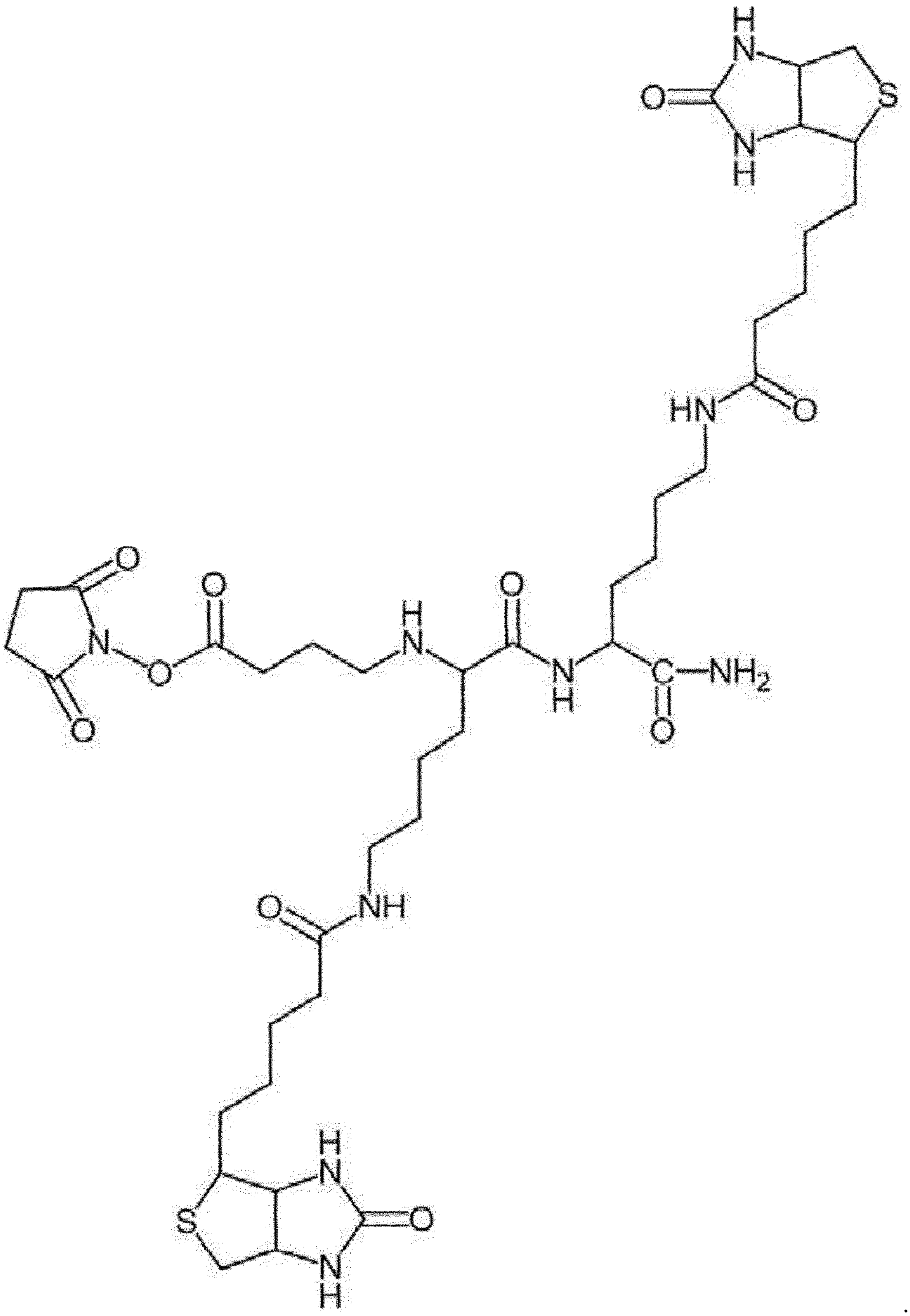

;

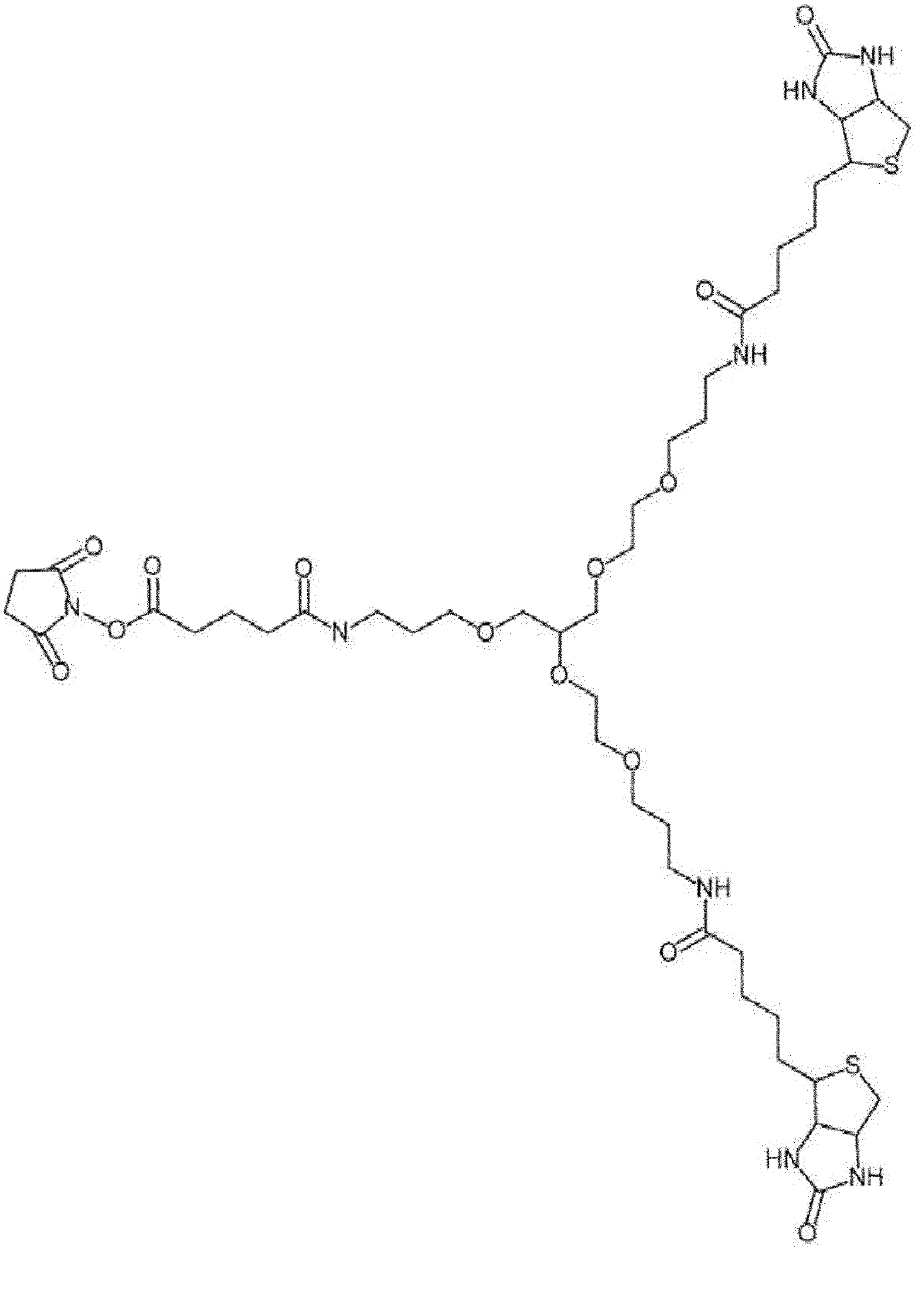

;

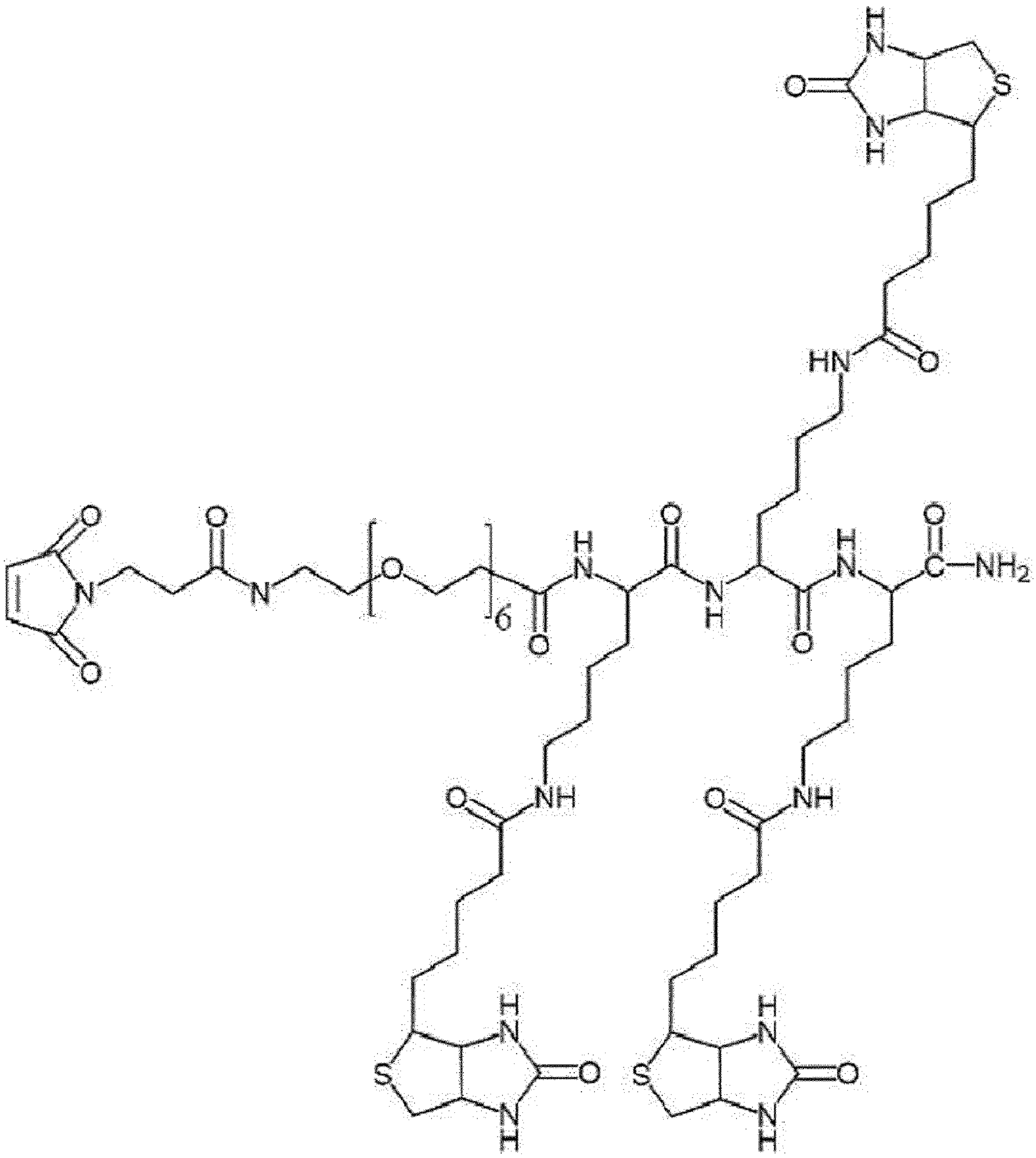

;

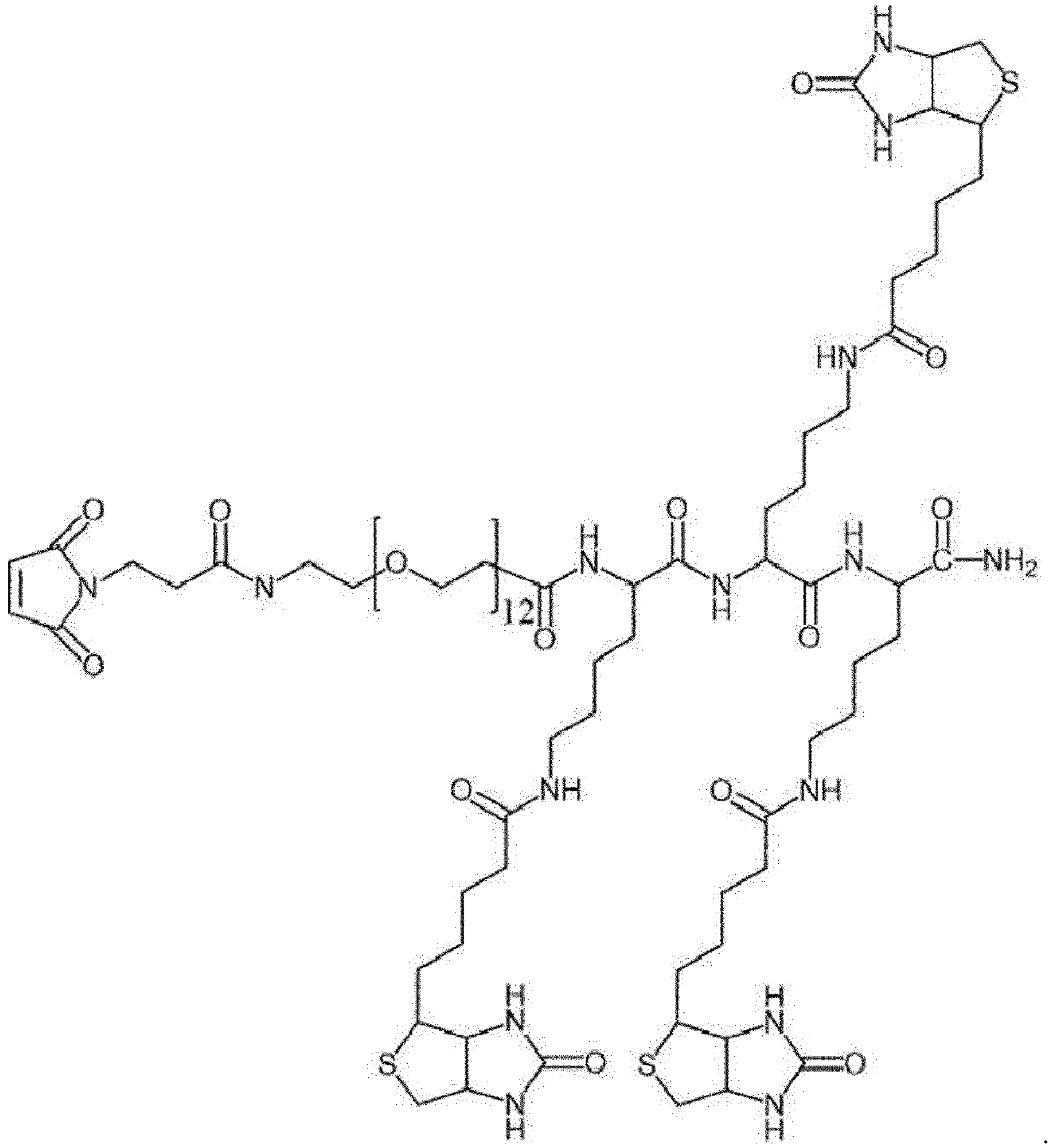

;

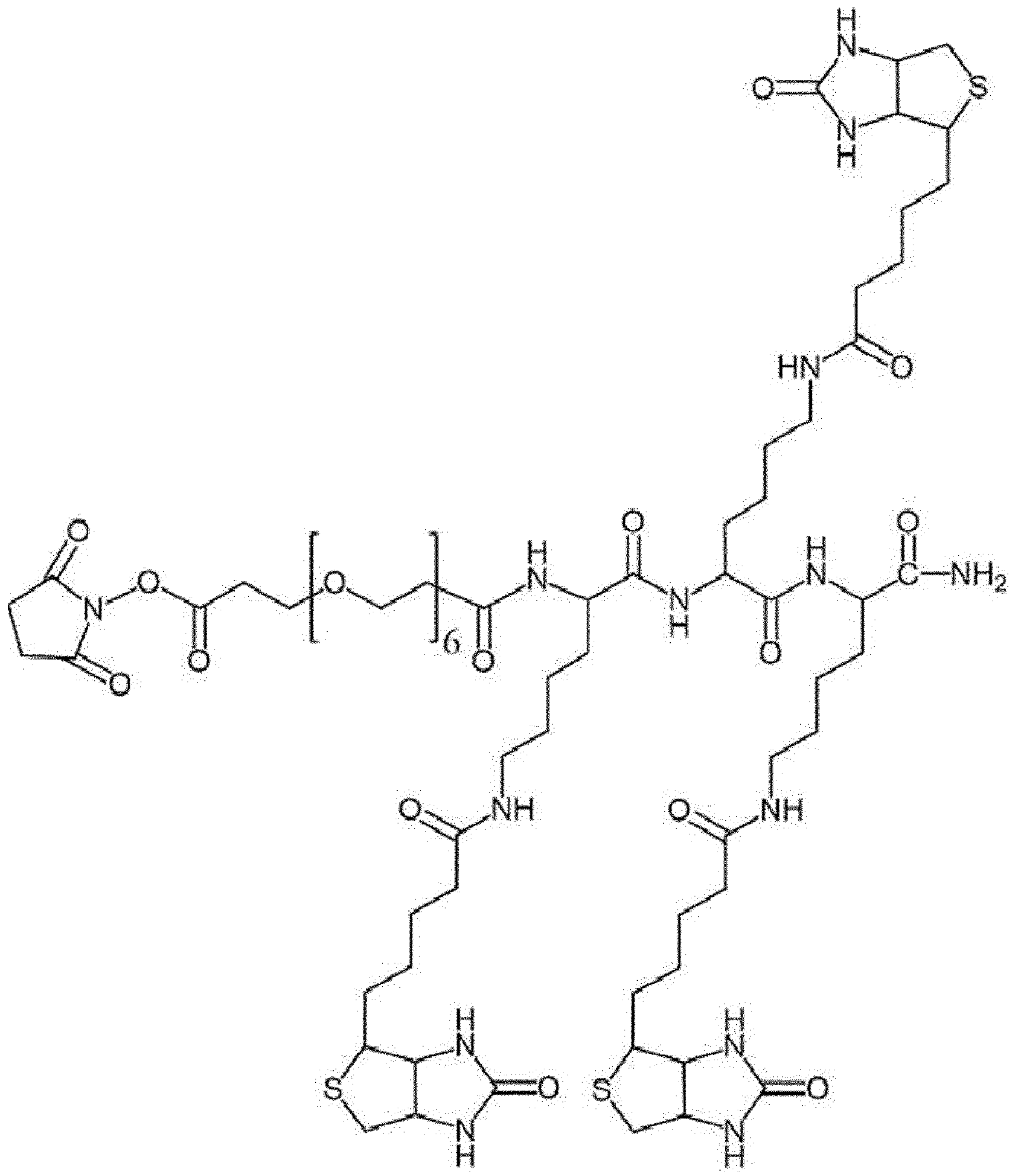

,

;

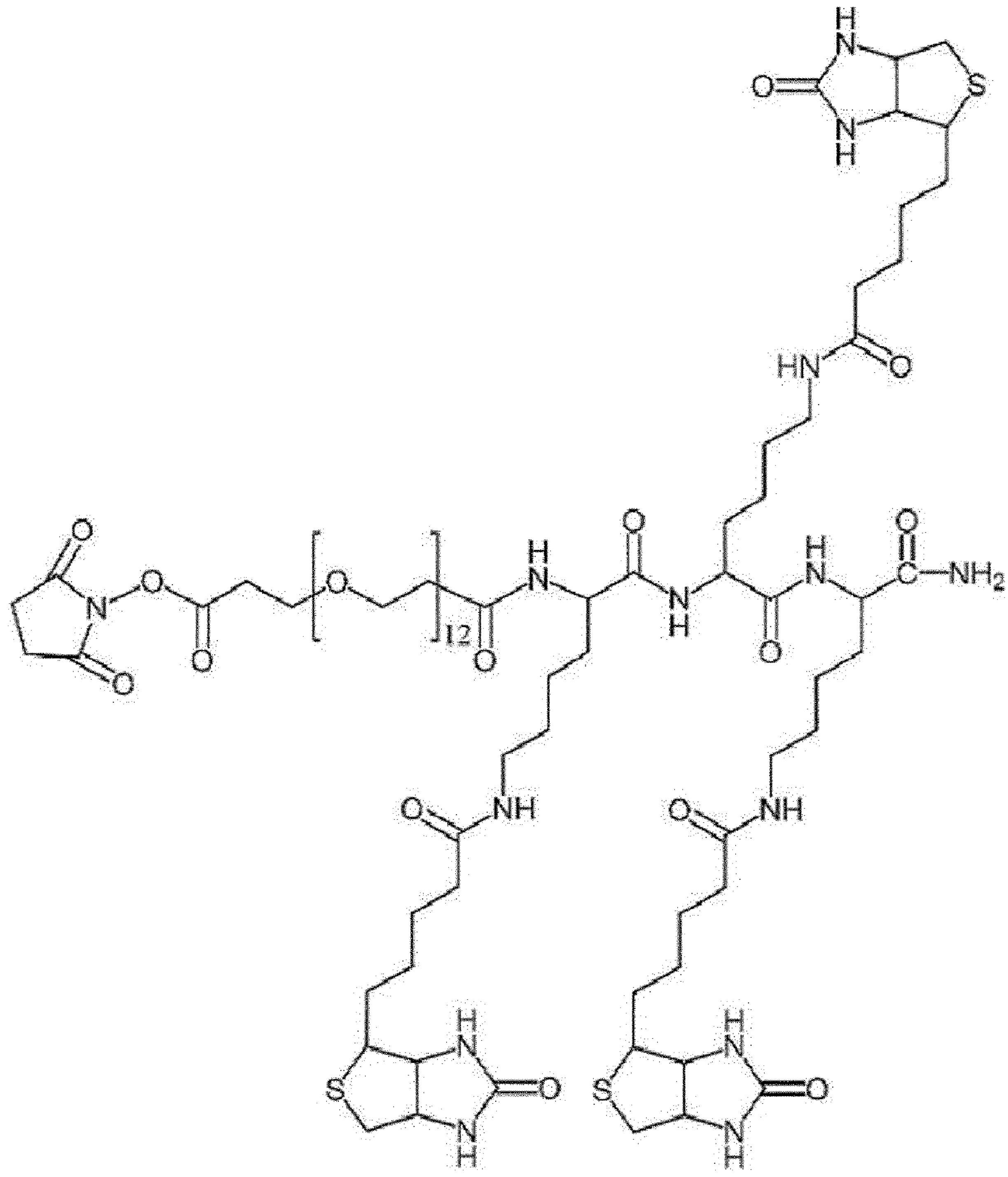

,

;

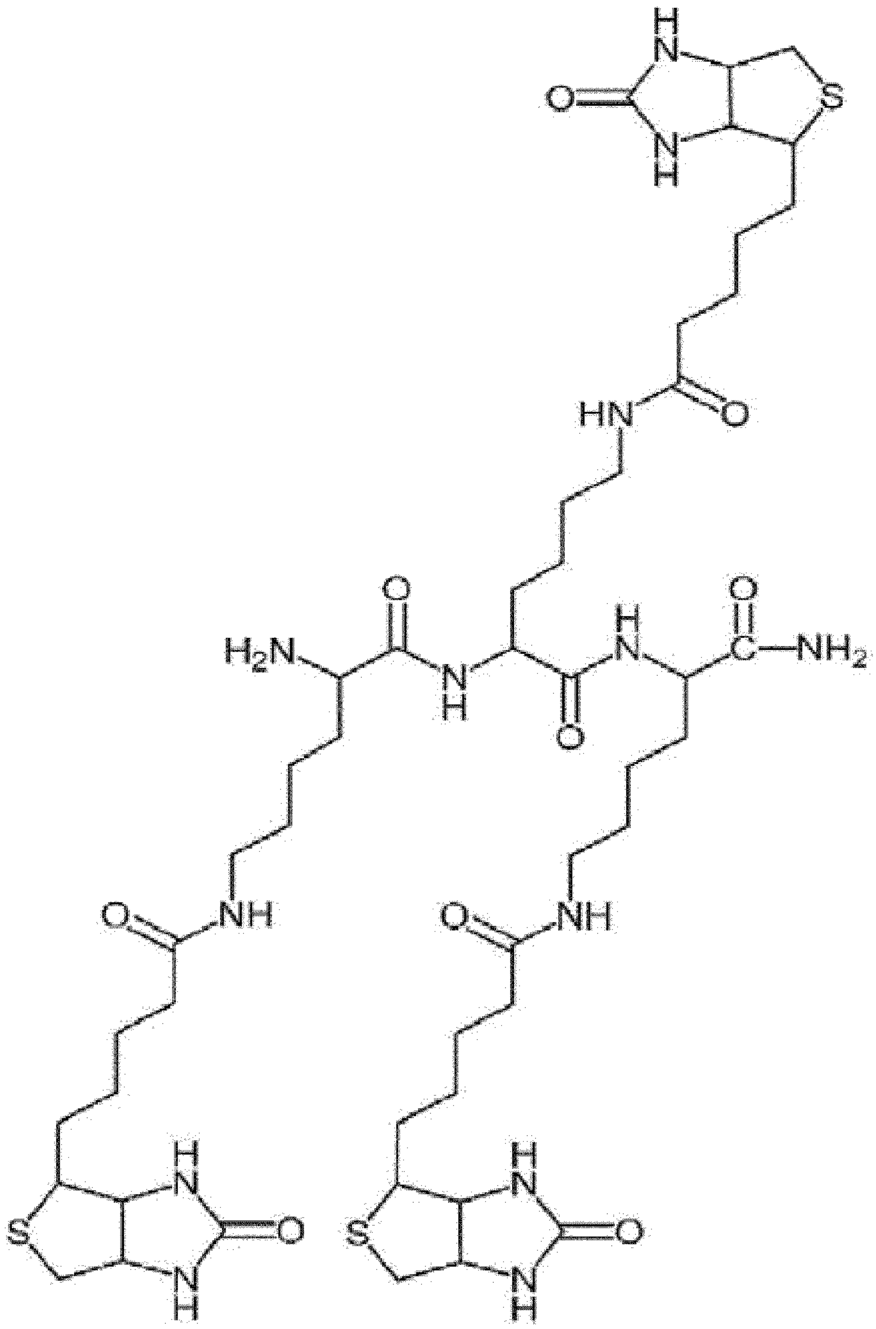

;

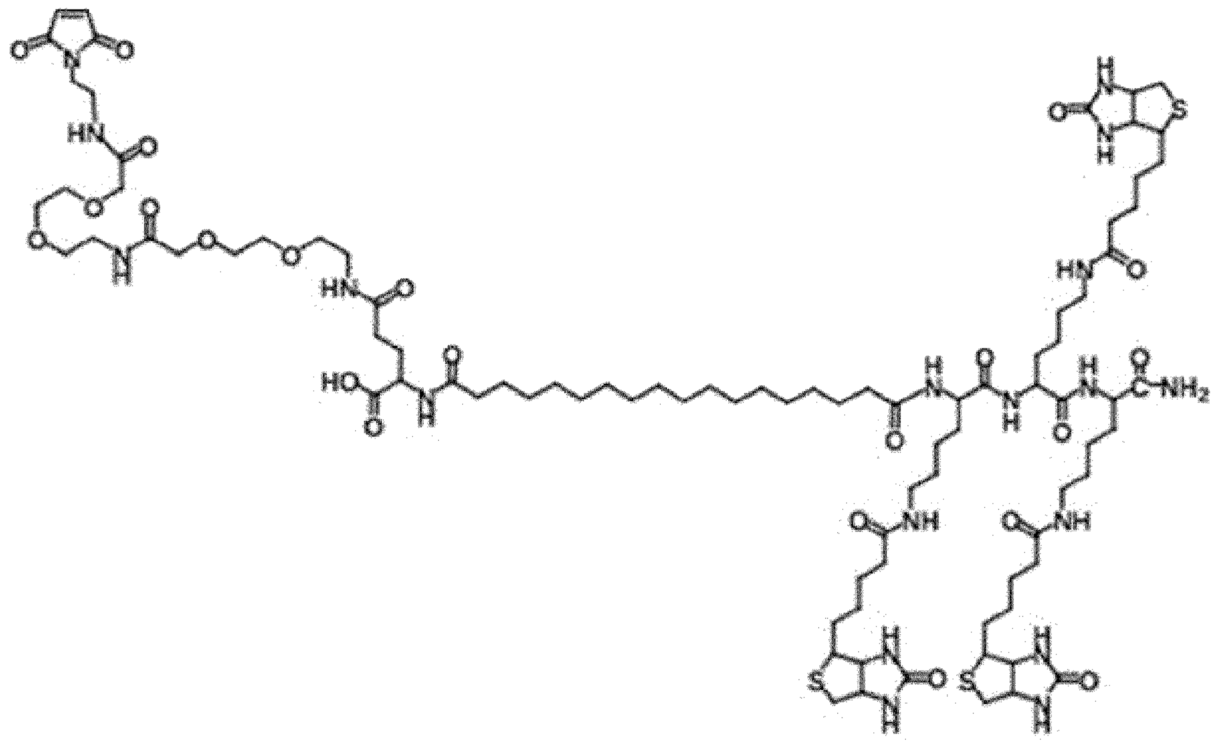

;

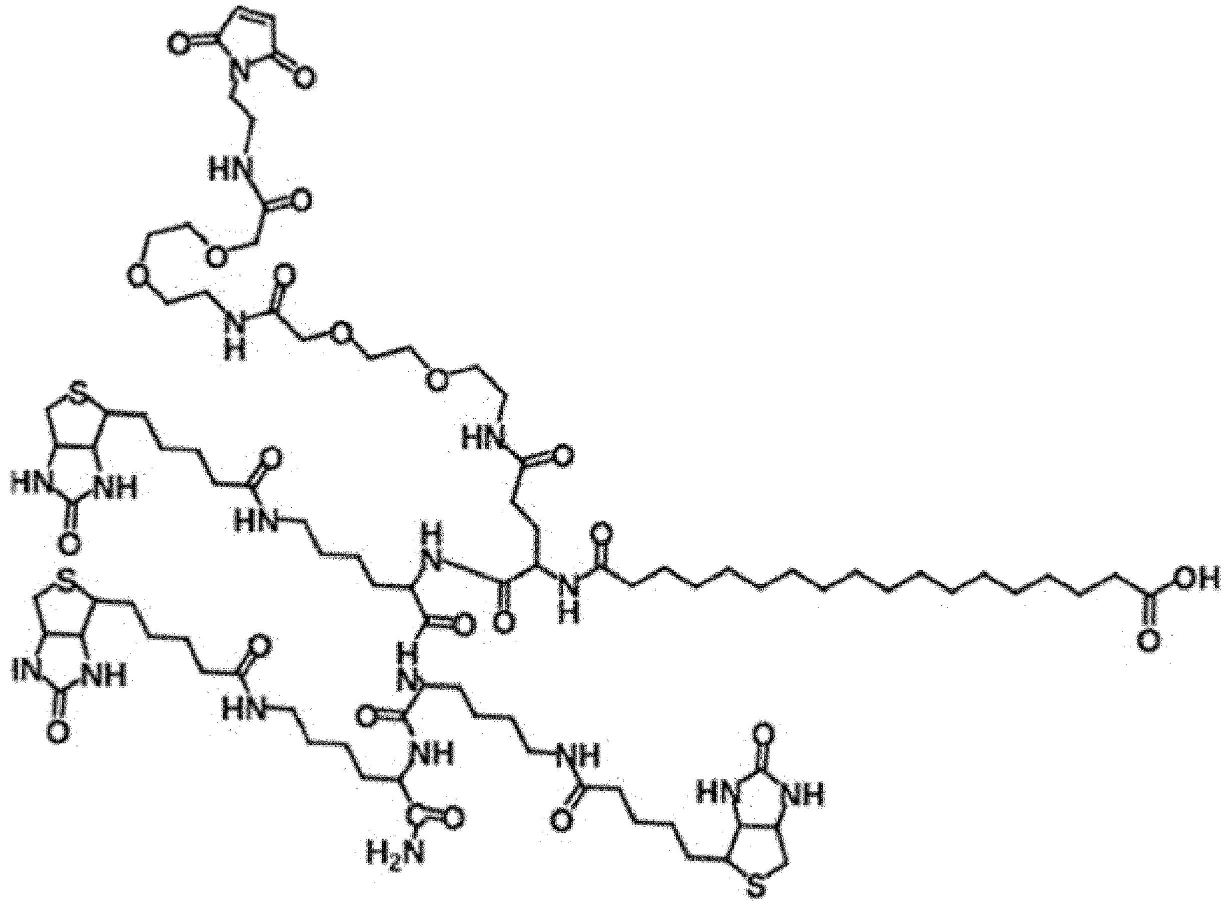

;

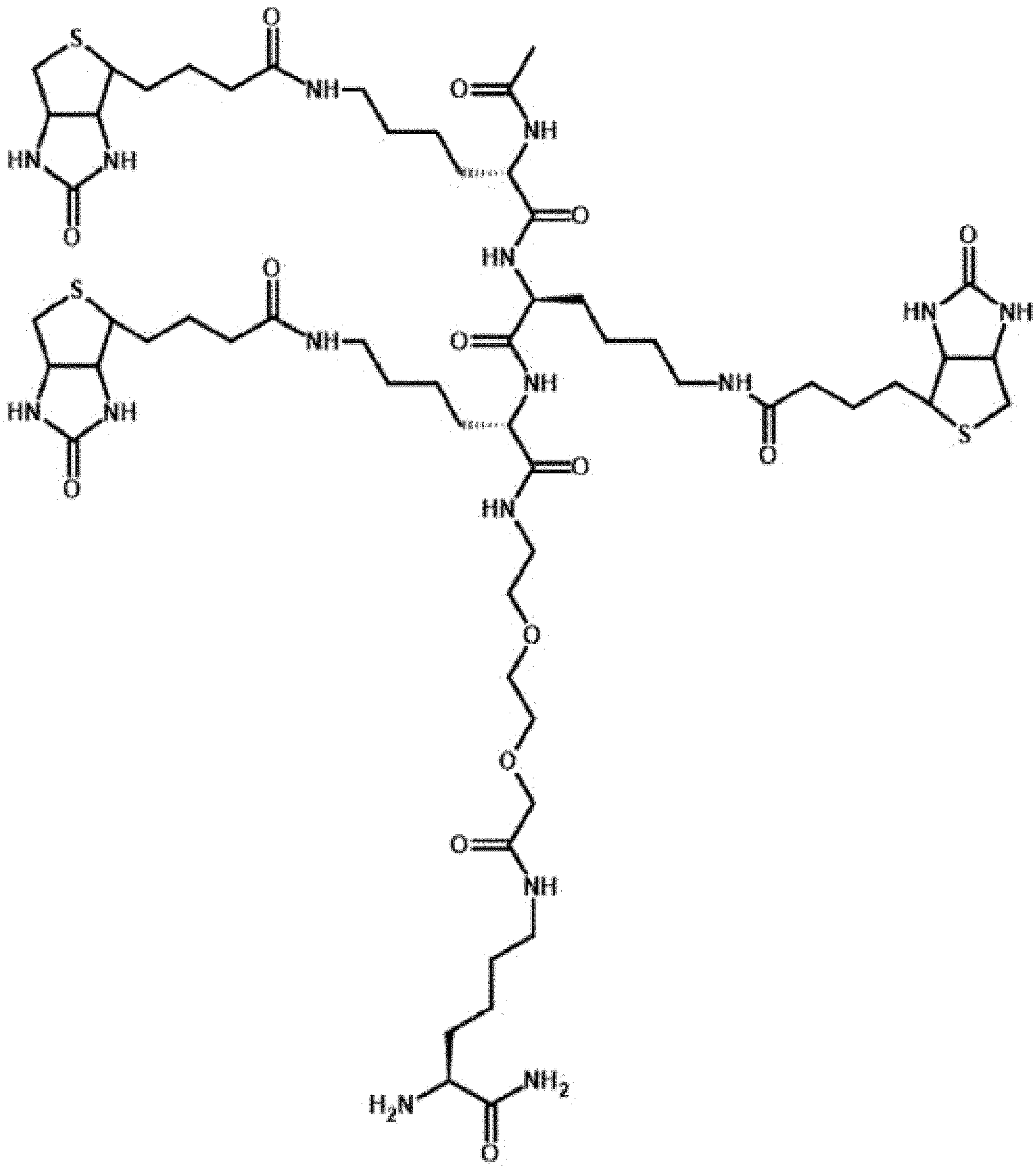

; and,

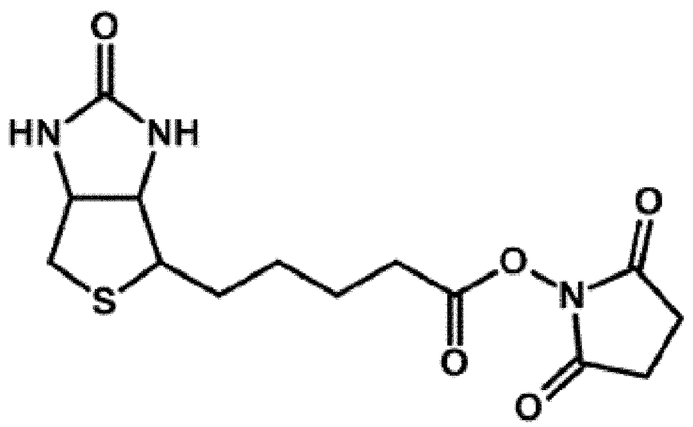

.

According to one embodiment of the present invention, the fatty acid moiety may be represented by General Formula B below:

[General Formula B] X'-Y'-W

where, in the above formula,
X' is a functional group capable of binding to a the biologically active material;
Y' is a spacer; and
W is a fatty acid.

In the present specification, the fatty acid comprises carboxylic acid having a long saturated or unsaturated aliphatic chain, comprising, for example, but not limited to, caprylic acid, lauric acid, which is a type of saturated fatty acid, Palmitic acid, Stearic acid, Arachidic acid, Cerotic acid, Myristoleic acid, which is a kind of unsaturated fatty acid, Palmitoleic acid, oleic acid, linoleic acid, alpha-linolenic acid, and the like.

According to one embodiment of the present invention, in General Formula B, X' is a functional group capable of binding to a biologically active material. Here, X' is the same as X in the General Formula A. Accordingly, in one embodiment of the present invention, the functional group X' may retain its structure or may be eliminated or modified when bound to a biologically active material.

According to one embodiment of the present invention, in General Formula B, W may correspond to a fatty acid. Here, the fatty acid includes all types of fatty acids, including simple, modified, added, deleted and the like.

In one aspect of the present invention, Y' is the same as Y in General Formula Y. Accordingly, in one embodiment of the present invention, the spacer Y' may be a direct bond, or may include a substituted or unsubstituted alkylene, -O-, -C(O), -C(O)NR-, -C(O)O- or -S-, -NR-, -NOR-, or the like. More specifically, Y may be a direct bond, or the structure of Y may comprise at least one of the group comprising substituted or unsubstituted $C_{1-50}$ linear alkylene, substituted or unsubstituted $C_{1-50}$ non-linear alkylene, substituted or unsubstituted $C_{1-50}$ Linear heteroalkylene, substituted or unsubstituted $C_{1-50}$ nonlinear heteroalkylene, substituted or unsubstituted $C_{1-50}$ arylene, substituted or unsubstituted $C_{1-50}$ heteroarylene, -O-, -C (O), -C(O)NR-, -C(O)O-, -S-, -NR- or -NOR-, wherein R is hydrogen, or unsubstituted $C_{1-50}$ alkyl, substituted or unsubstituted $C_{1-50}$ aryl, or an ethylene glycol repeating unit ($-(CH_2CH_2O)_n-$, where n is an integer of at least 1 but not more than 20).

In one aspect of the present invention, W is a substituted or unsubstituted linear or branched $C_{1-60}$ alkylene, substituted or unsubstituted linear or branched $C_{1-60}$ alkenylene, substituted or unsubstituted linear or branched $C_{1-60}$ heteroalkylene, or substituted or unsubstituted linear or branched $C_{1-60}$ heteroalkenylene, and if substituted, may be substituted by at least one selected from the group comprising =O, $-C(O)NH_2$, -OH, -COOH, -SH, =NH, $-NH_2$, and halo.

In one aspect of the present invention, W is a $C_{12-24}$ alkylene wherein at least one is substituted or a $C_{36-48}$ heteroalkylene wherein at least one is substituted, and if substituted, may comprise =O or-COOH.

In a specific aspect of the present invention, the W wherein at least one is substituted is a substituted or unsubstituted $C_{12-24}$ saturated fatty acid, and if substituted, comprises -COOH.

In one aspect of the present invention, the fatty acid moiety may have the chemical formula of General Formula B1 below:

[General Formula B1] $X'_1$ -Y'-C(O)-$F_1$

where, in the above formula,
$X'_1$ is maleimide, N-hydroxysuccinimide, aldehyde, amine, tetrafluorophenyl ester or nitrophenol;
Y' is a spacer;
$F_1$ is a $C_{6-28}$ substituted or unsubstituted linear or branched alkylene, or substituted or unsubstituted linear or branched heteroalkylene.

According to one aspect of the present invention, in General Formula B-1, Xi may be the same as X in General Formulas A and B. Therefore, in one aspect of the present invention, the functional group Xi may retain its structure or may be eliminated or modified when bound to a biologically active material.

Further, in General Formula B1, Y' may be the same as Y in General Formulae A and B.

In one aspect of the present invention, Y' is a substituted or unsubstituted $C_{6-50}$ linear or branched heteroalkylene, and if substituted, comprises at least one selected from the group comprising =O,$-C(O)NH_2$, -OH, -COOH, -SH, =NH and $-NH_2$.

In one aspect of the present invention, the Y' may comprise $-(CH_2CH_2O)-$ as a repeating unit.

In one aspect of the present invention, the Y' may comprise $-C(O)-(OCH_2CH_2)_u-NH-$ as a repeating unit, where u is an integer of 1 to 20.

In a specific aspect of the present invention, the Y' comprises -C(O)-$(OCH_2CH_2)_u$-NH- as a repeating unit, where u is an integer of 2 to 4.

In one aspect of the present invention, the Y' comprises an amino acid or a derivative thereof as a component.

In a specific aspect of the present invention, the Y' comprises glutamic acid, glutamine, glycine, isoleucine, or lysine as a component, where each amino acid may exist in bonded form.

In a specific aspect of the present invention, the Y' comprises glutamic acid or lysine as a component. In one aspect of the present invention, the $F_1$ may be a substituted or unsubstituted $C_{10-28}$ linear or branched alkylene.

In a specific aspect of the present invention,, the W wherein at least one is substituted is a substituted or unsubstituted $C_{12-24}$ saturated fatty acid, and if substituted, comprises -COOH.

In a specific aspect of the present invention, the $F_1$ is -$(CH_2)_v$-COOH, where v is an integer of 10 to 20.

In a specific aspect of the present invention, the $F_1$ is -C(O)-$(CH_2)_v$-COOH, where v is an integer of 10 to 20.

In a specific aspect of the present invention, the fatty acid moiety may be selected from the group composed of:

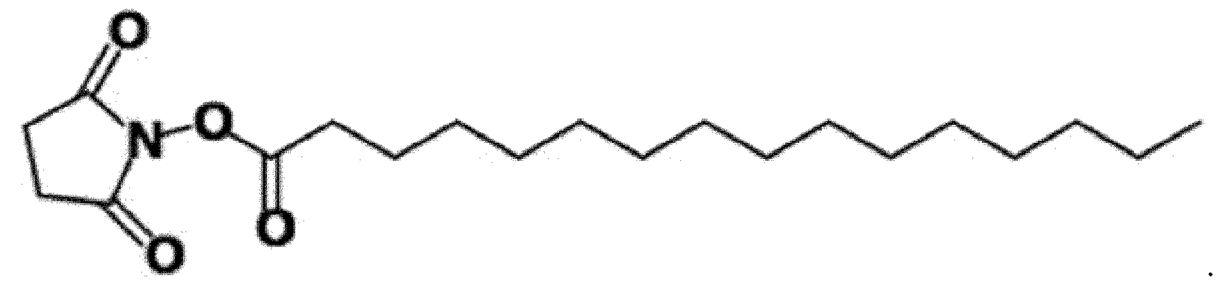

;

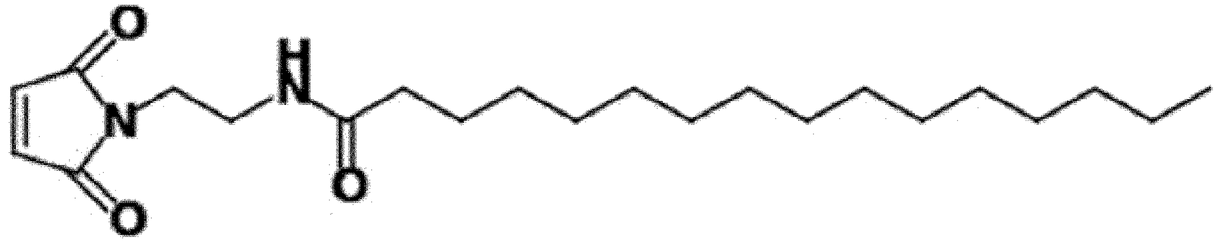

;

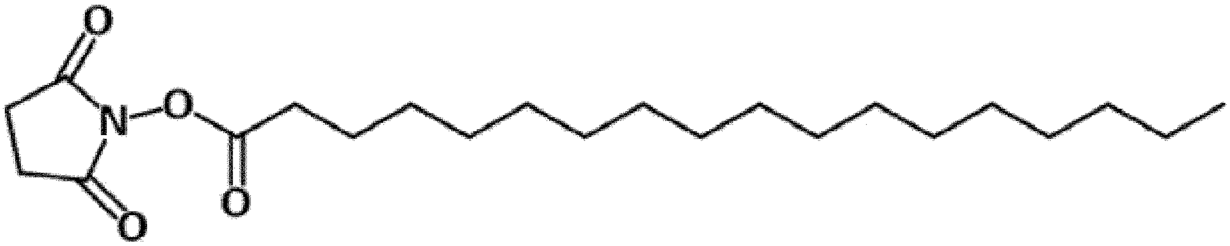

;

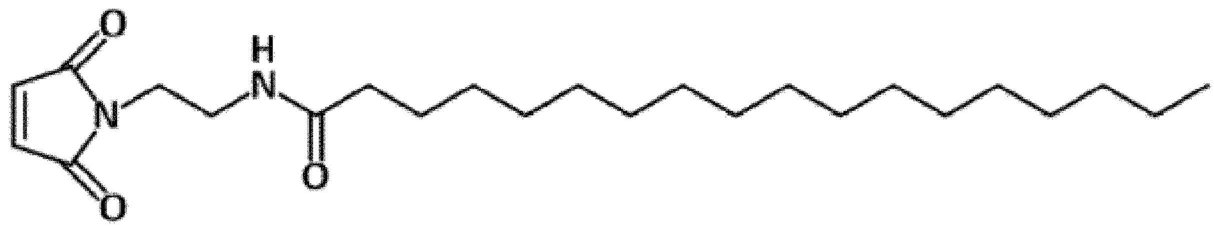

;

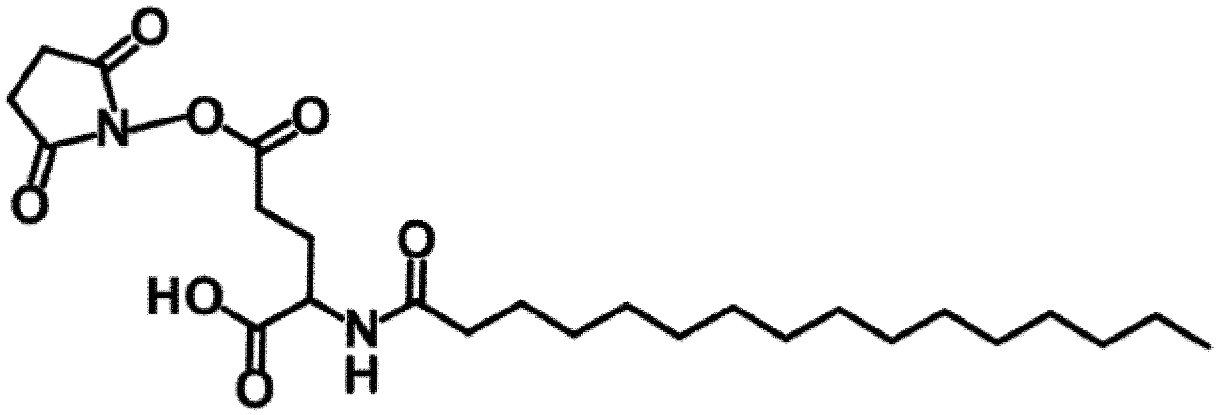

;

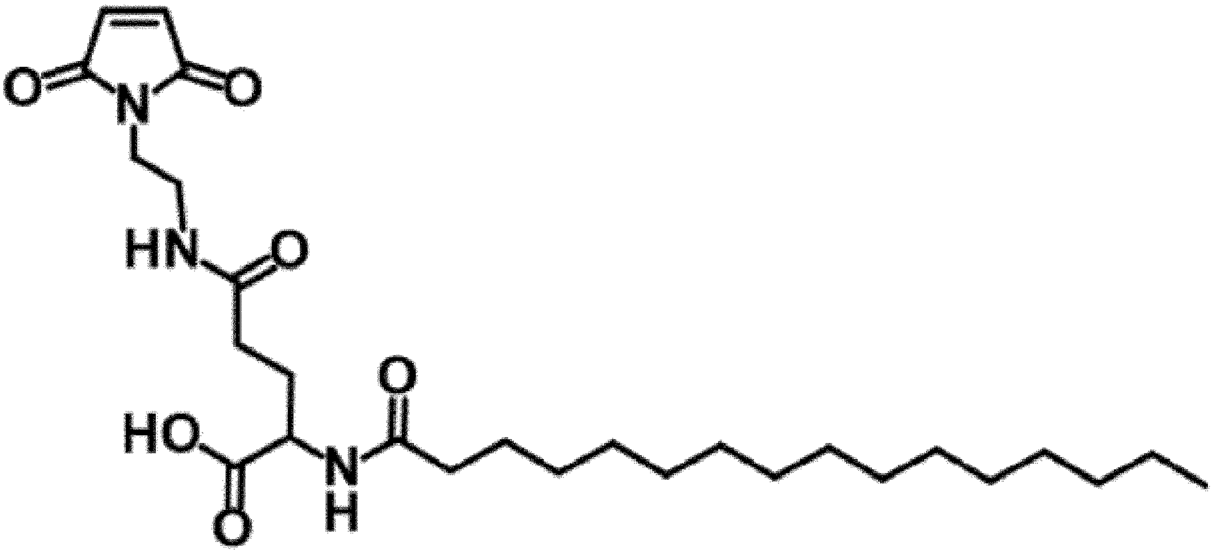

;

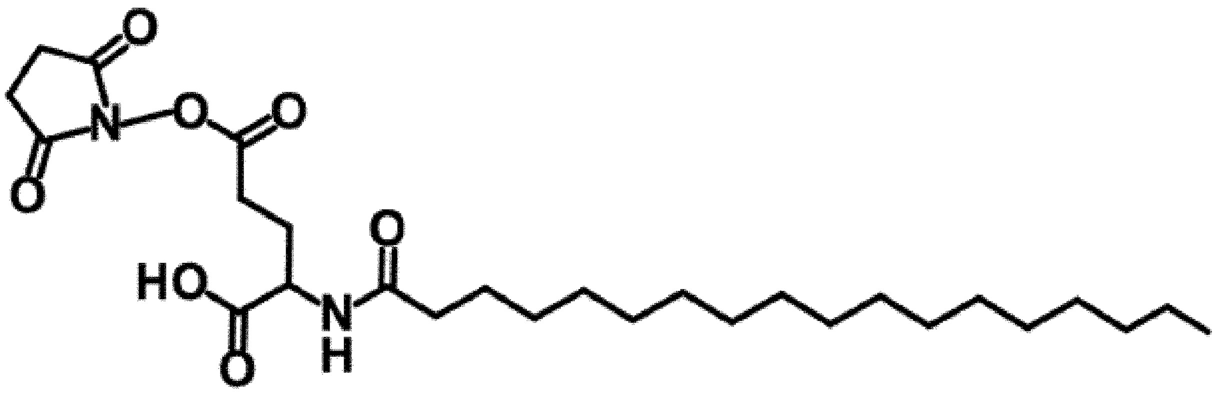

;

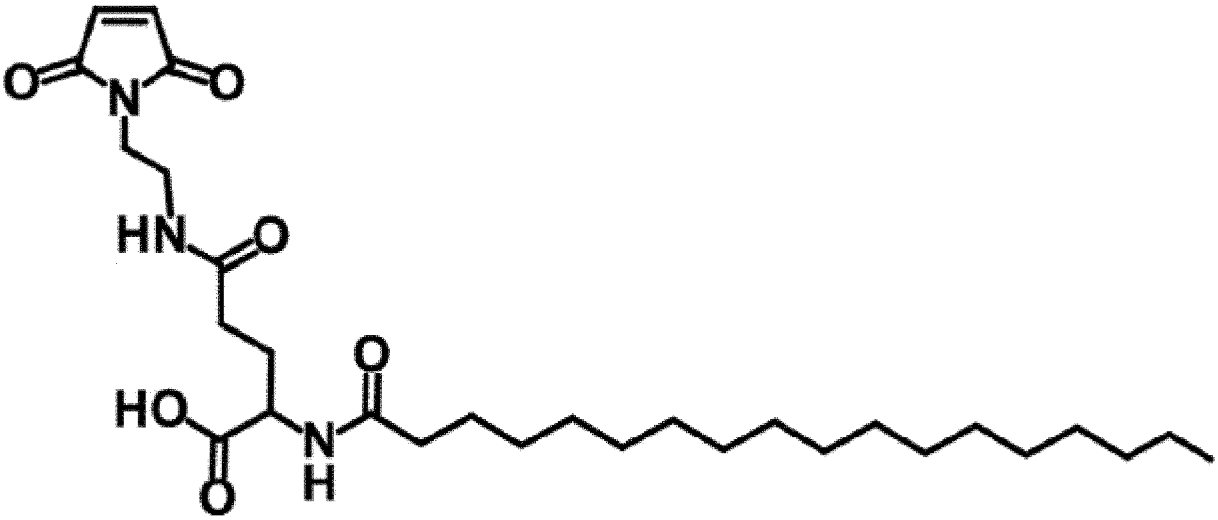

;

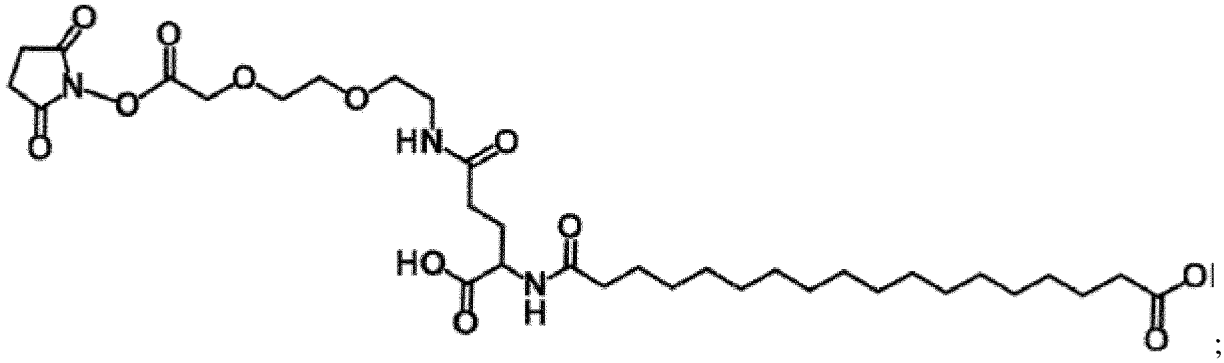

;

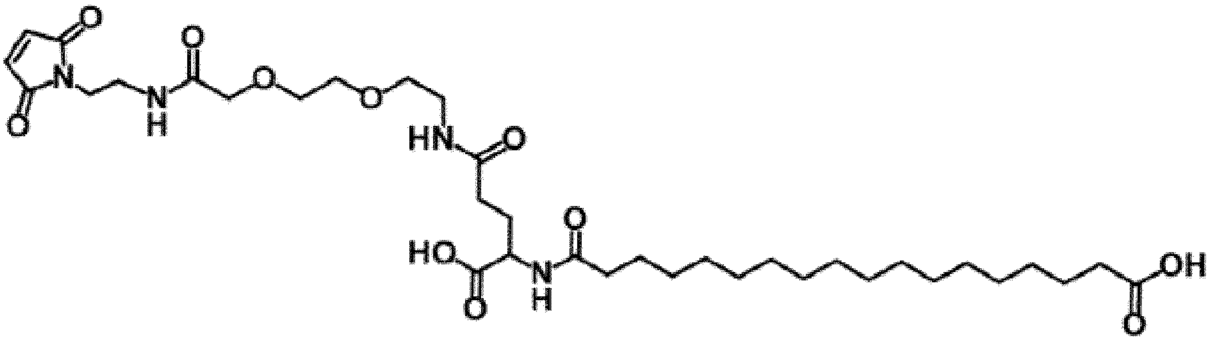

;

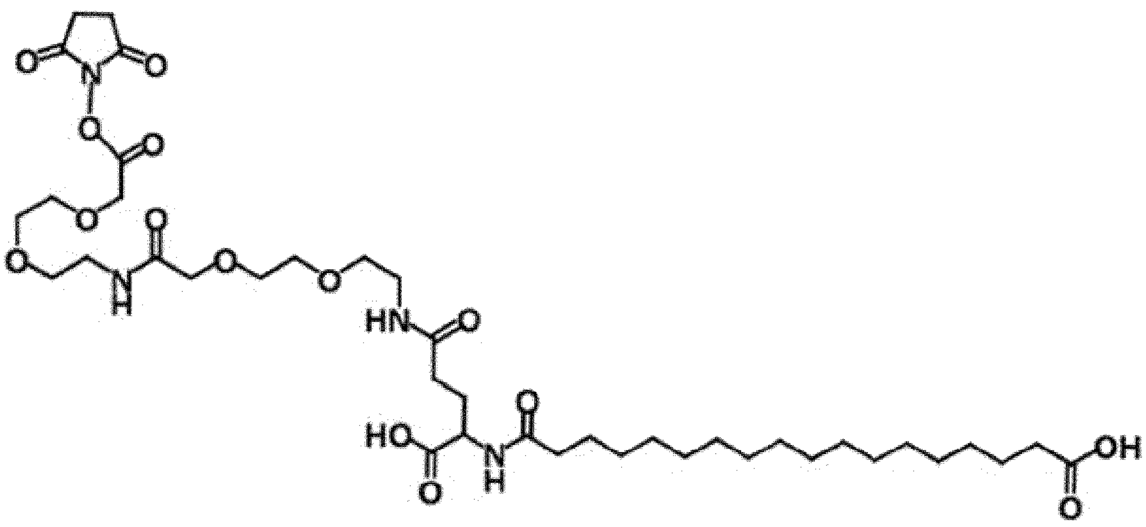

;

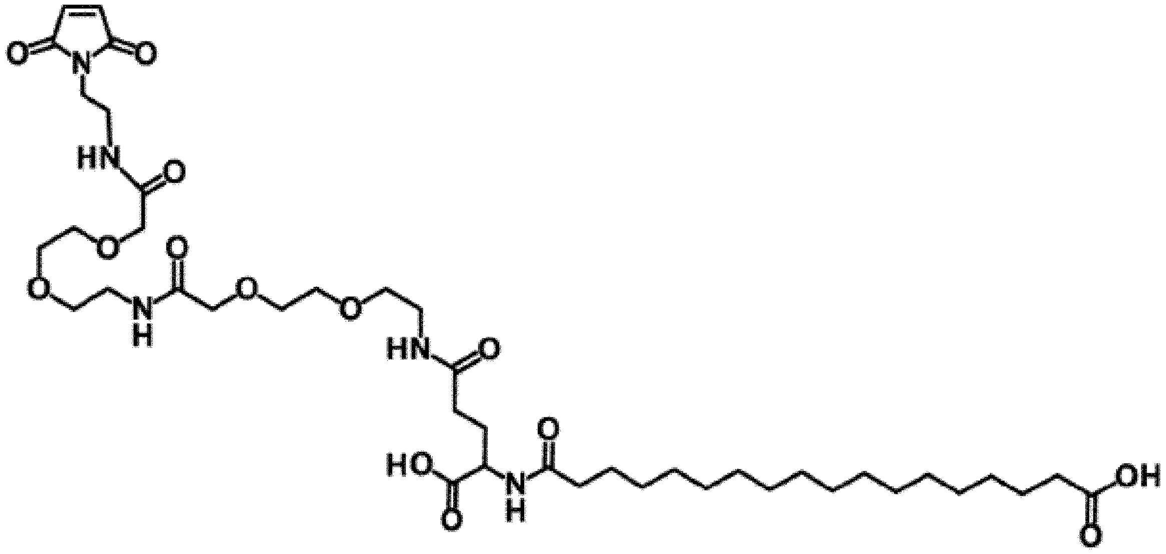

;

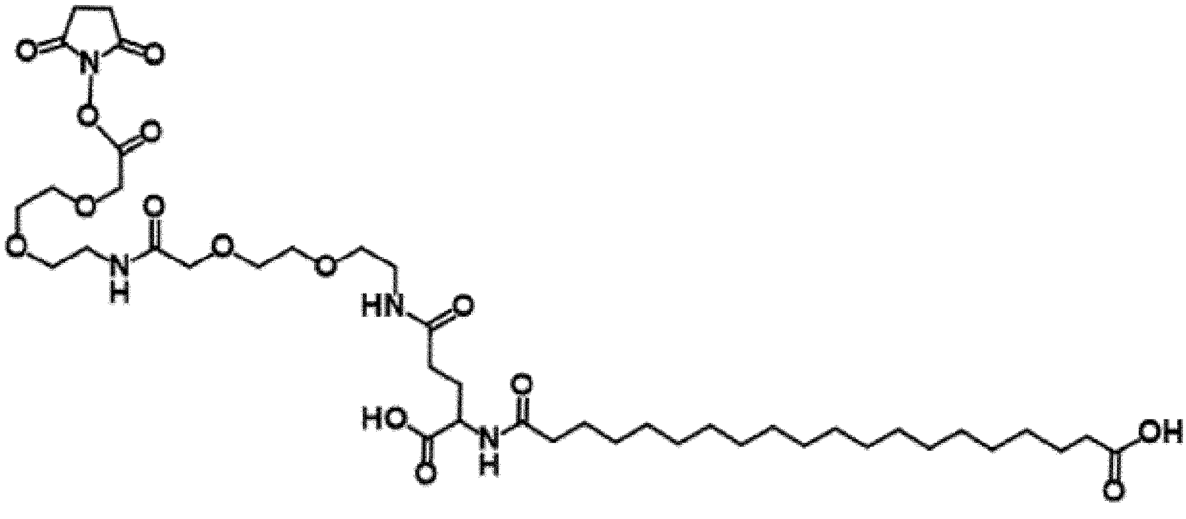

;

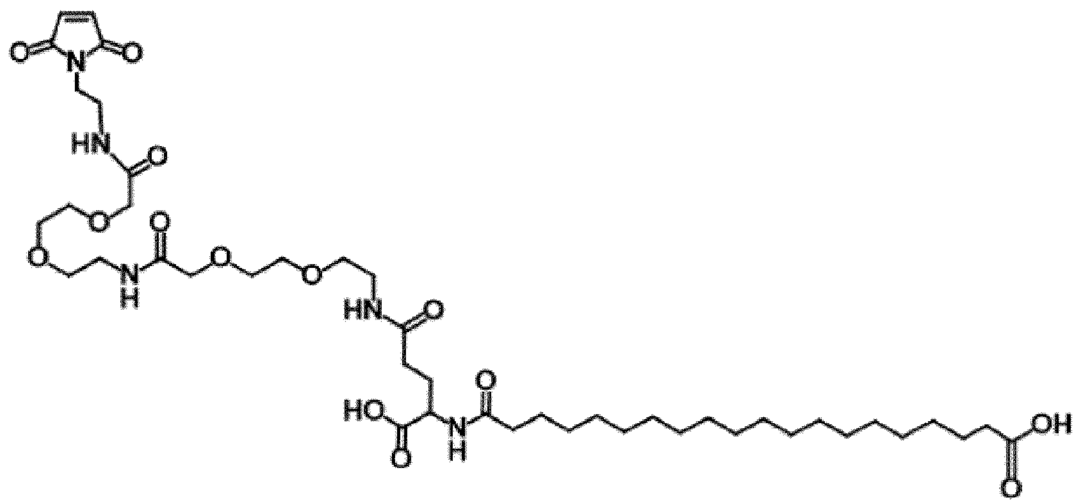

;

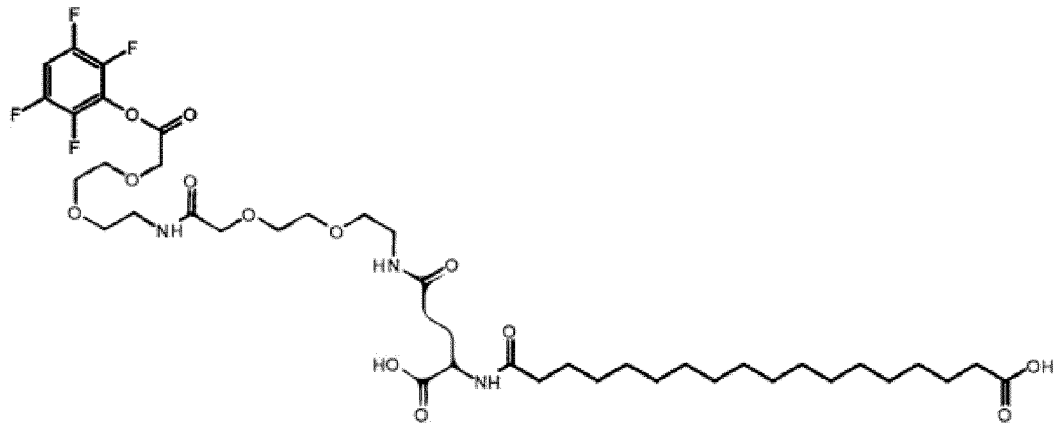

;

and

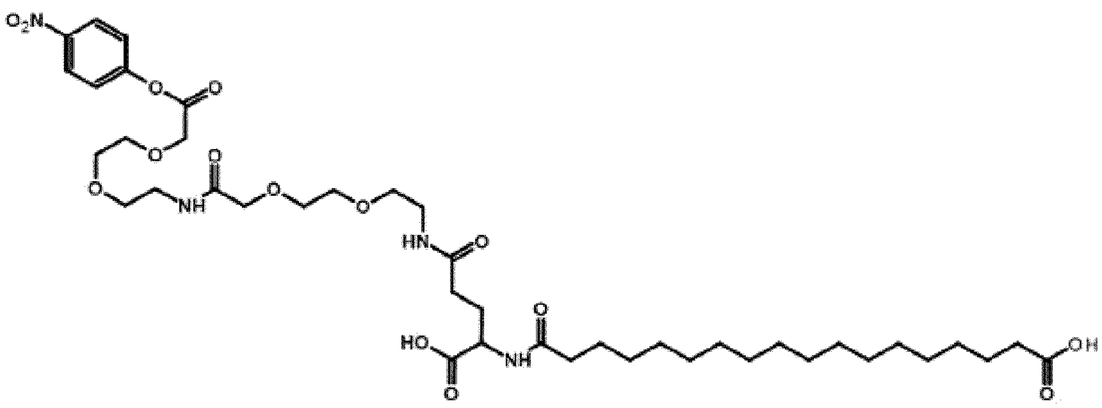

.

According to one embodiment of the present invention, the bond between biotin moiety and the biologically active material may be formed by various bonds. It may be formed by bonding a functional group of a biotin moiety with a functional group of a physiologically active material, and may be formed as, for example, but is not limited to, a thiol-ether bond or an amide bond.

In one specific example, the bond between the biotin moiety and the biologically active material may be formed by the method of Reaction Formula 1 below. In Reaction Formula 1,

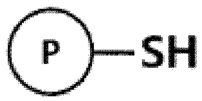

represents a biologically active material comprising a thiol group, and represents a reaction between a biotin moiety comprising maleimide according to an embodiment of the present invention and a thiol group (-SH) of a cysteine residue present in the biologically active material.

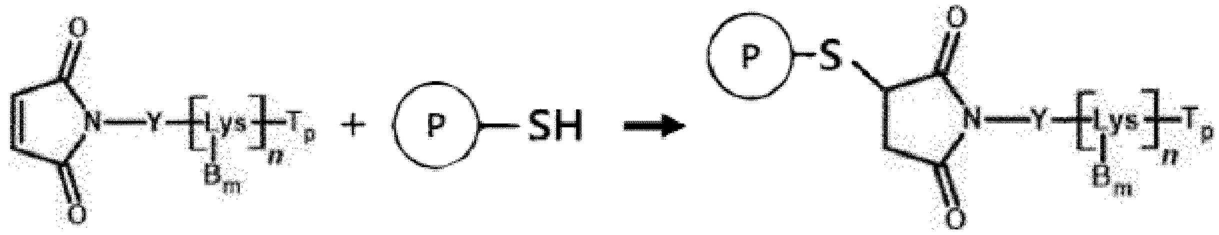

[Reaction Formula 1]

In one specific example, the bond between the biotin moiety and the biologically active material may be formed by the method of Reaction Formula 2 below. In Reaction Formula 2,

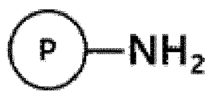

represents a biologically active material comprising an amine group, and represents a reaction between a biotin moiety comprising N-hydroxy succinimide according to an embodiment of the present invention and an amine group ($-NH_2$) present in the biologically active material.

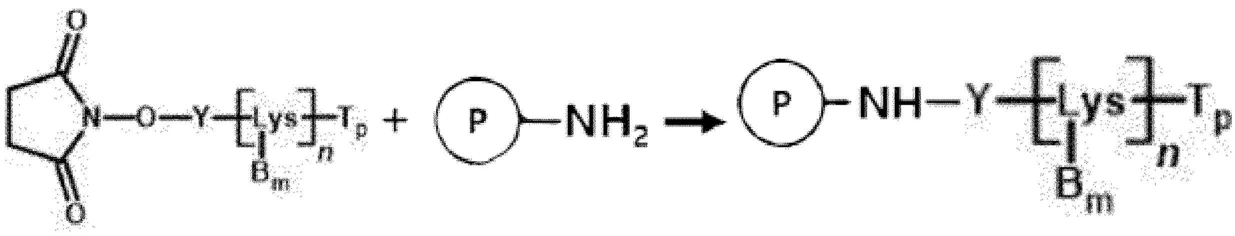

[Reaction Formula 2]

According to one embodiment of the present invention, there may be no particular limitation on the biologically active material.

In the present invention, a biologically active material is a material which may be administered to the body for a specific purpose, and which causes a physiological or biochemical reaction in the body.

According to an embodiment of the present invention, the biologically active material may be a material used in a pharmaceutical formulation. For example, it may be a material used for the prevention or treatment of diabetes, obesity, fatty liver disease, irritable bowel syndrome, neurodegenerative disease, bone disease, osteoporosis, human growth hormone deficiency, anticancer or non-alcoholic fatty liver disease. These are non-limiting examples, as the indications may vary depending on the type of the biologically active material.

According to one embodiment of the present invention, the biologically active material may be, but is not limited to, a polypeptide or a non-peptidic polymer. Non-limiting examples include polypeptide, protein, polysaccharide, or a derivative thereof. Non-limiting examples of the biologically active material include glucagon (Glugacon), GLP-1 (Glucagon-like peptide-1), GLP-2 (Glucagon-like peptide-2), GIP (glucose-dependent insulinotropic polypeptide) ), exendin-4, insulin, parathyroid hormone, interferon, erythropoietin, calcitonin, amylin, serotonin, rituximab, trastuzumab, uricase, tissue plasminogen activator, thymoglobin, vaccine, heparin or heparin analog, antithrombin III, filgrastim, pramlintide acetate, exenatide, eptifibatide, antivenin, IgG, IgM, HGH, thyroxine, blood clotting factors VII and VIII, glycolipids acting

as therapeutic agents, and derivatives thereof. According to one embodiment of the present invention, [the biologically active material] may be bonded to a biotin moiety.

By bonding a biotin moiety to the biologically active material, it is possible to not inhibit the biological activity of the biologically active material, and thereby it is possible to have the same biological activity as the biologically active material or an improved biological activity.

Although not limited hereto, the biologically active material may comprise an exposed -SH group, so that a biotin moiety may be bonded to the -SH group. In addition, the biologically active material may comprise an exposed $-NH_3^+$ group or a $-NH_2$ group, so that a biotin moiety may be bonded to the exposed $-NH_3^+$ group or $-NH_2$ group.

According to one embodiment of the present invention, the binding site of the biotin moiety with the biologically active material may be adjusted so as to bond while avoiding sites which exhibit activity. Further, according to one embodiment of the present invention, the fatty acid moiety may be bonded directly to the biologically active material. Further, part of the fatty acid moiety may be shared with the biotin moiety. For example, in one embodiment of the following embodiments, biotin moieties B35 and B36 share the fatty acid portion which is part of a fatty acid moiety. Provided, that this is only one example, and the present invention is not limited hereto.

Further, according to one embodiment of the present invention, the fatty acid moiety may be bonded to the biologically active material, at a site of the biologically active material other than the site at which the biotin moiety is bonded.

Further, the fatty acid moiety, like the biotin moiety, may be bonded to an active site or inactive site of the biologically active material, and may exhibit the same properties as stated above.

According to one embodiment of the present invention, both the biotin moiety and the fatty acid moiety may be bonded to the biologically active material, and a biologically active material conjugate to which both a biotin moiety and fatty acid moiety are bonded, when compared to a conjugate to which only a biotin moiety or only a fatty acid moiety is bonded, may exhibit superior oral absorption rate, pharmacokinetics, enzyme degradation inhibition, intestinal membrane permeation, and the like. According to one embodiment of the present invention, the biologically active material may be glucagon, calcitonin, GLP-1, GLP-2, GIP, exendin-4, parathyroid hormone, insulin, amylin, human growth hormone or a derivative thereof.

According to an embodiment of the present invention, the biologically active material may be a polypeptide having any one of the following amino acid sequences of SEQ ID NOs 1 to 7 or derivatives thereof. Specifically, the biologically active materials of SEQ ID Nos: 1 to 7 are, respectively glucagon derivatives (SEQ ID NO: 1), GLP-1 (SEQ ID NO: 2), GLP-2 (SEQ ID NO: 3), GIP (SEQ ID NO: 4), exendin-4 (SEQ ID NO: 5), parathyroid hormone (SEQ ID NO: 6), and glucagon (SEQ ID NO: 7).

SEQ ID NO: 1: H(Aib)QGTFTSDYSKYLDEQAAKEFVQWLMNT
SEQ ID NO: 2: HAEGTFTSDVSSYLEGQAAKEFIAWLVKGR
SEQ ID NO: 3: HADGSFSDEMNTILDNLAARDFINWLIQTKITD
SEQ ID NO: 4: YAEGTFISDYSIAMDKIHQQDFVNWLLAQKGKKKNDWKHNITQ
SEQ ID NO: 5: HGEGTFTSDLSKQMEEEAVRLFIEWLKNGGPSSGAPPPS
SEQ ID NO: 6: SVSEIQLMHNLGKHLNSMERVEWLRKKLQDVHNF
SEQ ID NO: 7: HSQGTFTSDYSKYLDSRRAQDFVQWLMNT

In addition, the biologically active material may be proteins having the amino acid sequence of SEQ ID NOs: 15 and 16 or a protein having the amino acid sequence of SEQ ID NOs: 17 and 16.

In addition, the biologically active material may be proteins having the amino acid sequence of SEQ ID NOs: 15 and 16 or a protein having the amino acid sequence of SEQ ID NOs: 17 and 16, wherein the proteins are joined through disulfide bonds between the 6$^{th}$ and 11$^{th}$ cysteine of SEQ ID NOs: 15 or 17; the 7$^{th}$ cysteine of SEQ ID NOs: 15 or 17 and the 7$^{th}$ cysteine of SEQ ID NO 16; and the 20$^{th}$ cysteine of SEQ ID NOs: 15 or 17 and the 19$^{th}$ cysteine of SEQ ID NO 16. Specifically, the proteins having the amino acid sequences of SEQ ID NOs: 15 and 16 or biologically active material having the amino acid sequences of SEQ ID NOs: 17 and 16 represent insulin (SEQ ID NO 15 (Insulin A chain derivative) and 16 (Insulin B chain) / SEQ ID NO 17 (Insulin A chain) and 16 (Insulin B chain)).

SEQ ID NO: 15 GIVEQCCTSICSLEQLENYCN
SEQ ID NO: 16: FVNQHLCGSHLVEALYLVCGERGFFYTPKT
SEQ ID NO: 17: GIVEQCCTSICSLYQLENYCN

According to one embodiment of the present invention, cysteine may be substituted or inserted into the polypeptide to adjust the site of binding with the biotin moiety.

In a non-limiting example, any at least one of the amino acids of a polypeptide selected from the group consisting of the amino acid sequences represented by SEQ ID NOs: 1 through 7 may be substituted or inserted with a cysteine

amino acid. Here, the biotin moiety bonds to the -SH group of the cysteine amino acid.

Further, any at least one of the amino acids of a polypeptide selected from the group consisting of the above amino acid sequences may be substituted or inserted with a lysine amino acid. Here, the biotin moiety bonds to the $-NH_2$ group of the lysine amino acid.

Further, the polypeptide into which the cysteine amino acid is inserted may be a polypeptide having any one of the amino acid sequences of SEQ ID NOs: 8 through 14 below. Specifically, the biologically active materials of SEQ ID NOs: 8 through 14 below represent the biologically active materials of SEQ ID NOs: 1 through 7, wherein a cysteine amino acid has been substituted or inserted (for example, in the biologically active material of SEQ ID NO 8, at least any one of the amino acids of the biologically active material of SEQ ID NO 1 has been substituted with cysteine)

SEQ ID NO: 8: H(Aib)QGTFTSDYSKYLDEQAAKEFVQWLMNTC
SEQ ID NO: 9: HAEGTFTSDVSSYLEGQAAKEFIAWLVKGRC
SEQ ID NO: 10: HADGSFSDEMNTILDNLAARDFINWLIQTKITDC
SEQ ID NO: 11:
YAEGTFISDYSIAMDKIHQQDFVNWLLAQKGKKNDWKHNITQC
SEQ ID NO: 12: HGEGTFTSDLSKQMEEEAVRLFIEWLKNGGPSSGAPPPSC
SEQ ID NO: 13: SVSEIQLMHNLGKHLNSMERVEWLRKKLQDVHNFC
SEQ ID NO: 14: HSQGTFTSDYSKYLDSRRAQDFVQWLMNTC

According to one embodiment of the present invention, a portion of the polypeptide may be substituted to adjust the site of binding with the biotin moiety.

Further, according to one embodiment of the present invention, the amino acid lysine may be substituted or inserted into the polypeptide to adjust the site of binding with the biotin moiety.

In a non-limiting example, any at least one of the amino acids of the amino acid sequence represented by SEQ ID NO 5 may be substituted or inserted with the amino acid lysine.

In another non-limiting example, any at least one of the amino acids of the amino acid sequence represented by SEQ ID NO 5 may be substituted with 2-aminoisobutyric acid (Aib), with the insertion of a lysine amino acid. Here, the biotin moiety bonds to the $-NH_2$ group of the lysine amino acid. Further, the polypeptide wherein a portion has been substituted, or wherein a lysine amino acid has been substituted or inserted may be a polypeptide having the amino acid sequence of any one of SEQ ID NOs: 18 through 21 below. Specifically, the biologically active materials of SEQ ID NOs: 18 through 21 below represent exendin-4 derivatives, wherein a portion of the amino acids of the biologically active material of SEQ ID NO 5 has been substituted or inserted.

SEQ ID NO: 18: HGEGTFTSDLSKQMEEEAVRLFIEWLKNGGPSSGAPPPSK
SEQ ID NO: 19:
HGEGTFTSDLSKQMEEEAVRLFIEWLKNGGPSSGAPPPSKKK
SEQ ID NO: 20:
H(Aib)EGTFTSDLSKQMEEEAVRLFIEWLKNGGPSSGAPPPSK
SEQ ID NO: 21:
H(Aib)EGTFTSDLSKQMEEEAVRLFIEWLKNGGPSSGAPPPSKKK

According to one embodiment of the present invention, the biologically active material may be a polypeptide having the amino acid sequence of SEQ ID NO 22 below, or a derivative thereof. The biologically active material of SEQ ID NO 22 below represents amylin.

SEQ ID NO: 22: KCNTATCATQRLANFLVHSSNNFGAILSSTNVGSNTY

According to one embodiment of the present invention, a portion of the amino acid sequence represented by SEQ ID NO 22 may be substituted or inserted to adjust the site of binding with the biotin moiety.

In a non-limiting example, any at least one of the amino acid having the amino acid sequence represented by SEQ ID NO 22 may be substituted with the amino acid proline, aspartic acid, or arginine. In another non-limiting example, any at least one of the amino acid having the amino acid sequence represented by SEQ ID NO 22 may be substituted with the amino acid lysine.

Further, the polypeptide wherein a portion of the amino acids represented by SEQ ID NO 22 have been substituted or inserted may be a polypeptide having the amino acid sequence of any one of SEQ ID NOs: 23 through 31 below. Specifically, the biologically active materials of SEQ ID NOs: 23 through 31 below represent amylin derivatives wherein a portion of the amino acids of the biologically active material of SEQ ID NO 22 has been substituted or inserted.

SEQ ID NO: 23: KCNTATCATQRLANFLVHSSNNFGAILSSTNVGSNTYK
SEQ ID NO: 24: KCNTATCATQRLANFLVHSSNNFGPILPPTNVGSNTY

SEQ ID NO: 25: KCNTATCATQRLANFLVHSSNNFGPILPPTNVGSNTYK
SEQ ID NO: 26: KCNTATCATQRLLADFLRHSSPNFGAIPSSTNVGSRTY
SEQ ID NO: 27: KCNTATCATQRLADFLLRHSSPNFGAIPSSTNVGSRTYK
SEQ ID NO: 28: KCNTATCATQRLADFLLRHSSNNFGAIPSSTNVGSRTY
SEQ ID NO: 29: KCNTATCATQRLADFLLRHSSNNFGAIPSSTNVGSRTYK
SEQ ID NO: 30: RCNTATCATQRLADFLLRHSSNNFGAIPSSTNVGSKTY
SEQ ID NO: 31: RCNTATCATQRLADFLLRHSSNNFGAIPSSTNVGSKTYK

According to one embodiment of the present invention, the biologically active material may be a polypeptide having the amino acid sequence of SEQ ID NO 32 below, or a derivative thereof. The biologically active material of SEQ ID NO 32 below represents exendin-4 derivatives.

SEQ ID NO: 32: H(Aib)QGTFTSDKSKYLDERAAQDFVQWLLDGGPSSGAPPPS

According to one embodiment of the present invention, a portion of the amino acid sequence of SEQ ID NO 32 may be deleted, substituted or inserted to adjust the site of binding with the biotin moiety. In a non-limiting example, any at least one of the amino acids of the amino acid sequence represented by SEQ ID NO 32 may be substituted with the amino acid methionine, lysine, isoleucine, tryptophan or glycine. In another non-limiting example, any at least one of the amino acids of the amino acid sequence represented by SEQ ID NO 32 may be deleted.

Further, the polypeptide in which a portion of the amino acids represented by SEQ ID NO 32 has been deleted, substituted or inserted may be a polypeptide having the amino acid sequence of any one of SEQ ID NOs: 33 through 37 below. Specifically, the biologically active materials of SEQ ID NOs: 33 through 37 below wherein a portion of amino acids of the biologically active material of SEQ ID NO 32 has been deleted, substituted or inserted represent exendin-4 derivatives.

SEQ ID NO: 33: H(Aib)QGTFTSDKSKYLDERAAQDFVQWLMDGGPSSGAPPPS
SEQ ID NO: 34: H(Aib)QGTFTSDKSKYLDKIAAQDFVQWLIDGGPSSGAPPPS
SEQ ID NO: 35: H(Aib)QGTFTSDKSWYLDKIAAQDFVQWLLGGGPSSGAPPPS
SEQ ID NO: 36: H(Aib)QGTFTSDKSWYLDERAAQDFVQWLMGGGPSSGAPPPS
SEQ ID NO: 37: H(Aib)QGTFTSDKSKWLDKIAAQDFVQWLIGGGPSSGAPPPS

According to one embodiment of the present invention, any at least one of the amino acids of the amino acid sequence represented by SEQ ID NO 12 may be substituted with 2-aminoisobutyric acid (Aib).

According to one embodiment of the present invention, a polypeptide wherein any at least one of the amino acids of the amino acid sequence represented by SEQ ID NO 12 has been substituted with 2-aminoisobutyric acid (Aib) and any at least one has been substituted with Des-amino-His(h) may be the polypeptide having the amino acid sequence of SEQ ID NOs: 38 through 39 below. Specifically, the biologically active materials of SEQ ID NOs: 38 or 39 below, wherein amino acids of the biologically active material of SEQ ID NO 12 have been substituted, represent exendin-4 derivatives. More specifically, the biologically active material having the amino acid sequence of SEQ ID NO 39 is a biologically active material wherein at least one of the amino acids has been substituted with Desamino-His(h).

SEQ ID NO: 38: H(Aib)EGTFTSDLSKQMEEEAVRLFIEWLKNGGPSSGAPPPSC
SEQ ID NO: 39: h(Aib)EGTFTSDLSKQMEEEAVRLFIEWLKNGGPSSGAPPPSC

According to one embodiment of the present invention, any at least one of the amino acids of the amino acid sequence represented by SEQ ID NO 8 may be substituted with lysine (Lys) or arginine (Arg).

A polypeptide wherein any at least one of the amino acids of the amino acid sequence represented by SEQ ID NO 8 has been substituted with lysine or arginine may be the polypeptide having the amino acid sequence of SEQ ID NOs: 40 through 41 below. Specifically, the biologically active materials of SEQ ID NOs: 40 or 41 below, wherein amino acids of the biologically active material of SEQ ID NO 8 have been substituted, represent glucagon derivatives.

SEQ ID NO: 40: H(Aib)QGTFTSDYSKYLDEQAAKEFVQWLMNTK
SEQ ID NO: 41: H(Aib)QGTFTSDYSKYLDEKRAKEFVQWLMNTC

According to one embodiment of the present invention, the biologically active material may be the polypeptide having the amino acid sequence of SEQ ID NO 42 or a derivative thereof. Specifically, the biologically active material of SEQ ID NO 42 represents a human growth hormone derivative.

SEQ ID NO: 42:

MFPTIPLSRLFDNAMLRAHRLHQLAFDTYQEFEEAYIPKEQKISFLQNPQTSLCFSESIPTPSN
REETQQKSNLELLRISLLLIQSWLEPVQFLRSVFANSLVYGASDSNVYDLLKDLEEGIQTLM
GRLEDGSPRTGQIFKQTYSKFDTNSHNDDALLKNYGLLYCFRKDMDKVETFLRIVQCRSVE
GSCGF

According to one embodiment of the present invention, the physiologically active material to which the biotin moiety, fatty acid moiety or a combination thereof is bonded may be covalently bonded with, or form an inclusion body (microsphere) with, any at least one selected from the group comprising peptide and non-peptidic polymer, fatty acid, cholesterol, antibody, antibody fragment, albumin and fragments thereof, nucleotide, fibronectin, transferrin, FcRn binding material, saccharide, elastin, heparin, and derivatives thereof.

The non-peptidic polymer may be selected from the group composed of polyethylene glycol (PEG), polypropylene glycol, copolymers of ethylene glycol and propylene glycol, polyoxyethylated polyols, polyvinyl alcohol (PVA), polysaccharides, dextran, polyvinylethyl ether, PLA (polylactic acid, polylactic acid), PLGA (polylactic-glycolic acid), lipid polymer, chitin, hyaluronic acid, and combinations thereof.

Another aspect of the present invention provides a method for preparing a biologically active material conjugated with a biotin moiety, the method comprising: a step of obtaining a biotin moiety; a step of reacting the biotin moiety with a biologically active material; and, a step of isolating the biologically active material conjugated with a biotin moiety after completion of the reaction.

Yet another aspect of the present invention provides a method for preparing a biologically active material conjugated with a biotin moiety and a fatty acid moiety, the method comprising: a step of obtaining a biotin moiety; a step of reacting the biotin moiety with a biologically active material; a step of, after completion of the reaction between the biotin moiety and the biologically active material, reacting [the product] with a fatty acid moiety; and, a step of isolating the biologically active material conjugated with a biotin moiety and a fatty acid moiety after completion of the reaction.

According to one embodiment of the present invention, in the step of obtaining the biotin moiety, the biotin moiety may be represented by General Formula A above.

According to one embodiment of the present invention, the fatty acid moiety may be represented by General Formula B above.

According to one embodiment of the present invention, in the step of obtaining the mixture, the reaction mole ratio of biotin moiety to the biologically active material may be 0.5 or greater. Specifically, the reaction mole ratio of biotin moiety to the biologically active material may be 0.5 to 30. The above reaction mole ratio may be appropriately selected giving consideration to the molecular structure, molecular weight or solubility of the biotin moiety, pH of the reaction mixture, reaction temperature, reaction time, and the like.

According to one embodiment of the present invention, in the step of obtaining the mixture, the reaction mole ratio of fatty acid moiety to the biologically active material may be 0.5 or greater. Specifically, the reaction mole ratio of fatty acid moiety to the biologically active material may be 0.5 to 20. The above reaction mole ratio may be appropriately selected giving consideration to the molecular structure, molecular weight or solubility of the fatty acid moiety, pH of the reaction mixture, reaction temperature, reaction time, and the like.

Depending on the form of the biologically active material, the reaction mole ratio of the biotin moiety to the biologically active material in the step of obtaining the mixture may be 20 or greater. Specifically, the reaction mole ratio of biotin moiety to the biologically active material may be 20 to 25.

Further, depending on the form of the biologically active material, the reaction mole ratio of the fatty acid moiety to the biologically active material in the step of obtaining the mixture may be 6 or greater. Specifically, the reaction mole ratio of fatty acid moiety to the biologically active material may be 6 to 12.

According to one embodiment of the present invention, the reaction may be carried out using a buffer solution or an organic solvent. There is no particular limitation on the buffer solution or organic solvent, and a buffer solution typically used in the art may be appropriately selected depending on the structure of the biotin moiety and the fatty acid moiety.

In one embodiment of the present invention, the temperature and duration of the reacting step may be appropriately adjusted depending on the characteristics of the biotin moiety, fatty acid moiety and biologically active material. Whereas the present invention is not limited hereto, the reaction may be carried out, for example, for 3 hours or longer at 4°C, or may be carried out for a shorter time at room temperature. This may be related to the degree of reactivity of the biotin moiety or fatty acid moiety being used, or the combination thereof. Once an appropriate reaction time has passed, the reaction may be stopped by reducing the pH of the reaction mixture.

According to one embodiment of the present invention, a step of removing unreacted material may carried out

after the reacting step. The step of removing unreacted material may be carried out using methods ordinarily used in the art. For example, whereas the present invention is not limited to the following, the removal may be carried out using dialysis, etc., with an appropriate buffer solution, for example, a solution such as PBS (phosphate buffered saline).

According to one embodiment of the present invention, a purifying step may be comprised after the isolating step. The isolating and purifying step may be carried out using size exclusion chromatography, reverse-phase high performance liquid chromatography, or ion exchange chromatography, but is not limited hereto.

Yet another aspect of the present invention provides a pharmaceutical formulation comprising a biologically active material conjugate bonded to the biotin moiety or fatty acid moiety described in the above, or to a combination thereof.

Another specific aspect of the present invention provides a pharmaceutical formulation comprising a biologically active material conjugate bonded to the biotin moiety and fatty acid moiety described in the above.

Here, the use of the pharmaceutical formulation may be determined depending on the type of the biologically active material. Further, the pharmaceutical formulation may be a formulation for oral administration.

According to one embodiment of the present invention, a pharmaceutical formulation used for the prevention or treatment of diabetes, obesity, fatty liver disease, irritable bowel syndrome, neurodegenerative disease, bone disease, osteoporosis, human growth hormone deficiency, cancer or non-alcoholic fatty liver disease may be provided.

According to one embodiment of the present invention, when the biologically active material is GLP-1, GLP-2, GIP, insulin, amylin or a derivative thereof, the conjugate can be used for the prevention or treatment of diabetes. Specifically, the conjugate comprising the biologically active material of SEQ ID NO 12 can be used for preventing or treating diabetes. However, this example is illustrative and the present invention is not limited hereto.

Further, according to one embodiment of the present invention, when the biologically active material is parathyroid hormone or a derivative thereof, the conjugate can be used for the prevention or treatment of bone diseases. Specifically, the conjugate comprising the biologically active material of SEQ ID NO 6 can be used for the prevention or treatment of bone diseases. However, this example is illustrative and the present invention is not limited hereto.

According to one embodiment of the present invention, when the biologically active material is hGH or a derivative thereof, the conjugate can be used for preventing or treating human growth hormone deficiency. Specifically, the conjugate comprising the biologically active material of SEQ ID NO 42 can be used for preventing or treating human growth hormone deficiency. However, this example is illustrative and the present invention is not limited hereto.

Another aspect of the present invention provides a formulation for oral administration, the formulation comprising a biologically active material conjugate bonded to the biotin moiety or fatty acid moiety described in the above, or to a combination thereof.

Another specific aspect of the present invention provides a formulation for oral administration, the formulation comprising a biologically active material conjugate bonded to the biotin moiety and fatty acid moiety described in the above.

The biologically active material conjugate bonded to a biotin moiety, fatty acid moiety or combination thereof according to one embodiment of the present invention, by being bonded to biotin, which is a type of water soluble vitamin, can be absorbed by active transport through a sodium-dependent multivitamin transporter, improving absorption in the intestine through the intestinal membrane. More specifically, an excellent effect is exhibited through the bonding with both a biotin moiety and a fatty acid moiety.

According to one embodiment of the present invention, the pharmaceutical formulation comprising the biologically active material bonded to a biotin moiety may be administered by formulating in various forms for oral or non-oral administration, but the present invention is not limited hereto. Further, according to one embodiment of the present invention, the pharmaceutical formulation comprising the biologically active material bonded to a biotin moiety, a fatty acid moiety or a combination thereof may be administered by formulating in various forms for oral or non-oral administration, but the present invention is not limited hereto.

When formulating, the formulation may be prepared by using commonly used diluents or excipients such as fillers, dissolution aids, extenders, binders, wetting agents, disintegrants, surfactants, and absorption enhancers.

Solid formulations for oral administration include tablets, pills, powders, granules, capsules, and the like, and such solid formulations may be prepared by mixing at least one excipient with the compound, for example, starch, calcium carbonate, sucrose, lactose, gelatin or the like.

Further, in addition to simple excipients, lubricants such as magnesium stearate or talc may be used. Liquid formulations for oral administration include suspensions, internal solutions, emulsions, syrups and the like, and may comprise, in addition to commonly used simple diluents such as water and liquid paraffin, various excipients, for example wetting agents, sweeteners, flavoring agents, fragrances, preservatives, and the like. Formulations for non-oral administration comprise sterile aqueous solutions, non-aqueous solvents, suspensions, emulsions, freeze-dried preparations, and suppositories. Non-aqueous solvents and suspensions may include propylene glycol (Propylene glycol), polyethylene glycol (PEG), vegetable oils such as olive oil, and injectable esters such as ethyl oleate. In addition, calcium or vitamin D3 may be added to enhance efficacy as a therapeutic agent for proliferative diseases or autoimmune diseases.

The dosage of the pharmaceutical formulation according to an embodiment of the present invention may vary

depending on the patient's weight, age, sex, health status, diet, administration time, administration method, excretion rate and severity of disease. However, in general, it may be administered once a day or divided into several doses within the effective daily dose range. In addition, it may be possible to administer an effective dose even by administration several times in 1 to 2 weeks. In the following, the present invention is described in detail by means of embodiments and experimental examples. Provided, that the following embodiments and experimental examples are intended to exemplify the present invention, and the present invention is not limited thereto.

**[Working Examples]**

**<Preparation of biotin moiety>**

List of abbreviations

HBTU: 3-[Bis(dimethylamino)methyliumyl]-3H-benzotriazol-1-oxide hexafluorophosphate) DIEA: Ethyldiisopropylamine
HATU: 1-[bis(dimethylamino)methylene]-1H-1,2,3-triacolo[4,5-b]pyridinium-3oxide hexafluorophosphate
DIC: Diisopropylcarbodiimide
HOBt:: 1-Hydroxybenzotriazole
MBHA: 4-Methylbenzhydrylamine hydrochloride
Fmoc: 9-Fluorenylmethyloxycarbonyl
DMF: dimethylformamide
SPPS: Solid Phase Peptide Synthesis
HPLC: High Performance Liquid Chromatography
LCMS: Liquid Chromatography Mass Spectrometry

**Typical SPPS method**

In some cases, solid phase synthesis of a peptide can be improved through use a di-peptide protected from di-peptide amide bonds having groups that can be cleaved under acidic conditions, for example, 2-Fmoc-oxy-4-methoxybenzyl, or 2,4,6-trimethoxybenzyl. The Fmoc-protected amino acid derivative used was the recommended standard, for example: Fmoc-Ala-OH, Fmoc-Arg(Pbf)-OH, Fmoc-Asn(Trt)-OH, Fmoc-Asp(OtBu)-OH, Fmoc-Cys(Trt)-OH, Fmoc-Gln(Trt)-OH, Fmoc-Glu(OtBu)-OH, Fmoc-Gly-OH, Fmoc-His(Trt )-OH, Fmoc-Ile-OH, Fmoc-Leu-OH, Fmoc-Lys(Boc)-OH, Fmoc-Met-OH, Fmoc-Phe-OH, Fmoc-Pro-OH, Fmoc-Ser(tBu)- OH, Fmoc-Thr(tBu)-OH, Fmoc-Trp(Boc)-OH, Fmoc-Tyr(tBu)-OH, or Fmoc-Val-OH and the like supplied by Anaspec, Bachem, Iris Biotech, or Novabiochem. The N-terminal amino acid was Boc protected at the alpha amino group. For example: Fmoc-8-amino-3,6-dioxaoctanoic acid, Fmoc-tranexamic acid, Fmoc-isonipecotic acid, Fmoc-Glu-OtBu, Fmoc-Lys(Fmoc)-OH supplied by Anaspec, Bachem, Iris Biotech, or Novabiochem was used.

**Peptide synthesis using SPPS**

Peptides can be synthesized using general Fmoc chemistry in link amide MBHA resins using HBTU/DIEA, HATU/DIEA, or DIC/HOBt as the coupling reagents. The combinations of reactants and coupling reagents used in synthesis comprise the following.

**[Table 1]**

| # | Reactant | Coupling Reagent |
|---|---|---|
| 1 | Fmoc-Lys (Biotin)-OH (1.5 *eq)* | HBTU (1.42 eq) and DIEA (3.0 eq) |
| 2 | Fmoc-Lys (Biotin)-OH (2.0 *eq)* | HBTU (1.9 eq) and DIEA (4.0 *eq)* |
| 3 | 3-(2,5-dioxo-2,5-dihydro-1H-pyrrol-1-yl) propanoic acid (3.0 *eq)* | DIC (3.0 eq) and HOBt (6.0 eq) |

(continued)

| # | Reactant | Coupling Reagent |
|---|---|---|
| 4 | 2,2-dimethyl-4-oxo-3,7,10,13,16,19,22-heptaoxapentacosan-25-oic acid (2.0 *eq)* | HATU (1.9 eq) and DIEA (4.0 eq) |
| 5 | Fmoc-21-amino-4,7,10,13,16,19-hexaoxaheneicosanoic acid (2.0 *eq)* | HATU (1.9 eq) and DIEA (4.0 eq) |

An exemplary protocol for the peptide synthesis process using SPPS comprises the following. 1) Add DMF to a vessel containing link amide MBHA resin and expand for 2 hours (sub: 0.68 mmol/g, 1.0 mmol, 1.47 g or 5 mmol, 7.35 g, sub: 0.68 mmol/g). 2) After adding 20% piperidine/DMF, mix for 30 minutes. 3) After removing the solvent of 1)-2), wash using DMF (30 seconds x 5 times). 4) Add the reactant (one of the reactants #1 to #5) and mix for 30 seconds, then add the coupling reagent (one of the coupling reagents #1 to #5) corresponding to the reactant, and carry out nitrogen bubbling for 1 hour. 5) After adding 20% piperidine/DMF, mix for 30 minutes. In the exemplary protocol of 1) to 5) above, iterative synthesis can be performed using combinations of the reactants and coupling reactants #1 to #5 more than once. To remove Fmoc, treatment with 20% piperidine/DMF solution for 30 minutes was used.

**Typical procedure for peptide purification and analysis**

Unpurified peptide was dissolved in an appropriate mixture of water, TFA and ACN, purified using preparative HPLC, dried and quantified. The conditions for purification using preparative HPLC include those shown in Table 2 below.

**[Table 2]**

| Purification Conditions | |
|---|---|
| Solvent | ACN/$H_2O$ |
| Equipment | SHIMADZU LC-8A, or Gilson GX-281 |
| Mobile Phase | A: $H_2O$ (0.075% TFA in $H_2O$) |
| | B: $CH_3CN$ |
| Gradient | 15-35%-60min. Retention time: 42 min, or<br>20-50%-60min. Retention time: 45 min, or<br>5-35%-60min. Retention time: 50 min |
| Column | Luna25*200mm, C18, 10um, 110A+Gemin150*30mm, C18, 5um, 110A, or Luna50*25mm, C18, 10um, 100A+Gemini(R)50*50mm, C8, 5um, 110A |
| Flow Rate | 80mL/Min or 20mL/Min |
| Wavelength | 220/254 nm |
| Oven Temp. | Room temperature |

After purification using preparative HPLC, the final product was characterized using analytical HPLC or LCMS. As a result of the analysis, the biotin moieties of Table 3 below were obtained.

[Table 3]

| Biotin Moiety | Designation |
|---|---|
| B1 | N-Biotinoyl-N'-(6-maleiidohexanoyl)hydrazide |
| B2 | 3-Maleimidopropionate-Lys(Biotin)-Lys(Biotin)-$CONH_2$ |
| B3 | 3-Maleimidopropionate-Lys(Biotin)-Lys(Biotin)-Lys (Biotin)-$CONH_2$ |
| B4 | propionate-N-hydroxysuccinimide ester-PEG-Lys(Biotin)-Lys (Biotin)-Lys(Biotin)-$CONH_2$ |

(continued)

| Biotin Moiety | Designation |
|---|---|

(continued)

| Biotin Moiety | Designation |
|---|---|
| B5 | 3-Maleimidopropionate-PEG-Lys(Biotin)-Lys(Biotin)-Lys (Biotin)-$CONH_2$ |

B1:

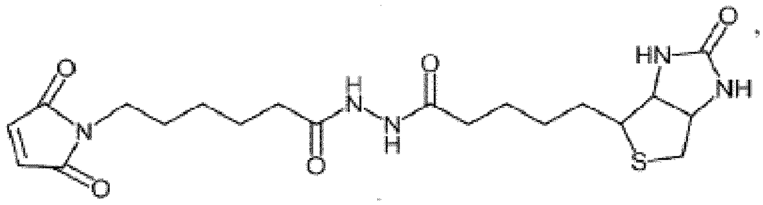

,

B2:

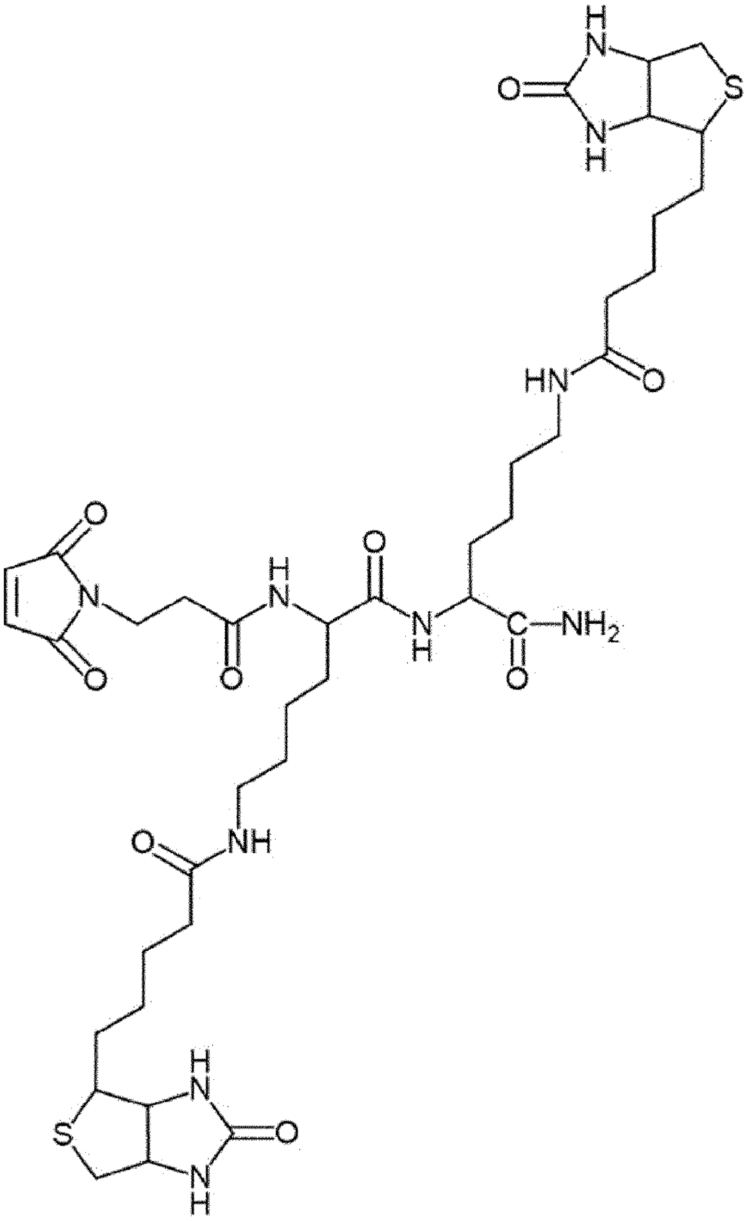

,

B3:

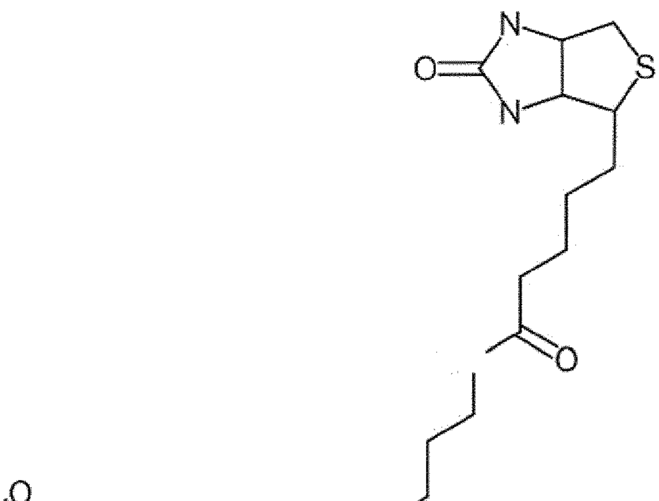

,

Further, through the protocol 1)~5) for peptide synthesis using SPPS, purification and analysis, the following biotin moieties B6 to B7 were obtained. In Table 4 below, X, Y, Z and B are included in the definition of General Formula A of the present specification.

[Table 4]

| Biotin Moiety | X | Y | Z | Number of B (Biotin) |
|---|---|---|---|---|
| B6 | Aldehyde | propane | Lysine | 2 |
| B7 | Maleimide | butyrate | Glycerol and PEG | 2 |
| B8 | Maleimide | butyrate | Glycerol and PEG | 2 |
| B9 | N-hydroxysuccinimide | butyrate | Lysine | 2 |
| B10 | N-hydroxysuccinimide | glutarate | Glycerol and PEG | 2 |
| B11 | Maleimide | PEG12 | Lysine | 3 |
| B12 | N-hydroxysuccinimide | PEG12 | Lysine | 3 |
| B13 | amine | - | Lysine | 3 |
| B14 | Aldehyde | pentane | Lysine | 2 |
| B15 | Maleimide | adipate | Glycerol and PEG | 2 |
| B16 | Maleimide | suberate | Glycerol and PEG | 2 |
| B17 | Maleimide | sebacate | Glycerol and PEG | 2 |
| B18 | N-hydroxysuccinimide | adipate | Glycerol and PEG | 2 |
| B19 | N-hydroxysuccinimide | suberate | Lysine | 4 |
| B20 | N-hydroxysuccinimide | sebacate | Lysine | 4 |
| B21 | N-hydroxysuccinimide | PEG6 | Glycerol and PEG | 2 |
| B22 | Succinimidyl carbonate | PEG6 | Lysine | 2 |
| B23 | Succinimidyl carbonate | PEG12 | Lysine | 3 |
| B24 | Succinimidyl carbonate | pentane | Lysine | 3 |
| B25 | Succinimidyl carbonate | hexane | Lysine | 3 |
| B26 | p-nitrophenyl carbonate | PEG6 | Lysine | 3 |
| B27 | p-nitrophenyl carbonate | PEG12 | Lysine | 4 |
| B28 | p-nitrophenyl carbonate | propane | Glycerol and PEG | 2 |
| B29 | p-nitrophenyl carbonate | pentane | Glycerol and PEG | 2 |
| B30 | amine | - | Glycerol and PEG | 2 |
| B31 | thiol | butyrate | Lysine | 2 |
| B32 | thiol | glutarate | Lysine | 3 |
| B33 | aminoxy | PEG6 | Lysine | 3 |
| B34 | iodoacetamide | PEG6 | Lysine | 3 |
| B35 | Maleimide | EG2-EG2-Glu-C18 | Lysine | 3 |
| B36 | Maleimide | EG2-EG2-Glu-C18 | Lysine | 3 |
| B37 | Amine | Lys-EG2 | Lysine | 3 |

(continued)

| Biotin Moiety | X | Y | Z | Number of B (Biotin) |
|---|---|---|---|---|

(continued)

| Biotin Moiety | X | Y | Z | Number of B (Biotin) |
|---|---|---|---|---|
| B38 | N-hydroxysuccinimide | - | - | 1 |

B35:

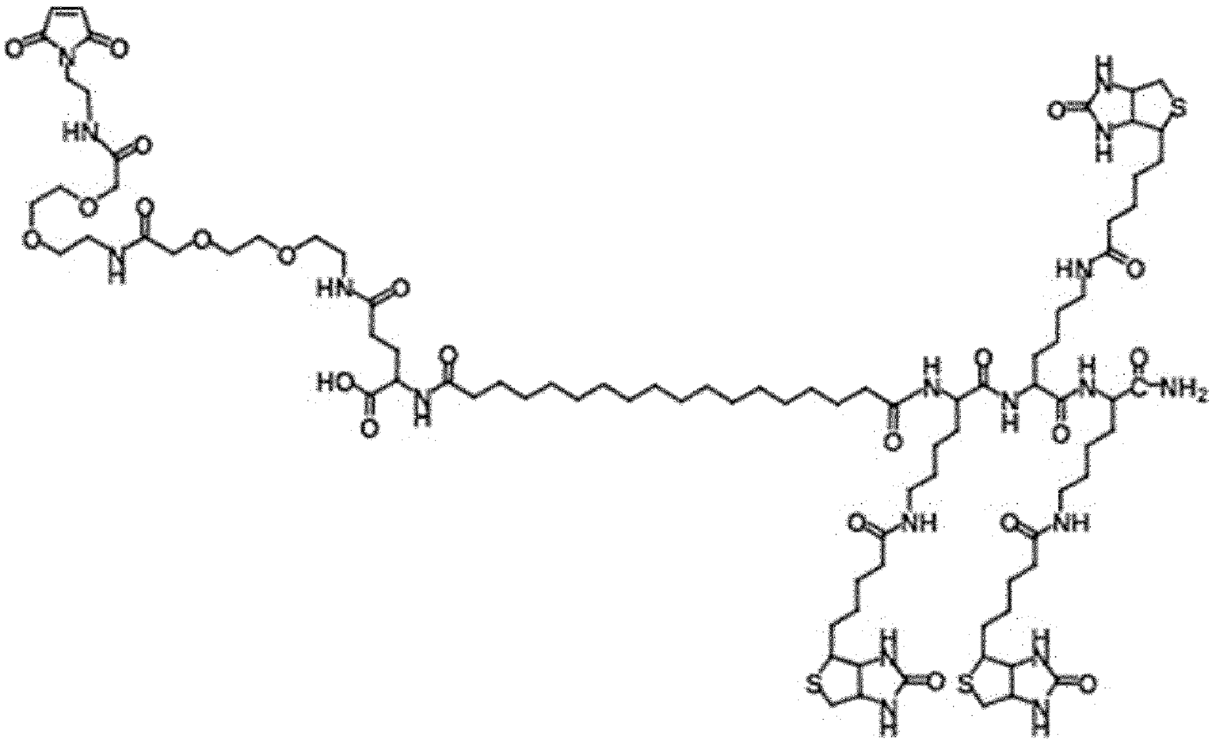

B36:

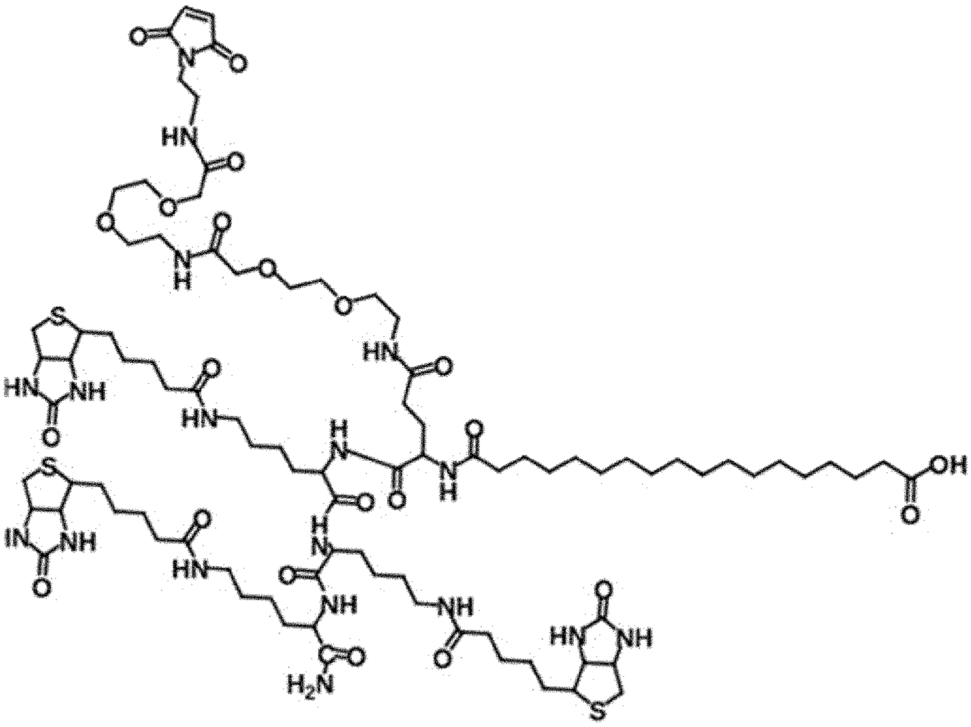

B37:

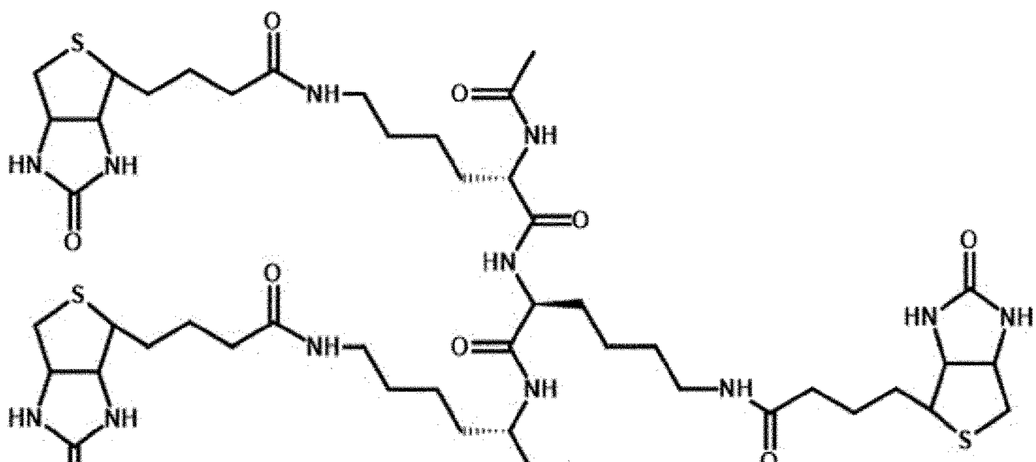

<Fatty Acid Moiety>

The fatty acid moiety may be prepared using methods known to the art, or a commercially obtained material may be used.

As the fatty acid moiety, the fatty acid moieties of Table 5 below were used.

[Table 5]

| Fatty Acid Moiety | Designation |
|---|---|
| F1 | C16-NHS |
| F2 | C16-MAL |
| F3 | C18-NHS |
| F4 | C18-MAL |
| F5 | C16-Glu-NHS |
| F6 | C16-Glu-MAL |
| F7 | C18-Glu-NHS |
| F8 | C18-Glu-MAL |
| F9 | C18-Glu-EG2-NHS |
| F10 | C18-Glu-EG2-MAL |
| F11 | C18-Glu-EG2-EG2-NHS |
| F12 | C18-Glu-EG2-EG2-MAL |
| F13 | C20-Glu-EG2-EG2-NHS |
| F14 | C20-Glu-EG2-EG2-MAL |
| F15 | C18-Glu-EG2-EG2-TFP |

(continued)

| Fatty Acid Moiety | Designation |
|---|---|
| F16 | C18-Glu-EG2-EG2-NPC |

F1:

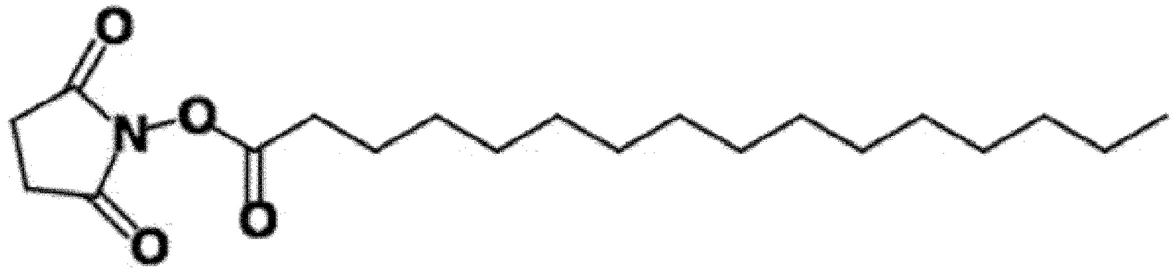

F2:

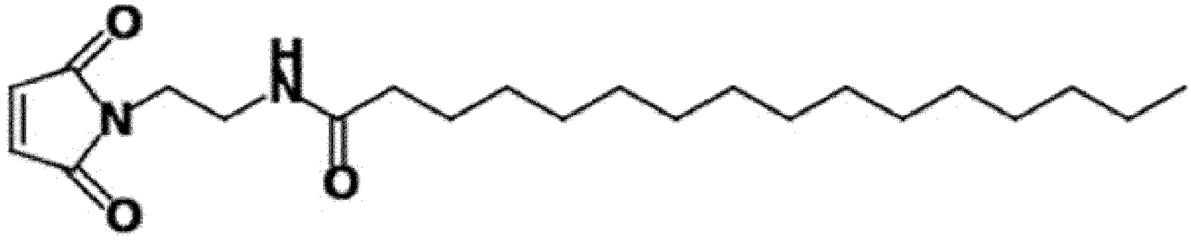

F3:

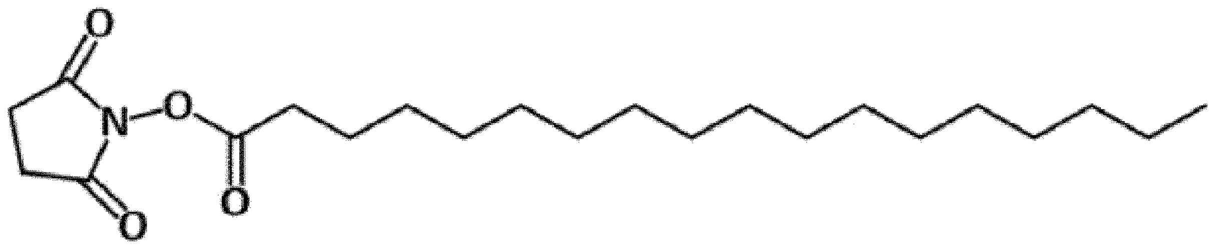

F4:

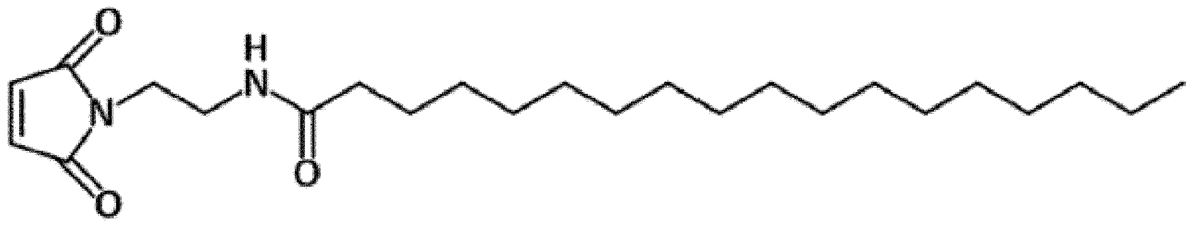

F5:

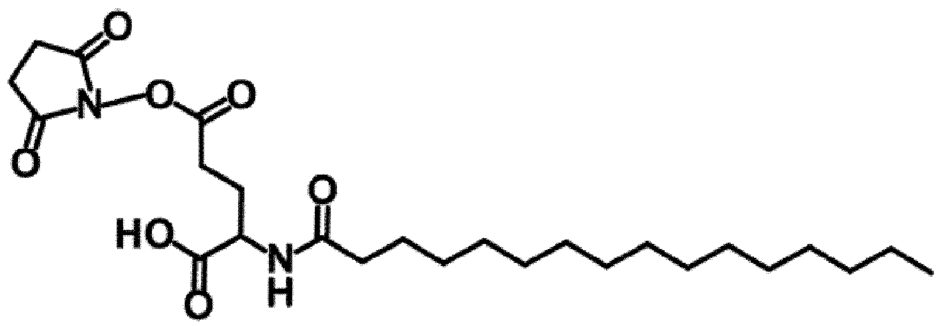

F6:

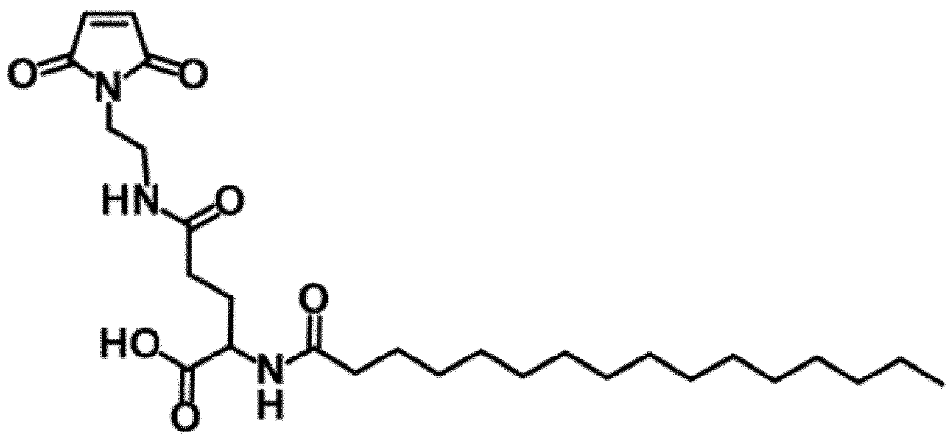

F7:

< Polypeptide >

The polypeptide may be prepared using methods known to the art, or commercially obtained materials may be used. In the present invention, the sequences of the biologically active materials bound to a biotin moiety, a fatty acid moiety, or a combination thereof are shown in Table 6 below.

**[Table 6]**

| Polype ptide | SEQ ID NO | Amino Acid Sequence |
|---|---|---|
| P1 | 1 | H(Aib)QGTFTSDYSKYLDEQAAKEFVQWLMNT |
| P2 | 2 | HAEGTFTSDVSSYLEGQAAKEFIAWLVKGR |
| P3 | 3 | HADGSFSDEMNTILDNLAARDFINWLIQTKITD |
| P4 | 4 | YAEGTFISDYSIAMDKIHQQDFVNWLLAQKGKKNDWKHNITQ |
| P5 | 5 | HGEGTFTSDLSKQMEEEAVRLFIEWLKNGGPSSGAPPPS |
| P6 | 6 | SVSEIQLMHNLGKHLNSMERVEWLRKKLQDVHNF |
| P7 | 7 | HSQGTFTSDYSKYLDSRRAQDFVQWLMNT |
| P8 | 8 | H(Aib)QGTFTSDYSKYLDEQAAKEFVQWLMNTC |
| P9 | 9 | HAEGTFTSDVSSYLEGQAAKEFIAWLVKGRC |
| P10 | 10 | HADGSFSDEMNTILDNLAARDFINWLIQTKITDC |
| P11 | 11 | YAEGTFISDYSIAMDKIHQQDFVNWLLAQKGKKNDWKHNITQC |
| P12 | 12 | HGEGTFTSDLSKQMEEEAVRLFIEWLKNGGPSSGAPPPSC |
| P13 | 13 | SVSEIQLMHNLGKHLNSMERVEWLRKKLQDVHNFC |
| P14 | 14 | HSQGTFTSDYSKYLDSRRAQDFVQWLMNTC |
| P15 | 15 | GIVEQCCTSICSLEQLENYCN |
| P16 | 16 | FVNQHLCGSHLVEALYLVCGERGFFYTPKT |
| P17 | 17 | GIVEQCCTSICSLYQLENYCN |
| P18 | 18 | HGEGTFTSDLSKQMEEEAVRLFIEWLKNGGPSSGAPPPSK |
| P19 | 19 | HGEGTFTSDLSKQMEEEAVRLFIEWLKNGGPSSGAPPPSKKK |
| P20 | 20 | H(Aib)EGTFTSDLSKQMEEEAVRLFIEWLKNGGPSSGAPPPSK |
| P21 | 21 | H(Aib)EGTFTSDLSKQMEEEAVRLFIEWLKNGGPSSGAPPPSKKK |
| P22 | 22 | KCNTATCATQRLANFLVHSSNNFGAILSSTNVGSNTY |
| P23 | 23 | KCNTATCATQRLANFLVHSSNNFGAILSSTNVGSNTYK |
| P24 | 24 | KCNTATCATQRLANFLVHSSNNFGPILPPTNVGSNTY |
| P25 | 25 | KCNTATCATQRLANFLVHSSNNFGPILPPTNVGSNTYK |

(continued)

| Polype ptide | SEQ ID NO | Amino Acid Sequence |
|---|---|---|
| P26 | 26 | KCNTATCATQRLADFLLRHSSPNFGAIPSSTNVGSRTY |
| P27 | 27 | KCNTATCATQRLADFLLRHSSPNFGAIPSSTNVGSRTYK |
| P28 | 28 | KCNTATCATQRLADFLLRHSSNNFGAIPSSTNVGSRTY |
| P29 | 29 | KCNTATCATQRLADFLLRHSSNNFGAIPSSTNVGSRTYK |
| P30 | 30 | RCNTATCATQRLADFLLRHSSNNFGAIPSSTNVGSKTY |
| P31 | 31 | RCNTATCATQRLADFLLRHSSNNFGAIPSSTNVGSKTYK |
| P32 | 32 | H(Aib)QGTFTSDKSKYLDERAAQDFVQWLLDGGPSSGAPPPS |
| P33 | 33 | H(Aib)QGTFTSDKSKYLDERAAQDFVQWLMDGGPSSGAPPPS |
| P34 | 34 | H(Aib)QGTFTSDKSKYLDKIAAQDFVQWLIDGGPSSGAPPPS |
| P35 | 35 | H(Aib)QGTFTSDKSWYLDKIAAQDFVQWLLGGGPSSGAPPPS |
| P36 | 36 | H(Aib)QGTFTSDKSWYLDERAAQDFVQWLMGGGPSSGAPPPS |
| P37 | 37 | H(Aib)QGTFTSDKSKWLDKIAAQDFVQWLIGGGPSSGAPPPS |
| P38 | 38 | H(Aib)EGTFTSDLSKQMEEEAVRLFIEWLKNGGPSSGAPPPSC |
| P39 | 39 | h(Aib)EGTFTSDLSKQMEEEAVRLFIEWLKNGGPSSGAPPPSC |
| P40 | 40 | H(Aib)QGTFTSDYSKYLDEQAAKEFVQWLMNTK |
| P41 | 41 | H(Aib)QGTFTSDYSKYLDEKRAKEFVQWLMNTC |
| P42 | 42 | MFPTIPLSRLFDNAMLRAHRLHQLAFDTYQEFEEAYIPKEQKISFLONT<br>SLCFSESIPTPSYREETQQKSNLELLRISLLLIQSWLEPVQFLRSVFANSL<br>VYGASDSNVYDLLKDLEEGIQTLMGRLEDGSPRTGQIFKQTYSKFDT<br>NSHNDDALLKNYGLLYCFRKDMDKVETFLRIVQCRSVEGSCGF |

**<Embodiment: Preparation of a biologically active material combined with a biotin moiety, a fatty acid moiety, or a combination thereof>**

**[Preparation Example]**

Using DMSO solution with 0.3% trimethylamine (TEA, Sigma) added as the reaction solvent, molar ratio mixtures of 1:X between the polypeptides of Table 6 and the biotin moieties of Tables 3 through 4 were reacted for at least 30 minutes each at room temperature. Then, molar ratio mixtures of 1:Y (1: 0.5~20) between the polypeptide-biotin moiety mixtures and the fatty acid moieties of Table 5 were prepared and reacted for at least 90 minutes each at room temperature. The reactions were stopped by adding 1% Trifluoroacetic acid solution of the same volume as the volume of each mixture.

**[Isolation, Purification and Confirmation]**

The reaction products were isolated and purified using reverse phase high performance liquid chromatography. As the column, a SUPERSIL ODS- 1 column (10x250mm, 5um, LB Science, South Korea) was used.

The mobile phase condition was changed linearly while maintaining a flow rate of 4.7ml/min with 30-50% Solvent B (acetonitrile with 0.1% TFA added) and Solvent A (distilled water with 0.1% TFA added). Monitoring with a UV absorption spectrometer at 280nm, peaks detected between 10 minutes and 20 minutes were collected. The collected peaks were concentrated and purified using ultracentrifugal filters having an appropriate molecular weight cut-off, after volatilizing organic solvents and TFA under vacuum. The purity of the purified materials was confirmed using the HPLC analysis method. Analysis was carried out at a constant temperature near room temperature using a Gemini C18 column (4.6 x 250mm, 5um; Phenomenex, CA, USA). Analysis was carried out using the gradient elution method at a flow rate of 1mL/min using a mobile phase comprised of trifluoroacetic acid solution: acetonitrile mixture (at varying mix ratios). UV absorbance was observed at 280nm.

[Confirming molecular weight]

The molecular weight of the reaction product was measured.

The molecular weight was measured using the MALDI-TOF mass spectrometry method. As the matrix solution, a solution of 50% acetonitrile containing 0.1% TFA saturated with CHCA (a-Cyano-4-hydrocinnamic acid) was used. The mass spectrum was confirmed in linear and positive mode, and the molecular weight was confirmed by setting the concentration of the final material to 0.1 mg/mL. Through this, it was confirmed that the molecular weight of the separated materials was consistent with the theoretical molecular weight.

**<Embodiment: Polypeptide combined with a biotin moiety, a fatty acid moiety or a combination thereof>**

The materials stated above were used as the biotin moiety, fatty acid moiety and polypeptide. Methods known to the art or the method of the above embodiment was used for binding the polypeptide to the biotin moiety, fatty acid moiety or a combination thereof.

The polypeptides bound to a biotin moiety, fatty acid moiety or a combination thereof are as shown in Table 7 below. (Here, the molecular weights represent the measured molecular weights or the theoretical molecular weights).

**[Table 7]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 1 | 12 | HGEGTFTSDLSKQ MEEEA VRLFIEWL KNGGPSSGAPPSC | B1 | C40 | - | - | 4744.5 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 2 | 12 | HGEGTFTSDLSKQ MEEEA VRLFIEWL KNGGPSSGAPPPSC | B2 | C40 | - | - | 5167.1 |
| 3 | 12 | HGEGTFTSDLSKQ MEEEA VRLFIEWL KNGGPSSGAPPPSC | B3 | C40 | - | - | 5521.6 |
| 4 | 8 | H(Aib )QGTFTSDYS KYLDEQAAKEFVQ WLMNTC | B1 | C30 | - | - | 3963.5 |
| 5 | 8 | H(Aib )QGTFTSDYS KYLDEQAAKEFVQ WLMNTC | B2 | C30 | - | - | 4386.1 |
| 6 | 8 | H(Aib )QGTFTSDYS KYLDEQAAKEFVQ WLMNTC | B3 | C30 | - | - | 4740.6 |
| 7 | 13 | SVSEIQLMHNLGK HLNSMERVEWLRK KLQDVHNFC | B1 | C35 | - | - | 4675.4 |
| 8 | 13 | SVSEIQLMHNLGK HLNSMERVEWLRK KLQDVHNFC | B2 | C35 | - | - | 5098.0 |
| 9 | 13 | SVSEIQLMHNLGK HLNSMERVEWLRK KLQDVHNFC | B3 | C35 | - | - | 5452.5 |
| 10 | 5 | HGEGTFTSDLSKQ MEEEAVRLFIEWL KNGGPSSGAPPPS | B3 | C35 | - | - | 5452.5 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 11 | 12 | HGEGTFTSDLSKQ MEEEAVRLFIEWL KNGGPSSGAPPPSC | B5 | C40 | - | - | 5857.0 |
| 12 | 15 | GIVEQCCTSICSLE QLENYCN (Chain A) | B4 | K29 of Chain B | - | - | 7200.9 |
| | 16 | FVNQHLCGSHLVEA LYLVCGERGFFYTPKT (Chain B) | | | - | - | |
| 13 | 15 | GIVEQCCTSICSLE QLENYCN (Chain A) | B4 | F1/K29 of Chain B | - | - | 8627.8 |
| | 16 | FVNQHLCGSH LVEALYLVCGER GFFYTPKT (Chain B) | | | - | - | |
| 14 | 12 | HGEGTFTSDLSKQ MEEEAVRLFIEWLK NGGPSSGAPPPSC | B3 | C40 | F1 | K27 | 5759.4 |
| 15 | 12 | HGEGTFTSDLSKQ MEEEAVRLFIEWL KNGGPSSGAPPPSC | B38 | K12, K27 | F2 | C40 | 5120.9 |
| 16 | 12 | HGEGTFTSDLSKQ MEEEAVRLFIEWL KNGGPSSGAPPPSC | B3 | C40 | F11 | K27 | 6237.2 |
| 17 | 12 | HGEGTFTSDLSKQ MEEEAVRLFIEWL KNGGPSSGAPPPSC | B38 | K12, K27 | F12 | C40 | 5598.6 |
| 18 | 12 | HGEGTFTSDLSKQ | B35 | C40 | - | - | 6208.4 |
| | | MEEEAVRLFIEWL KNGGPSSGAPPPSC | | | | | |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 19 | 12 | HGEGTFTSDLSKQ MEEEAVRLFIEWL KNGGPSSGAPPPSC | B36 | C40 | - | - | 6208.4 |
| 20 | 18 | HGEGTFTSDLSKQ MEEEAVRLFIEWLKN GGPSSGAPPPSK | B37 | K40 | - | - | 5565.4 |
| 21 | 19 | HGEGTFTSDLSKQ MEEEAVRLFIEWLKNG GPSSGAPPPSKKK | B | K39, K40, K 41 | - | - | 5251.1 |
| 22 | 19 | HGEGTFTSDLSKQ MEEEAVRLFIEWLKNG GPSSGAPPPSKKK | B | K39, K 40, K41 | F11 | K27 | 6241.6 |
| 23 | 19 | HGEGTFTSDLSKQ MEEEAVRLFIEWLKNG GPSSGAPPPSKKK | B | K39, K40, K41 | F11 | F12 | 6241.6 |
| 24 | 20 | H(Aib)EGTFTSDLSKQ MEEEAVRLFIEW LKNGGPSSGAPPPSK | B37 | K40 | - | - | 5278.1 |
| 25 | 21 | H(Aib)EGTFTSDLSKQ MEEEAVRLFIEW LKNGGPSSGAPPPSKKK | B | K39, K 40, K41 | - | - | 5279.0 |
| 26 | 21 | H(Aib)EGTFTSDLSKQ MEEEAVRLFIEW LKNGGPSSGAPPPSKKK | B | K39, K 40, K41 | F11 | K27 | 6241.6 |
| 27 | 21 | H(Aib)EGTFTSDLSKQ MEEEAVRLFIEW LKNGGPSSGAPPPSKKK | B | K39, K40, K41 | F11 | K12 | 5759.0 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 28 | 8 | H(Aib )QGTFTSDYS KYLDEQAAKEFVQ WLMNTC | B2 | K12 | C30 | F6 | 5259.0 |
| 29 | 8 | H(Aib )QGTFTSDYS KYLDEQAAKEFVQ WLMNTC | B5 | C30 | K12 | F5 | 4800.0 |
| 30 | 22 | KCNTATCATQRLAN FLVHSSNNFGAIL SSTNVGSNTY | B38 | K1 | - | - | 4129.6 |
| 31 | 22 | KCNTATCATQRLAN FLVHSSNNFGAIL SSTNVGSNTY | B39 | K1 | - | - | 5842.7 |
| 32 | 23 | KCNTATCATQRLAN FLVHSSNNFGAIL SSTNVGSNTYK | B2 | K38 | F11 | K1 | 5627.5 |
| 33 | 24 | KCNTATCATQRLAN FLVHSSNNFGPIL PPTNVGSNTY | B38 | K1 | - | - | 4175.7 |
| 34 | 24 | KCNTATCATQRLAN FLVHSSNNFGPIL PPTNVGSNTY | B39 | K1 | - | - | 5888.8 |
| 35 | 25 | KCNTATCATQRLAN FLVHSSNNFGPIL PPTNVGSNTYK | B2 | K38 | F11 | K1 | 5673.6 |
| 36 | 26 | KCNTATCATQRLAD FLLRHSSPNFGAIP SSTNVGSRTY | B38 | K1 | - | - | 4196.7 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 37 | 26 | KCNTATCATQRLAD FLLRHSSPNFGAIP SSTNVGSRTY | B39 | K1 | - | - | 5908.8 |
| 38 | 27 | KCNTATCATQRLAD FLLRHSSPNFGAIP SSTNVGSRTYK | B2 | K38 | F11 | K1 | 5694.5 |
| 39 | 28 | KCNTATCATQRLAD FLLRHSSNNFGAIP SSTNVGSRTY | B38 | K1 | - | - | 4213.7 |
| 40 | 28 | KCNTATCATQRLAD FLLRHSSNNFGAIP SSTNVGSRTY | B39 | K1 | - | - | 5926.8 |
| 41 | 29 | KCNTATCATQRLAD FLLRHSSNNFGAIP SSTNVGSRTYK | B2 | K38 | F11 | K1 | 5711.5 |
| 42 | 30 | RCNTATCATQRLAD FLLRHSSNNFGAIP SSTNVGSKTY | B38 | K35 | - | - | 4213.7 |
| 43 | 30 | RCNTATCATQRLAD FLLRHSSNNFGAIP SSTNVGSKTY | B39 | K35 | - | - | 5926.8 |
| 44 | 31 | RCNTATCATQRLAD FLLRHSSNNFGAIP SSTNVGSKTYK | B2 | K35 | F11 | K38 | 5711.5 |
| 45 | 32 | H(Aib)QGTFTSDKSKYL DERAAQDFVQWLLD GGPSSGAPPPS | B38 | K12 | - | - | 6112.9 |
| 46 | 33 | H(Aib)QGTFTSDKSKYL DERAAQDFVQWLMD GGPSSGAPPPS | B39 | K12 | - | - | 6131.0 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 47 | 34 | H(Aib)QGTFTSDKSKYL DKIAAQDFVQWLID GGPSSGAPPPS | B38 | K10 | - | - | 6069.0 |
| 48 | 35 | H(Aib)QGTFTSDKSWYL DKIAAQDFVQWLLG GGPSSGAPPPS | B39 | K10 | - | - | 6069.0 |
| 49 | 36 | H(Aib)QGTFTSDKSWYL DERAAQDFVQWLM GGGPSSGAPPPS | B3 | K12 | F11 | K40 | 6142.0 |
| 50 | 37 | H(Aib)QGTFTSDKSKWL DKIAAQDFVQWLIG GGPSSGAPPPS | B3 | K12 | F12 | C40 | 6220.2 |
| 51 | 20 | H(Aib)EGTFTSDLSKQ MEEEAVRLFIEW LKNGGPSSGAPPPSK | B37 | K40 | F11 | K27 | 6309.3 |
| 52 | 20 | H(Aib)EGTFTSDLSKQ MEEEAVRLFIEW LKNGGPSSGAPPPSK | B38 | K12, K27 | F11 | K40 | 5511.3 |
| 53 | 38 | H(Aib)EGTFTSDLSKQ MEEEAVRLFIEW LKNGGPSSGAPPPSC | B38 | K12, K27 | F12 | C40 | 5626.5 |
| 54 | 39 | H(Aib)EGTFTSDLSKQ MEEEAVRLFIEW LKNGGPSSGAPPPSC | B38 | K12, K27 | F12 | C40 | 5597.4 |
| 55 | 8 | H(Aib )QGTFTSDYS KYLDEQAAKEFVQ WLMNTC | B38 | K12 | F12 | C30 | 4591.3 |
| 56 | 40 | H(Aib)QGTFTSDYSKYL DEQAAKEFVQW LMNTK | B38 | K12 | F11 | K30 | 4475.1 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 57 | 41 | H(Aib)QGTFTSDYSKYL DEKRAKEFVQW LMNTC | B38 | K12 | F12 | C30 | 4647.6 |
| 58 | 8 | H(Aib )QGTFTSDYS KYLDEQAAKEFVQ WLMNTC | B1 | C30 | F16 | K12 | 4679.4 |
| 59 | 8 | H(Aib )QGTFTSDYS KYLDEQAAKEFVQ WLMNTC | B38 | K12 | F14 | C30 | 4619.4 |
| 60 | 22 | KCNTATCATQRLAN FLVHSSNNFGAIL SSTNVGSNTY | B38 | K1 | F11 | K1 | 4845.5 |
| 61 | 24 | KCNTATCATQRLAN FLVHSSNNF GPILPPTNVGSNTY | B38 | K1 | F11 | K1 | 4891.6 |
| 62 | 26 | KCNTATCATQRLAD FLLRHSSPNF GAIPSSTNVGSRTY | B38 | K1 | F11 | K1 | 4912.6 |
| 63 | 28 | KCNTATCATQRLAD FLLRHSSNNFGAIP SSTNVGSRTY | B38 | K1 | F11 | K1 | 4929.6 |
| 64 | 30 | RCNTATCATQRLAD FLLRHSSNNFGAIP SSTNVGSKTY | B38 | K35 | F11 | K1 | 4929.6 |
| 65 | 15 | GIVEQCCTSICSLE QLENYCN (Chain A) | B38 | K29 of Chain B | - | - | 6000.3 |
| | 16 | FVNQHLCGSHLVEA LYLVCGERGFFYTPKT (Chain B) | B38 | K29 of Chain B | | | 6000.3 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 66 | 15 | GIVEQCCTSICSLE QLENYCN (Chain A) | B38 | K29 of Chain B | | | 6716.2 |
| | 16 | FVNQHLCGSHLVEA LYLVCGERGFFYTPKT (Chain B) | B38 | K29 of Chain B | F11 | F1 of Chain B | 6716.2 |
| 67 | 13 | SVSEIQLMHNLGK HLNSMERVEWL RKKLQDVHNF | B38 | K13, K26, K27 | - | - | 4796.7 |
| 68 | 42 | MFPTIPLSRLFDNA MLRAHRLHQLAFD TYQEFEEAYIPKEQ KISFLQNPQTSLCFS ESIPTPSNREETQQ KSNLELLRISLLLIQ SWLEPVQFLRSVF ANSL VYGASDSNV YDLLKDLEEGIQTL MGRLEDGSPRTGQ IFKQTYSKFDTNSH NDDALLKNYGLLY CFRKDMDKVETFL RIVQCRSVEGSCGF | B38 | Lys random | - | - | |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 69 | 42 | MFPTIPLSRLFDNA MLRAHRLHQLAFD TYQEFEEAYIPKEQ KISFLQNPQTSLCFS ESIPTPSNREETQQ KSNLELLRISLLLIQ SWLEPVQFLRSVF ANSL VYGASDSNV YDLLKDLEEGIQTL MGRLEDGSPRTGQ IFKQTYSKFDTNSH NDDALLKNYGLLY CFRKDMDKVETFL RIVQCRSVEGSCGF | B38 | Lys random | F16 | Lys random | |
| 70 | 5 | HGEGTFTSDLSKQ MEEEAVRLFIEWL KNGGPSSGAPPPS | B38 | K12, K27 | - | - | |
| 71 | 12 | HGEGTFTSDLSKQ MEEEAVRLFIEWL KNGGPSSGAPPPSC | - | - | F1 | C40 | |
| 72 | 12 | HGEGTFTSDLSKQ MEEEAVRLFIEWL KNGGPSSGAPPPSC | - | - | F12 | C40 | |
| (In the above table, B represents native biotin.) | | | | | | | |

**Conjugates 14 through 17**

1) Preparation example

The conjugates of Table 8 below were prepared using the following method.

Using DMSO solution with 0.3% trimethylamine (TEA, Sigma) added as the reaction solvent, the polypeptide of SEQ ID NO: 12 from Table 6 above and the biotin moieties of Table 3 and Table 4 above were mixed into a mixture of molar ratio 1:2, and reacted for at least 30 minutes at room temperature. Then, mixtures of the polypeptide-biotin

moiety mixture and the fatty acid moieties of Table 5 above at a molar ratio of 1:1 to 1:2 were prepared, and reacted for at least 90 minutes at room temperature. The reaction was stopped by adding a 1% trifluoroacetic acid solution of the same volume as the mixture.

**[Table 8]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 14 | 12 | HGEGTFTSDLSKQ MEEEAVRLFIEWLK NGGPSSGAPPPSC | B3 | C40 | F1 | K27 | 5759.4 |
| 15 | 12 | HGEGTFTSDLSKQ MEEEAVRLFIEWL KNGGPSSGAPPPSC | B1 | K12, K27 | F2 | C40 | 5120.9 |
| 16 | 12 | HGEGTFTSDLSKQ MEEEAVRLFIEWL KNGGPSSGAPPPSC | B3 | C40 | F11 | K27 | 6237.2 |
| 17 | 12 | HGEGTFTSDLSKQ MEEEAVRLFIEWL KNGGPSSGAPPPSC | B1 | K12, K27 | F12 | C40 | 5598.6 |

2) Isolation, purification and purity check The reaction products of Conjugates 14 through 17 were isolated and purified using reverse phase high-performance liquid chromatography (hereinafter HPLC).

A SUPERSIL ODS-1 column (10 x 250mm, 5um, LB Science, South Korea) was used. Maintaining solvent A (distilled water with 0.1% TFA added) and solvent B (acetonitrile with 0.1% TFA added) at a flow rate of 4.7ml/min, the mobile phase condition was changed linearly as follows: Conjugate 14: 30-80%, Conjugate 15: 40-70%, Conjugate 16: 30-60%, Conjugate 17:40-60%. Then, monitoring at 280nm with a UV spectroscope, the peaks detected at between 10 and 17 minutes were collected (Conjugate 14: 15 minutes, Conjugate 15: 16 minutes, Conjugate 16: 16 minutes, Conjugate 17: 11 minutes). The collected peaks were concentrated and purified using ultracentrifugal filters having an appropriate molecular weight cut-off, after volatilizing organic solvents and TFA under vacuum. The purity of the purified substances was confirmed using the HPLC analysis method. Analysis was carried out at a constant temperature near room temperature using a Gemini C18 column (4.6 x 250mm, 5um; Phenomenex, CA, USA). Analysis was carried out using the gradient elution method at a flow rate of 1mL/min using a mobile phase comprised of trifluoroacetic acid solution: acetonitrile mixture (at a mix ratio of 70:30 and 20:80 at 20 minutes later). UV absorbance was observed at 280nm.

The measured purification chromatograms of Conjugates 14 through 17 are as shown in FIG. 1. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugates 14 through 17 was at least 95%.

3) Confirming molecular weight

The molecular weight of the final material obtained from Conjugates 14 through 17 was measured.

The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 8 above.

**Conjugates 18 through 19**

1) Preparation example

The conjugates of Table 9 below were prepared using the following method.

Using DMSO solution with 0.3% trimethylamine (TEA, Sigma) added as the reaction solvent, the polypeptide of SEQ ID NO: 12 from Table 6 above and the biotin moieties of Table 3 and Table 4 above were mixed into a mixture of molar ratio 1:2, and reacted for at least 30 minutes at room temperature. The reaction was stopped by adding a 1% trifluoroacetic acid solution of the same volume as the mixture.

**[Table 9]**

| Conjugate | Polypeptide | | Biotin Moiety | | Fatty Acid Moiety | | (g/mol) |
|---|---|---|---|---|---|---|---|
| | SEQ ID NO | Sequence | No. | Binding Site | No. | Binding Site | |
| 18 | 12 | HGEGTFTSDLSKQ MEEEAVRLFIEWL KNGGPSSGAPPPSC | B35 | C40 | - | - | 6208.4 |
| 19 | 12 | HGEGTFTSDLSKQ MEEEAVRLFIEWL KNGGPSSGAPPPSC | B36 | C40 | - | - | 6208.4 |

2) Isolation, purification and purity check

The reaction products of Conjugates 18 through 19 were isolated and purified using reverse phase high-performance liquid chromatography (hereinafter HPLC).

A SUPERSIL ODS-1 column (10 x 250mm, 5um, LB Science, South Korea) was used and the mobile phase condition were linearly changed from 30% to 80% while maintaining solvent A (distilled water with 0.1% TFA added) and solvent B (acetonitrile with 0.1% TFA added) at a flow rate of 4.7m1/min. Then, monitoring at 280nm with a UV spectroscope, the peaks detected at between 12 and 13 minutes were collected. The collected peaks were concentrated and purified using ultracentrifugal filters having an appropriate molecular weight cut-off, after volatilizing organic solvents and TFA under vacuum. The purity of the purified substances was confirmed using the HPLC analysis method. Analysis was carried out at a constant temperature near room temperature using a Gemini C18 column (4.6 x 250mm, 5um; Phenomenex, CA, USA). Analysis was carried out using the gradient elution method at a flow rate of 1mL/min using a mobile phase comprised of trifluoroacetic acid solution: acetonitrile mixture (at a mix ratio of 70:30 and 20:80 at 20 minutes later). UV absorbance was observed at 280nm.

The measured purification chromatograms of Conjugates 18 through 19 are as shown in FIG. 2. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugates 18 through 19 was at least 95%.

3) Confirming molecular weight

The molecular weight of the final material obtained from Conjugates 18 through 19 was measured. The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 9 above.

**Conjugates 20 and 24**

1) Preparation example

For Conjugates 20 and 24, the typical SPPS method described above was used to synthesize the conjugates

of Table 10 below.

[Table 10]

| Conjuga te | Polypeptide | | Biotin Moiety | | Fatty Acid Moiety | | (g/mol) |
|---|---|---|---|---|---|---|---|
| | SEQ ID NO | Sequence | No. | Binding Site | No. | Binding Site | |
| 20 | 18 | HGEGTFTSDLSKQ MEEEAVRLFIEWLKN GGPSSGAPPPSK | B37 | K40 | - | - | 5565.4 |
| 24 | 20 | H(Aib)EGTFTSDLSKQ MEEEAVRLFIEW LKNGGPSSGAPPPSK | B37 | K40 | - | - | 5278.1 |

2) The purity of the purified Conjugates 20 and 24 was confirmed using the HPLC analysis method. Analysis was carried out at a constant temperature near room temperature using a Gemini C18 column (4.6 x 250mm, 5um; Phenomenex, CA, USA). Analysis was carried out using the gradient elution method at a flow rate of 1mL/min using a mobile phase comprised of trifluoroacetic acid solution: acetonitrile mixture (at a mix ratio of 70:30 and 50:50 at 20 minutes later). UV absorbance was observed at 280nm.

The measured purification chromatograms of Conjugates 20 and 24 are as shown in FIG. 3. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugates 20 and 24 was at least 95%.

3) Confirming molecular weight

The molecular weight of the final material obtained from Conjugates 20 and 24 was measured.

The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 10 above.

**Conjugates 51 through 54**

1) Preparation example

For Conjugates 51 through 54, the standard Fmoc preparation method was used to synthesize the conjugates of Table 12 below.

The standard Fmoc preparation method means the method that is carried out as follows:

DMF was added to a vessel containing Fmoc Rink amid AM resin, and allowed to swell for 2 hours. After washing with DMF, 20% piperidine/DMF was added and mixed for 30 minutes. After washing with DMF, a Fmoc-amino acid solution was mixed for 30 minutes, then an activating buffer solution was added, and nitrogen gas was applied for 1 hour. The above process was repeated until the target peptide sequences were completed. Here, the Fmoc-amino acid reagent included the materials of Table 11 below.

[Table 11]

| # | Materials | Coupling reagents |
|---|---|---|
| 1 | Fmoc-Lys(Alloc)-OH (2.0 eq) | HATU (1.95 eq) and DIEA (4.0 eq) |
| 2 | Fmoc-Ser(tBu)-OH (3.0 eq) | HBTU (2.85 eq) and DIEA (6.0 eq) |
| 3 | Fmoc-Pro-OH (3.0 eq) | HBTU (2.85 eq) and DIEA (6.0 eq) |
| 4 | Fmoc-Pro-OH (3.0 eq) | HBTU (2.85 eq) and DIEA (6.0 eq) |
| 5 | Fmoc-Pro-OH (3.0 eq) | HBTU (2.85 eq) and DIEA (6.0 eq) |
| 6 | Fmoc-Ala-OH (3.0 eq) | HBTU (2.85 eq) and DIEA (6.0 eq) |
| 7 | Fmoc-Gly-OH (3.0 eq) | HBTU (2.85 eq) and DIEA (6.0 eq) |
| 8 | Fmoc-Ser(tBu)-OH (3.0 eq) | HBTU (2.85 eq) and DIEA (6.0 eq) |
| 9 | Fmoc-Ser(tBu)-OH (3.0 eq) | HBTU (2.85 eq) and DIEA (6.0 eq) |
| 10 | Fmoc-Pro-OH (3.0 eq) | HOBt (3.00 eq) and DIC (3.0 eq) |
| 11 | Fmoc-Gly-OH (3.0 eq) | HBTU (2.85 eq) and DIEA (6.0 eq) |
| 12 | Fmoc-Gly-OH (3.0 eq) | HBTU (2.85 eq) and DIEA (6.0 eq) |
| 13 | Fmoc-Asn(Trt)-OH (3.0 eq) | HBTU (2.85 eq) and DIEA (6.0 eq) |
| 14 | Fmoc-Lys(Dde)-OH (3.0 eq) | HBTU (2.85 eq) and DIEA (6.0 eq) |
| 15 | Fmoc-Leu-OH (3.0 eq) | HBTU (2.85 eq) and DIEA (6.0 eq) |
| 16 | Fmoc-Trp(Boc)-OH (3.0 eq) | HBTU (2.85 eq) and DIEA (6.0 eq) |
| 17 | Fmoc-Glu(OtBu)-OH (3.0 eq) | HBTU (2.85 eq) and DIEA (6.0 eq) |
| 18 | Fmoc-Ile-OH (3.0 eq) | HBTU (2.85 eq) and DIEA (6.0 eq) |
| 19 | Fmoc-Phe-OH (3.0 eq) | HBTU (2.85 eq) and DIEA (6.0 eq) |
| 20 | Fmoc-Leu-OH (3.0 eq) | HBTU (2.85 eq) and DIEA (6.0 eq) |
| 21 | Fmoc-Arg-OH (3.0 eq) | HBTU (2.85 eq) and DIEA (6.0 eq) |
| 22 | Fmoc-Val-OH (6.0 eq) | HATU (5.70 eq) and DIEA (12.0 eq) |
| 23 | Fmoc-Ala-OH (6.0 eq) | HBTU (5.70 eq) and DIEA (12.0 eq) |
| 24 | Fmoc-Glu(OtBu)-OH (6.0 eq) | HBTU (5.70 eq) and DIEA (12.0 eq) |
| 25 | Fmoc-Glu(OtBu)-OH (6.0 eq) | HBTU (5.70 eq) and DIEA (12.0 eq) |
| 26 | Fmoc-Glu(OtBu)-OH (6.0 eq) | HBTU (5.70 eq) and DIEA (12.0 eq) |
| 27 | Fmoc-Met-OH (6.0 eq) | HATU (5.70 eq) and DIEA (12.0 eq) |
| 28 | Fmoc-Gln(Trt)-OH (6.0 eq) | HATU (5.70 eq) and DIEA (12.0 eq) |
| 29 | Fmoc-Lys(Dde)-OH (3.0 eq) | HATU (2.85 eq) and DIEA (6.0 eq) |
| 30 | Fmoc-Ser(tBu)-OH (6.0 eq) | HATU (5.70 eq) and DIEA (12.0 eq) |
| 31 | Fmoc-Leu-OH (6.0 eq) | HATU (5.70 eq) and DIEA (12.0 eq) |
| 32 | Fmoc-Asp(OtBu)-OH (6.0 eq) | HOBt (6.00 eq) and DIC (6.0 eq) |
| 33 | Fmoc-Ser(tBu)-OH (6.0 eq) | HATU (5.70 eq) and DIEA (12.0 eq) |
| 34 | Fmoc-Thr(tBu)-OH (6.0 eq) | HATU (5.70 eq) and DIEA (12.0 eq) |
| 35 | Fmoc-Phe-OH (6.0 eq) | HATU (5.70 eq) and DIEA (12.0 eq) |
| 36 | Fmoc-Thr(tBu)-OH (6.0 eq) | HATU (5.70 eq) and DIEA (12.0 eq) |
| 37 | Fmoc-Gly-OH (9.0 eq) | HATU (8.55 eq) and DIEA (18.0 eq) |
| 38 | Fmoc-Glu(OtBu)-OH (9.0 eq) | HATU (8.55 eq) and DIEA (18.0 eq) |
| 39 | Fmoc-Aib-OH (3.0 eq) | HATU (2.85 eq) and DIEA (6.0 eq) |
| 40 | Fmoc-His(Trt)-OH (6.0 eq) | HATU (5.70 eq) and DIEA (12.0 eq) |
| 41 | $(Boc)_2O$ (5.0 eq) | DIEA (10.0 eq) |
| | De-Alloc | PhSiH3 (10 eq) and $Pd(PPh_3)_4$ (0.1 eq) |
| 42 | Fmoc-AEEA-OH (3.0 eq) | HATU (2.85 eq) and DIEA (6.0 eq) |
| 43 | Fmoc-AEEA-OH (3.0 eq) | HATU (2.85 eq) and DIEA (6.0 eq) |
| 44 | Fmoc-γ (3.0 eq) | HATU (2.85 eq) and DIEA (6.0 eq) |
| 45 | C18DA-tBu (3.0 eq) | HATU (2.85 eq) and DIEA (6.0 eq) |

| | | |
|---|---|---|
| | De-Dde | 3% Hydrazine hydrate |
| 46 | Biotin-OSu (4.0 eq) | DIEA (12.0 eq) |

- Conjugates 53 through 54 were prepared using the following method: Using DMSO solution with 0.3% trimethylamine (TEA, Sigma) added as the reaction solvent, the polypeptides of SEQ ID NOs: 39 through 40 from Table 6 above and the biotin moieties of Table 3 and Table 4 above were mixed into a mixture of molar ratio 1:2, and reacted for at least 10 minutes at room temperature. Then, mixtures of the polypeptide-biotin moiety mixture and the fatty acid moieties of Table 5 above at a molar ratio of 1:3 were prepared, and reacted for at least 90 minutes at room temperature. The reaction was stopped by adding a 1% trifluoroacetic acid solution of the same volume as the mixture.

**[Table 12]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 51 | 20 | H(Aib)EGTFTSDLSKQ MEEEAVRLFIEW LKNGGPSSGAPPPSK | B37 | K40 | F11 | K27 | 6309.3 |
| 52 | 20 | H(Aib)EGTFTSDLSKQ MEEEAVRLFIEW LKNGGPSSGAPPPSK | B38 | K12, K27 | F11 | K40 | 5511.3 |
| 53 | 38 | H(Aib)EGTFTSDLSKQ MEEEAVRLFIEW LKNGGPSSGAPPPSC | B38 | K12, K27 | F12 | C40 | 5626.5 |
| 54 | 39 | H(Aib)EGTFTSDLSKQ MEEEAVRLFIEW LKNGGPSSGAPPPSC | B38 | K12, K27 | F12 | C40 | 5597.4 |

2) Isolation, purification and purity check

The reaction products of Conjugates 51 through 54 were isolated and purified using reverse phase high performance liquid chromatography.

A Gemini C18 column (10 x 250mm, 5um; Phenomenex, CA, USA) was used. Maintaining solvent A (distilled water with 0.1% TFA added) and solvent B (acetonitrile with 0.1% TFA added) at a flow rate of 4.7ml/min, the mobile phase condition was changed linearly from 40 to 60%. Then, monitoring at 280nm with a UV spectroscope, the peaks detected at between 11 and 13 minutes (Conjugate 53: 12 minutes, Conjugate 54: 13 minutes) were collected. The collected peaks were concentrated and purified using ultracentrifugal filters having an appropriate molecular weight cut-off, after volatilizing organic solvents and TFA under vacuum. The purity of the purified materials was confirmed using the HPLC analysis method. Analysis was carried out with a Gemini C18 column (4.6 x 250mm, 5um; Phenomenex, CA, USA) at a constant temperature near room temperature. Analysis was carried out using the gradient elution method at a flow rate of 1mL/min using a mobile phase comprised of trifluoroacetic acid solution: acetonitrile mixture (at a mix ratio of 70:30 and 20:80 at 20 minutes later). UV absorbance was observed at 280nm.

The measured purification chromatograms for the final products from Conjugates 51 through 52 are as shown in FIG. 5, and the purification chromatograms of Conjugate 53 through 54 are as shown in FIG. 6. Further, analyzing with a UV detector, peaks other than that of the polypeptide bonded to a biotin moiety and the fatty acid moiety were not observed. Representing the area of the peaks from the respective chromatograms as a percentage, it was confirmed

that the purity of Conjugates 51 through 54 was at least 95%.

3) Confirming molecular weight

The molecular weight of the final material obtained from Conjugates 51 through 54 was measured.

The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 12.

**Conjugates 55 through 59**

- Conjugate 56 was synthesized using the standard Fmoc preparation method used for Conjugates 51 through 54 above.
- Conjugate 55 and Conjugates 57 through 59 were prepared using the following method. Using DMSO solution with 0.3% trimethylamine (TEA, Sigma) added as the reaction solvent, the polypeptides of SEQ ID NOs: 8, 40 and 41 from Table 6 above and the biotin moieties of Table 3 and Table 4 above were mixed into a mixture of molar ratio 1:2, and reacted for at least 10 minutes at room temperature. Then, mixtures of the polypeptide-biotin moiety mixture and the fatty acid moieties of Table 5 above at a molar ratio of 1:1 or 1:2 were prepared, and reacted for at least 90 minutes at room temperature. The reaction was stopped by adding a 1% trifluoroacetic acid solution of the same volume as the mixture.

**[Table 13]**

| Conjugat e (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 55 | 8 | H(Aib )QGTFTSDYS KYLDEQAAKEFVQ WLMNTC | B38 | K12 | F12 | C30 | 4591.3 |
| 56 | 40 | H(Aib)QGTFTSDYSKYL DEQAAKEFVQW LMNTK | B38 | K12 | F11 | K30 | 4475.1 |
| 57 | 41 | H(Aib)QGTFTSDYSKYL DEKRAKEFVQW LMNTC | B38 | K12 | F12 | C30 | 4647.6 |
| 58 | 8 | H(Aib )QGTFTSDYS KYLDEQAAKEFVQ WLMNTC | B1 | C30 | F11 | K12 | 4679.4 |
| 59 | 8 | H(Aib )QGTFTSDYS KYLDEQAAKEFVQ WLMNTC | B38 | K12 | F14 | C30 | 4619.4 |

2) Isolation, purification and purity check

The reaction products of Conjugates 55 through 59 were isolated and purified using reverse phase high performance liquid chromatography.

A Gemini C18 column (10 x 250mm, 5um; Phenomenex, CA, USA) was used. Maintaining solvent A (distilled water with 0.1% TFA added) and solvent B (acetonitrile with 0.1% TFA added) at a flow rate of 4.7ml/min, the mobile phase condition was changed linearly as follows: Conjugates 55 and 57: 40-60%, Conjugates 58 through 59: 40-70%. Then, monitoring at 280nm with a UV spectroscope, the peaks detected at between 10 and 15 minutes (Conjugate 55: 13 minutes, Conjugate 57: 10 minutes, Conjugate 58: 13 minutes, Conjugate 59: 15 minutes) were collected. The collected peaks were concentrated and purified using ultracentrifugal filters having an appropriate molecular weight cut-off, after volatilizing organic solvents and TFA under vacuum. The purity of the purified materials was confirmed using the HPLC analysis method. Analysis was carried out with a Gemini C18 column (4.6 x 250mm, 5um; Phenomenex, CA, USA) at a constant temperature (35°C). Analysis was carried out using the gradient elution method at a flow rate of 1mL/min using a mobile phase comprised of trifluoroacetic acid solution: acetonitrile mixture (at a mix ratio of 60:40 and 30:70 at 20 minutes later). UV absorbance was observed at 280nm.

The measured purification chromatograms for the final products from Conjugate 55 and Conjugates 57 through 59 are as shown in FIG. 8, and the purification chromatograms of Conjugate 56 is as shown in FIG. 9. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugates 55 through 59 was at least 95%.

3) Confirming molecular weight

The molecular weight of the final material obtained from Conjugates 55 through 59 was measured.

The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 13.

**Conjugates 60 through 64**

1) Preparation example

The conjugates of Table 14 below were prepared using the following method: Using DMSO solution with 0.3% trimethylamine (TEA, Sigma) added as the reaction solvent, the polypeptides of SEQ ID NOs: 22, 24, 26, 28 and 30 from Table 6 above and the biotin moieties of Table 3 and Table 4 above were mixed into a mixture of molar ratio 1:1, and reacted for at least 30 minutes at room temperature. Then, mixtures of the polypeptide-biotin moiety mixture and the fatty acid moieties of Table 5 above at a molar ratio of 1:2 were prepared, and reacted for at least 120 minutes at room temperature. The reaction was stopped by adding a 1% trifluoroacetic acid solution of the same volume as the mixture.

**[Table 14]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 60 | 22 | KCNTATCATQRLAN FLVHSSNNFGAIL SSTNVGSNTY | B38 | K1 | F11 | K1 | 4845.5 |
| 61 | 24 | KCNTATCATQRLAN FLVHSSNNF GPILPPTNVGSNTY | B38 | K1 | F11 | K1 | 4891.6 |

(continued)

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 62 | 26 | KCNTATCATQRLAD FLLRHSSPNF GAIPSSTNVGSRTY | B38 | K1 | F11 | K1 | 4912.6 |
| 63 | 28 | KCNTATCATQRLAD FLLRHSSNNFGAIP SSTNVGSRTY | B38 | K1 | F11 | K1 | 4929.6 |
| 64 | 30 | RCNTATCATQRLAD FLLRHSSNNFGAIP SSTNVGSKTY | B38 | K35 | F11 | K1 | 4929.6 |

2) Isolation, purification and purity check

The reaction products of Conjugates 60 through 64 were isolated and purified using reverse phase high performance liquid chromatography.

A Gemini C18 column (10 x 250mm, 5um; Phenomenex, CA, USA) was used. Maintaining solvent A (distilled water with 0.1% TFA added) and solvent B (acetonitrile with 0.1% TFA added) at a flow rate of 4.7ml/min, the mobile phase condition was changed linearly as follows: Embodiment 18 through 20: 30-60%, Embodiments 21 through 22: 25-50%. Then, monitoring at 280nm with a UV spectroscope, the peaks detected at between 5 and 18 minutes (Embodiment 18: 20 minutes, Embodiment 19: 15 minutes, Embodiment 20: 13 minutes, Embodiment 21: 17 minutes) were collected. The collected peaks were concentrated and purified using ultracentrifugal filters having an appropriate molecular weight cut-off, after volatilizing organic solvents and TFA under vacuum. The purity of the purified materials was confirmed using the HPLC analysis method. Analysis was carried out with a Gemini C18 column (4.6 x 250mm, 5um; Phenomenex, CA, USA) at a constant temperature (35°C). Analysis was carried out using the gradient elution method at a flow rate of 1mL/min using a mobile phase comprised of trifluoroacetic acid solution: acetonitrile mixture (at a mix ratio of 75:25 and 50:50 at 20 minutes later). UV absorbance was observed at 280nm.

The measured purification chromatograms for the final products from Conjugates 60 through 64 are as shown in FIG. 11.

Analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugates 60 through 64 was at least 95%.

3) Confirming molecular weight

The molecular weight of the final material obtained from Conjugates 60 through 64 was measured.

The molecular weights were confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 14.

**Conjugate 66**

1) Preparation example

The conjugate of Table 15 below was prepared using the following method: Using DMSO solution with 0.3%

trimethylamine (TEA, Sigma) added as the reaction solvent, the polypeptide from Table 6 above wherein the proteins are joined through disulfide bonds between the 6th and 11th cysteine of SEQ ID NO 15; the 7th cysteine of SEQ ID NO 15 and the 7th cysteine of SEQ ID NO 16; and the 20th cysteine of SEQ ID NO 15 and the 19th cysteine of SEQ ID NO 16, and the biotin moieties of Table 3 and Table 4 above, were mixed into a mixture of molar ratio 1:1, and reacted for at least 30 minutes at room temperature. Then, mixtures of the polypeptide-biotin moiety mixture and the fatty acid moieties of Table 5 above at a molar ratio of 1:2 were prepared, and reacted for at least 120 minutes at room temperature. The reaction was stopped by adding a 1% trifluoroacetic acid solution of the same volume as the mixture.

**[Table 15]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 66 | 15 | GIVEQCCTSICSLE QLENYCN (Chain A) | B38 | K29 of Chain B | F11 | F1 of Chain B | 6716.2 |
| | 16 | FVNQHLCGSHLVEA LYLVCGERGFFYTPKT (Chain B) | | | | | |

2) Isolation, purification and purity check

The reaction products of Conjugate 66 were isolated and purified using reverse phase high performance liquid chromatography. A Gemini C18 column (10 x 250mm, 5um; Phenomenex, CA, USA) was used. Maintaining solvent A (distilled water with 0.1% TFA added) and solvent B (acetonitrile with 0.1% TFA added) at a flow rate of 4.7ml/min, the mobile phase condition was changed linearly from 35 to 45%. Then, monitoring at 280nm with a UV spectroscope, the peaks detected at between 13 and 14 minutes were collected. The collected peaks were concentrated and purified using ultracentrifugal filters having an appropriate molecular weight cut-off, after volatilizing organic solvents and TFA under vacuum. The purity of the purified materials was confirmed using the HPLC analysis method. Analysis was carried out with a Gemini C18 column (4.6 x 250mm, 5um; Phenomenex, CA, USA) at a constant temperature (35°C). Analysis was carried out using the gradient elution method at a flow rate of 1mL/min using a mobile phase comprised of trifluoroacetic acid solution: acetonitrile mixture (at a mix ratio of 70:30 and 40:60 at 20 minutes later). UV absorbance was observed at 280nm.

The measured purification chromatogram for Conjugate 66 is as shown in FIG. 13. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugate 66 was at least 95%.

3) Confirming molecular weight

The molecular weight of the final material obtained from Conjugate 66 was measured.

The molecular weight was confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. The results of analysis are as shown in Table 15.

**Conjugate 69**

1) Preparation example

The conjugate of Table 16 below was prepared using the following method:
After dissolving the polypeptide of SEQ ID NO: 42 from Table 6 above in a pH 7.8 phosphate buffer solution, biotin moieties were prepared by dissolving in DMSO, and the materials were reacted at a volume ratio of 90: 10. They were

reacted for at least 60 minutes at room temperature with a mole ratio of 1:22. Then, a mixture of the polypeptide-biotin moiety mixture and the fatty acid moieties from Table 5 above was prepared at a mole ratio of 1:9, and reacted for at least 60 minutes at room temperature. The reaction was slowed by lowering the storage temperature of the reaction product to 4 degrees, followed immediately by purification.

**[Table 16]**

| Conjugate (SEQ ID NO) | Polypeptide | | Biotin Moiety | | Fatty Acid Moiety | | Molecular Weight (g/mol) |
|---|---|---|---|---|---|---|---|
| | SEQ ID NO | Sequence | No. | Binding Site | No. | Binding Site | |
| 69 | 42 | MFPTIPLSRLFDNA MLRAHRLHQLAFD TYQEFEEAYIPKEQ KISFLQNPQTSLCFS ESIPTPSNREETQQ KSNLELLRISLLLIQ SWLEPVQFLRSVF ANSL VYGASDSNV YDLLKDLEEGIQTL MGRLEDGSPRTGQ IFKQTYSKFDTNSH NDDALLKNYGLLY CFRKDMDKVETFL RIVQCRSVEGSCGF | B38 | Lys random | F16 | Lys random | |

2) Isolation, purification and purity check

The reverse phase high performance liquid chromatography method was used to confirm that the reaction had proceeded and the applicable materials had been generated for Conjugate 69. Purification was carried out using an ultracentrifugal filter having an appropriate molecular weight cutoff.

The measured purification chromatogram for Conjugate 69 is as shown in FIG. 15. Further, analyzing with a UV detector, peaks other than that of the polypeptide bonded to a biotin moiety and the fatty acid moiety were not observed. Representing the area of the peaks from the respective chromatograms as a percentage, it was confirmed that the purity of Conjugate 69 was at least 95%.

3) Confirming molecular weight

The molecular weight of the final material obtained from Conjugate 69 was measured.

The molecular weight was confirmed using the MALDI-TOF mass spectrometry method. As the matrix solution, a solution of CHCA (a-Cyano-4-hydroxycinnamic acid) saturated in 50% acetonitrile containing 0.1% TFA was used. The mass spectrum was confirmed in linear and positive mode, and the molecular weight was confirmed by setting the concentration of the final material to 0.1 mg/mL. Using the mean molecular weight before and after the reaction, the average number of bonds of the biotin moiety was found. Conjugate 69 was found to have an average number of biotin bonds of 10 to 20. The results of analysis are as shown in Table 16.

**Experimental example. In vitro activity test**

**Confirmation of potency against GLP-1 receptor**

Conjugates 3 and 14 through 20

The *in vitro* activity of Conjugates 3 and 14 through 20 was measured.

First, to confirm potency against GLP-1 receptor, CHO-K1 cells wherein human GLP-1 receptors are expressed were purchased from Eurofins and used. The $7 \times 10^3$ cells were distributed in each well of a 96-well plate, then cultured in a $CO_2$ incubator under a temperature condition of 37°C. After at least 24 hours, the culture fluid was removed from each well and treated for 15 minutes with 30μl 0.5mM IBMX. Then, each embodiment was treated with 0.001-1000 nM and Exendin-4, then cultured in a $CO_2$ incubator under a temperature condition of 37°C for 30 minutes. Then, a HitHunter® cAMP assay kit was used to measure the amount of cAMP (Luciferase activity) generated to calculate an $EC_{50}$ value against GLP-1 receptor. The results of measurement were as shown in Table 17.

**[Table 17]**

| Item | Activity (nM) |
|---|---|
| Exendin-4 | 1.991 |
| Conjugate 3 | 0.301 |
| Conjugate 14 | 1.155 |
| Conjugate 15 | 0.307 |
| Conjugate 16 | 0.882 |
| Conjugate 17 | 1.109 |
| Conjugate 18 | 0.347 |
| Conjugate 19 | 0.237 |
| Conjugate 20 | NA |

**Measuring absorption rate**

Conjugates 3 and 14 through 20

To confirm the absorption rates of the drug, pharmacokinetic behavior was compared.

The specimens were respectively administered into the duodenum of experimental rats (SD rat) of around 200g body weight at amounts of 100 and 500 ug/kg, then blood samples were taken. The administration dose of each specimen was dissolved in 10mM PBS (pH 7.4) containing 0.02% Polysorbate 80, then 68mg/kg and 34mg/kg of NaCDC and PG were added, respectively, then mixed for 20 minutes at 1,000rpm before administering. The change in concentration of drug in blood over time was measured using the enzyme-linked immunoassay (ELISA) method. The blood samples were collected from the jugular vein. Results were calculated as averages, and the results were as shown in Table 18.

[Table 18]

| Item | Half-life (hours) | Cmax (ng/mL) | AUC (hr*ng/mL) | Bioavailability (%) |
|---|---|---|---|---|
| Conjugate 3 | - | 34 ± 24 | 9 ± 3 | 2.4 |
| Conjugate 14 | 1.6 ± 0.3 | 65 ± 16 | 146 ± 50 | - |
| Conjugate 16 | 5.3 ± 2.1 | 1204 ± 275 | 8919 ± 7009 | 17.4 |
| Conjugate 17 | 7.9 ± 1.8 | 1961 ± 752 | 11879 ± 3709 | 5.2 |
| Conjugate 18 | 0.9 ± 0.2 | 4736 ± 2534 | 4411 ± 1593 | - |
| Conjugate 19 | 1.4 ± 0.4 | 693 ± 249 | 880 ± 368 | - |

Conjugates 15 through 17 were intravenously administered into experimental rats (SD Rat), and pharmacokinetic behavior was observed. Experimental results were as shown in Table 19 below.

**[Table 19]**

| Test Material | | Half-life (hr) | AUClast (hr*ng/mL) |
|---|---|---|---|
| Conjugate | Administration Dose (mg/kg) | | |
| 15 | 0.154 | 1.1 ± 0.1 | 2223.1 ± 81.9 |
| 16 | 0.187 | 6.5 ± 0.7 | 7439.9 ± 508.0 |
| 17 | 0.168 | 6.5 ± 1.1 | 29612.4 ± 2495.4 |

**Measuring blood glucose regulating ability**

Conjugates 3, 17 and 20

To observe glucose tolerance, an oral formulation of GLP-1 agonist was orally administered to mice, and an intraperitoneal glucose tolerance test (IPGTT) was carried out to measure blood glucose regulating efficacy.

To measure intraperitoneal glucose tolerance in an animal model, 100 μl of specimen (10ug/mouse for SEQ ID NO 1) was administered orally at -60 minutes to male mice (C57BL/6) at 9 weeks old, followed by intraperitoneal injection of 200μl glucose (2g/kg). Changes in blood glucose in blood samples collected from the tail vein at -60, 0, 20, 40, 60, 90 and 120 minutes were observed. Control Group 1 was subcutaneously administered the polypeptide having the amino acid sequence of SEQ ID NO 5, and Control Group 2 was orally administered the same.

**[Table 20]**

| Item | AUC (%) |
|---|---|
| Vehicle | 100 |
| Conjugate 3 | 56.7 ± 6.8 |
| Conjugate 17 | 68.8 ± 9.1 |
| Conjugate 20 | 53.0 ± 7.0 |

Conjugates 20 and 24

Conjugates 20 and 24 were orally administered to mice, and a glucose tolerance test was carried out to measure blood glucose regulating efficacy.

The results of measurement were as shown in FIG. 4. It was confirmed that blood glucose was substantially reduced in the groups administered Conjugates 20 and 24 compared to the untreated group.

Conjugates 53 through 54

The blood glucose regulating efficacy of Conjugates 53 through 54 were measured through glucose tolerance tests. The conjugates were orally administered to mice, followed by intraperitoneal administration of glucose after 6 hours, after which changes in blood glucose were measured.

The results of measurement were as shown in Table 7. It was confirmed that blood glucose was substantially reduced in the groups administered Conjugates 53 and 54 compared to the untreated group.

Conjugates 65 and 66

Conjugates 65 and 66 were orally administered to mice, then their blood glucose regulating efficacy was measured through glucose tolerance tests.

To measure intraperitoneal glucose tolerance in an animal model, the sample was administered orally at -20 minutes to male mice (C57BL/6) at 8 weeks old, followed by intraperitoneal injection of glucose (2g/kg). Changes in blood glucose in blood samples collected from the tail vein at -20, 0, 20, 40, 60, 90 and 120 minutes were observed. The Control Group was orally administered a polypeptide wherein the proteins are joined through disulfide bonds between the 6th and 11th cysteine of SEQ ID NO 15; the 7th cysteine of SEQ ID NO 15 and the 7th cysteine of SEQ ID NO 16;

and the 20th cysteine of SEQ ID NO 15 and the 19th cysteine of SEQ ID NO 16.

The results of measurement were as shown in FIG. 14. As shown in FIG. 14, blood glucose was substantially reduced in the group administered with conjugate compared to the untreated group.

**Measuring Caco-2 cell membrane permeability**

Conjugates 17, 51, 72 and 73

Conjugate 51 was dissolved in Hanks Balanced Salt Solution (HBSS), and Caco-2 cell membrane permeability was measured. First, to form a Caco-2 cell monolayer, $1.5 \times 10^5$ cells were dispensed per well in a 12-transwell plate, and cultured for 3 to 4 weeks under 37°C $CO_2$ conditions. For the first week, the culture medium was changed once every 2 days, and thereafter, culturing was performed changing the culture medium at 3-day intervals. Cells between 3 and 4 weeks after seeding were used for the experiment. To verify formation of a cell monolayer, the TEER value and Lucifer yellow values were measured, using only cell monolayers where the TEER value was $300\Omega \cdot cm^2$ or greater and the measured value of Lucifer yellow permeability was within 3%. The transwells to be used in the experiments were washed with transport medium (HBSS) then cultured for 1 hour in an incubator at 37°C $CO_2$, after which 200μl each of the formulation comprising the prepared agent and excipient were added to the apical side, treating the basolateral side with 1mL transport medium not containing the agent. This was followed by incubation for 2 hours in an incubator at 37°C $CO_2$. 2 hours later, samples of 1mL each were taken from the basolateral side, and the permeability coefficient (Papp value) was measured using the enzyme-linked immunoassay (ELISA) method. The permeability coefficient (Papp value) was calculated as follows, and the results of analysis are as shown in Table 21. (Here, the respective conjugates were prepared using the same method as the above preparation examples)

$$[Papp(10^{-6}, cm/s) = (dCr / dt) \times Vr / (A \times C_0)]$$

(* dCr - concentration of permeated sample, dt - duration of treatment with agent, Vr - basolateral volume, A - Transwell area, $C_0$ - initial concentration of agent added)

**[Table 21]**

| Test Material | | Permeability Coefficient ($\times 10^7$ cm/s) | Fold |
|---|---|---|---|
| Test Material | Dose (ug/mL) | | |
| Polypeptide SEQ ID NO 5 | 214.5 | 0.01 | 1 |
| Conjugate 70 | 232.0 | 1.10 | 110 |
| Conjugate 72 | 234.8 | 0.99 | 99 |
| Conjugate 17 | 280.0 | 1.55 | 155 |
| Conjugate 52 | 275.6 | 1.33 | 133 |

**Conjugates 68 and 69**

Conjugates 68 and 69 were dissolved in Hanks Balanced Salt Solution (HBSS), and accumulation in Caco-2 cells was measured. First, to measure intracellular accumulation, Caco-2 cells were dispensed in a 96-well plate at $7 \times 10^4$ per well, then cultured in a $CO_2$ incubator under a temperature condition of 37°C. 24 hours later, the culture fluid was removed from each well and washed with HBSS, followed by addition of 100μl each of the prepared drug and culturing in a $CO_2$ incubator at 37°C. 8 minutes later, each well was washed with PBS and treated with 100μl 10% formalin each, followed by reacting at room temperature. 10 minutes later, each well was washed with PBS then treated with 100μl 0.1% TRITON X-100, and reacted at room temperature. 10 minutes later, each well was washed with PBS and blocked for 1 hour using 1% BSA, then treated with HRP Anti-Growth Hormone antibody (1:1000). After 1 hour, each well was washed with PBST, and Ultra TMB substrate solution was added. 10 minutes later, each well was treated with 2N HCL stop solution, and absorbance was measured at 450nM to measure the intracellular accumulation of each material. The results are relative to hGH at 100%, and the results of measurement are as shown in Table 22 and FIG. 16. (Here, the respective conjugates were prepared using the same method as the above preparation examples)

**[Table 22]**

| Test Material | | Relative Uptake (%) |
|---|---|---|
| Test Material | Dose (ug/mL) | |
| Polypeptide SEQ ID NO 42 | 0.66 | 100 |
| Conjugate 68 | 0.69 | 110 |
| Conjugate 69 | 0.74 | 707 (*P*<0.001) |

**Measuring body weight change and feed intake**

Conjugates 58 and 59

Conjugates 58 and 59 were subcutaneously administered for 2 weeks into an obese mouse model, then body weight changes were measured.

As shown in FIG. 10, it was confirmed that body weight was substantially reduced after administering Conjugates 58 and 59 compared to the untreated group.

Conjugates 33, 36, 39, 42 and 61 through 64

After orally administering polypeptide, Conjugates 61 through 64, and Conjugates 33, 36, 39 and 42 to mice, body weight reduction and feed intake were measured over 24 hours (Here, the respective conjugates were prepared using the same method as the above preparation examples).

The results for weight reduction were as shown in Table 23 below, and the results for feed intake were as shown in FIG. 12. Whereas body weight and feed intake were reduced in the group administered single polypeptide compared to the untreated group, the group administered the conjugates exhibited outstanding body weight reduction and feed intake compared to the group administered single polypeptide.

**[Table 23]**

| Item | Weight loss (%) compared to untreated group |
|---|---|
| Polypeptide SEQ ID NO 24 | -2.63 ± 1.06 |
| Polypeptide SEQ ID NO 26 | -3.13 ± 1.05 |
| Polypeptide SEQ ID NO 28 | -1.11 ± 0.84 |
| Polypeptide SEQ ID NO 30 | -1.17 ± 0.76 |
| Conjugate 33 | -4.07 ± 1.30 |
| Conjugate 36 | -4.53 ± 0.86 |
| Conjugate 39 | -1.99 ± 0.94 |
| Conjugate 42 | -2.60 ± 0.62 |
| Conjugate 61 | -2.96 ± 4.22 |
| Conjugate 62 | -3.32 ± 1.31 |
| Conjugate 63 | -2.41 ± 0.78 |
| Conjugate 64 | -3.38 ± 1.26 |

Persons skilled in the art will be able to become aware of or confirm various equivalents to the specific examples of the present invention stated in the present specification through routine experimentation. Such equivalents are intended to be included in the appended claims. The foregoing descriptions of the present application are meant to be exemplary, and a person skilled in the art shall understand that the present application may be easily modified into other specific forms without altering the technical idea or essential characteristics. Therefore, all embodiments described in the foregoing shall be understood to be exemplary and non-limiting in all aspects. For example, component elements described as being single elements may be carried out in a distributed manner, and likewise component elements described as being distributed may be carried out in a combined manner. All changed or modified forms deduced from the meanings

and scope of the appended claims and their equivalents may be interpreted as being included in the scope of the present invention.

**[Commercial applicability]**

The present invention, having improved oral absorption, can be usefully used in the pharmaceutical field.

## Claims

1. A biologically active material conjugate wherein a biotin moiety, a fatty acid moiety or a combination thereof is bonded to a biologically active material.

2. The biologically active material conjugate of Claim 1, wherein the biologically active material is selected from the group consisting of: glucagon (Glugacon), GLP-1 (Glucagon-like peptide-1), GLP-2 (Glucagon-like peptide-2), GIP (glucose-dependent insulinotropic polypeptide) ), exendin-4, insulin, parathyroid hormone, interferon, erythropoietin, calcitonin, amylin, serotonin, rituximab, trastuzumab, uricase, tissue plasminogen activator, thymoglobin, vaccine, heparin or heparin analog, antithrombin III, filgrastim, pramlintide acetate, exenatide, eptifibatide, antivenin, IgG, IgM, HGH, thyroxine, blood clotting factors VII and VIII, glycolipids acting as therapeutic agents, and derivatives thereof.

3. The biologically active material conjugate of Claim 1, wherein the biologically active material is selected from the group consisting of: polypeptides comprised of the amino acid sequences represented by SEQ ID NOs: 1 through 14 and SEQ ID NOs: 18 through 42, and derivatives thereof.

4. The biologically active material conjugate of Claim 1, wherein the biologically active material is a polypeptide consisting of the amino acid sequences represented by SEQ ID NOs: 15 and 16 or a derivative thereof; or a polypeptide consisting of the amino acid sequences represented by SEQ ID NOs: 17 and 16 or a derivative thereof.

5. The biologically active material conjugate of Claim 1, wherein the biotin moiety is represented by General Formula A below:

[General Formula A]

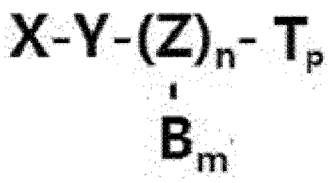

where, in General Formula A,
X is a functional group capable of binding to a biologically active material;
Y is a spacer;
Z is a binding unit;
B is represented by the following Chemical Formula A-1;

[Chemical Formula A-1]

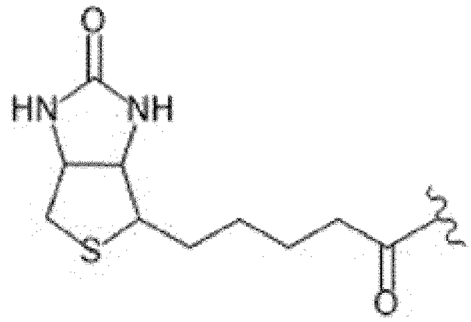

;

Z is connected with the 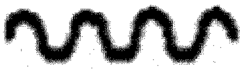 of Chemical Formula A-1;
T is a terminal group;
m is an integer of 1 to 10;
n is 0 or an integer of 1 to 10, where, when n=0, Y bonds directly with B or T; and,
p is an integer of 0 or 1.

**6.** The biologically active material conjugate of Claim 5, wherein the X is selected from the group consisting of: maleimide, succinimide, N-hydroxysuccinimide, succinimidyl succinate, succinimidyl glutarate, succinimidyl methyl ester, succinimidyl pentyl ester, succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyl, $C_{6-20}$ aryldisulfide, $C_{5-20}$ heteroaryldisulfide, isocyanate, thioester, iminoester, and derivatives thereof.

**7.** The biologically active material conjugate of Claim 5, wherein the Y is absent, or is a substituted or unsubstituted linear or branched $C_{1-50}$ alkylene, substituted or unsubstituted linear or branched $C_{1-50}$ heteroalkylene, substituted or unsubstituted $C_{6-50}$ arylene, or substituted or unsubstituted $C_{6-50}$ heteroarylene; and if substituted, comprises at least one selected from the group comprising =O, $-C(O)NH_2$, -OH, -COOH, -SH, =NH and $-NH_2$.

**8.** The biologically active material conjugate of Claim 5, wherein the Y comprises $-C(O)-(OCH_2CH_2)_u-NH-$ as a repeating unit, where u is an integer of 1 to 20.

**9.** The biologically active material conjugate of Claim 5, wherein the Y comprises glutamic acid, glutamine, glycine, isoleucine, or lysine as a component.

**10.** The biologically active material conjugate of Claim 5, wherein the Z is any one of the following, each of which may be independently selected:

A) forms an amino acid or a derivative thereof together with X or separately from X;
B) is a substituted or unsubstituted linear or branched $C_{1-50}$ heteroalkyene,

where, if substituted, comprises at least one selected from the group composed of =O, $-C(O)NH_2$, - OH, -COOH, -SH, =NH and $-NH_2$

**11.** The biologically active material conjugate of Claim 5, wherein the T is selected from the group composed of: amine, $C_{1-8}$ alkyl, $C_{1-8}$ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.

**12.** The biologically active material conjugate of Claim 1, wherein the biotin moiety is selected from the group composed of:

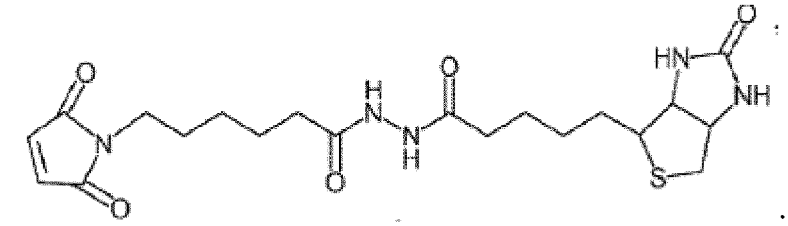

;

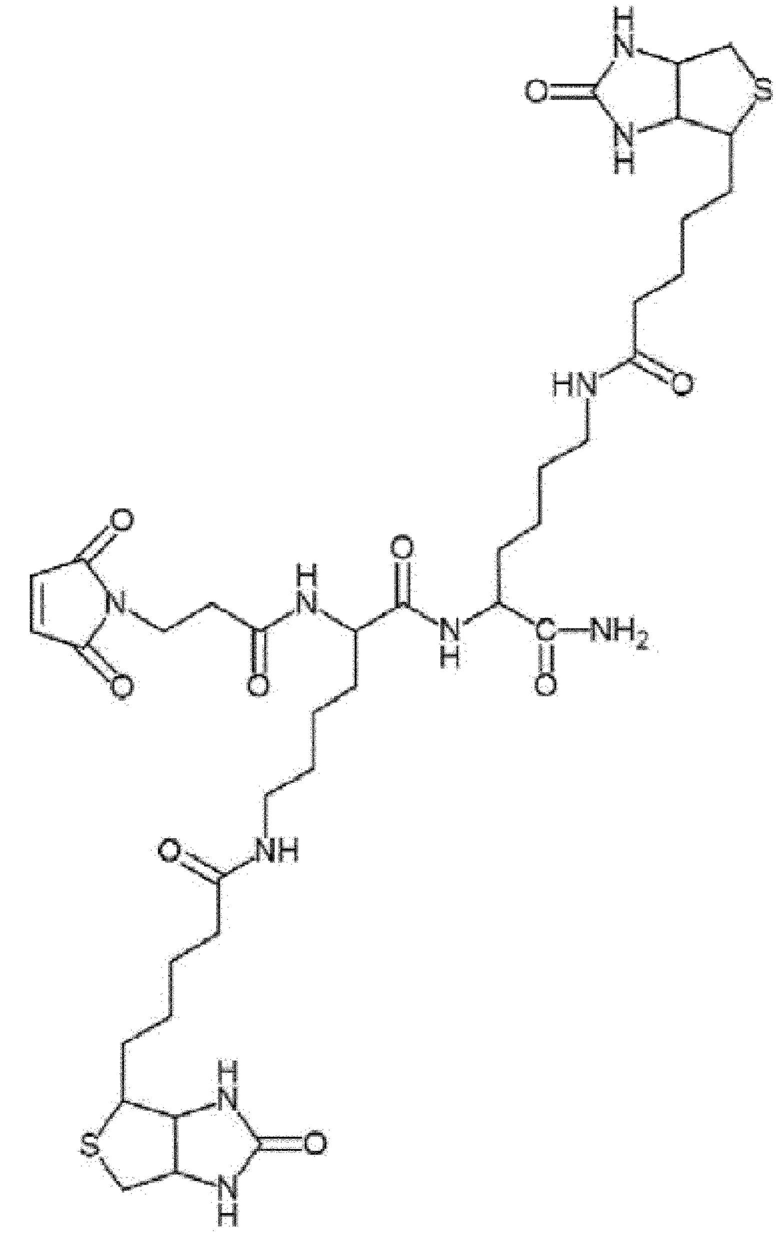

;

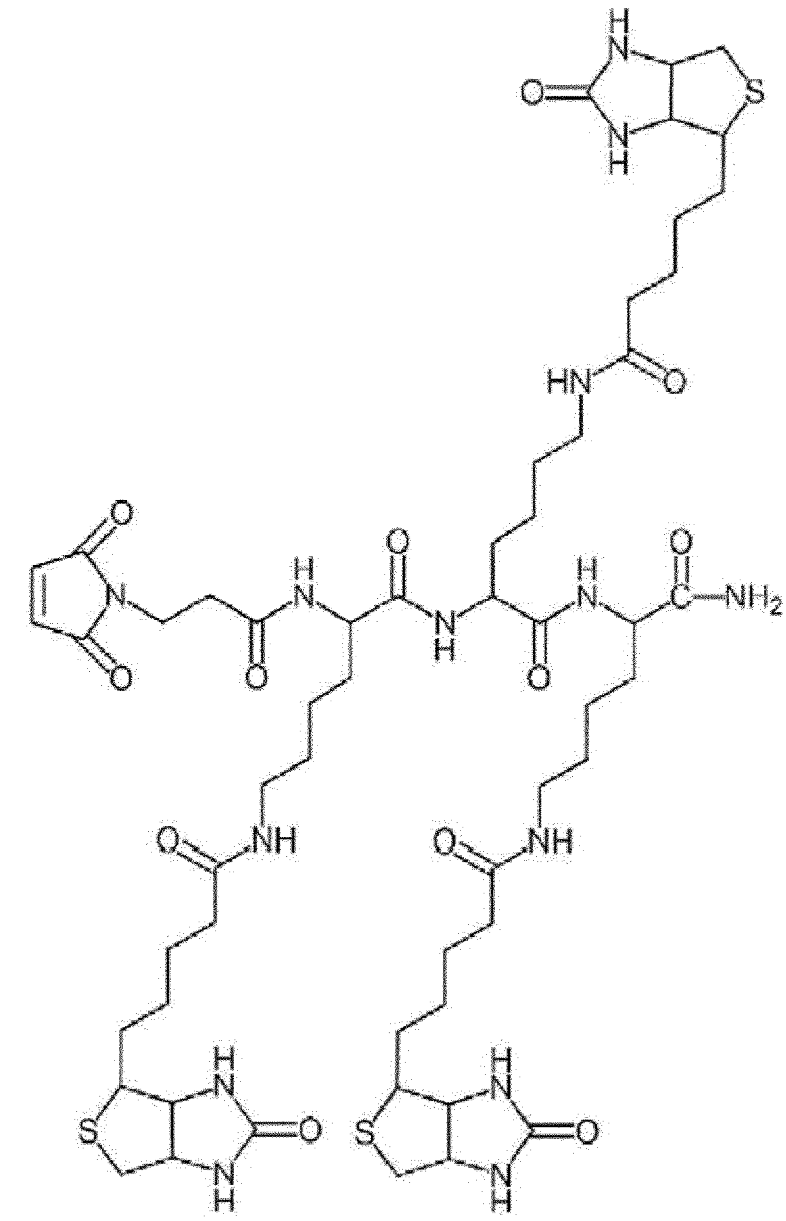

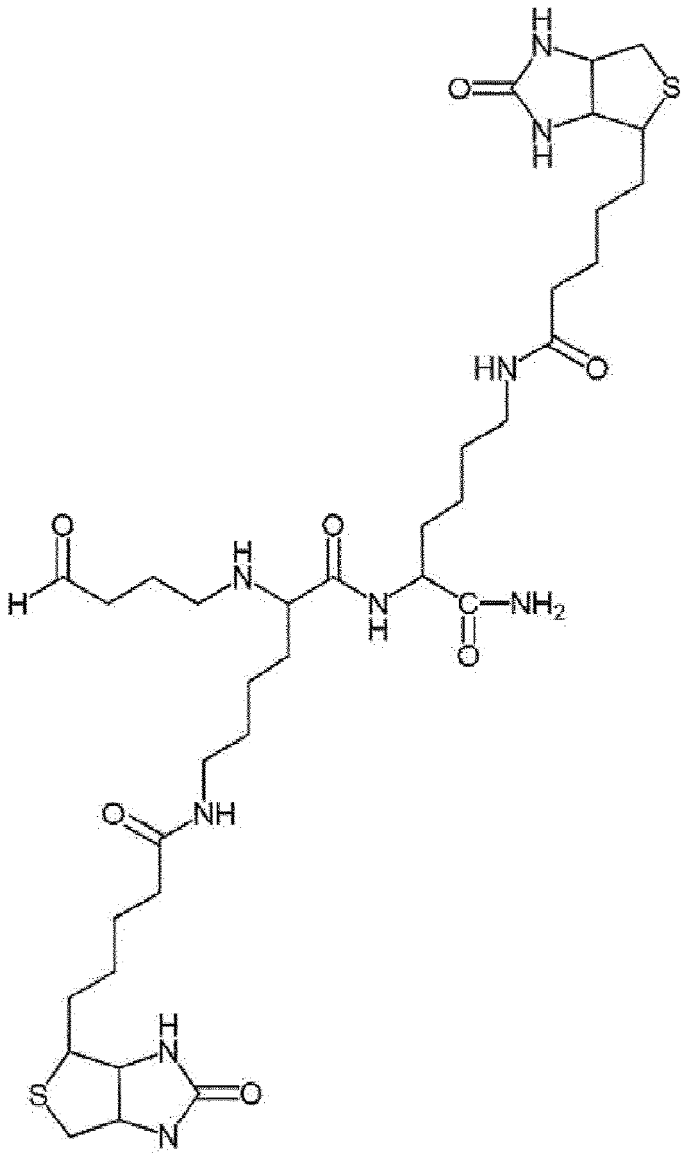

;

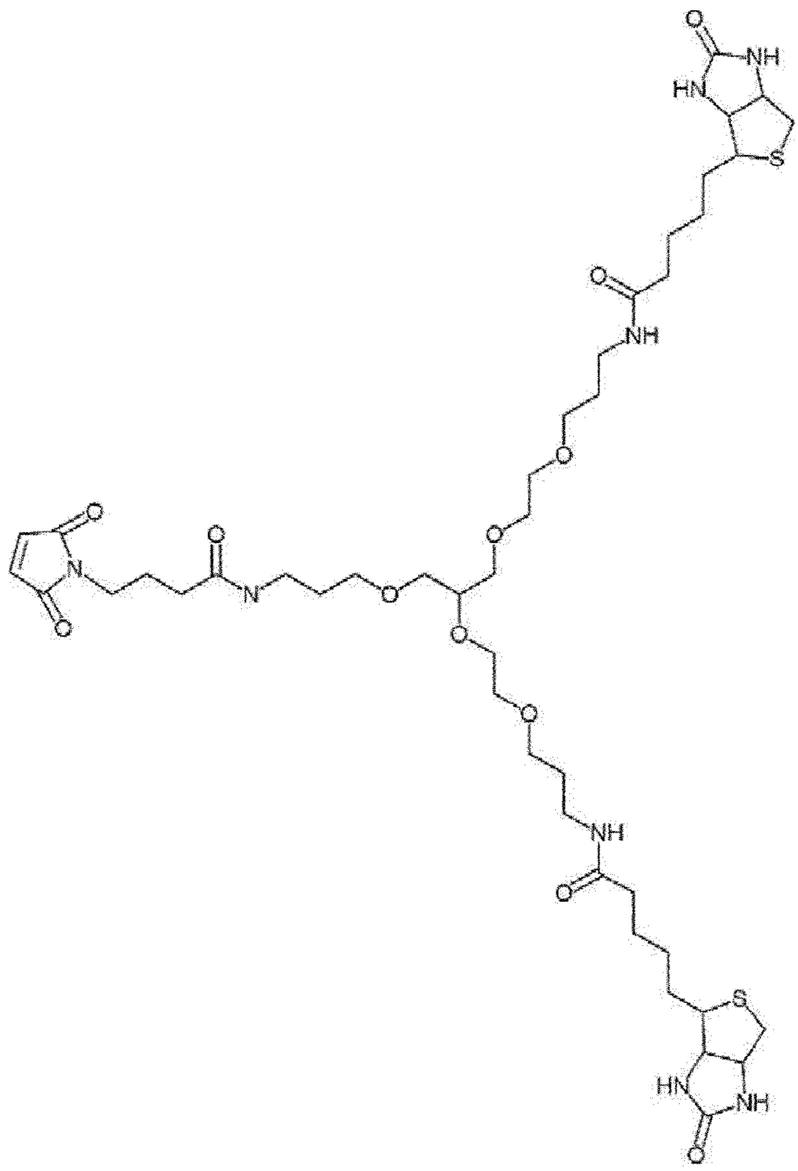

;

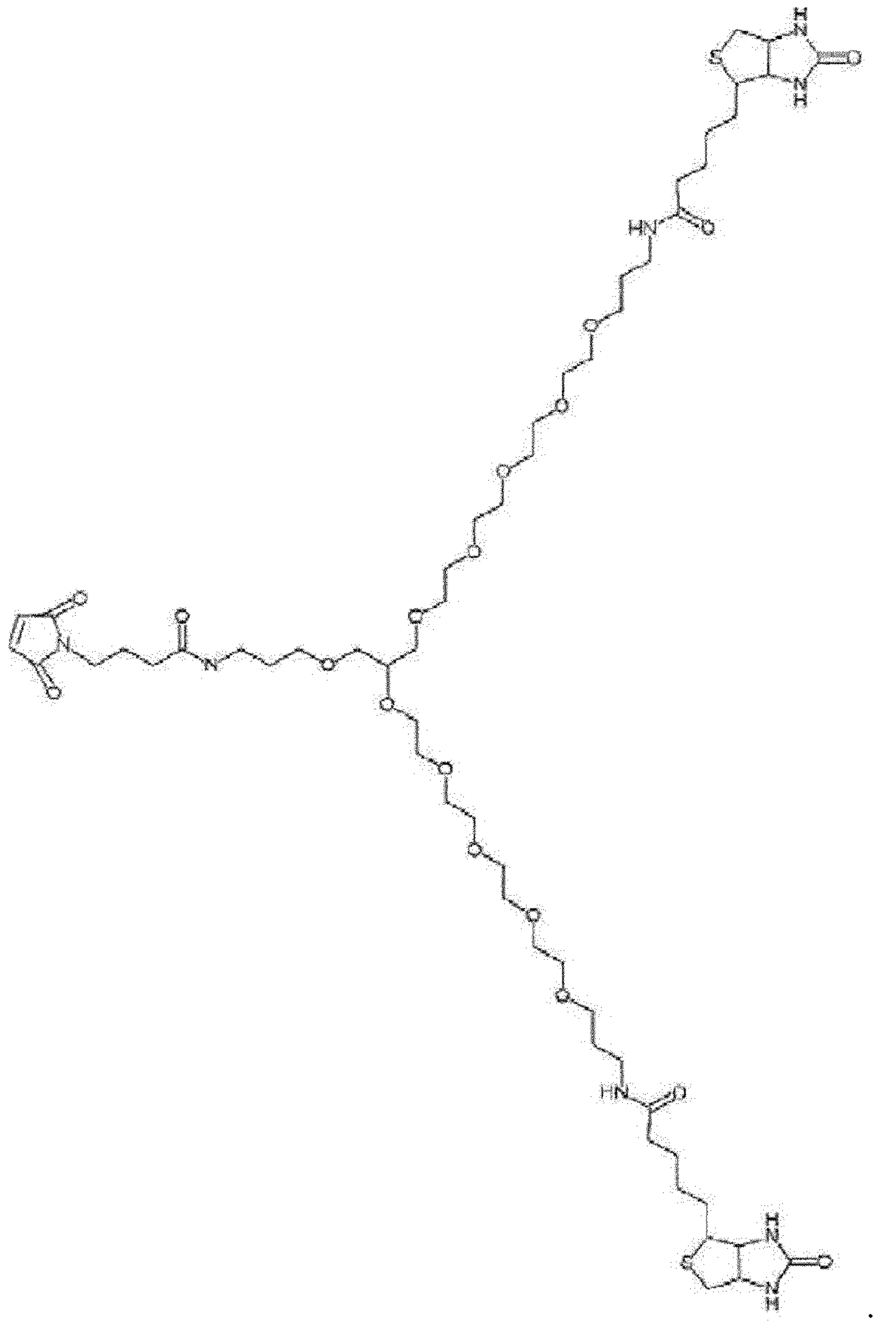

;

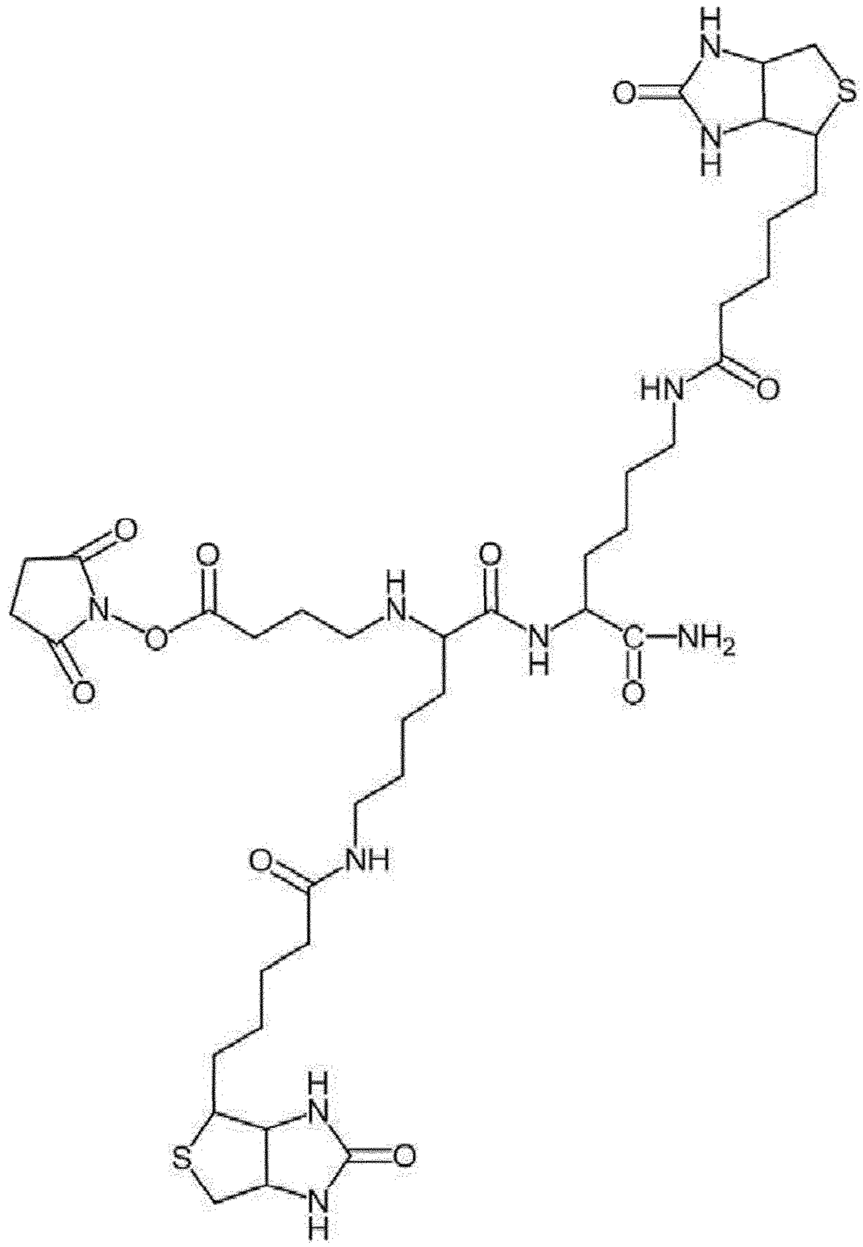

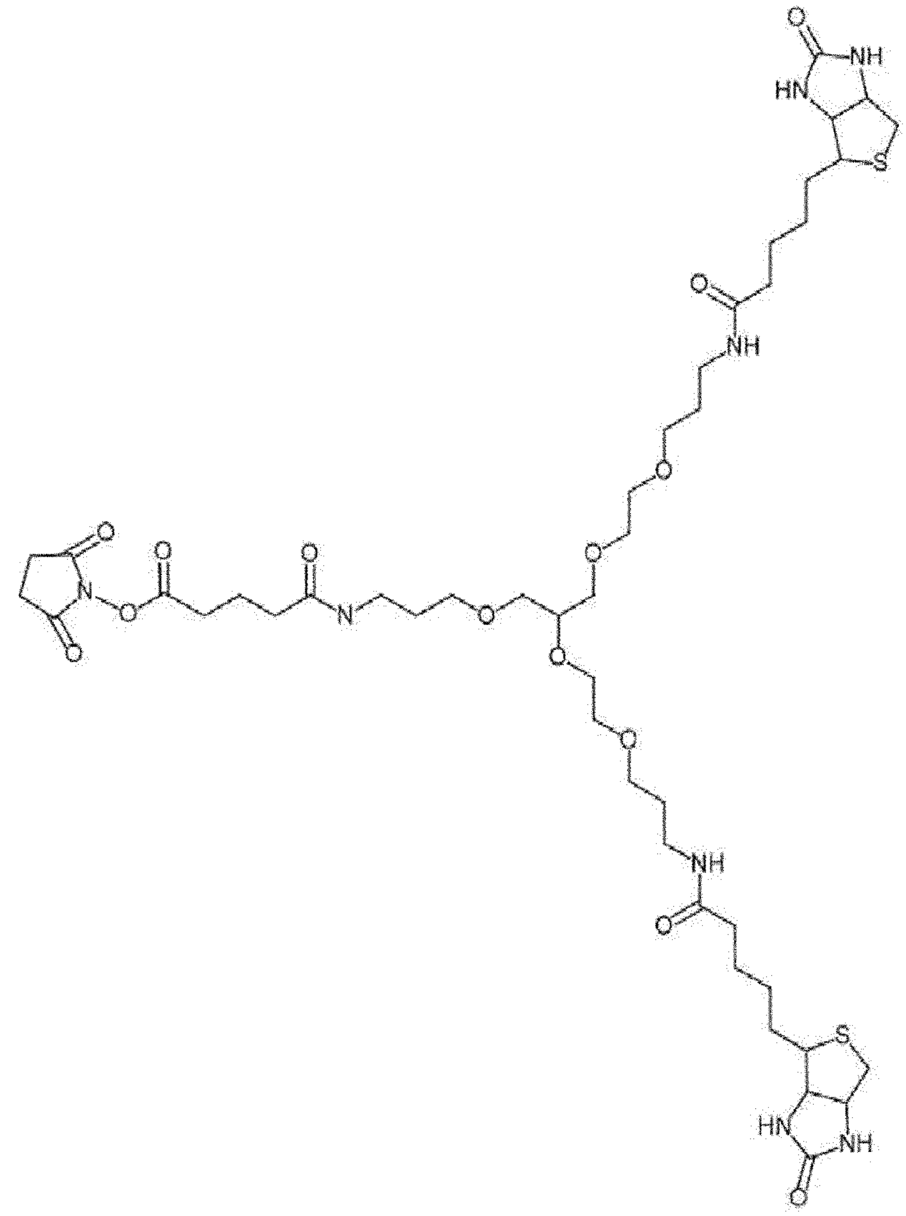

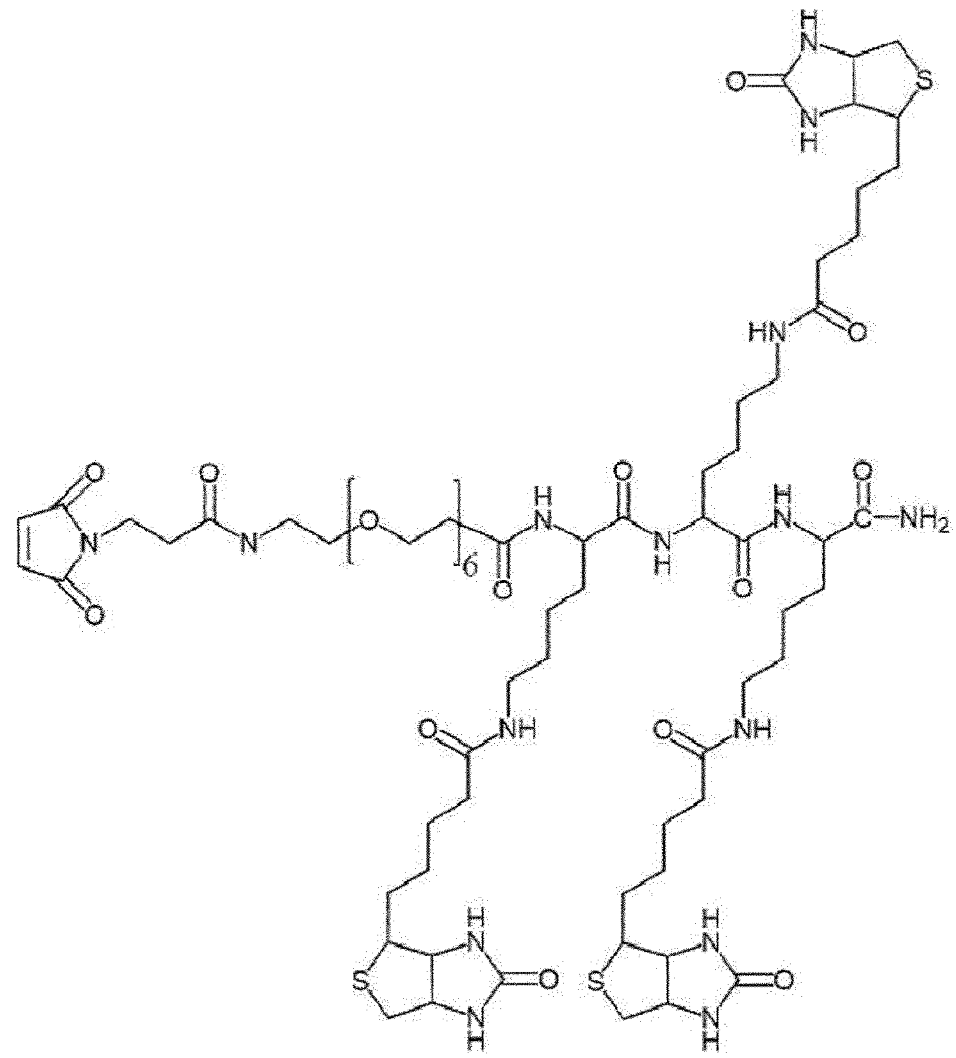

;

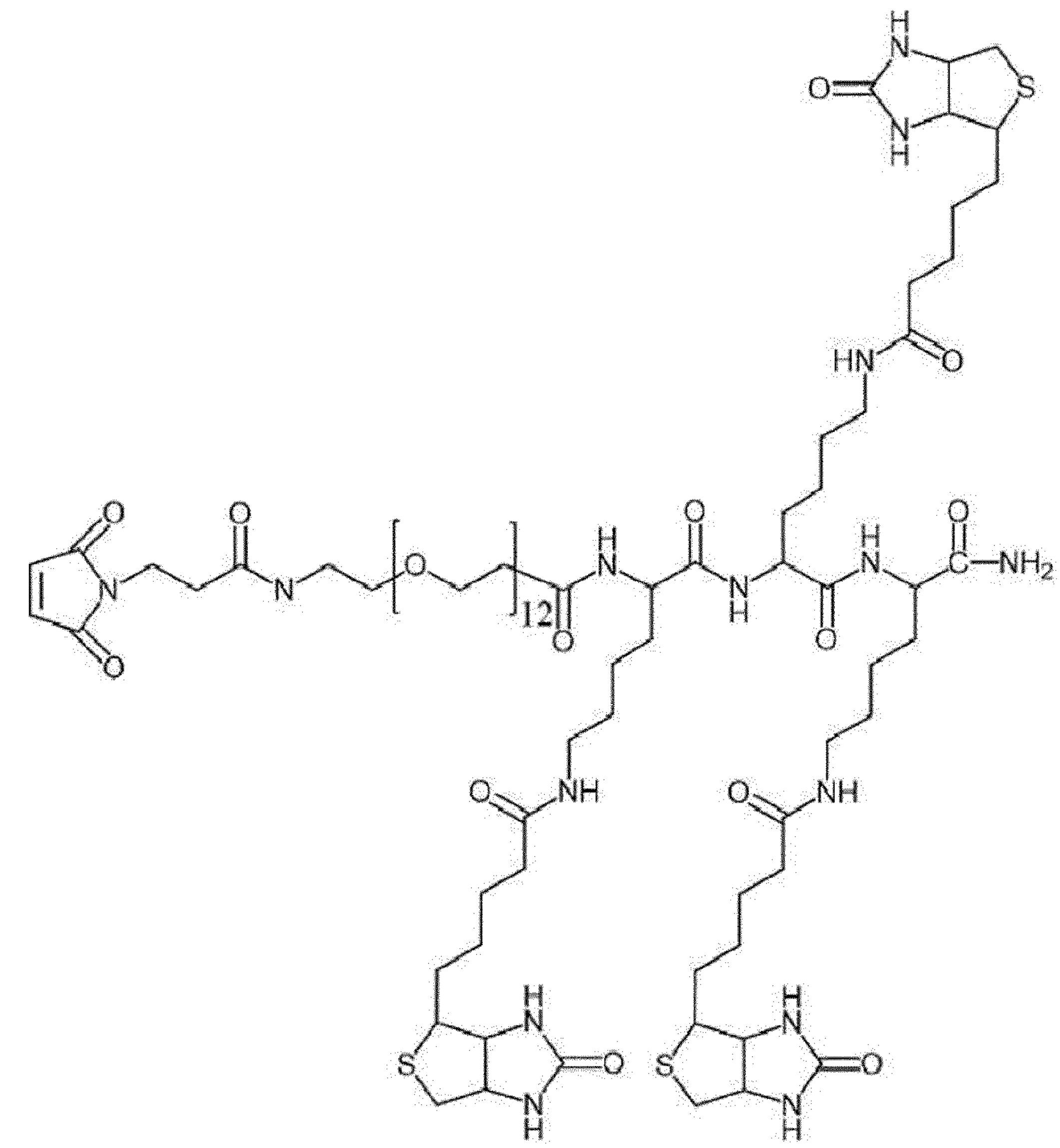

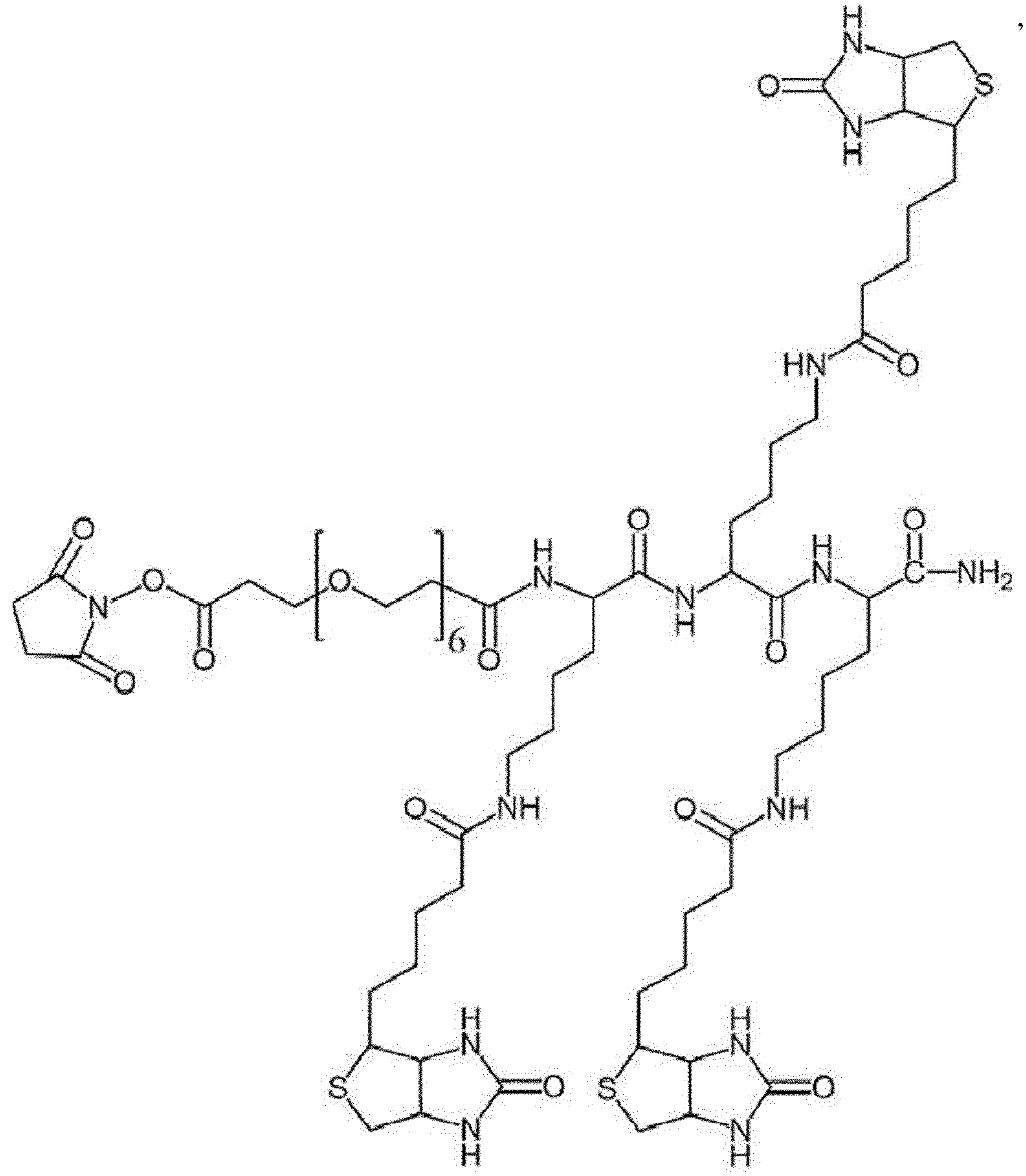

,

;

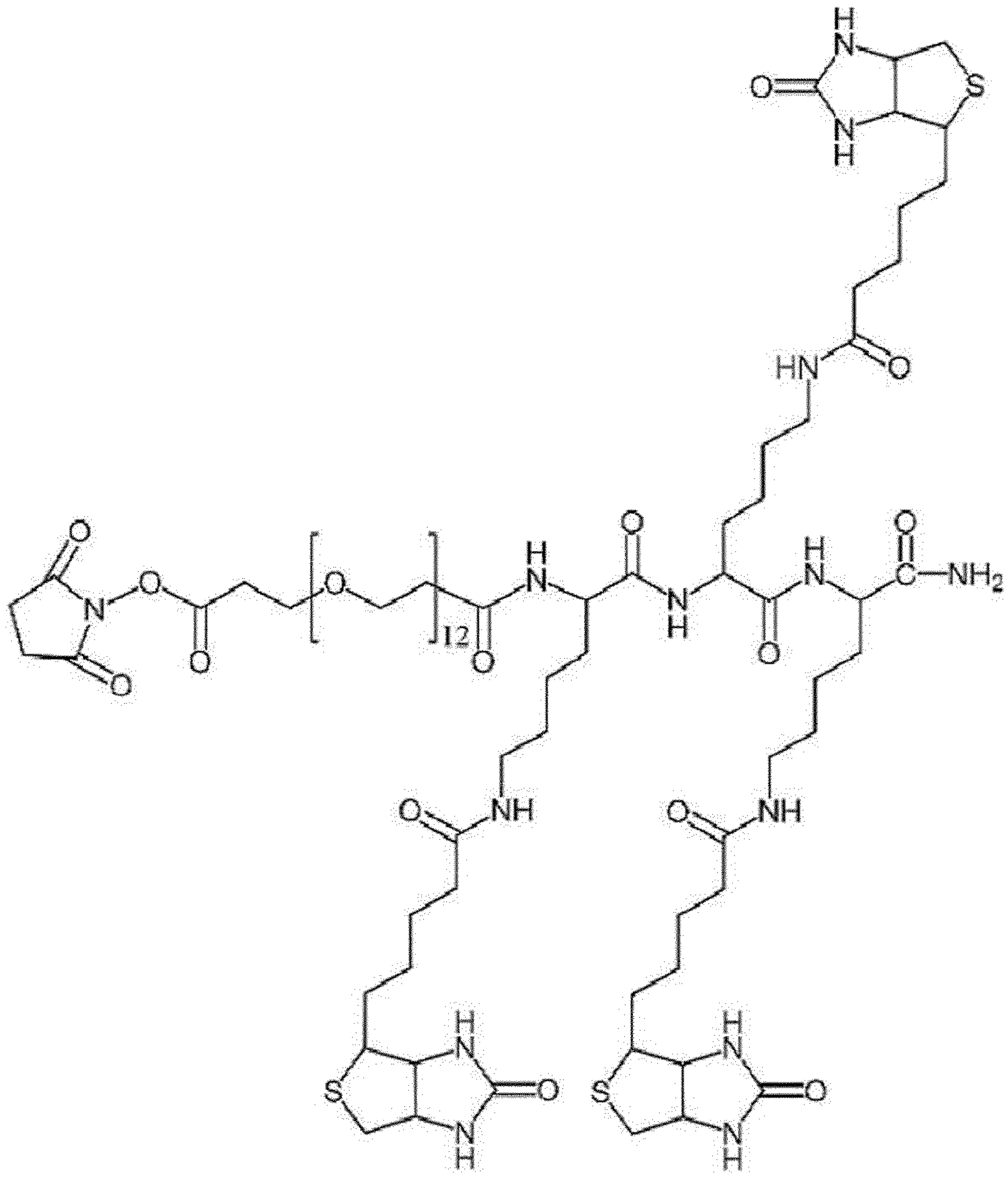

,

;

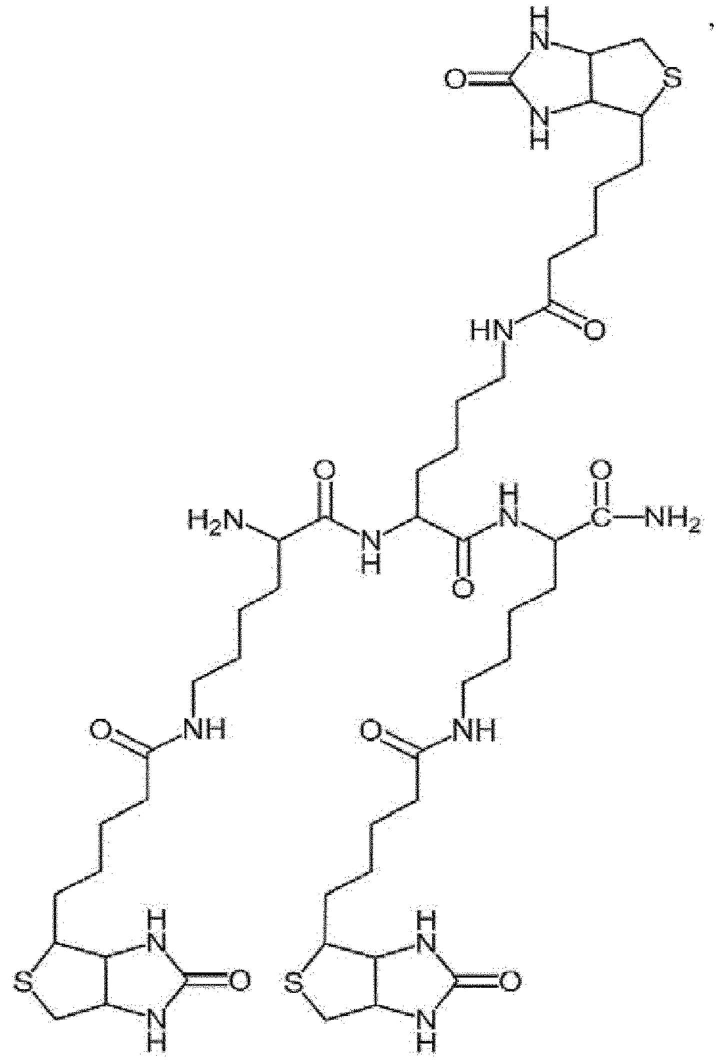

;

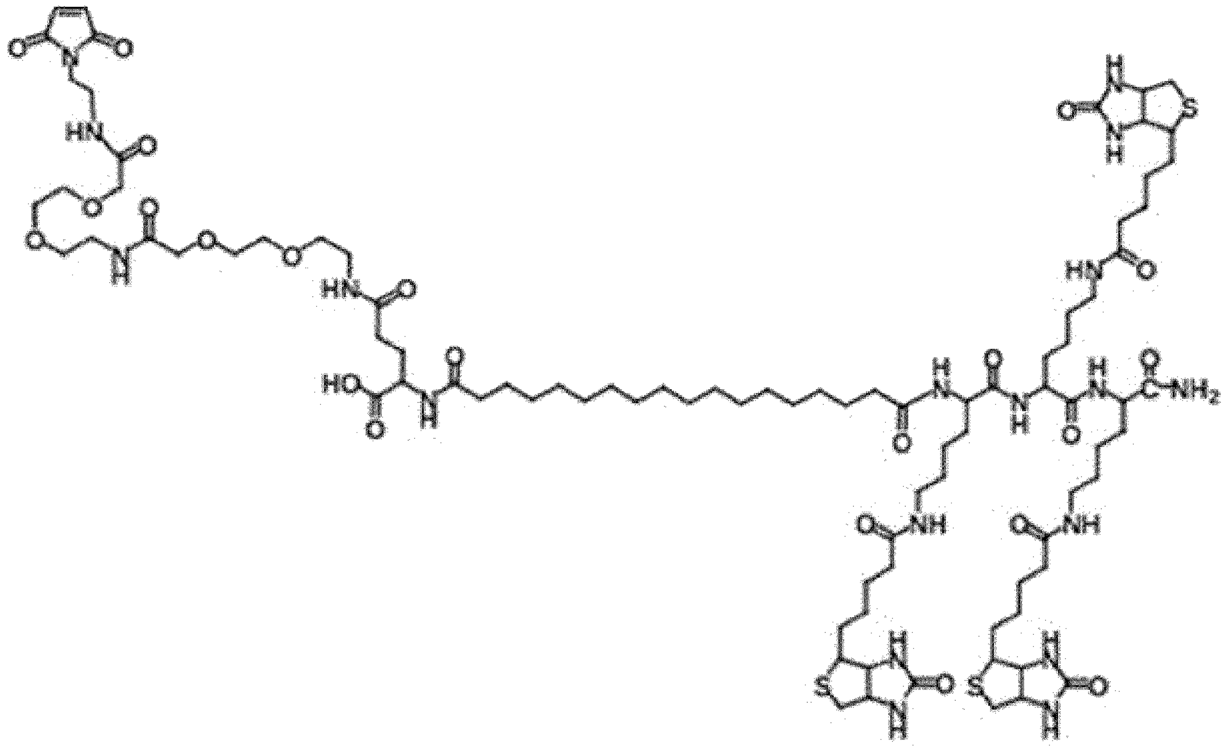

;

,

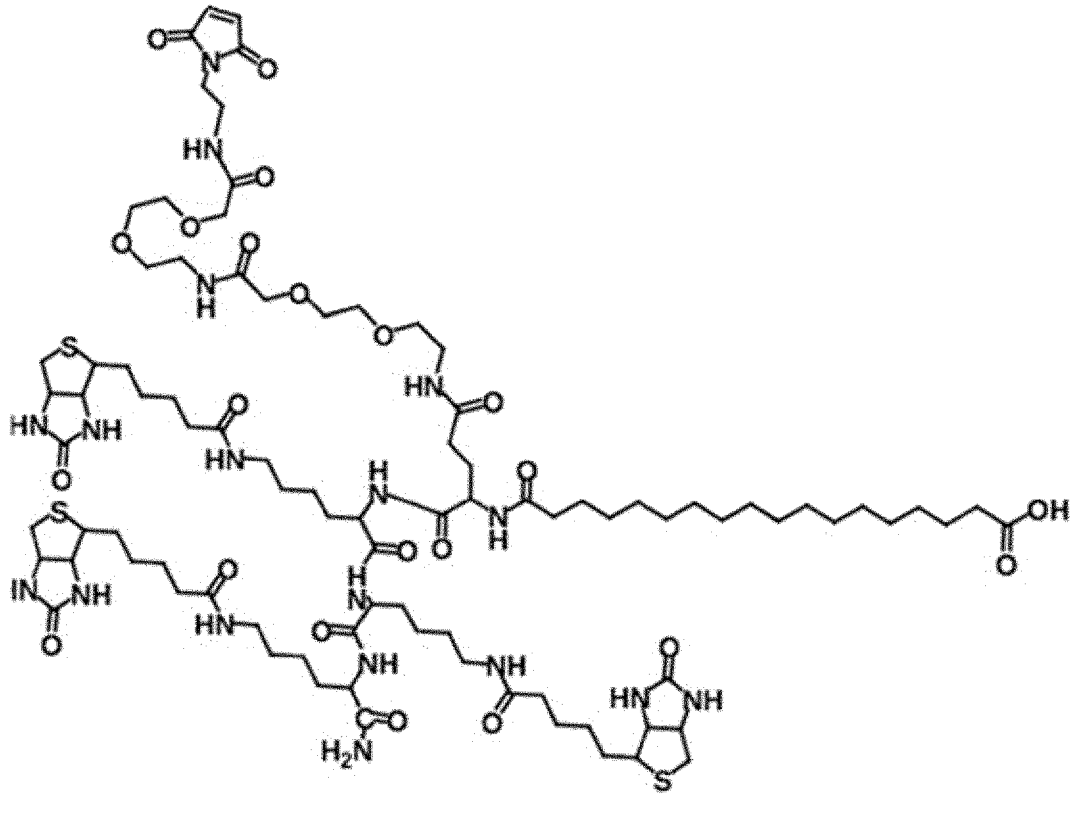

,

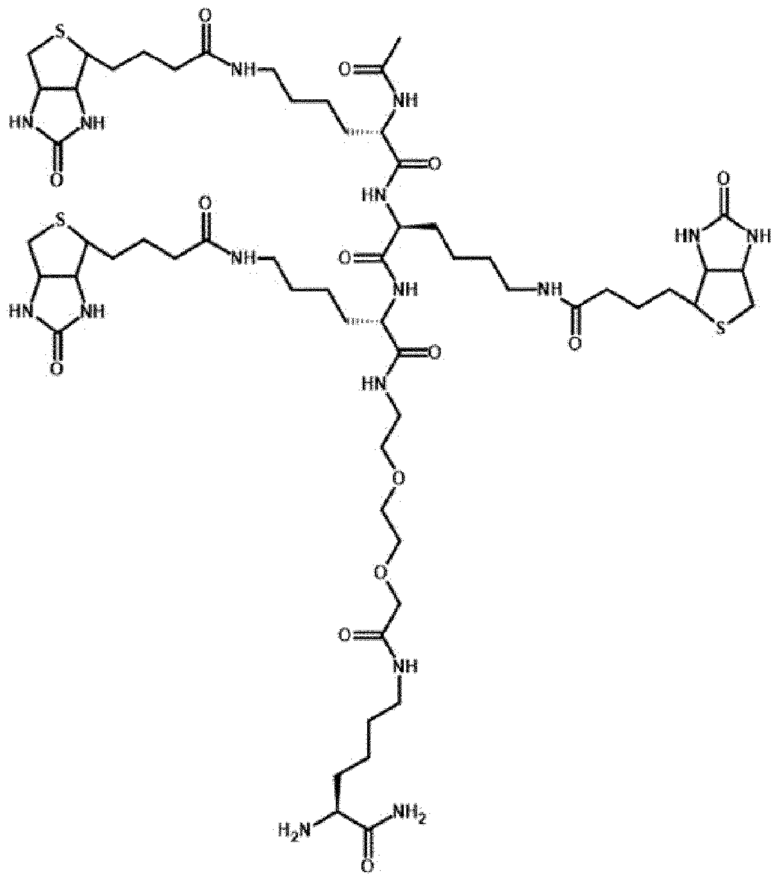

;

and

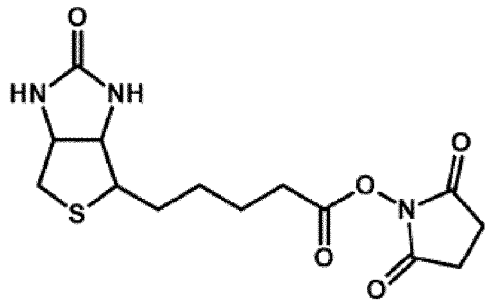

.

**13.** The biologically active material conjugate of Claim 1, wherein the fatty acid moiety is bonded to the biologically active material, and is bonded to a site of the biologically active material other than the site to which the biotin moiety is bonded.

**14.** The biologically active material conjugate of Claim 1, wherein the fatty acid moiety is represented by General Formula B below:

[General Formula B] X'-Y'-W

where, in the above formula,
X' is a functional group capable of binding to a the biologically active material;
Y' is a spacer; and
W is a fatty acid.

**15.** The biologically active material conjugate of Claim 14, wherein the X' is selected from the group consisting of maleimide, succinimide, N-hydroxysuccinimide, succinimidyl succinate, succinimidyl glutarate, succinimidyl methyl ester, succinimidyl pentyl ester, succinimidyl carbonate, p-nitrophenyl carbonate, aldehyde, amine, thiol, oxyamine, iodoacetamide, aminooxyl, hydrazide, hydroxy, propionate, pyridyl, alkyl halide, vinyl sulfone, carboxyl, hydrazide, halogen acetamide, $C_{2-5}$ alkynyl, $C_{6-20}$ aryldisulfide, $C_{5-20}$ heteroaryldisulfide, isocyanate, thioester, iminoester, tetrafluorophenyl ester, nitrophenyl carbonate, nitrophenyl and derivatives thereof.

**16.** The biologically active material conjugate of Claim 14, wherein the W is a substituted or unsubstituted linear or branched $C_{1-60}$ alkylene, substituted or unsubstituted linear or branched $C_{1-60}$ alkenylene, substituted or unsubstituted linear or branched $C_{1-60}$ heteroalkylene, or substituted or unsubstituted linear or branched $C_{1-60}$ heteroalkenylene, and if substituted, is substituted by at least one selected from the group comprising =O, -C(O)$NH_2$, - OH, -COOH, -SH, =NH, -$NH_2$, and halo.

**17.** The biologically active material conjugate of Claim 1, wherein the fatty acid moiety is represented by General Formula B1 below:

[General Formula B1] $X'_1$ -Y'-C(O)-$F_1$

where, in the above formula,
$X'_1$ is maleimide, N-hydroxysuccinimide, aldehyde, amine, tetrafluorophenyl ester or nitrophenol;
Y' is a spacer; and,
$F_1$ is a $C_{6-28}$ substituted or unsubstituted linear or branched alkylene, or substituted or unsubstituted linear or branched heteroalkylene.

**18.** The biologically active material conjugate of Claim 14 or Claim 17, wherein the Y' is a direct bond, or the structure of Y comprises at least one of the group comprising substituted or unsubstituted $C_{1-50}$ linear alkylene, substituted or unsubstituted $C_{1-50}$ non-linear alkylene, substituted or unsubstituted $C_{1-50}$ Linear heteroalkylene, substituted or unsubstituted $C_{1-50}$ nonlinear heteroalkylene, substituted or unsubstituted $C_{1-50}$ arylene, substituted or unsubstituted $C_{1-50}$ heteroarylene, -O-, -C (O), -C(O)NR-, -C(O)O-, -S-, -NR- or -NOR-, wherein R is hydrogen, or unsubstituted $C_{1-50}$ alkyl, substituted or unsubstituted $C_{1-50}$ aryl, or an ethylene glycol repeating unit (-$(CH_2CH_2O)_n$-, where n is an integer of at least 1 but not more than 20).

**19.** The biologically active material conjugate of Claim 14 or Claim 17, wherein the Y' comprises -C(O)-$(OCH_2CH_2)_u$-NH- as a repeating unit, where u is an integer of 1 to 20.

**20.** The biologically active material conjugate of Claim 14 or Claim 17, wherein the Y' comprises glutamic acid, glutamine, glycine, isoleucine, or lysine as a component.

**21.** The biologically active material conjugate of Claim 1, wherein the fatty acid moiety is selected from the group composed of:

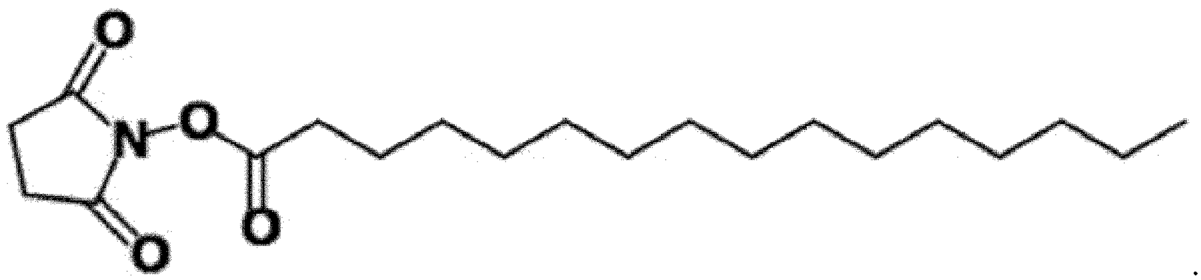

;

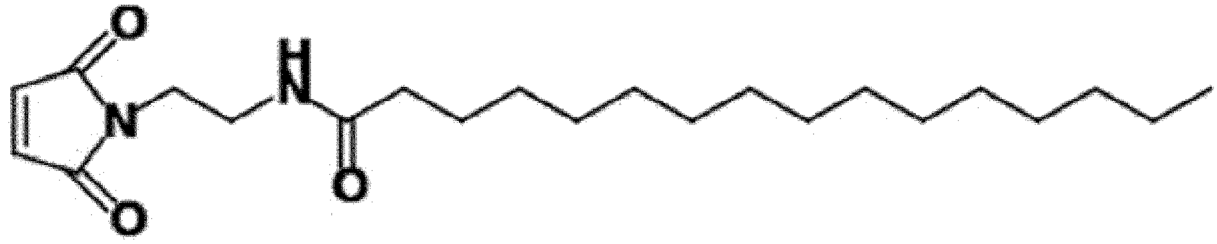

;

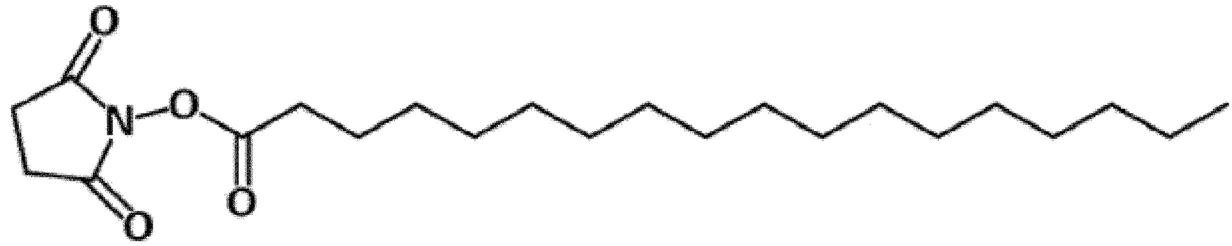

;

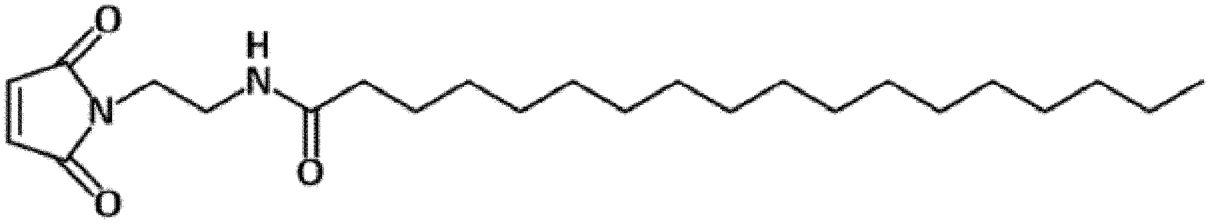

;

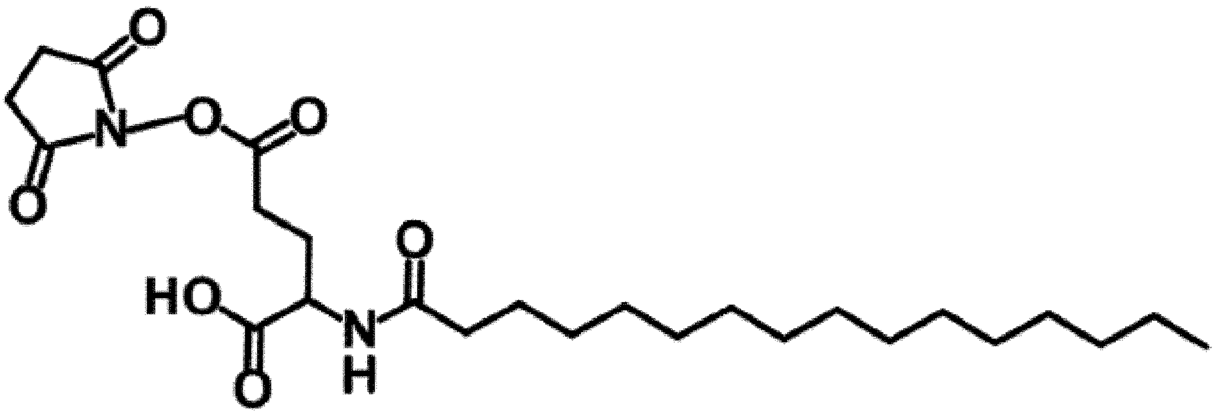

;

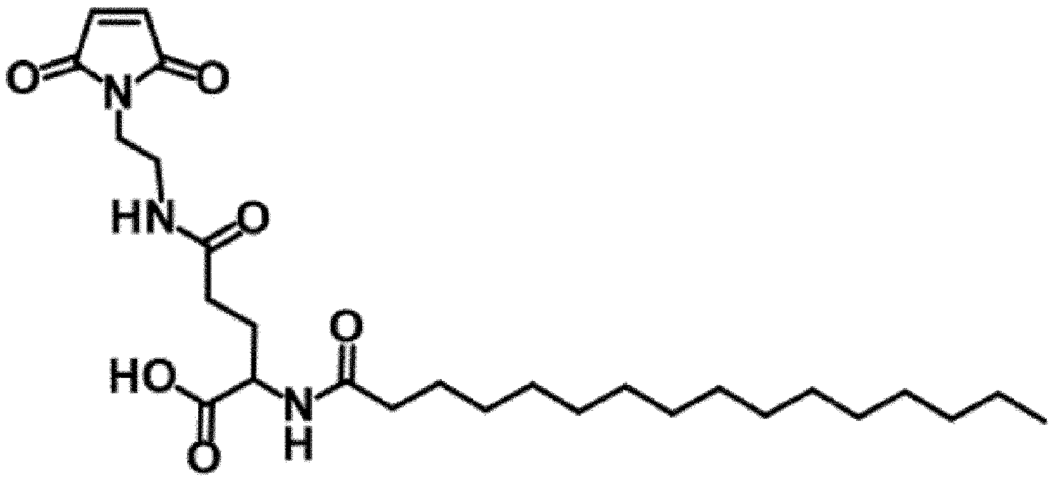

;

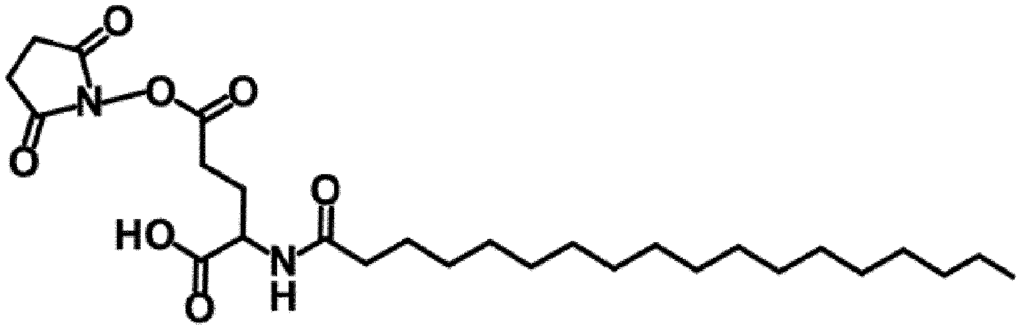

;

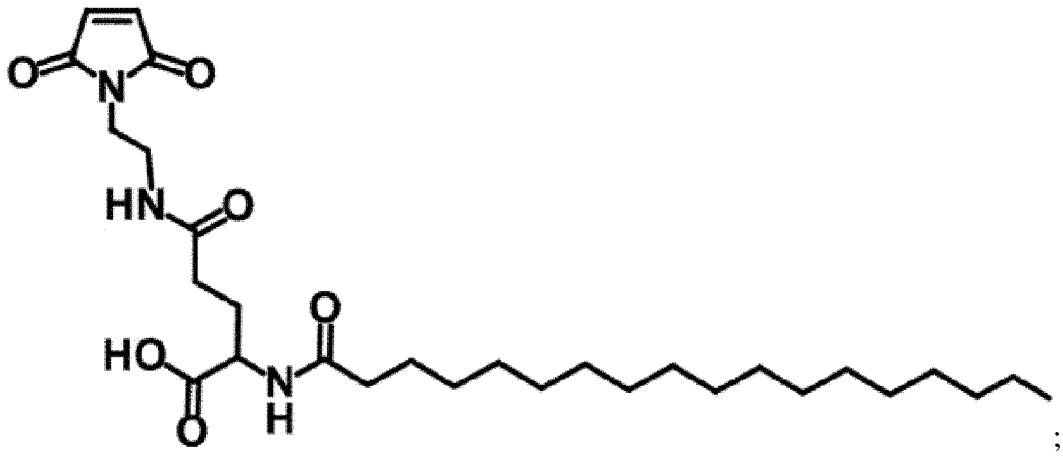

;

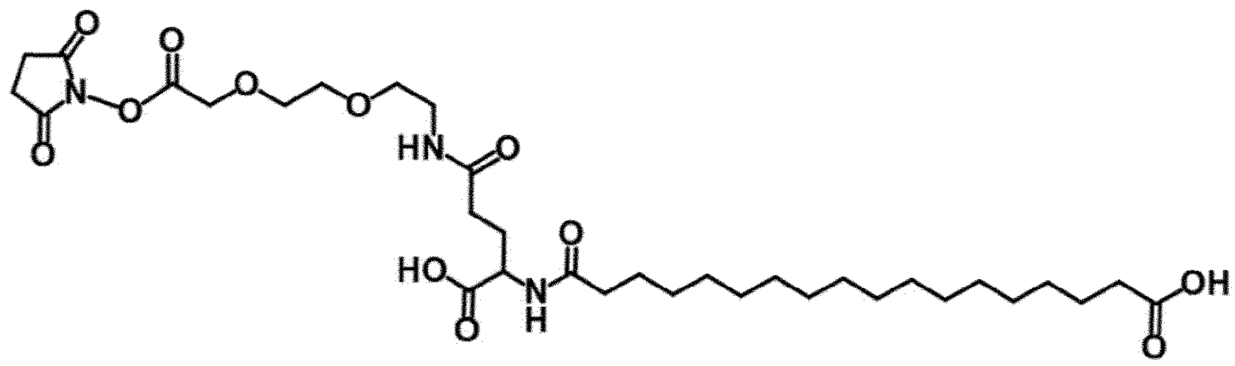

;

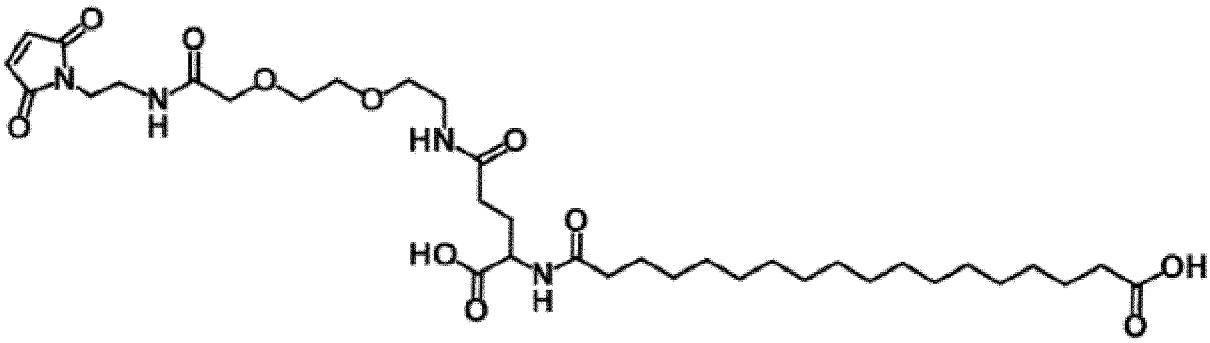

;

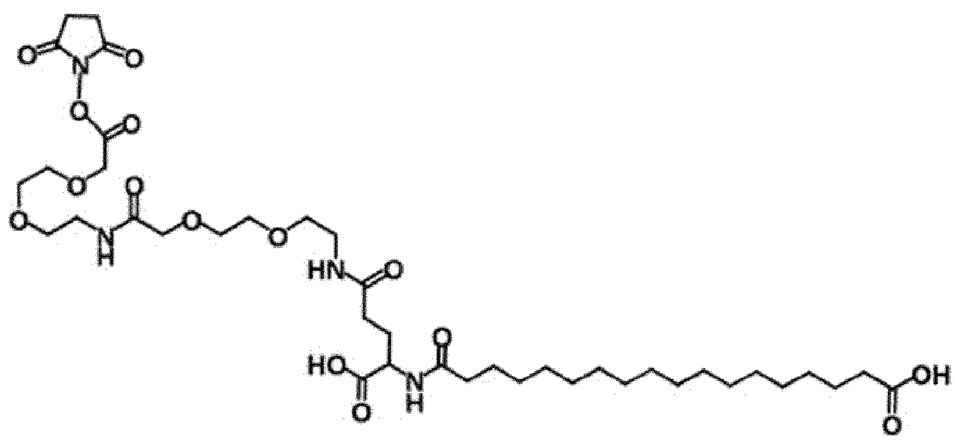

,

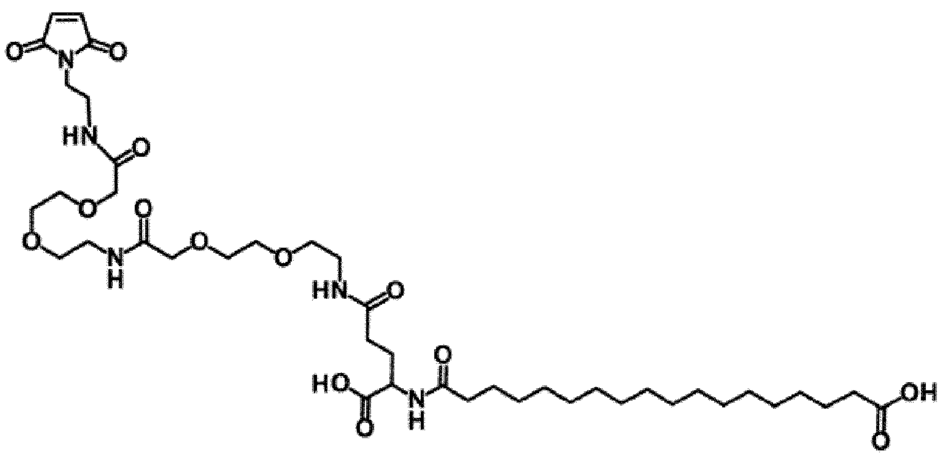

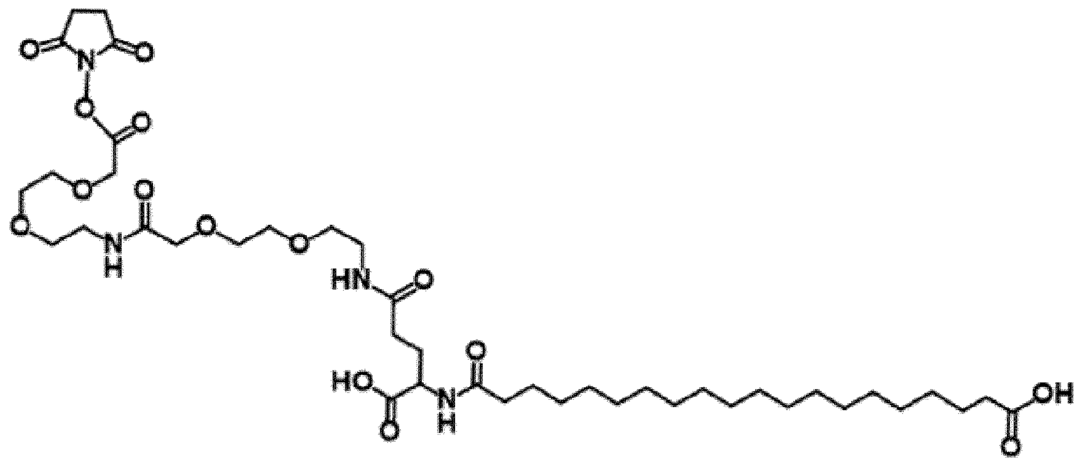

;

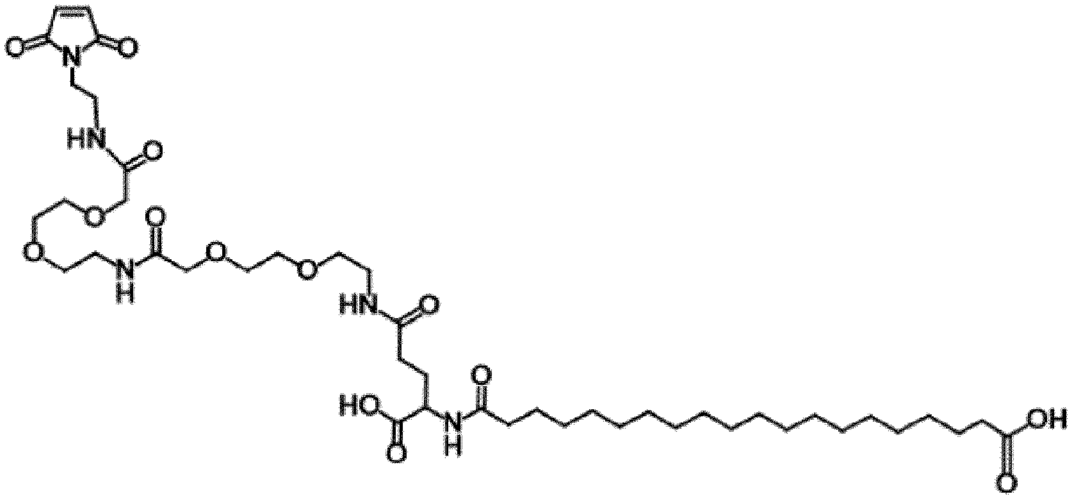

;

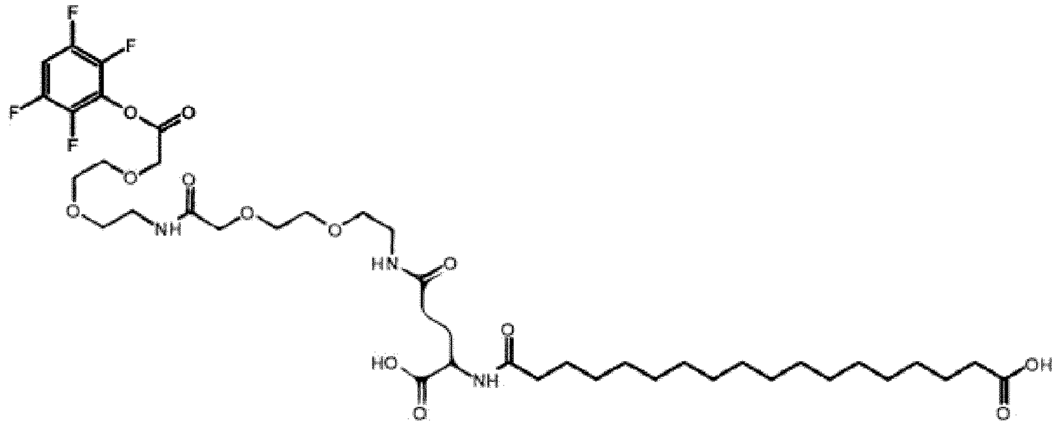

;

and

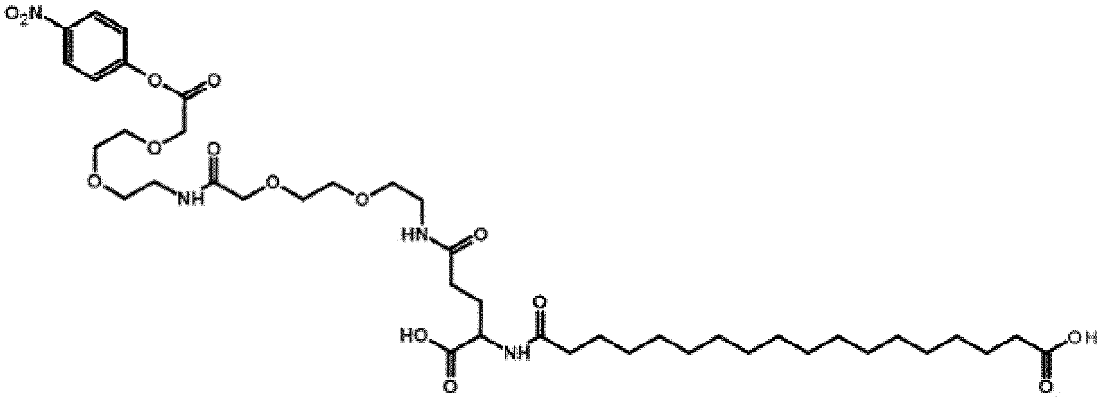

.

**22.** A pharmaceutical formulation for preventing or treating diabetes, obesity, fatty liver disease, irritable bowel syndrome, neurodegenerative disease, bone disease, osteoporosis, human growth hormone deficiency, cancer or non-alcoholic fatty liver disease, the formulation comprising the biologically active material conjugate of any one of Claim 1 through Claim 21.

**23.** A formulation for oral administration comprising the biologically active material conjugate of any one of Claim 1 through Claim 21.

CHROMATOGRAM

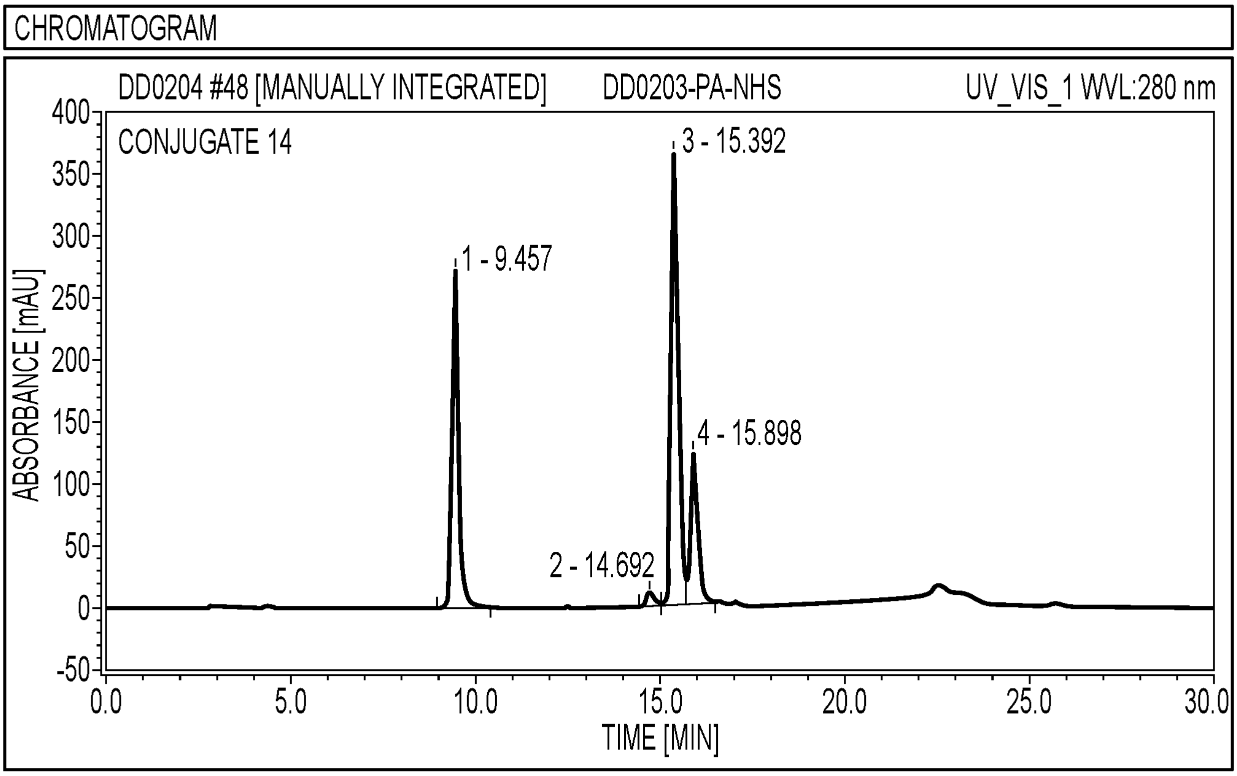


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 9.457 | 55.698 | 271.548 | 32.95 | 35.39 | N.A. |
| 2 | | 14.692 | 2.766 | 10.837 | 1.64 | 1.41 | N.A. |
| 3 | | 15.392 | 80.389 | 363.431 | 47.55 | 47.37 | N.A. |
| 4 | | 15.898 | 30.204 | 121.463 | 17.87 | 15.83 | N.A. |
| TOTAL: | | | 169.057 | 767.278 | 100.00 | 100.00 | |

*FIG. 1A*

CHROMATOGRAM

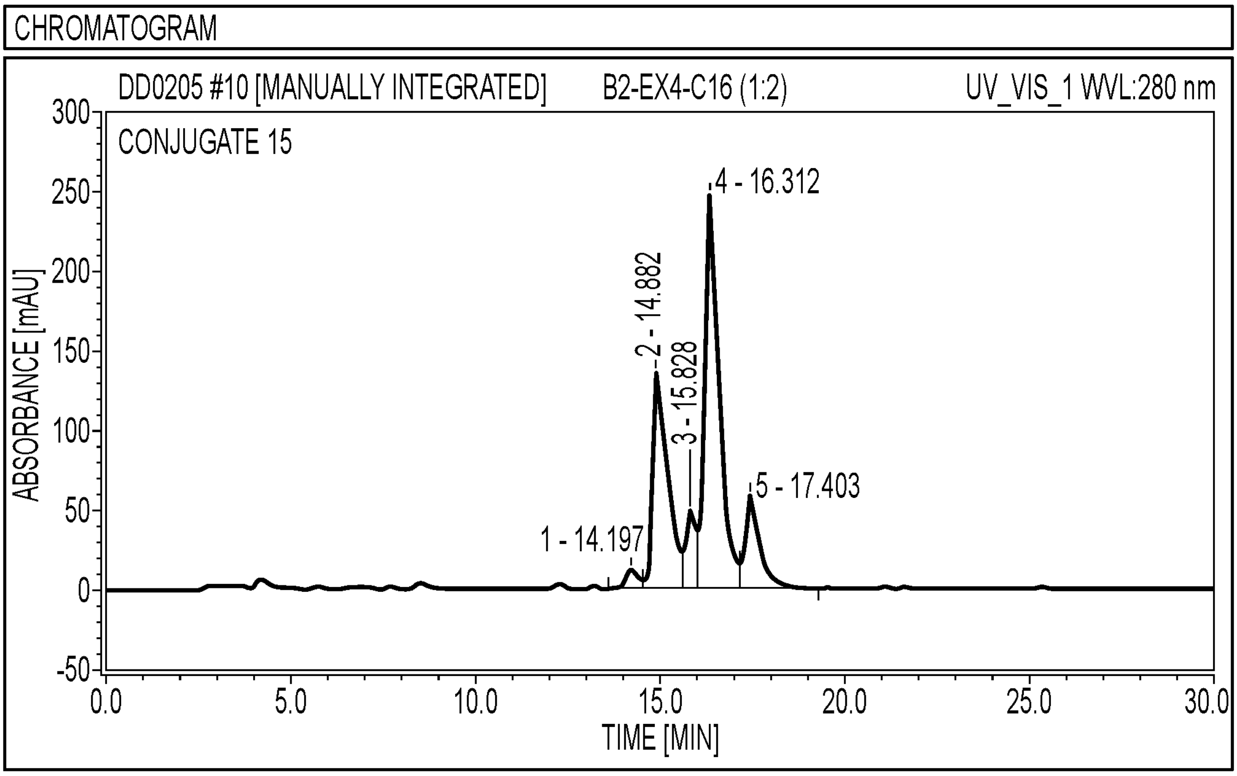


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 14.197 | 4.634 | 11.616 | 1.98 | 2.33 | N.A. |
| 2 | | 14.882 | 69.044 | 135.270 | 29.52 | 27.16 | N.A. |
| 3 | | 15.828 | 15.217 | 46.787 | 6.51 | 9.39 | N.A. |
| 4 | | 16.312 | 117.218 | 246.032 | 50.12 | 49.39 | N.A. |
| 5 | | 17.403 | 27.780 | 58.419 | 11.88 | 11.73 | N.A. |
| TOTAL: | | | 233.893 | 498.124 | 100.00 | 100.00 | |

*FIG. 1B*

CHROMATOGRAM

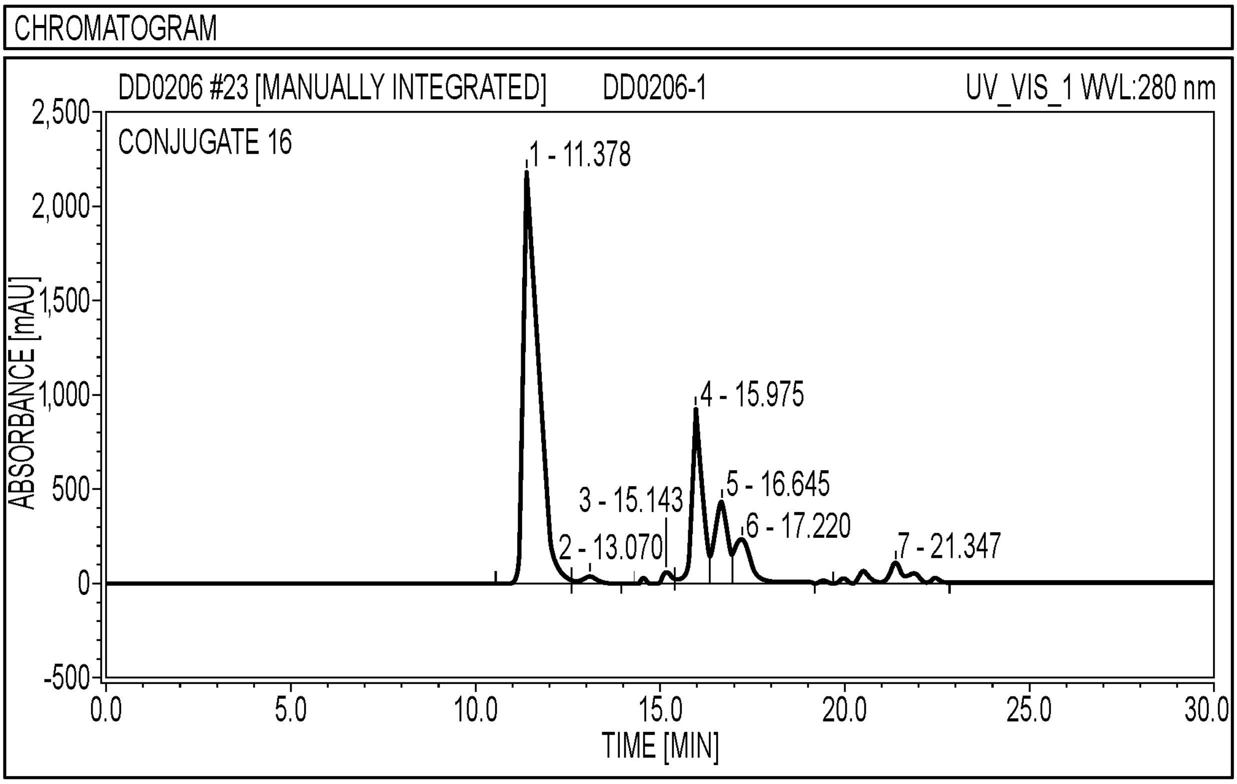


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 11.378 | 1104.407 | 2176.515 | 60.82 | 55.29 | N.A. |
| 2 | | 13.070 | 17.556 | 25.597 | 0.97 | 0.65 | N.A. |
| 3 | | 15.143 | 19.633 | 59.284 | 1.08 | 1.51 | N.A. |
| 4 | | 15.975 | 313.065 | 913.948 | 17.24 | 23.22 | N.A. |
| 5 | | 16.645 | 166.236 | 432.264 | 9.16 | 10.98 | N.A. |
| 6 | | 17.220 | 118.750 | 228.021 | 6.54 | 5.79 | N.A. |
| 7 | | 21.347 | 76.080 | 101.028 | 4.19 | 2.57 | N.A. |
| TOTAL: | | | 1815.726 | 3936.657 | 100.00 | 100.00 | |

*FIG. 1C*

CHROMATOGRAM

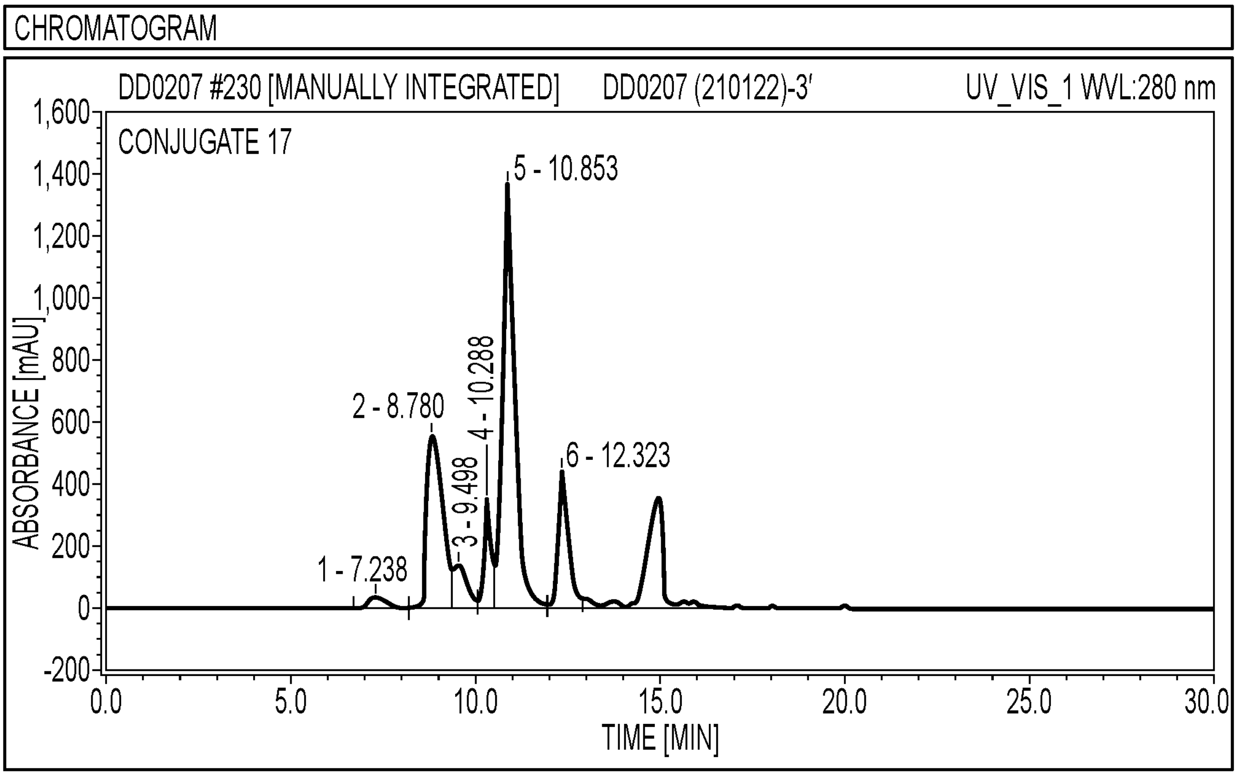


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 7.238 | 23.223 | 38.241 | 2.07 | 1.33 | N.A. |
| 2 | | 8.780 | 266.689 | 554.070 | 23.75 | 19.26 | N.A. |
| 3 | | 9.498 | 63.995 | 138.154 | 5.70 | 4.80 | N.A. |
| 4 | | 10.288 | 76.952 | 351.168 | 6.85 | 12.21 | N.A. |
| 5 | | 10.853 | 547.034 | 1357.532 | 48.71 | 47.19 | N.A. |
| 6 | | 12.323 | 145.191 | 437.871 | 12.93 | 15.22 | N.A. |
| TOTAL: | | | 1123.084 | 2877.036 | 100.00 | 100.00 | |

*FIG. 1D*

CHROMATOGRAM

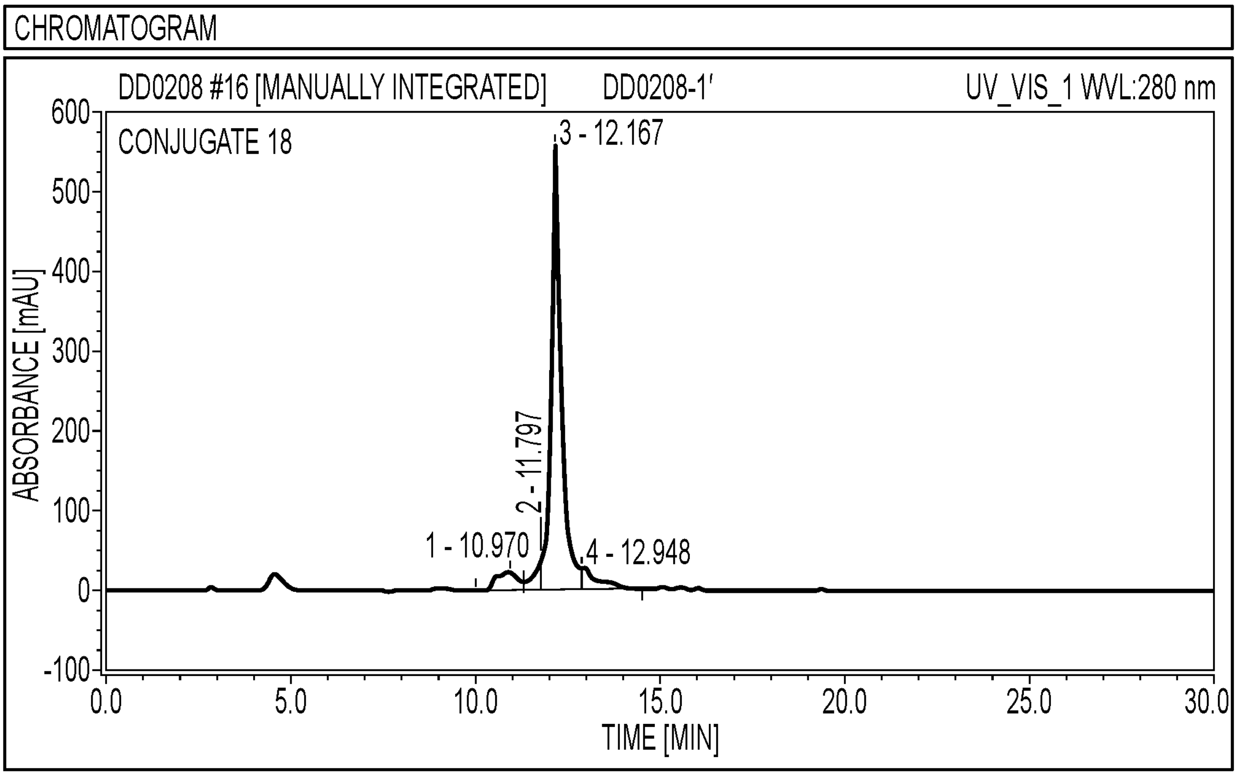


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 10.970 | 14.729 | 21.024 | 6.66 | 3.29 | N.A. |
| 2 | | 11.797 | 9.223 | 37.332 | 4.17 | 5.85 | N.A. |
| 3 | | 12.167 | 183.622 | 551.170 | 83.08 | 86.34 | N.A. |
| 4 | | 12.948 | 13.435 | 28.845 | 6.08 | 4.52 | N.A. |
| TOTAL: | | | 221.008 | 638.371 | 100.00 | 100.00 | |

*FIG. 2A*

CHROMATOGRAM

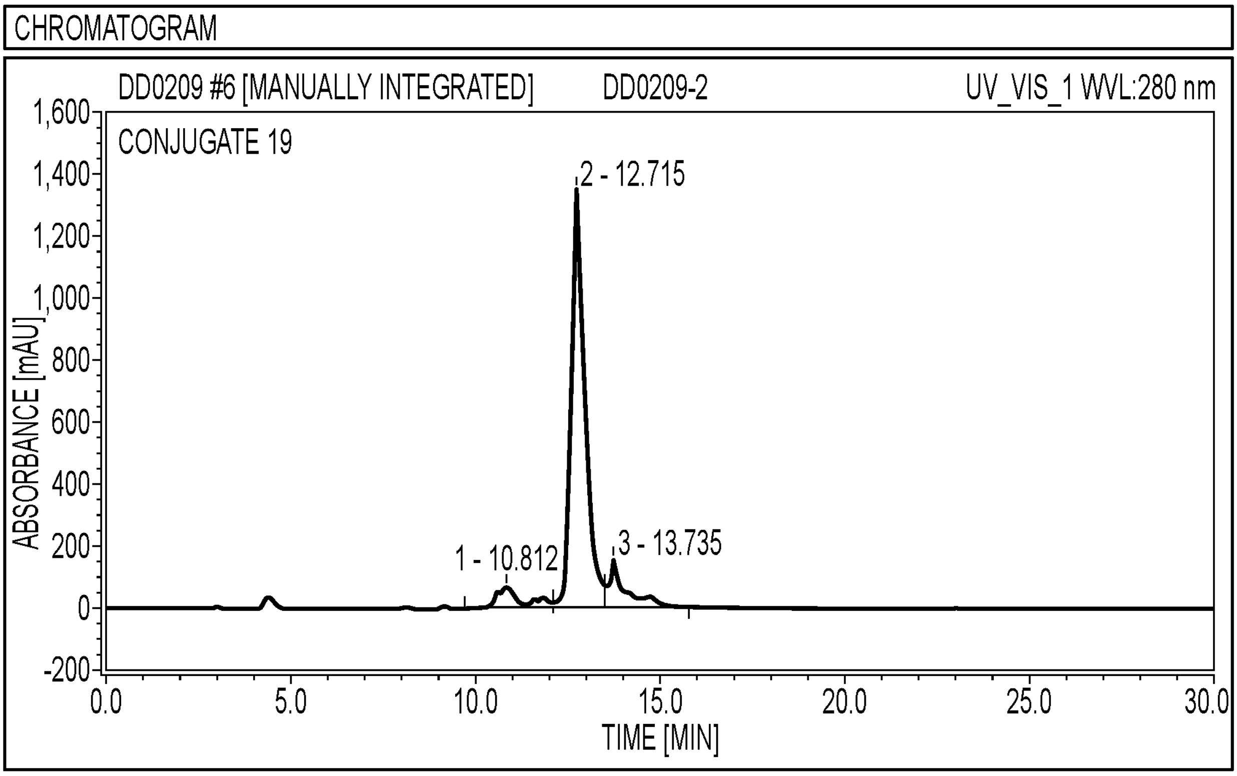


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 10.812 | 57.799 | 68.875 | 8.01 | 4.41 | N.A. |
| 2 | | 12.715 | 582.780 | 1342.386 | 80.76 | 85.95 | N.A. |
| 3 | | 13.735 | 81.021 | 150.532 | 11.23 | 9.64 | N.A. |
| TOTAL: | | | 721.600 | 1561.793 | 100.00 | 100.00 | |

# *FIG. 2B*

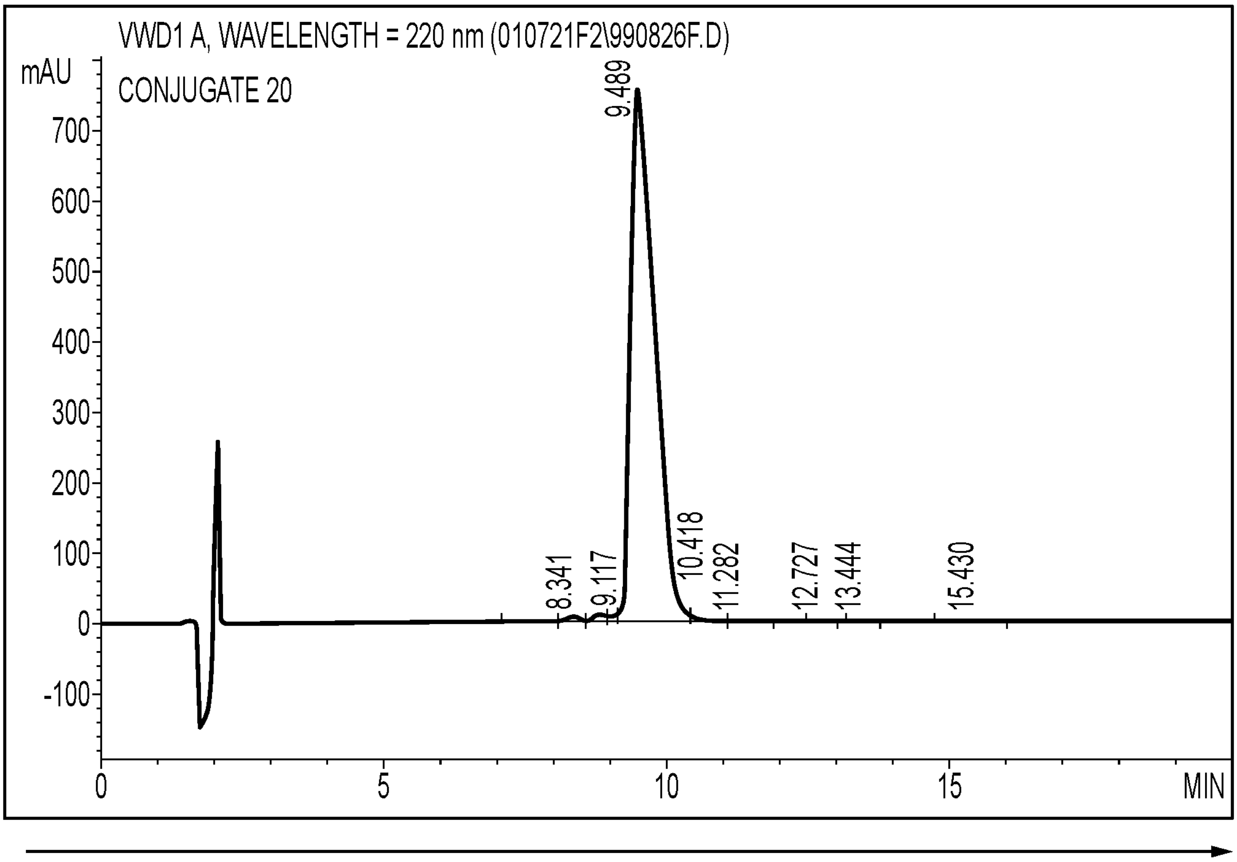


SIGNAL 1: VWD1 A, WAVELENGTH = 220 nm

| PEAK # | RT [MIN] | TYPE | HEIGHT | WIDTH [MIN] | AREA | AREA % |
|---|---|---|---|---|---|---|
| 1 | 7.964 | PV | 1.286 | 0.284 | 27.079 | 0.121 |
| 2 | 8.341 | VV | 5.588 | 0.294 | 110.299 | 0.493 |
| 3 | 8.788 | VV | 8.115 | 0.256 | 141.627 | 0.633 |
| 4 | 9.117 | VV | 9.480 | 0.146 | 83.105 | 0.371 |
| 5 | 9.489 | VF | 755.808 | 0.451 | 21845.049 | 97.619 |
| 6 | 10.418 | VV | 7.485 | 0.223 | 100.198 | 0.448 |
| 7 | 11.282 | VBA | 0.804 | 0.373 | 23.273 | 0.104 |
| 8 | 12.727 | BBA | 1.299 | 0.216 | 17.536 | 0.078 |
| 9 | 13.444 | BBA | 0.659 | 0.246 | 11.417 | 0.051 |
| 10 | 15.430 | PBA | 0.541 | 0.436 | 18.328 | 0.082 |

*FIG. 3A*

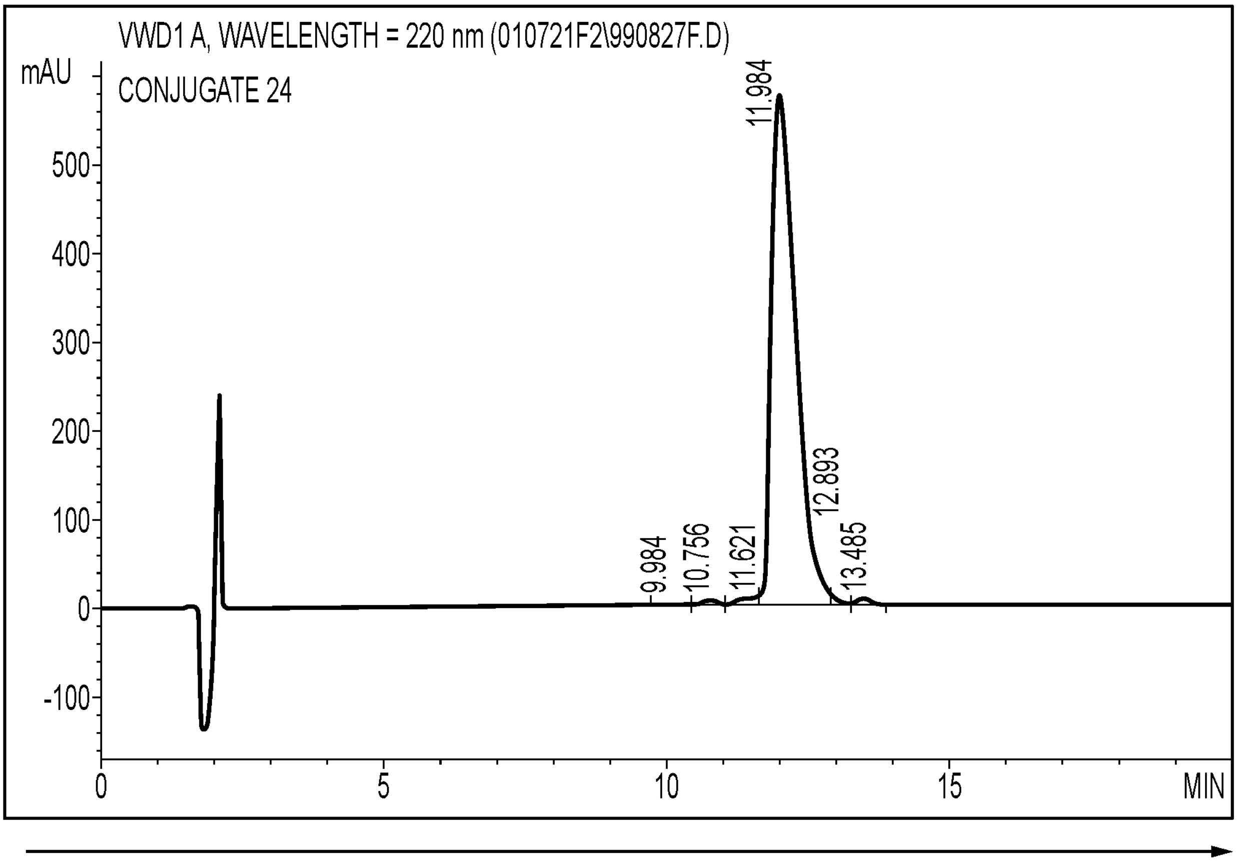


SIGNAL 1: VWD1 A, WAVELENGTH = 220 nm

| PEAK # | RT [MIN] | TYPE | HEIGHT | WIDTH [MIN] | AREA | AREA % |
|---|---|---|---|---|---|---|
| 1 | 9.984 | BV | 0.290 | 0.769 | 18.552 | 0.107 |
| 2 | 10.756 | VV | 4.555 | 0.361 | 113.789 | 0.656 |
| 3 | 11.621 | VV | 8.432 | 0.362 | 182.982 | 1.054 |
| 4 | 11.984 | VF | 576.686 | 0.448 | 16830.783 | 96.991 |
| 5 | 12.893 | VV | 11.265 | 0.161 | 108.685 | 0.626 |
| 6 | 13.485 | VBA | 6.038 | 0.245 | 98.098 | 0.565 |

*FIG. 3B*

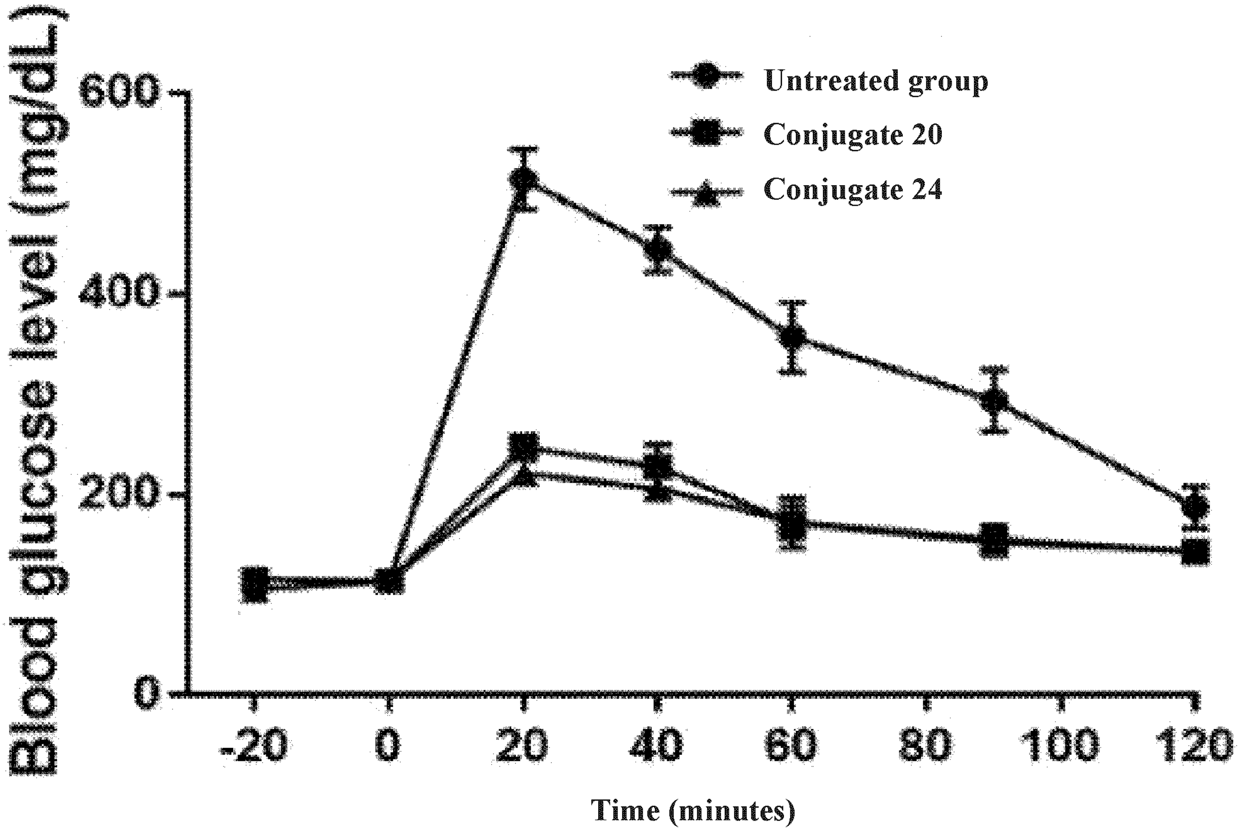
Blood glucose level (mg/dL)
600
400
200
0
-20
0
20
40
60
80
100
120
Time (minutes)
Untreated group
Conjugate 20
Conjugate 24

*FIG. 4*

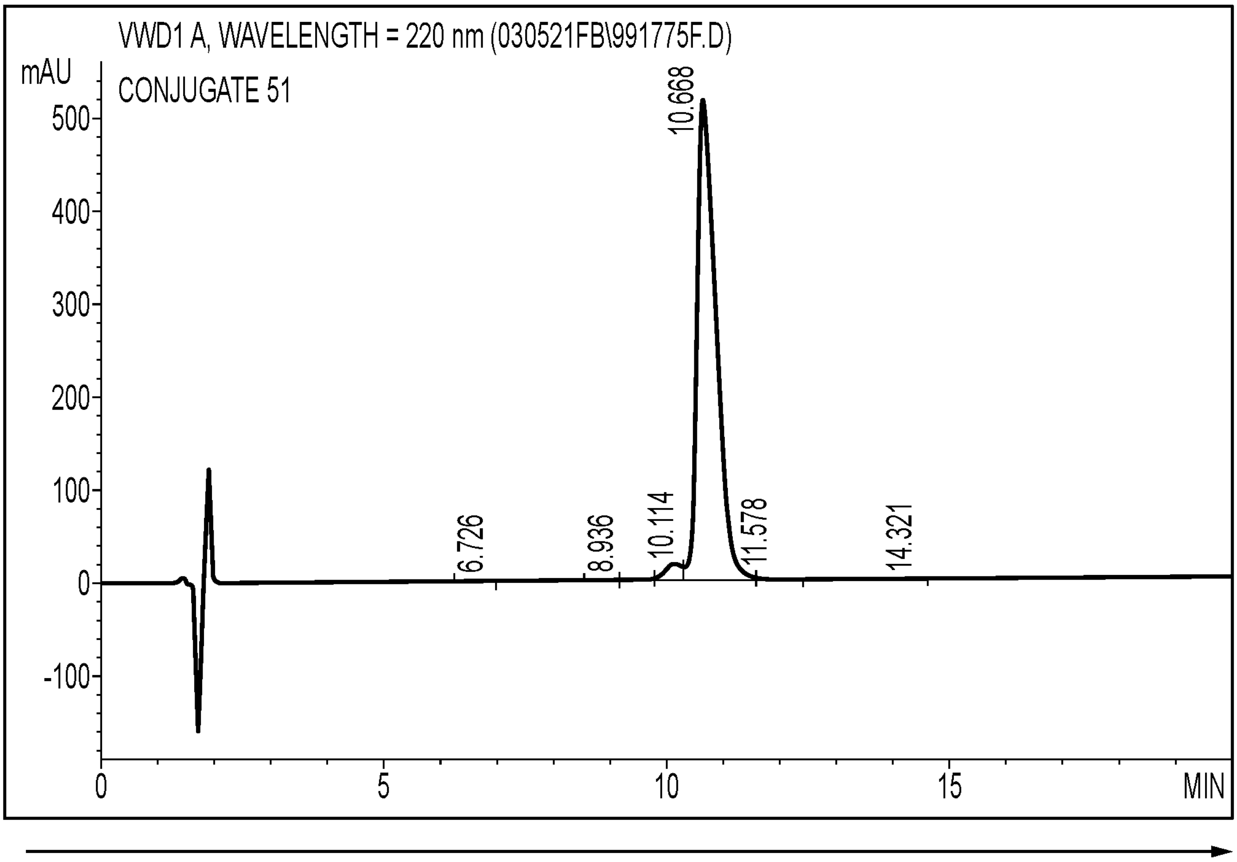


SIGNAL 1: VWD1 A, WAVELENGTH = 220 nm

| PEAK # | RT [MIN] | TYPE | HEIGHT | WIDTH [MIN] | AREA | AREA % |
|---|---|---|---|---|---|---|
| 1 | 6.726 | PBA | 0.810 | 0.292 | 14.206 | 0.118 |
| 2 | 8.936 | BV | 1.047 | 0.187 | 13.092 | 0.109 |
| 3 | 9.772 | VV | 2.119 | 0.269 | 34.140 | 0.284 |
| 4 | 10.114 | VV | 16.485 | 0.299 | 331.599 | 2.759 |
| 5 | 10.668 | VF | 520.097 | 0.336 | 11599.659 | 96.497 |
| 6 | 11.578 | VBA | 2.064 | 0.180 | 22.244 | 0.185 |
| 7 | 14.321 | BBA | 0.399 | 0.235 | 5.857 | 0.049 |

# *FIG. 5A*

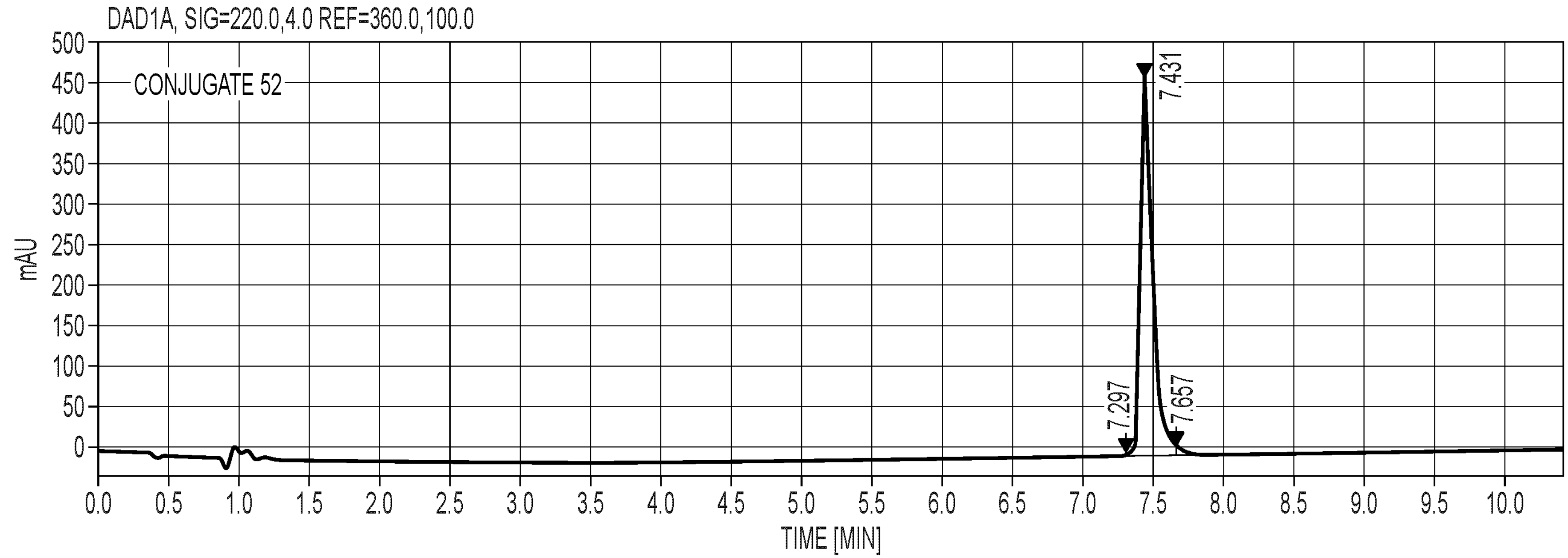


**SIGNAL:** DAD1A, SIG=220.0,4.0 REF=360.0,100.0

| RT [MIN] | TYPE | WIDTH [MIN] | AREA | HEIGHT | AREA% | NAME |
|---|---|---|---|---|---|---|
| 7.297 | BM m | 0.66 | 8.17 | 1.98 | 0.27 | |
| 7.431 | MM m | 0.35 | 2940.21 | 466.17 | 98.80 | |
| 7.657 | MB m | 0.20 | 27.59 | 9.42 | 0.93 | |
| | | **SUM** | **2975.96** | | | |

*FIG. 5B*

CHROMATOGRAM

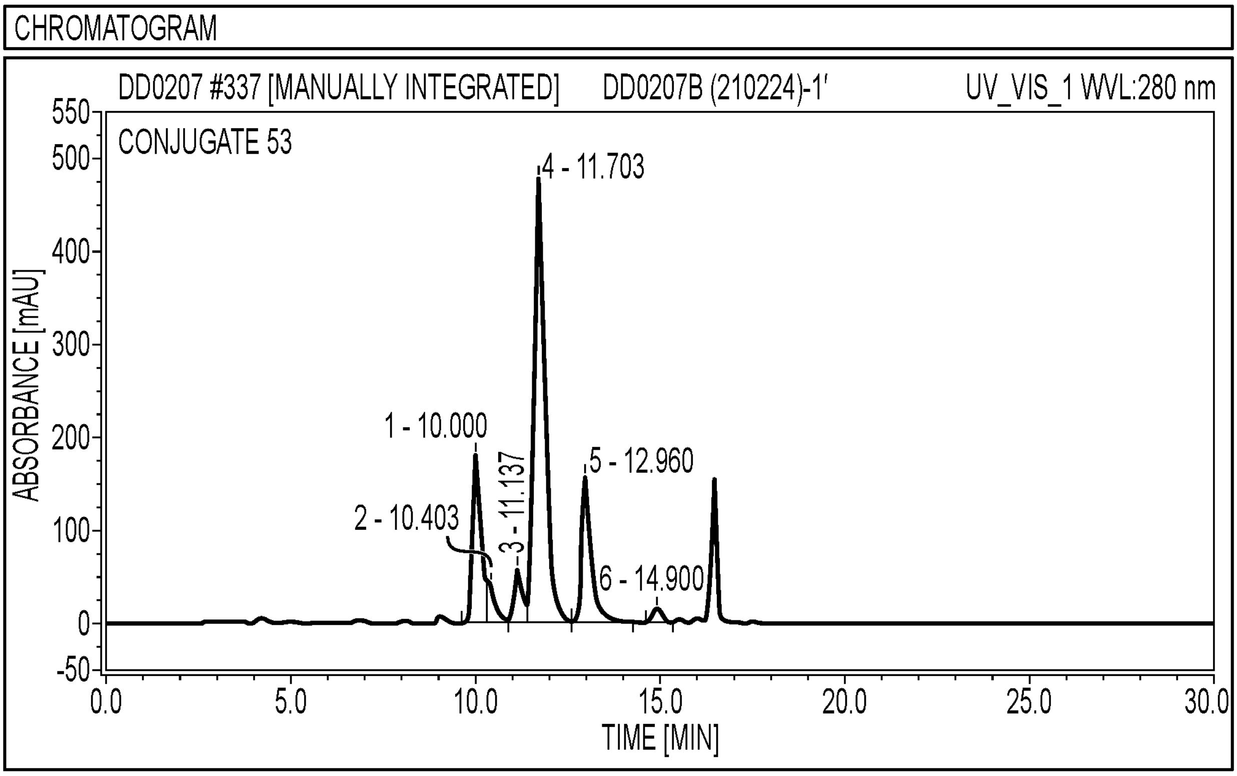


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 10.000 | 50.826 | 181.187 | 18.00 | 19.54 | N.A. |
| 2 | | 10.403 | 10.968 | 39.899 | 3.88 | 4.30 | N.A. |
| 3 | | 11.137 | 15.439 | 57.956 | 5.47 | 6.25 | N.A. |
| 4 | | 11.703 | 152.881 | 478.386 | 54.15 | 51.60 | N.A. |
| 5 | | 12.960 | 48.390 | 155.693 | 17.14 | 16.79 | N.A. |
| 6 | | 14.900 | 3.826 | 14.015 | 1.35 | 1.51 | N.A. |
| TOTAL: | | | 282.330 | 927.136 | 100.00 | 100.00 | |

*FIG. 6A*

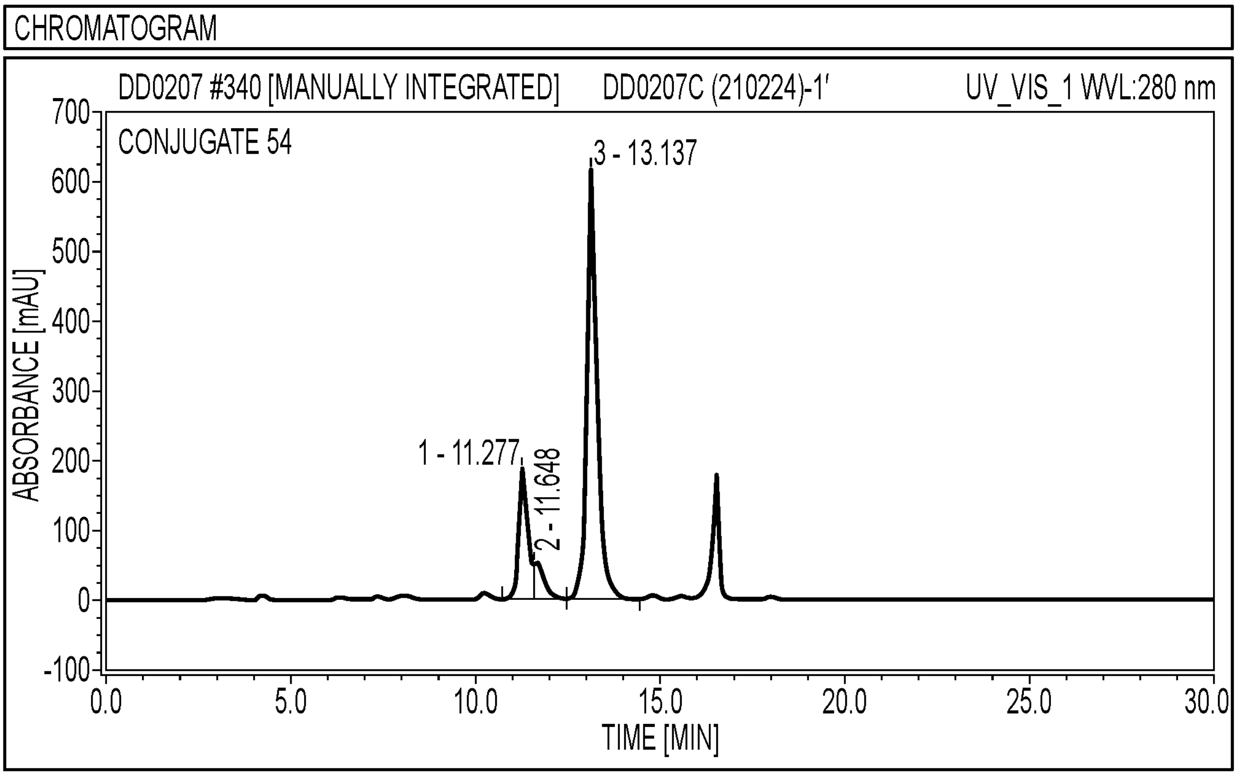


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 11.277 | 53.393 | 185.891 | 20.05 | 21.83 | N.A. |
| 2 | | 11.648 | 16.535 | 51.979 | 6.21 | 6.10 | N.A. |
| 3 | | 13.137 | 196.428 | 613.546 | 73.75 | 72.06 | N.A. |
| TOTAL: | | | 266.356 | 851.416 | 100.00 | 100.00 | |

*FIG. 6B*

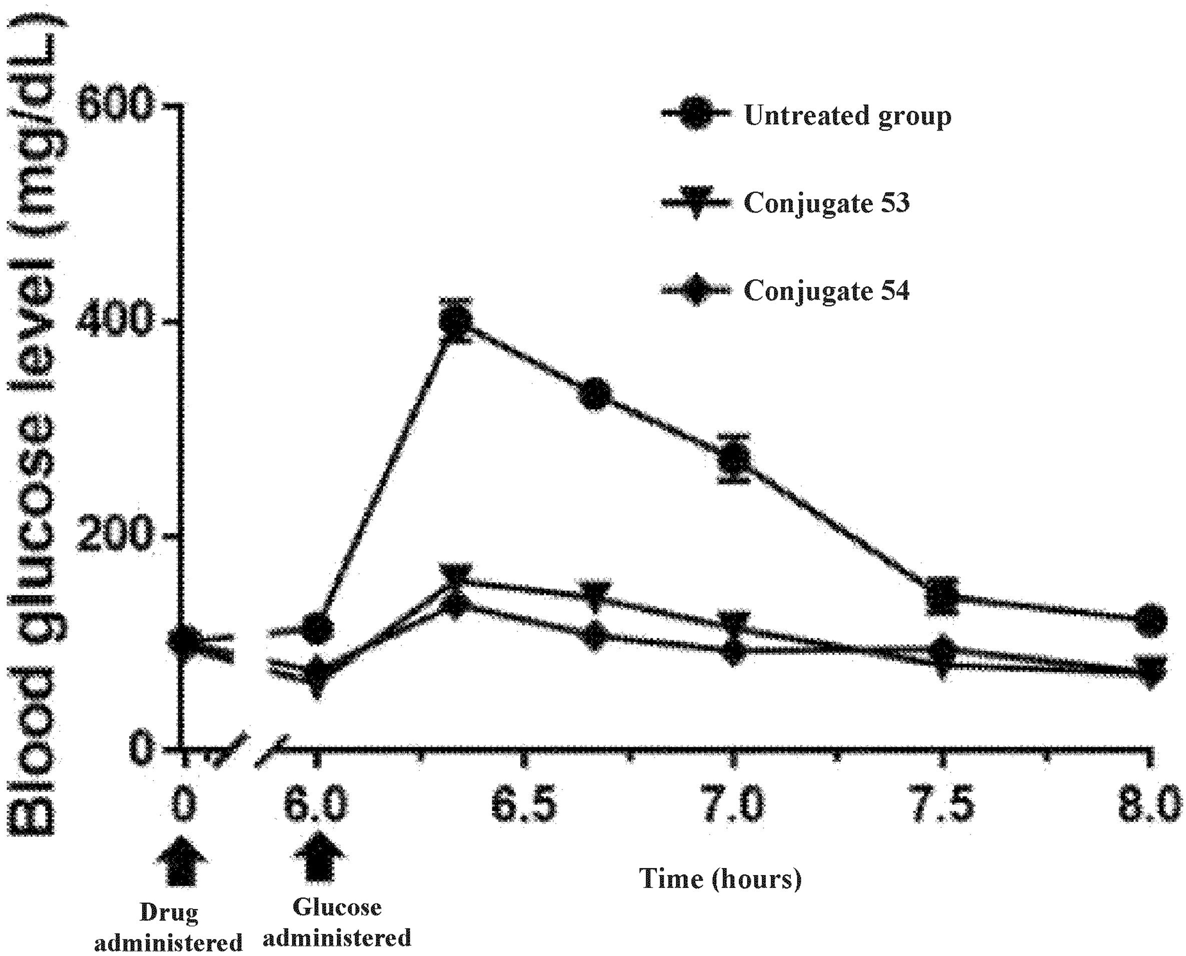
Blood glucose level (mg/dL)
600
400
200
0
Untreated group
Conjugate 53
Conjugate 54
0
6.0
6.5
7.0
7.5
8.0
Time (hours)
Drug administered
Glucose administered

FIG. 7

CHROMATOGRAM

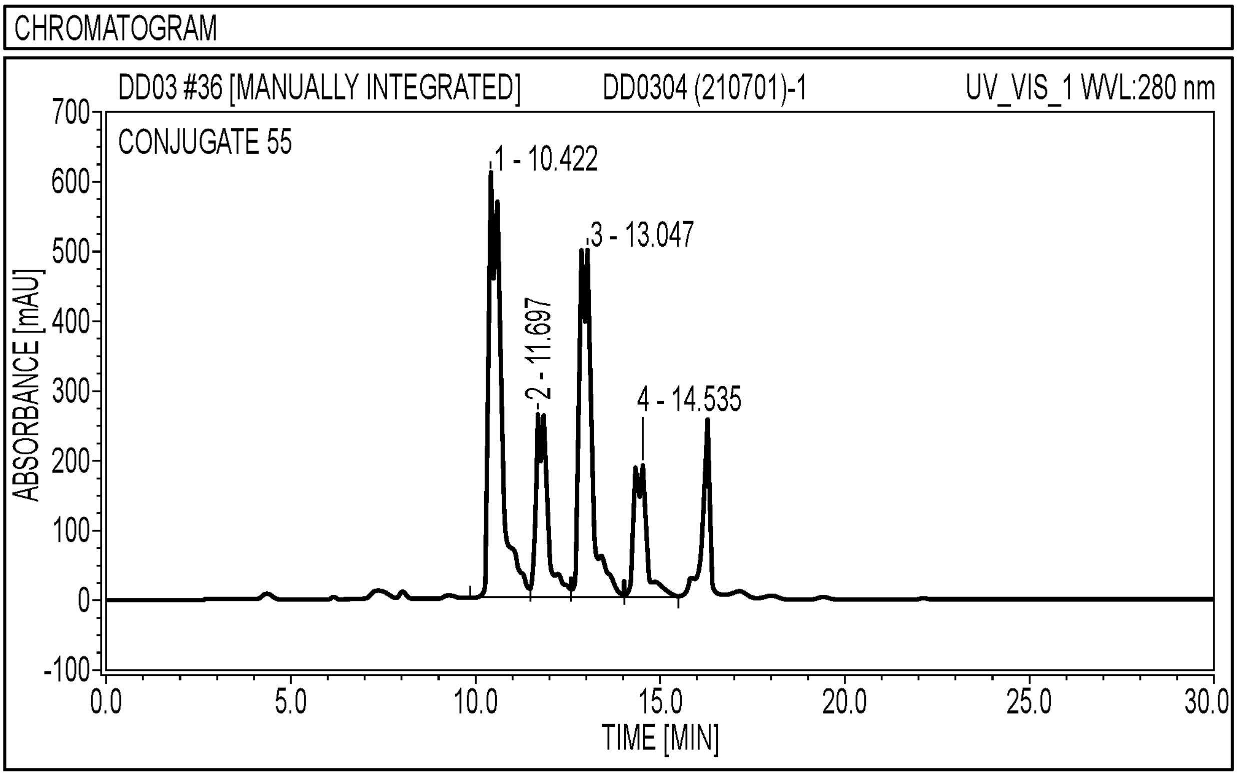


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 10.422 | 236.768 | 607.751 | 39.71 | 39.04 | N.A. |
| 2 | | 11.697 | 96.895 | 262.792 | 16.25 | 16.88 | N.A. |
| 3 | | 13.047 | 192.032 | 497.108 | 32.21 | 31.93 | N.A. |
| 4 | | 14.535 | 70.483 | 189.050 | 11.82 | 12.14 | N.A. |
| TOTAL: | | | 596.178 | 1556.700 | 100.00 | 100.00 | |

*FIG. 8A*

CHROMATOGRAM

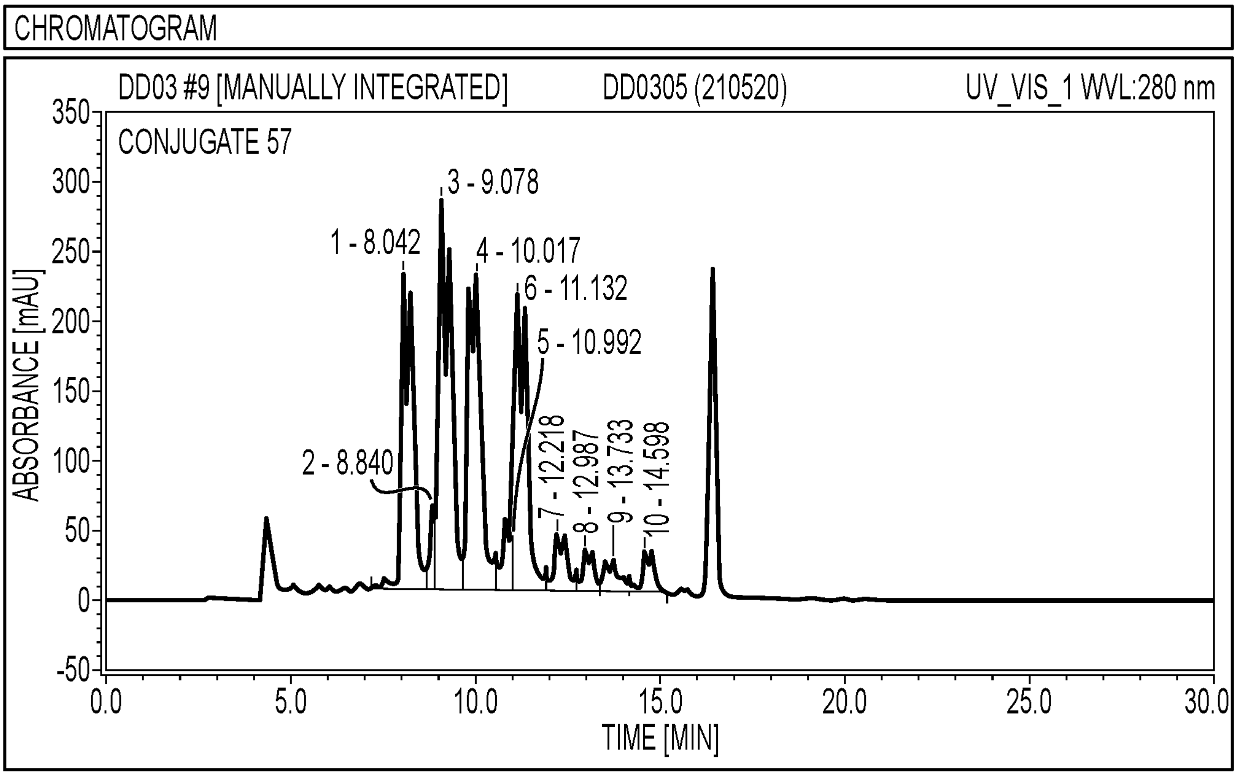


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 8.042 | 79.621 | 226.203 | 18.60 | 19.01 | N.A. |
| 2 | | 8.840 | 9.906 | 59.898 | 2.31 | 5.03 | N.A. |
| 3 | | 9.078 | 102.196 | 279.233 | 23.88 | 23.47 | N.A. |
| 4 | | 10.017 | 94.121 | 226.556 | 21.99 | 19.04 | N.A. |
| 5 | | 10.992 | 15.026 | 62.227 | 3.51 | 5.23 | N.A. |
| 6 | | 11.132 | 77.639 | 212.765 | 18.14 | 17.88 | N.A. |
| 7 | | 12.218 | 16.397 | 39.522 | 3.83 | 3.32 | N.A. |
| 8 | | 12.987 | 11.151 | 30.075 | 2.61 | 2.53 | N.A. |
| 9 | | 13.733 | 10.480 | 23.448 | 2.45 | 1.97 | N.A. |
| 10 | | 14.598 | 11.473 | 29.813 | 2.68 | 2.51 | N.A. |
| TOTAL: | | | 428.011 | 1189.741 | 100.00 | 100.00 | |

*FIG. 8B*

CHROMATOGRAM

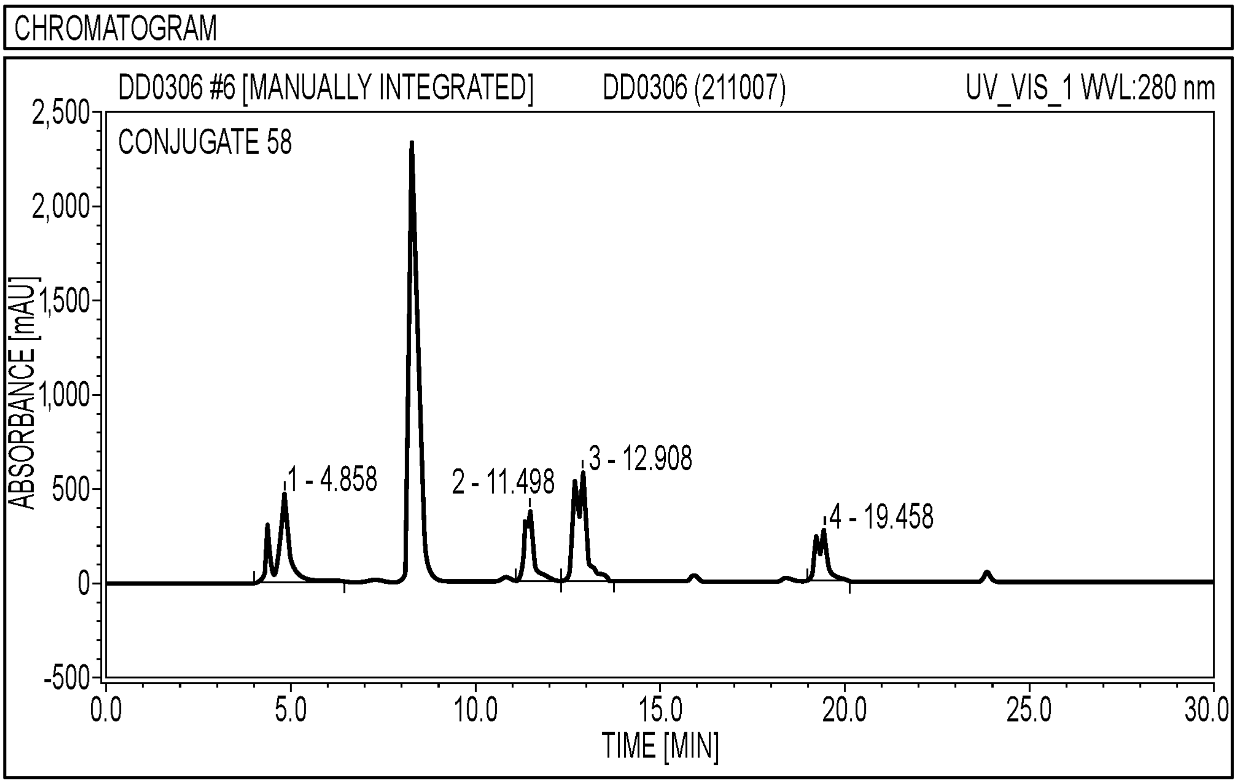


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 4.858 | 184.853 | 467.687 | 30.68 | 27.68 | N.A. |
| 2 | | 11.498 | 109.660 | 367.835 | 18.20 | 21.77 | N.A. |
| 3 | | 12.908 | 212.889 | 580.898 | 35.33 | 34.38 | N.A. |
| 4 | | 19.458 | 95.110 | 273.408 | 15.79 | 16.18 | N.A. |
| TOTAL: | | | 602.511 | 1689.828 | 100.00 | 100.00 | |

*FIG. 8C*

CHROMATOGRAM

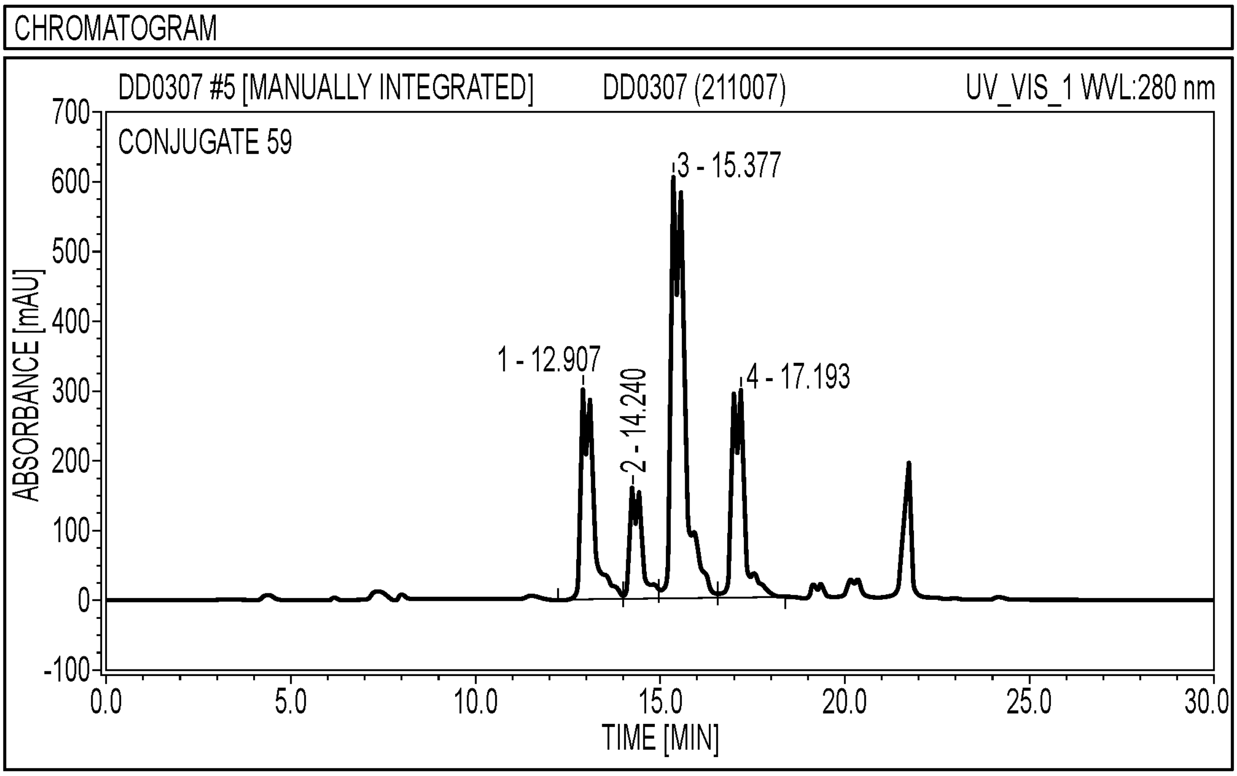


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 12.907 | 121.312 | 299.756 | 22.15 | 22.11 | N.A. |
| 2 | | 14.240 | 59.752 | 159.480 | 10.91 | 11.76 | N.A. |
| 3 | | 15.377 | 249.859 | 600.698 | 45.62 | 44.30 | N.A. |
| 4 | | 17.193 | 116.804 | 295.986 | 21.33 | 21.83 | N.A. |
| TOTAL: | | | 547.727 | 1355.919 | 100.00 | 100.00 | |

*FIG. 8D*

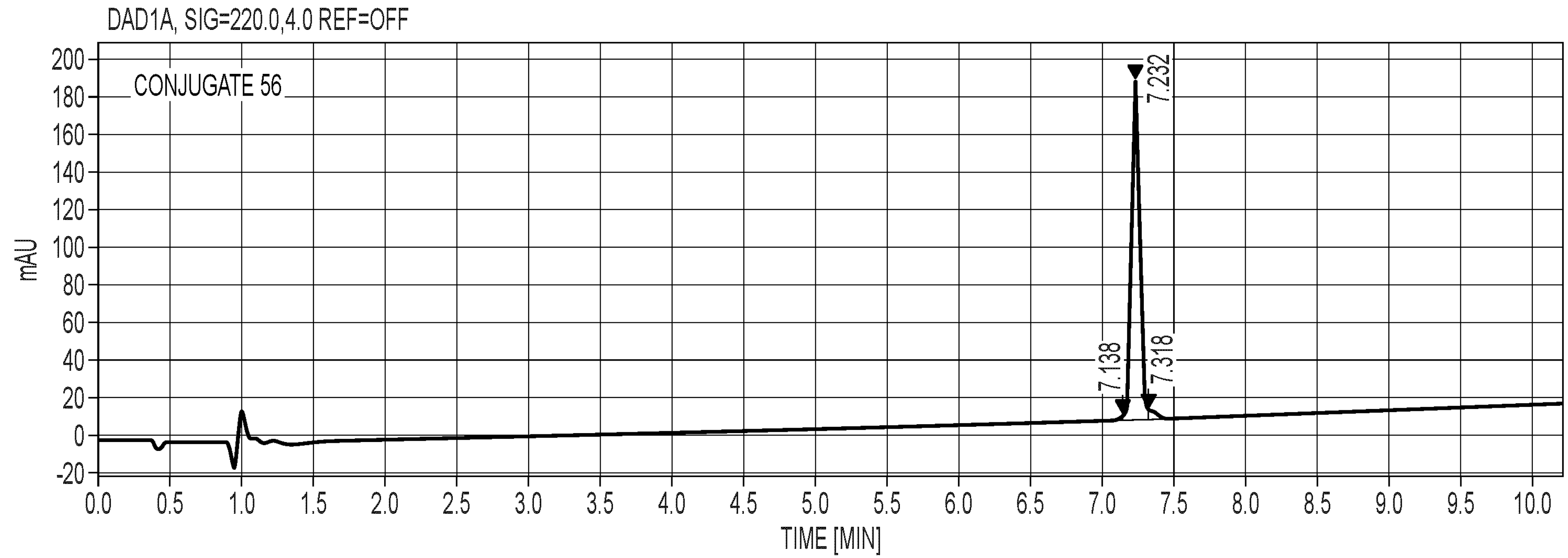


**SIGNAL:** DAD1A, SIG=220.0,4.0 REF=OFF

| RT [MIN] | TYPE | WIDTH [MIN] | AREA | HEIGHT | AREA% | NAME |
|---|---|---|---|---|---|---|
| 7.138 | MM m | 0.27 | 11.22 | 2.44 | 1.50 | |
| 7.232 | MM m | 0.17 | 713.33 | 180.14 | 95.61 | |
| 7.318 | MV m | 0.16 | 21.56 | 5.61 | 2.89 | |
| | | **SUM** | **746.11** | | | |

*FIG. 9*

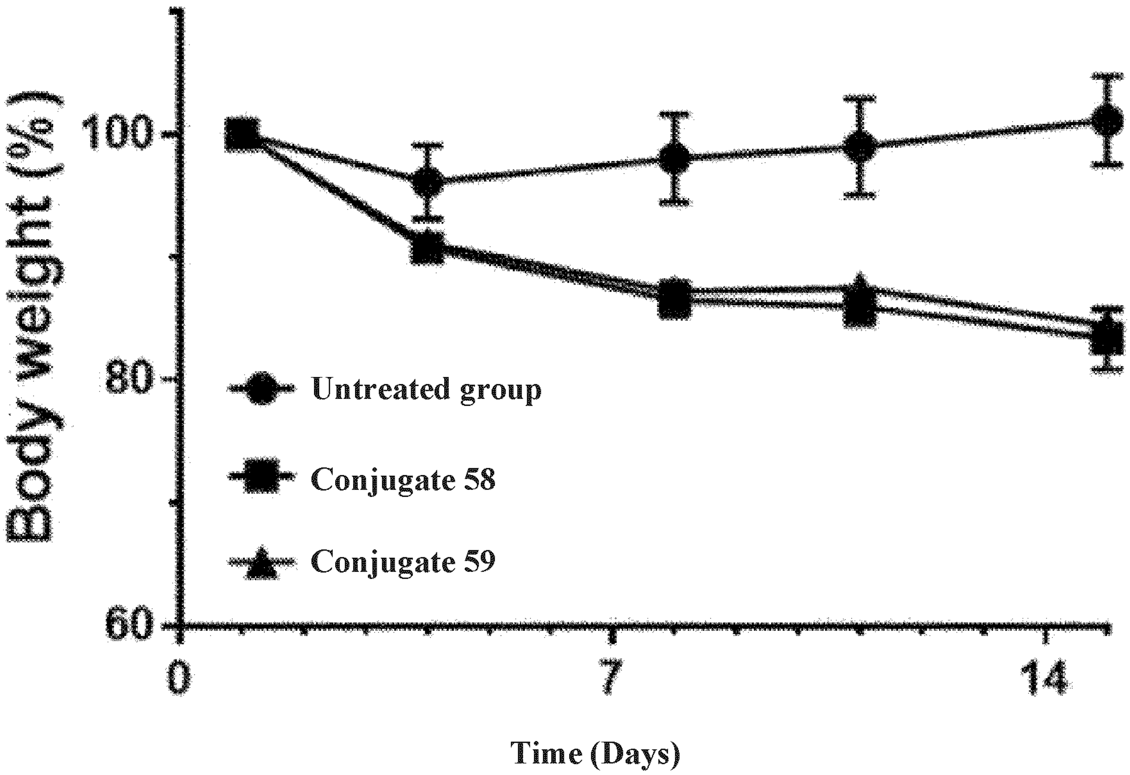
Body weight (%)
100
80
60
0
7
14
Time (Days)
Untreated group
Conjugate 58
Conjugate 59

*FIG. 10*

CHROMATOGRAM

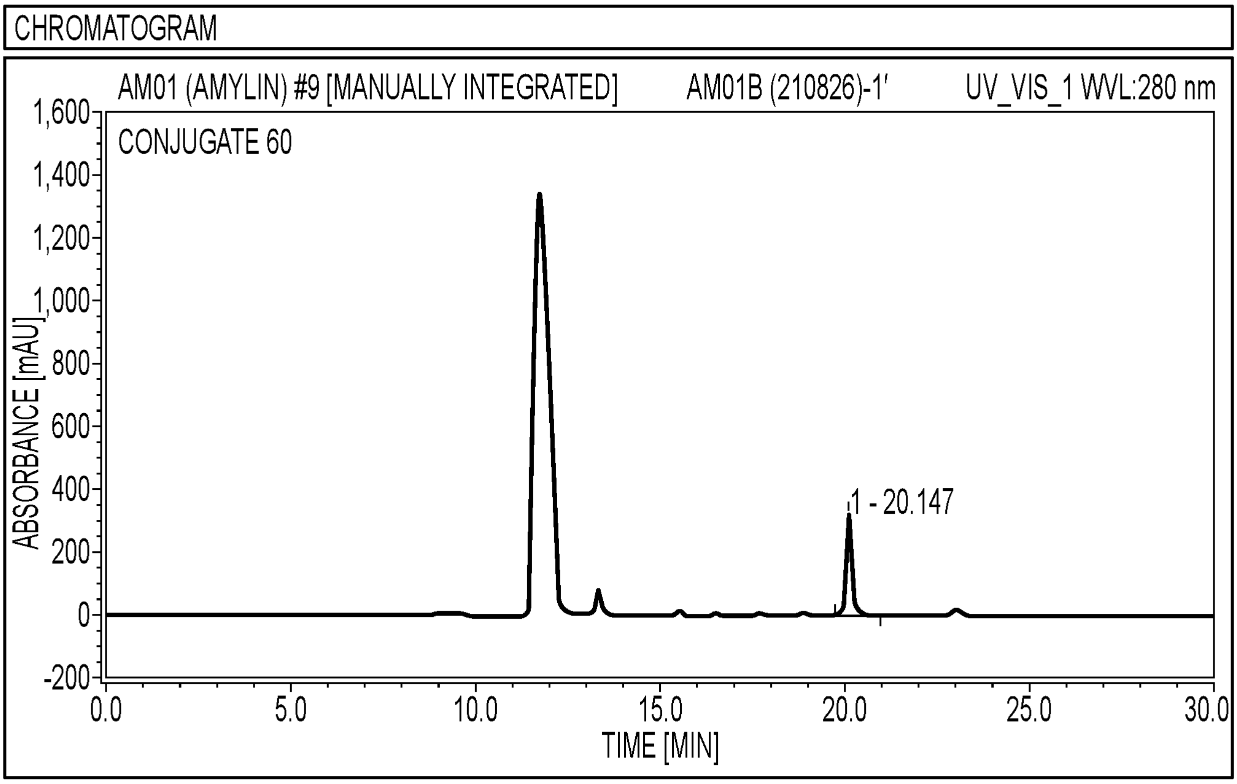


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 20.147 | 59.609 | 321.253 | 100.00 | 100.00 | N.A. |
| TOTAL: | | | 59.609 | 321.253 | 100.00 | 100.00 | |

FIG. 11A

CHROMATOGRAM

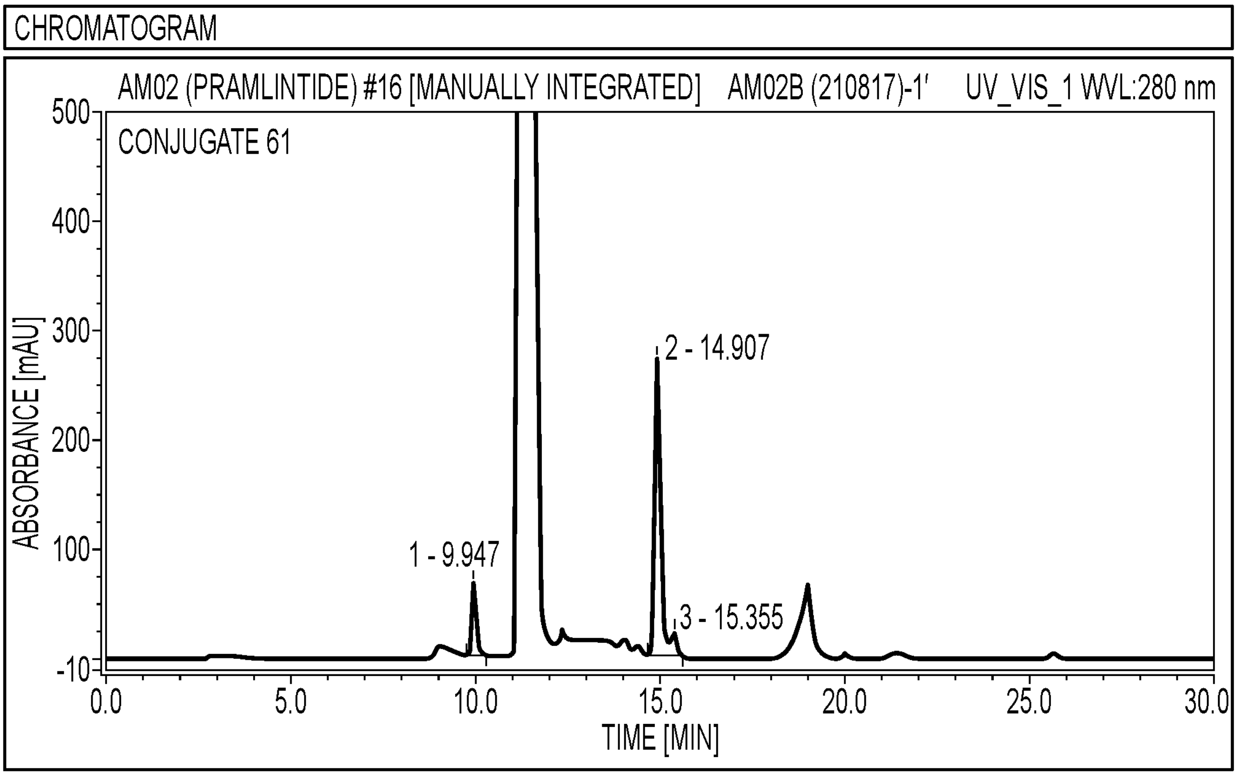


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 9.947 | 10.228 | 66.757 | 16.50 | 18.66 | N.A. |
| 2 | | 14.907 | 47.525 | 269.978 | 76.66 | 75.47 | N.A. |
| 3 | | 15.355 | 4.242 | 20.989 | 6.84 | 5.87 | N.A. |
| TOTAL: | | | 61.995 | 357.724 | 100.00 | 100.00 | |

FIG. 11B

CHROMATOGRAM

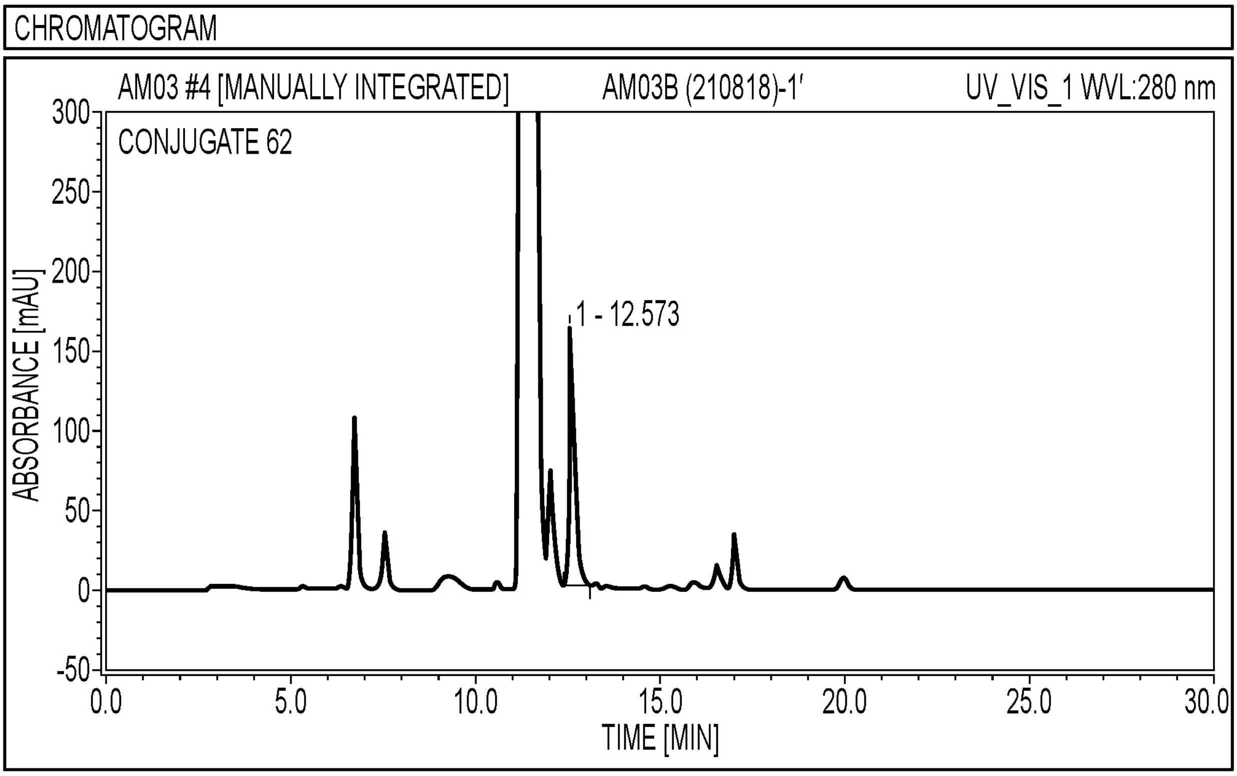


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 12.573 | 31.562 | 162.366 | 100.00 | 100.00 | N.A. |
| TOTAL: | | | 31.562 | 162.366 | 100.00 | 100.00 | |

*FIG. 11C*

CHROMATOGRAM

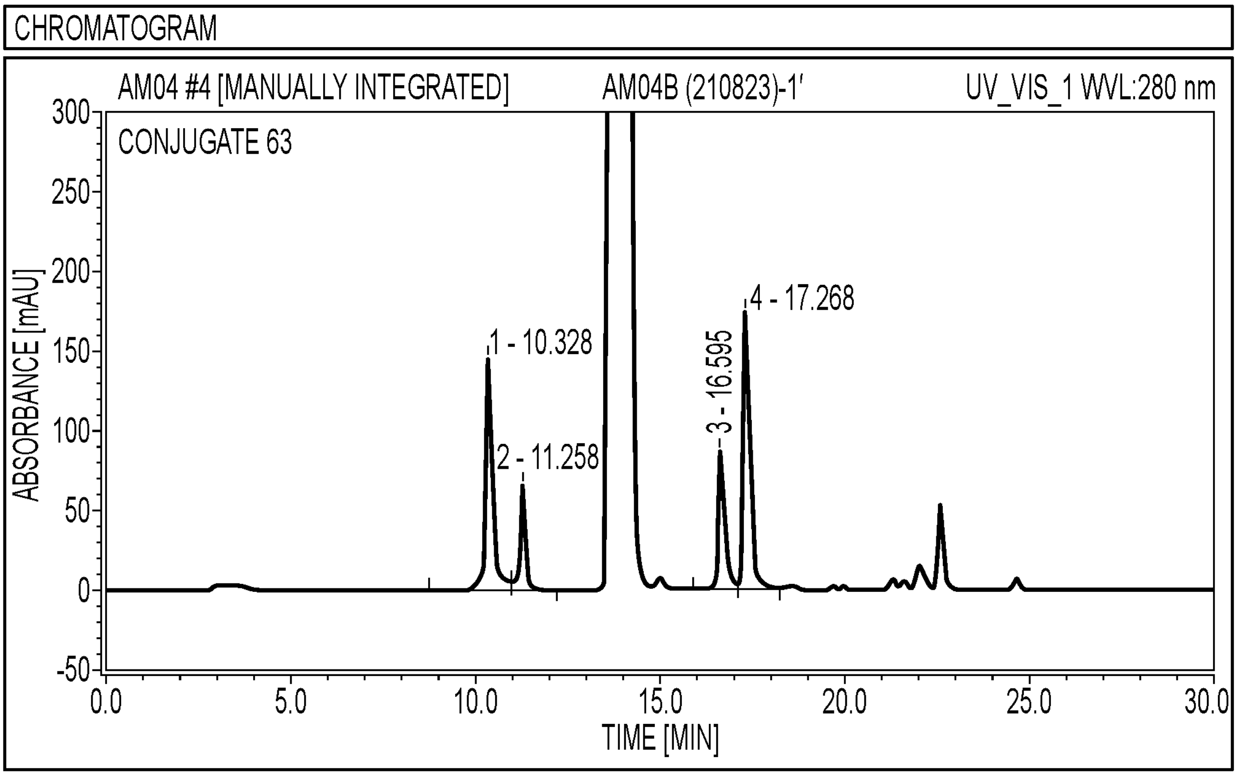


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 10.328 | 30.687 | 147.056 | 30.47 | 31.24 | N.A. |
| 2 | | 11.258 | 10.753 | 64.926 | 10.68 | 13.79 | N.A. |
| 3 | | 16.595 | 19.220 | 85.650 | 19.09 | 18.20 | N.A. |
| 4 | | 17.268 | 40.042 | 173.042 | 39.76 | 36.76 | N.A. |
| TOTAL: | | | 100.702 | 470.675 | 100.00 | 100.00 | |

*FIG. 11D*

CHROMATOGRAM

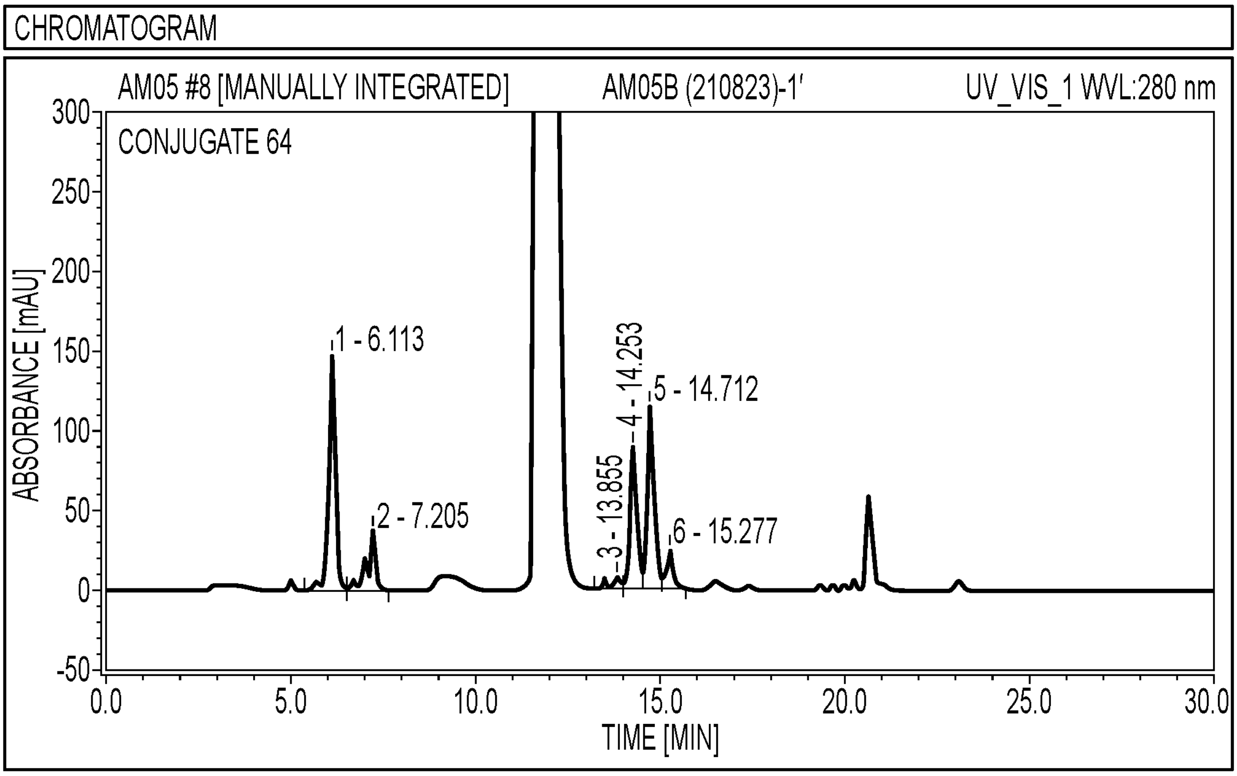


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 6.113 | 29.216 | 145.413 | 31.85 | 34.98 | N.A. |
| 2 | | 7.205 | 9.551 | 37.041 | 10.41 | 8.91 | N.A. |
| 3 | | 13.855 | 2.322 | 6.825 | 2.53 | 1.64 | N.A. |
| 4 | | 14.253 | 20.730 | 89.256 | 22.60 | 21.47 | N.A. |
| 5 | | 14.712 | 24.349 | 113.473 | 26.54 | 27.30 | N.A. |
| 6 | | 15.277 | 5.561 | 23.678 | 6.06 | 5.70 | N.A. |
| TOTAL: | | | 91.729 | 415.687 | 100.00 | 100.00 | |

*FIG. 11E*

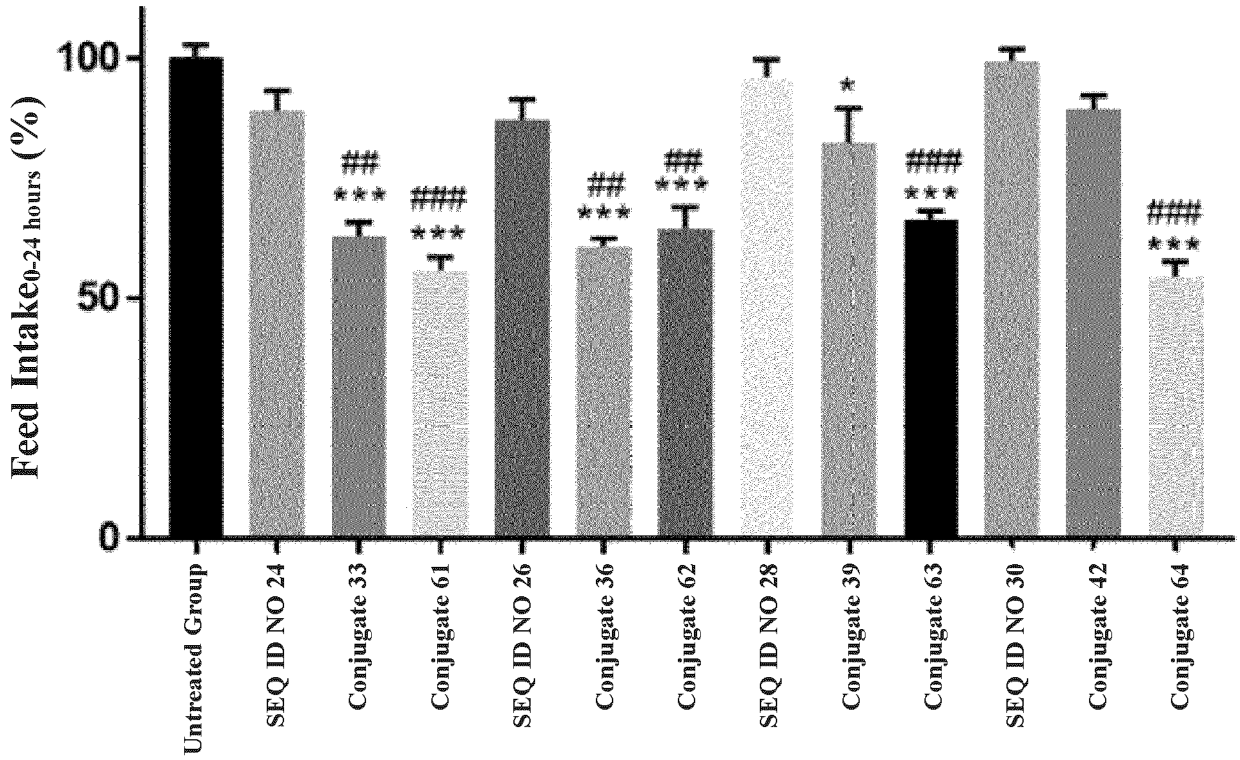


*p vs. untreated group, #p vs. cyclic peptide (SEQ ID NOs: 24, 26, 28, 30)

*FIG. 12*

CHROMATOGRAM

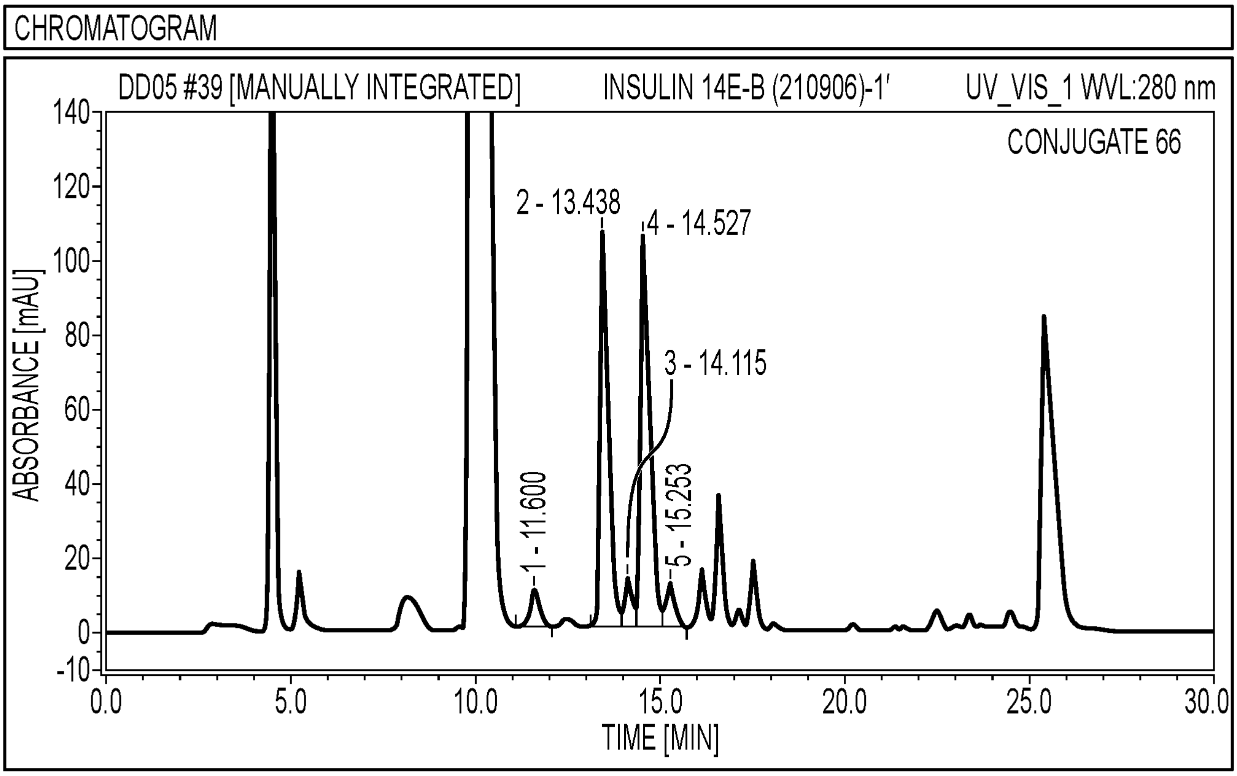


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 11.600 | 2.814 | 9.971 | 3.89 | 4.04 | N.A. |
| 2 | | 13.438 | 30.318 | 106.490 | 41.94 | 43.11 | N.A. |
| 3 | | 14.115 | 3.122 | 13.228 | 4.32 | 5.36 | N.A. |
| 4 | | 14.527 | 32.653 | 105.557 | 45.17 | 42.73 | N.A. |
| 5 | | 15.253 | 3.379 | 11.768 | 4.67 | 4.76 | N.A. |
| TOTAL: | | | 72.286 | 247.015 | 100.00 | 100.00 | |

*FIG. 13*

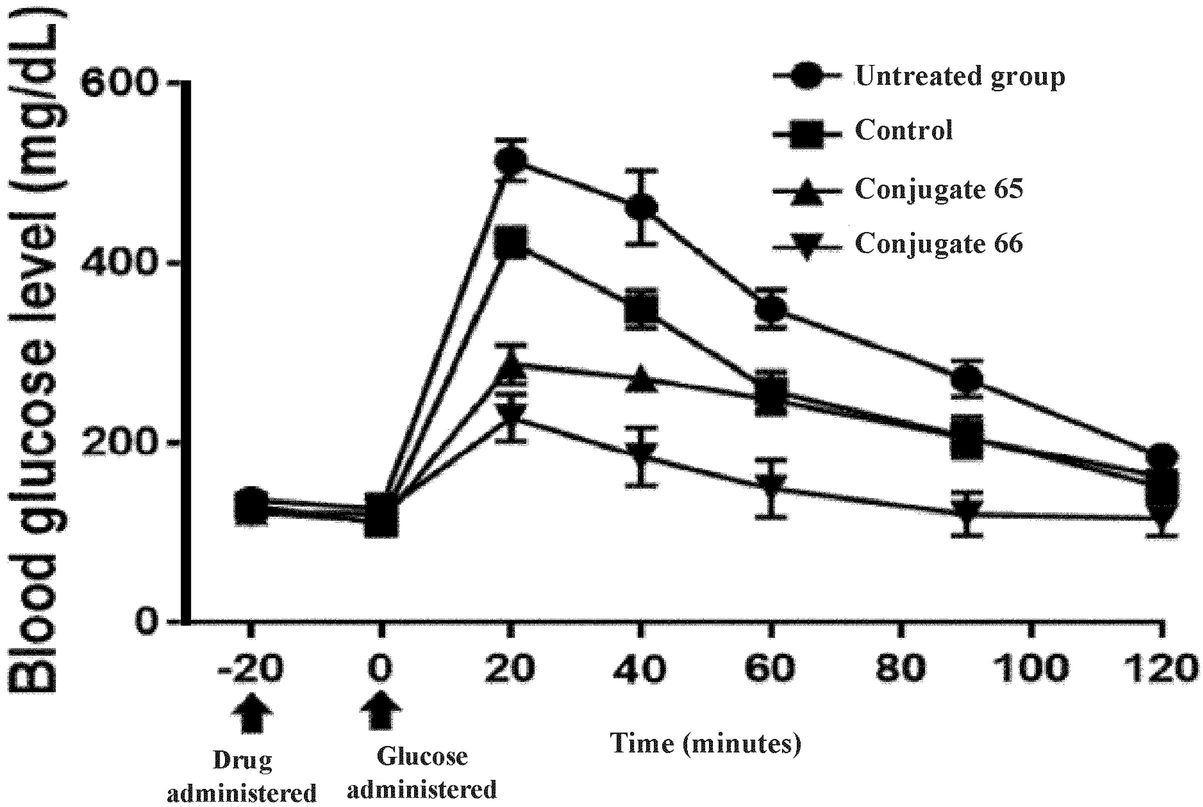
Blood glucose level (mg/dL)
600
400
200
0
-20
0
20
40
60
80
100
120
Drug administered
Glucose administered
Time (minutes)
Untreated group
Control
Conjugate 65
Conjugate 66

FIG. 14

CHROMATOGRAM

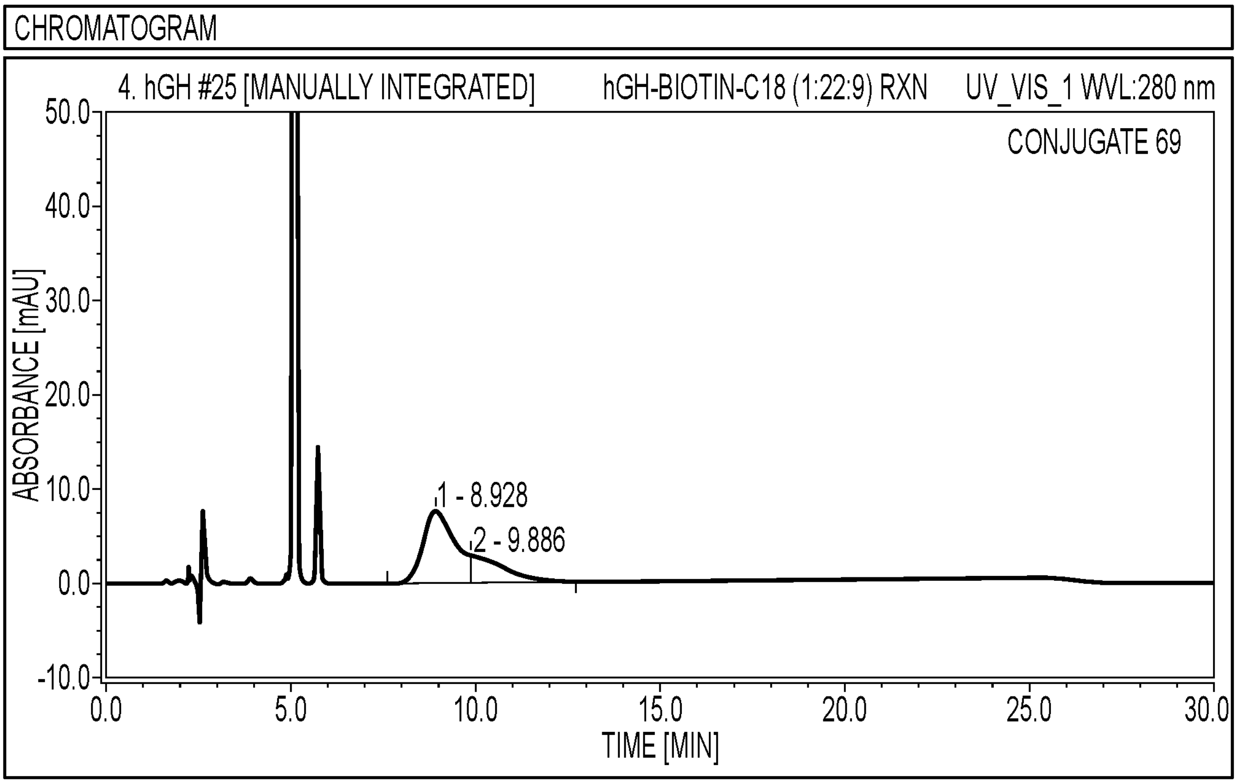


INTEGRATION RESULTS

| NO. | PEAK NAME | RETENTION TIME MIN | AREA mAU*MIN | HEIGHT mAU | RELATIVE AREA % | RELATIVE HEIGHT % | AMOUNT N.A. |
|---|---|---|---|---|---|---|---|
| 1 | | 8.928 | 7.420 | 7.504 | 73.34 | 73.65 | N.A. |
| 2 | | 9.886 | 2.698 | 2.685 | 26.66 | 26.35 | N.A. |
| TOTAL: | | | 10.117 | 10.189 | 100.00 | 100.00 | |

FIG. 15

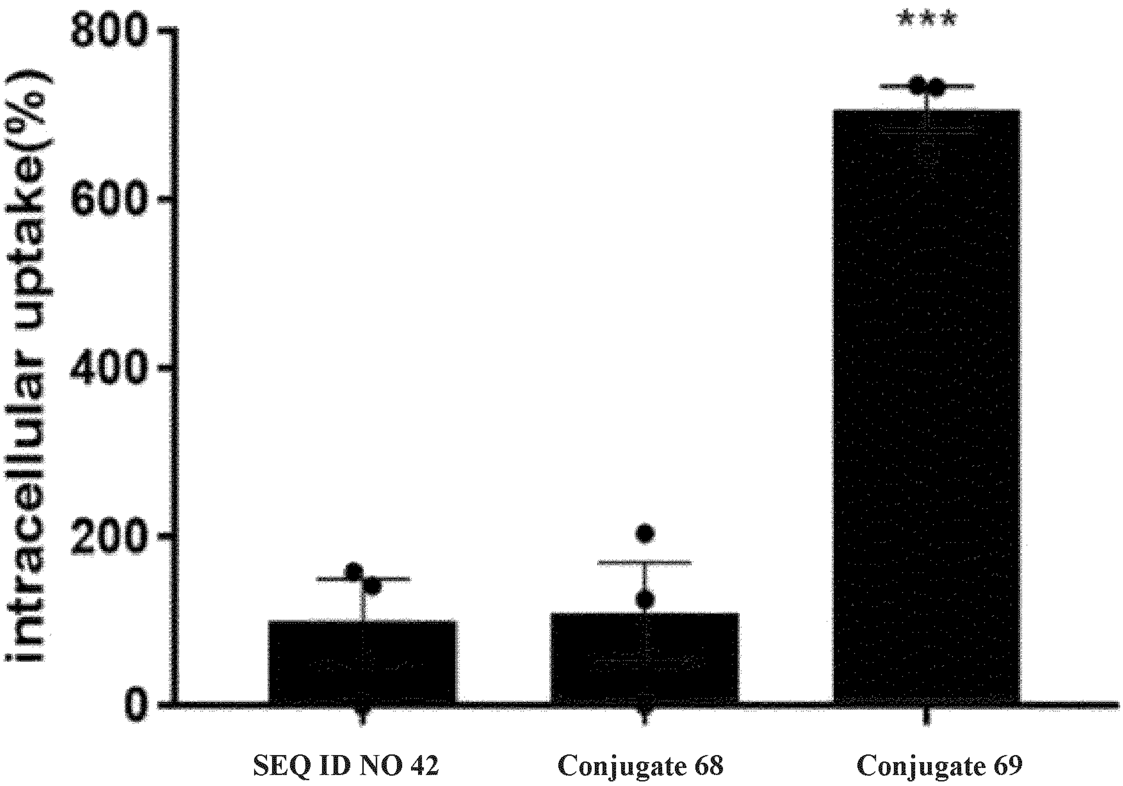
intracellular uptake(%)
800
600
400
200
0
***
SEQ ID NO 42
Conjugate 68
Conjugate 69

FIG. 16

**INTERNATIONAL SEARCH REPORT**

International application No.
**PCT/IB2021/022237**

**A. CLASSIFICATION OF SUBJECT MATTER**

**A61K 47/54**(2017.01)i; **A61K 38/00**(2006.01)i; **A61K 38/16**(2006.01)i; **A61K 9/00**(2006.01)i

According to International Patent Classification (IPC) or to both national classification and IPC

**B. FIELDS SEARCHED**

Minimum documentation searched (classification system followed by classification symbols)

A61K 47/54(2017.01); A61K 38/18(2006.01); A61K 38/21(2006.01); A61K 38/28(2006.01); A61K 38/52(2006.01); A61K 47/42(2006.01); A61K 47/61(2017.01); A61K 47/64(2017.01); A61K 47/68(2017.01); A61K 9/127(2006.01); C07K 14/605(2006.01); C07K 14/62(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), PubChem, Google & keywords: 비오틴 모이어티 (biotin moiety), 지방산 모이어티 (fatty acid moiety), 생리활성 물질 (bioactive material)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2010-0109896 A (SDG, INC.) 11 October 2010 (2010-10-11)<br>See paragraphs [0081], [0082], [0120], [0122] and [0137]; and claims 1-5, 9, 17 and 18. | 1,2,5-12,22,23<br>4 |
| Y | KR 10-2018-0039726 A (THE CALIFORNIA INSTITUTE FOR BIOMEDICAL RESEARCH) 18 April 2018 (2018-04-18)<br>See claims 1, 44, 45, 48 and 55. | 4 |
| X<br>Y | KR 10-2011-0004366 A (NOVO NORDISK A/S) 13 January 2011 (2011-01-13)<br>See abstract; paragraphs [0730] and [0731]; and claims 1, 9 and 10. | 1,2,14-19,21-23<br>4 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 March 2022** | **30 March 2022** |

Name and mailing address of the ISA/KR

**Korean Intellectual Property Office**
**Government Complex-Daejeon Building 4, 189 Cheongsa-ro, Seo-gu, Daejeon 35208**

Facsimile No. **+82-42-481-8578**

Authorized officer

Telephone No.

Form PCT/ISA/210 (second sheet) (July 2019)

**INTERNATIONAL SEARCH REPORT**

International application No.
**PCT/IB2021/022237**

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2003-0199451 A1 (MOGENSEN, J. P. et al.) 23 October 2003 (2003-10-23)<br>See paragraphs [0870], [1058] and [1162]; and claims 1, 69, 70 and 72. | 1,2,14-18,20-23<br>3 |
| Y | KR 10-2008-0064840 A (BIOCOMPATIBLES UK LIMITED) 09 July 2008 (2008-07-09)<br>See claims 1, 17, 18, 20, 21 and 35. | 3 |
| X | KR 10-2008-0042045 A (SDG, INC.) 14 May 2008 (2008-05-14)<br>See paragraphs [0131] and [0155]; and claims 1, 9-11, 38 and 45. | 1,2,5-12,23 |
| X | KR 10-2006-0032140 A (CENTOCOR, INC.) 14 April 2006 (2006-04-14)<br>See claims 1, 13, 15, 16 and 21. | 1,2,13 |
| PX | KR 10-2020-0138084 A (D&D PHARMATECH INC.) 09 December 2020 (2020-12-09)<br>See claims 1-52; and paragraphs [0116] and [0347]-[0361]. | 1-12,22,23 |
| PX | KR 10-2193211 B1 (D&D PHARMATECH INC.) 18 December 2020 (2020-12-18)<br>See claims 1-20; and experimental examples 6 and 7. | 1-12,22,23 |

Form PCT/ISA/210 (second sheet) (July 2019)

**INTERNATIONAL SEARCH REPORT**

International application No.
**PCT/IB2021/022237**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| KR 10-2010-0109896 A | 11 October 2010 | AU 2008-304269 A1 | 02 April 2009 |
| | | AU 2008-304269 B2 | 09 July 2015 |
| | | AU 2011-230564 A1 | 11 October 2012 |
| | | AU 2011-230564 B2 | 09 July 2015 |
| | | AU 2015-238887 A1 | 29 October 2015 |
| | | AU 2015-238887 B2 | 06 July 2017 |
| | | AU 2015-238890 A1 | 29 October 2015 |
| | | AU 2015-238890 B2 | 03 August 2017 |
| | | AU 2017-239513 A1 | 26 October 2017 |
| | | BR PI0817478 A2 | 08 September 2015 |
| | | CA 2704712 A1 | 02 April 2009 |
| | | CA 2704712 C | 31 May 2016 |
| | | CA 2794540 A1 | 29 September 2011 |
| | | CA 2794540 C | 04 December 2018 |
| | | CN 101917971 A | 15 December 2010 |
| | | CN 104666246 A | 03 June 2015 |
| | | DK 2205217 T3 | 12 March 2018 |
| | | EP 2205217 A1 | 14 July 2010 |
| | | EP 2205217 A4 | 23 January 2013 |
| | | EP 2205217 B1 | 13 December 2017 |
| | | EP 2410991 A1 | 01 February 2012 |
| | | EP 2410991 A4 | 07 October 2015 |
| | | EP 2552202 A1 | 06 February 2013 |
| | | EP 2552202 A4 | 17 February 2016 |
| | | EP 3360540 A1 | 15 August 2018 |
| | | ES 2661325 T3 | 28 March 2018 |
| | | HK 1211205 A1 | 20 May 2016 |
| | | HU E037002 T2 | 28 August 2018 |
| | | JP 2010-540559 A | 24 December 2010 |
| | | JP 2015-044883 A | 12 March 2015 |
| | | JP 6066982 B2 | 25 January 2017 |
| | | KR 10-1747433 B1 | 14 June 2017 |
| | | KR 10-2016-0005130 A | 13 January 2016 |
| | | LT 2205217 T | 26 March 2018 |
| | | MX 2010003396 A | 12 November 2010 |
| | | MX 337138 B | 02 February 2016 |
| | | NO 2205217 T3 | 12 May 2018 |
| | | PL 2205217 T3 | 30 May 2018 |
| | | PT 2205217 T | 15 March 2018 |
| | | US 10568835 B2 | 25 February 2020 |
| | | US 10751418 B2 | 25 August 2020 |
| | | US 2009-0087479 A1 | 02 April 2009 |
| | | US 2010-0080773 A1 | 01 April 2010 |
| | | US 2010-0247625 A1 | 30 September 2010 |
| | | US 2010-0310599 A1 | 09 December 2010 |
| | | US 2013-0183270 A1 | 18 July 2013 |
| | | US 2014-0243430 A1 | 28 August 2014 |
| | | US 2015-0125518 A1 | 07 May 2015 |
| | | US 2020-0405638 A1 | 31 December 2020 |
| | | US 8846053 B2 | 30 September 2014 |

Form PCT/ISA/210 (patent family annex) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| | | US 8962015 B2 | 24 February 2015 |
| | | US 9145453 B2 | 29 September 2015 |
| | | US 9943602 B2 | 17 April 2018 |
| | | WO 2009-042945 A1 | 02 April 2009 |
| | | WO 2010-111271 A1 | 30 September 2010 |
| | | WO 2011-119953 A1 | 29 September 2011 |
| | | ZA 201002177 B | 29 June 2011 |
| KR 10-2018-0039726 A | 18 April 2018 | AU 2018-315889 A1 | 22 March 2018 |
| | | CA 2996716 A1 | 09 March 2017 |
| | | CN 108348581 A | 31 July 2018 |
| | | EP 3344278 A2 | 11 July 2018 |
| | | EP 3344278 A4 | 23 January 2019 |
| | | HK 1258125 A1 | 08 November 2019 |
| | | JP 2018-537399 A | 20 December 2018 |
| | | JP 6951825 B2 | 20 October 2021 |
| | | US 10501546 B2 | 10 December 2019 |
| | | US 2019-0023794 A1 | 24 January 2019 |
| | | US 2020-0115458 A1 | 16 April 2020 |
| | | WO 2017-041001 A2 | 09 March 2017 |
| | | WO 2017-041001 A3 | 04 May 2017 |
| KR 10-2011-0004366 A | 13 January 2011 | AU 2009-226910 A1 | 24 September 2009 |
| | | AU 2009-226910 B2 | 06 February 2014 |
| | | BR PI200910348 A2 | 11 August 2020 |
| | | BR PI200910348 B1 | 29 June 2021 |
| | | CA 2009738 A1 | 24 September 2009 |
| | | CA 2718738 C | 07 May 2019 |
| | | CN 102037008 A | 27 April 2011 |
| | | CN 102037008 B | 31 August 2016 |
| | | CN 102227213 A | 26 October 2011 |
| | | CN 102245633 A | 16 November 2011 |
| | | DK 2254906 T3 | 23 January 2017 |
| | | DK 2910570 T3 | 30 January 2017 |
| | | EP 2254906 A1 | 01 December 2010 |
| | | EP 2254906 B1 | 05 October 2016 |
| | | EP 2370059 A1 | 05 October 2011 |
| | | EP 2376531 A1 | 19 October 2011 |
| | | EP 2910569 A1 | 26 August 2015 |
| | | EP 2910569 B1 | 05 October 2016 |
| | | EP 2910570 A1 | 26 August 2015 |
| | | EP 2910570 B1 | 12 October 2016 |
| | | EP 2910571 A1 | 26 August 2015 |
| | | EP 2910571 B1 | 05 October 2016 |
| | | ES 2609288 T3 | 19 April 2017 |
| | | ES 2611007 T3 | 04 May 2017 |
| | | HU E032284 T2 | 28 September 2017 |
| | | HU E032287 T2 | 28 September 2017 |
| | | IL 250548 A | 31 March 2019 |
| | | IL 250549 A | 31 December 2018 |
| | | JP 2011-515358 A | 19 May 2011 |
| | | JP 2012-510438 A | 10 May 2012 |

Form PCT/ISA/210 (patent family annex) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| | | JP 2012-511506 A | 24 May 2012 |
| | | JP 2015-147781 A | 20 August 2015 |
| | | JP 2015-147782 A | 20 August 2015 |
| | | JP 2015-155421 A | 27 August 2015 |
| | | JP 5749155 B2 | 15 July 2015 |
| | | KR 10-1755434 B1 | 10 July 2017 |
| | | KR 10-1755529 B1 | 07 July 2017 |
| | | KR 10-2016-0073431 A | 24 June 2016 |
| | | KR 10-2016-0124929 A | 28 October 2016 |
| | | MX 2010009850 A | 30 September 2010 |
| | | PL 2254906 T3 | 28 April 2017 |
| | | PL 2910570 T3 | 30 June 2017 |
| | | PT 2254906 T | 03 January 2017 |
| | | PT 2910570 T | 24 January 2017 |
| | | RU 2010141481 A | 27 April 2012 |
| | | RU 2571857 C2 | 20 December 2015 |
| | | US 10259856 B2 | 16 April 2019 |
| | | US 2011-0105720 A1 | 05 May 2011 |
| | | US 2011-0293714 A1 | 01 December 2011 |
| | | US 2011-0294729 A1 | 01 December 2011 |
| | | US 2014-0073564 A1 | 13 March 2014 |
| | | US 2015-0210747 A1 | 30 July 2015 |
| | | US 2015-0210748 A1 | 30 July 2015 |
| | | US 2019-0112348 A1 | 18 April 2019 |
| | | US 8691759 B2 | 08 April 2014 |
| | | US 9045560 B2 | 02 June 2015 |
| | | US 9688737 B2 | 27 June 2017 |
| | | WO 2009-115469 A1 | 24 September 2009 |
| | | WO 2010-060667 A1 | 03 June 2010 |
| | | WO 2010-066636 A1 | 17 June 2010 |
| | | ZA 201006126 B | 30 November 2011 |
| US 2003-0199451 A1 | 23 October 2003 | AU 2831801 A | 07 August 2001 |
| | | BR 0107902 A | 05 November 2002 |
| | | CA 2396372 A1 | 02 August 2001 |
| | | CN 1396904 A | 12 February 2003 |
| | | EP 1254102 A1 | 06 November 2002 |
| | | HU 0204247 A2 | 28 April 2003 |
| | | HU 0204247 A3 | 28 October 2003 |
| | | JP 2003-520839 A | 08 July 2003 |
| | | KR 10-2002-0090211 A | 30 November 2002 |
| | | MX PA02007295 A | 29 November 2002 |
| | | NO 20023567 L | 25 September 2002 |
| | | PL 357017 A1 | 12 July 2004 |
| | | RU 2002123050 A | 10 January 2004 |
| | | US 2001-0041709 A1 | 15 November 2001 |
| | | US 6569901 B2 | 27 May 2003 |
| | | US 7202213 B2 | 10 April 2007 |
| | | WO 01-55086 A1 | 02 August 2001 |
| | | ZA 200204800 B | 26 March 2003 |
| KR 10-2008-0064840 A | 09 July 2008 | AT 448247 T | 15 November 2009 |

Form PCT/ISA/210 (patent family annex) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| | | AU 2006-299134 A1 | 12 April 2007 |
| | | AU 2006-299134 B2 | 23 February 2012 |
| | | BR PI0616107 A2 | 07 June 2011 |
| | | CA 2619053 A1 | 12 April 2007 |
| | | CN 101273058 A | 24 September 2008 |
| | | CY 1109778 T1 | 10 September 2014 |
| | | DK 1767545 T3 | 15 March 2010 |
| | | EA 013796 B1 | 30 June 2010 |
| | | EA 200800699 A1 | 30 October 2008 |
| | | EP 1767545 A1 | 28 March 2007 |
| | | EP 1767545 B1 | 11 November 2009 |
| | | EP 1926748 A1 | 04 June 2008 |
| | | EP 1926748 B1 | 29 August 2012 |
| | | EP 2045265 A1 | 08 April 2009 |
| | | EP 2045265 B1 | 21 November 2012 |
| | | EP 2174952 A2 | 14 April 2010 |
| | | EP 2174952 A3 | 17 November 2010 |
| | | EP 2261245 A1 | 15 December 2010 |
| | | ES 2336575 T3 | 14 April 2010 |
| | | ES 2394218 T3 | 23 January 2013 |
| | | ES 2397289 T3 | 06 March 2013 |
| | | HR P20100062 T1 | 31 March 2010 |
| | | JP 2009-508505 A | 05 March 2009 |
| | | JP 2013-099352 A | 23 May 2013 |
| | | JP 5222729 B2 | 26 June 2013 |
| | | PL 1767545 T3 | 30 April 2010 |
| | | PT 1767545 E | 05 February 2010 |
| | | RS 51319 B | 31 December 2010 |
| | | SG 165414 A1 | 28 October 2010 |
| | | SI 1767545 T1 | 31 March 2010 |
| | | US 2011-0130329 A1 | 02 June 2011 |
| | | US 2012-0238497 A1 | 20 September 2012 |
| | | US 8431533 B2 | 30 April 2013 |
| | | US 8853159 B2 | 07 October 2014 |
| | | WO 2007-039140 A1 | 12 April 2007 |
| | | ZA 200803488 B | 28 October 2009 |
| KR 10-2008-0042045 A | 14 May 2008 | AU 2006-249479 A1 | 30 November 2006 |
| | | AU 2006-249480 A1 | 30 November 2006 |
| | | AU 3999699 A | 06 December 1999 |
| | | CA 2609376 A1 | 30 November 2006 |
| | | CA 2609402 A1 | 30 November 2006 |
| | | CA 2609402 C | 21 October 2014 |
| | | CA 2864366 A1 | 30 November 2006 |
| | | CA 2864366 C | 05 April 2016 |
| | | CN 101237854 A | 06 August 2008 |
| | | CN 101237854 B | 27 March 2013 |
| | | CN 101237855 A | 06 August 2008 |
| | | DK 1883394 T3 | 25 June 2018 |
| | | DK 3391876 T3 | 14 June 2021 |
| | | EP 1087753 A1 | 04 April 2001 |

Form PCT/ISA/210 (patent family annex) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| | | EP 1087753 A4 | 30 June 2004 |
| | | EP 1883394 A2 | 06 February 2008 |
| | | EP 1883394 A4 | 21 August 2013 |
| | | EP 1883394 B1 | 28 March 2018 |
| | | EP 1883395 A2 | 06 February 2008 |
| | | EP 1883395 A4 | 04 July 2012 |
| | | EP 3391876 A1 | 24 October 2018 |
| | | EP 3391876 B1 | 10 March 2021 |
| | | ES 2675042 T3 | 05 July 2018 |
| | | HU E039023 T2 | 28 December 2018 |
| | | JP 2002-515417 A | 28 May 2002 |
| | | JP 2008-542270 A | 27 November 2008 |
| | | JP 2009-507761 A | 26 February 2009 |
| | | JP 5414270 B2 | 12 February 2014 |
| | | KR 10-1389226 B1 | 25 April 2014 |
| | | KR 10-2008-0043742 A | 19 May 2008 |
| | | LT 1883394 T | 11 June 2018 |
| | | PL 1883394 T3 | 28 September 2018 |
| | | PT 1883394 T | 06 June 2018 |
| | | US 10004686 B2 | 26 June 2018 |
| | | US 10463616 B2 | 05 November 2019 |
| | | US 2006-0222697 A1 | 05 October 2006 |
| | | US 2006-0222698 A1 | 05 October 2006 |
| | | US 2007-0104777 A1 | 10 May 2007 |
| | | US 2007-0218117 A1 | 20 September 2007 |
| | | US 2010-0129428 A1 | 27 May 2010 |
| | | US 2010-0209492 A1 | 19 August 2010 |
| | | US 2011-0135725 A1 | 09 June 2011 |
| | | US 2011-0135727 A1 | 09 June 2011 |
| | | US 2013-0267462 A1 | 10 October 2013 |
| | | US 2015-0031608 A1 | 29 January 2015 |
| | | US 2019-0054021 A1 | 21 February 2019 |
| | | US 2020-0261363 A1 | 20 August 2020 |
| | | US 7169410 B1 | 30 January 2007 |
| | | US 7858116 B2 | 28 December 2010 |
| | | US 7871641 B2 | 18 January 2011 |
| | | US 8257735 B2 | 04 September 2012 |
| | | US 8303983 B2 | 06 November 2012 |
| | | US 9034372 B2 | 19 May 2015 |
| | | WO 2006-127360 A2 | 30 November 2006 |
| | | WO 2006-127360 A3 | 10 January 2008 |
| | | WO 2006-127361 A2 | 30 November 2006 |
| | | WO 2006-127361 A3 | 24 May 2007 |
| | | WO 99-59545 A1 | 25 November 1999 |
| | | WO 99-59545 A9 | 27 April 2000 |
| | | ZA 200710055 B | 25 March 2009 |
| | | ZA 200710056 B | 25 March 2009 |
| KR 10-2006-0032140 A | 14 April 2006 | BR PI0410863 A | 04 July 2006 |
| | | CA 2527665 A1 | 16 December 2004 |
| | | CN 1826131 A | 30 August 2006 |

Form PCT/ISA/210 (patent family annex) (July 2019)

**INTERNATIONAL SEARCH REPORT**
**Information on patent family members**

International application No.
**PCT/IB2021/022237**

| Patent document cited in search report | Publication date (day/month/year) | Patent family member(s) | Publication date (day/month/year) |
|---|---|---|---|
| | | EP 1641481 A2 | 05 April 2006 |
| | | EP 1641481 A4 | 15 October 2008 |
| | | JP 2007-537986 A | 27 December 2007 |
| | | MX PA05012936 A | 17 May 2006 |
| | | NO 20056225 L | 24 February 2006 |
| | | US 2004-0266690 A1 | 30 December 2004 |
| | | US 2006-0116322 A1 | 01 June 2006 |
| | | US 6995245 B2 | 07 February 2006 |
| | | WO 2004-108667 A2 | 16 December 2004 |
| | | WO 2004-108667 A3 | 24 March 2005 |
| KR 10-2020-0138084 A | 09 December 2020 | CN 113924124 A | 11 January 2022 |
| | | WO 2020-242268 A1 | 03 December 2020 |
| KR 10-2193211 B1 | 18 December 2020 | WO 2021-107519 A1 | 03 June 2021 |

Form PCT/ISA/210 (patent family annex) (July 2019)